(19) Europäisches Patentamt
European Patent Office
Office européen des brevets

(11) EP 2 812 435 B1

(12) FASCICULE DE BREVET EUROPEEN

(45) Date de publication et mention de la délivrance du brevet:
**03.04.2019 Bulletin 2019/14**

(21) Numéro de dépôt: **13709486.8**

(22) Date de dépôt: **08.02.2013**

(51) Int Cl.:
***C07K 16/28*** ^(2006.01) ***C07K 16/34*** ^(2006.01)
***C12N 15/85*** ^(2006.01)

(86) Numéro de dépôt international:
**PCT/FR2013/050267**

(87) Numéro de publication internationale:
**WO 2013/117871 (15.08.2013 Gazette 2013/33)**

# (54) UNITÉS DE TRANSCRIPTION ET LEUR UTILISATION DANS DES VECTEURS D'EXPRESSION

TRANSKRIPTIONSEINHEITEN UND VERWENDUNG DAVON IN EXPRESSIONSVEKTOREN

TRANSCRIPTION UNITS AND THE USE THEREOF IN EXPRESSION VECTORS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.02.2012 FR 1251185**

(43) Date de publication de la demande:
**17.12.2014 Bulletin 2014/51**

(73) Titulaire: **Laboratoire Français du Fractionnement et des Biotechnologies**
**91940 Les Ulis (FR)**

(72) Inventeurs:
- **FONTAYNE, Alexandre**
**F-59110 La Madeleine (FR)**
- **COUTARD, François**
**F-30100 Ales (FR)**

(74) Mandataire: **Grosset-Fournier, Chantal Catherine et al**
**Grosset-Fournier & Demachy**
**54, rue Saint-Lazare**
**75009 Paris (FR)**

(56) Documents cités:
**WO-A1-2008/044869 WO-A1-2011/062298**
**US-A1- 2003 224 477**

- **RAMEZANI A ET AL: "Lentiviral vectors for enhanced gene expression in human hematopoietic cells", MOLECULAR THERAPY, ACADEMIC PRESS, SAN DIEGO, CA, US, vol. 2, no. 5, 1 novembre 2000 (2000-11-01), pages 458-469, XP002307687, ISSN: 1525-0016, DOI: 10.1006/MTHE.2000.0190**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 812 435 B1

## Description

La présente invention concerne de nouvelles unités de transcription susceptibles d'être utilisées dans des vecteurs d'expression.

A ce jour, l'expression des protéines recombinantes est toujours une des méthodes majeures pour la production des protéines thérapeutiques, telles que des anticorps pharmacologiques.

Les gènes codant les protéines recombinantes sont généralement introduits dans un vecteur d'expression circulaire.

La demande WO2011/062298 A1 divulgue une unité de transcription permettant l'expression d'une protéine recombinante, comprenant un promoteur β-actine, un enhancer CMV, et pouvant également comprendre une région régulatrice R (RU5'), notamment une région régulatrice R du 5' Long terminal repeat (LTR) du virus HTLV-1, directement en amont de la séquence codante de la protéine à exprimer.

L'un des buts de l'invention est de fournir une unité de transcription permettant de produire des anticorps dont le gain de productivité n'est pas lié à un anticorps de cible antigénique particulière et donc par extrapolation à une protéine recombinante donnée, ni lié au milieu de culture.

L'un des buts de l'invention est de mettre à disposition une unité de transcription universelle permettant de fournir une meilleure capacité de transcription et d'expression d'une protéine d'intérêt par rapport aux vecteurs d'expression traditionnels.

L'un des autres buts de l'invention est de fournir une unité de transcription permettant de limiter la taille de vecteur d'expression, afin de limiter les problèmes de clonage ou d'efficacité de transfection dans les lignées d'expression.

Enfin, l'un des autres buts est de fournir une unité de transcription dépourvue des promoteurs viraux, afin de limiter les risques sanitaires potentiels.

La présente invention concerne des unités de transcription pour construire les vecteurs d'expression.

La présente description concerne notamment une unité de transcription constituée d'un polynucléotide comprenant les éléments régulateurs suivants :

(i) - l'enhancer du virus hCMVie (E2), ledit enhancer ayant la séquence nucléotidique SEQ ID NO : 1, ou

- un acide nucléotidique ayant au moins 70% d'identité de séquence avec la séquence SEQ ID NO : 1 et possédant essentiellement des propriétés d'activation de la transcription, et

(ii) - la région promotrice de la cycline dépendante des kinases 9 (CDK9), ladite région promotrice ayant la séquence nucléotidique SEQ ID NO : 2, ou

- un acide nucléotidique ayant au moins 70% d'identité de séquence avec la séquence SEQ ID NO : 2 et possédant essentiellement une activité promotrice, ou
- la région promotrice de la β-actine, ladite région promotrice ayant la séquence nucléotidique SEQ ID NO : 3, ou
- un acide nucléotidique ayant au moins 70% d'identité de séquence avec la séquence SEQ ID NO : 3 et possédant essentiellement une activité promotrice.

On entend par « **éléments régulateurs** » dans le sens de la présente invention, des éléments génomiques non codant permettant de contrôler la transcription ou la traduction d'un acide nucléique codant.

On entend par « **unité de transcription** » un polynucléotide sur lequel peut se fixer une ARN polymérase, qui permet de synthétiser un ARNm à partir d'un gène d'intérêt lié à ladite unité de transcription.

On entend par « **région promotrice** », une région d'ADN qui contient en général une séquence d'ADN particulière permettant d'initialiser la transcription d'un gène d'intérêt particulier.

Au sens de la présente invention, les termes « région promotrice » et « promoteur » peuvent être remplacés l'un par l'autre.

La région promotrice est la zone de l'ADN sur laquelle se fixe initialement l'ARN polymérase, avant de déclencher la synthèse de l'ARN.

Un promoteur est en général proche (d'une vingtaine à une centaine de nucléotides) du gène d'intérêt à contrôler et est situé en amont du site de démarrage de la transcription d'un gène. La présence d'un promoteur est essentielle pour la transcription d'un gène particulier.

Le promoteur du gène CDK9 représenté par la séquence SEQ ID NO : 2 est un promoteur GC riche et dépourvu de TATA box.

« Un acide nucléotidique ayant au moins 70% d'identité de séquence avec la séquence SEQ ID : NO 2 et possédant essentiellement une activité promotrice » contenu dans une unité de transcription selon la présente invention est un acide nucléotidique possédant essentiellement la même capacité pour initialiser la transcription de gène que celle de la région promotrice du gène CDK9, représentée par la séquence SEQ ID NO : 2.

La capacité de la région promotrice du gène CDK9 pour initialiser la transcription d'un gène peut être déterminée selon la méthode décrite par Liu et al. (Gene 252, 51-59 (2000)).

« Un acide nucléotidique ayant au moins 70% d'identité de séquence avec la séquence SEQ ID NO : 3 et possédant essentiellement une activité promotrice » contenu dans une unité de transcription selon la présente invention est un acide nucléotidique possédant essentiellement la même capacité pour initialiser la transcription de gène que celle de la région promotrice du gène de la β-actine, représentée par la séquence SEQ ID NO : 3.

La capacité de la région promotrice du gène de la β-actine pour initialiser la transcription d'un gène peut être déterminée selon la méthode décrite par Liu et al. (Gene 252, 51-59 (2000)).

On entend par « **enhancer** », un segment d'ADN court qui peut fixer des protéines pour stimuler la transcription d'un gène. Un enhancer n'est pas nécessairement proche du gène d'intérêt à contrôler, et peut être situé en 5' ou en 3', ou même au milieu du gène à contrôler ou dans un intron.

La présence d'un enhancer dans un vecteur d'expression permet d'augmenter le niveau de transcription d'un gène.

« Un acide nucléotidique ayant au moins 70% d'identité de séquence avec la séquence SEQ ID : NO 1 et possédant essentiellement des propriétés d'activation de la transcription » est un acide nucléotidique possédant essentiellement la même capacité pour stimuler la transcription de gène que celui de l'enhancer du virus hCMVie représenté par la séquence SEQ ID NO : 1, désigné ci-après également E2.

Les propriétés d'activation de la transcription d'un gène peuvent être déterminées selon la méthode décrite par l'utilisation des gènes rapporteurs comme la luciférase.

Plusieurs enhancers peuvent coexister dans une unité de transcription selon la présente invention ; cela permet de stimuler davantage la transcription de gène.

Par conséquent, une unité de transcription selon la présente invention peut comprendre :

- l'enhancer du virus hCMVie, ledit enhancer ayant la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
- au moins un autre enhancer choisi parmi un SV40 enhancer et un Eμ enhancer.

Le pourcentage d'identité entre deux séquences d'acides nucléiques peut être calculé selon la formule suivante :

$$\frac{\text{le nombre des résidus identiques x 100}}{\text{le nombre des résidus de la séquence la plus courte}}$$

Dans un mode de réalisation particulier de l'invention, l'enhancer se situe en amont de la région promotrice. Autrement dit, l'enhancer est situé en extrémité 5' de l'ADN de la région promotrice.

La présente description divulgue également une unité de transcription constituée d'un polynucléotide comprenant les éléments régulateurs suivants :

(i) - l'enhancer du virus hCMVie, ledit enhancer ayant la séquence nucléotidique SEQ ID NO : 1, ou

- un acide nucléotidique ayant au moins 70% d'identité de séquence avec la séquence SEQ ID NO : 1 et possédant essentiellement des propriétés d'activation de la transcription, et

(ii) - la région promotrice de la cycline dépendante des kinases 9 (CDK9), ladite région promotrice ayant la séquence nucléotidique SEQ ID NO : 2, ou

- un acide nucléotidique ayant au moins 70% d'identité de séquence avec la séquence SEQ ID : NO 2 et possédant essentiellement une activité promotrice.

L'unité de transcription obtenue est désignée par CMV-CDK9.

La présente description divulgue également une unité de transcription constituée d'un polynucléotide comprenant les éléments régulateurs suivants :

(i) - l'enhancer du virus hCMVie, ledit enhancer ayant la séquence nucléotidique SEQ ID NO : 1, ou

- un acide nucléotidique ayant au moins 70% d'identité de séquence avec la séquence SEQ ID NO : 1 et possédant essentiellement des propriétés d'activation de la transcription, et

(ii) - la région promotrice de la cycline dépendante des kinases 9 (CDK9), ladite région promotrice ayant la séquence nucléotidique SEQ ID NO : 2, ou

- un acide nucléotidique ayant au moins 70% d'identité de séquence avec la séquence SEQ ID : NO 2 et possédant essentiellement une activité promotrice, l'enhancer étant situé en amont de la région promotrice.

La présente description divulgue également dans un autre mode de réalisation particulier, une unité de transcription constituée d'un polynucléotide comprenant les éléments régulateurs suivants :

(i) - l'enhancer du virus hCMVie, ledit enhancer ayant la séquence nucléotidique SEQ ID NO : 1, ou

- un acide nucléotidique présentant au moins 70% d'identité de séquence avec la séquence SEQ ID NO : 1 et possédant essentiellement des propriétés d'activation de la transcription, et

(ii) - la région promotrice de la β-actine, ladite région promotrice ayant la séquence nucléotidique SEQ ID NO : 3, ou

- un acide nucléotidique présentant au moins 70% d'identité de séquence avec la séquence SEQ ID NO : 3 et possédant essentiellement une activité promotrice.

L'unité de transcription obtenue est désignée par CMV-βactine.

La présente description divulgue également dans un mode de réalisation plus particulier, une unité de transcription constituée d'un polynucléotide comprenant les éléments régulateurs suivants :

(i) - l'enhancer du virus hCMVie, ledit enhancer ayant la séquence nucléotidique SEQ ID NO : 1, ou

- un acide nucléotidique présentant au moins 70% d'identité de séquence avec la séquence SEQ ID NO : 1 et possédant essentiellement des propriétés d'activation de la transcription, et

(ii) - la région promotrice de la β-actine, ladite région promotrice ayant la séquence nucléotidique SEQ ID NO : 3, ou

- un acide nucléotidique présentant au moins 70% d'identité de séquence avec la séquence SEQ ID NO : 3 et possédant essentiellement une activité promotrice, l'enhancer étant situé en amont de la région promotrice.

Une unité de transcription selon la présente invention peut comprendre également un acide nucléotidique situé en aval de la région promotrice et en amont du site d'initiation de la traduction, ledit acide nucléotidique comprenant au moins l'une des régions 5' non traduite (5' UTR) choisie parmi les suivantes :

(i) - la région régulatrice R du Long Terminal Repeat (LTR) (RU-5') du virus HTLV-1 ayant la séquence nucléotidique SEQ ID NO : 4 (U1), ou

- un acide nucléotidique ayant au moins 70% d'identité de séquence avec la séquence SEQ ID NO : 4,

(ii) - la région 5' UTR du gène NF-κB Repressing Factor (NRF) ayant la séquence nucléotidique SEQ ID NO : 5 (U2), ou

- un acide nucléotidique ayant au moins 70% d'identité de séquence avec la séquence SEQ ID NO : 5,

(iii) - la région 5' UTR du gène eukaryotic Initiation Factor 4GI (eIF4GI) ayant la séquence nucléotidique SEQ ID NO : 6 (U3), ou

- un acide nucléotidique ayant au moins 70% d'identité de séquence avec la séquence SEQ ID NO : 6,

les susdits acides nucléotidiques ayant au moins 70% d'identité de séquence avec l'une des séquences représentées par les séquences SEQ ID NO : 4, SEQ ID NO : 5 ou SEQ ID NO : 6 et ayant essentiellement des propriétés de stabilisation des ARNm et de facilitateur de la traduction.

Les propriétés de stabilisation des ARNm et de facilitateur de la traduction peuvent être mesurées par Fritz et al (Sci. STKE, 5 December 2000, Vol. 2000, Issue 61, p.11).

La région 5' non traduite dans un gène correspond à la portion de l'ARN messager (ARNm) placée en amont du site d'initiation de la traduction. Cette région permet la fixation de ribosome et peut être impliquée dans la régulation de l'expression du gène concerné.

Le site d'initiation de la traduction est un triplet de nucléotides qui dirige l'initiation de la traduction protéique. Ce triplet est souvent le triplet ATG.

« Les acides nucléotidiques ayant au moins 70% d'identité de séquence avec l'une des séquences représentées par les séquences SEQ ID NO : 4, SEQ ID NO : 5 ou SEQ ID NO : 6 » contenus dans les unités de transcription selon la présente invention permettent la fixation de ribosome et la stabilisation des ARNm.

Le susdit acide nucléotidique situé en aval de la région promotrice et en amont du site d'initiation de la traduction peut comprendre une seule région 5'UTR choisie parmi :

(i) - la région R du Long Terminal Repeat (LTR) du virus HTLV-1 ayant la séquence nucléotidique SEQ ID NO : 4 (U1), ou

- un acide nucléotidique ayant au moins 70% d'identité de séquence avec la séquence SEQ ID NO : 4,

(ii) - la région 5' UTR du gène NF-κB Repressing Factor (NRF) ayant la séquence nucléotidique SEQ ID NO : 5 (U2), ou

- un acide nucléotidique ayant au moins 70% d'identité de séquence avec la séquence SEQ ID NO : 5,

(iii) - la région 5' UTR du gène eukaryotic Initiation Factor 4GI (eIF4GI) ayant la séquence nucléotidique SEQ ID NO : 6 (U3), ou

- un acide nucléotidique ayant au moins 70% d'identité de séquence avec la séquence SEQ ID NO : 6.

On entend par une région 5'UTR « situé en aval de la région promotrice et en amont du site d'initiation de la traduction» une région 5'UTR situé après l'extrémité 3' de l'ADN de la région promotrice et avant l'extrémité 5'de l'ADN du site d'initiation de la traduction.

Le susdit acide nucléotidique situé en aval de la région promotrice et en amont du site d'initiation de la traduction peut comprendre deux régions 5'UTR.

La présence de deux régions 5'UTR dans une unité de transcription selon l'invention permet d'accumuler ou de synergiser les effets positifs sur la stabilité des ARNm et l'efficacité de la traduction.

Un susdit acide nucléotidique utilisé dans une unité de transcription selon la présente invention peut comprendre la région R du Long Terminal Repeat (LTR) du virus HTLV-1 et la région 5' UTR du gène NF-κB Repressing Factor (NRF), ledit acide nucléotidique étant représenté par la séquence SEQ ID NO : 7, ou étant un acide nucléotidique ayant au moins 70% d'identité de séquence avec la séquence SEQ ID NO : 7. L'acide nucléotidique obtenu est désigné par U1U2.

Un susdit acide nucléotidique utilisé dans une unité de transcription selon la présente invention peut également comprendre la région R du Long Terminal Repeat (LTR) du virus HTLV-1 et la région 5' UTR du gène eukaryotic Initiation Factor 4GI (eIF4GI), ledit acide nucléotidique étant représenté par la séquence SEQ ID NO : 8, un étant acide nucléotidique ayant au moins 70% d'identité de séquence avec la séquence SEQ ID NO : 8. L'acide nucléotidique obtenu est désigné par U1U3.

Un susdit acide nucléotidique utilisé dans une unité de transcription peut également comprendre la région 5' UTR du gène NF-κB Repressing Factor (NRF) et la région 5' UTR du gène eukaryotic Initiation Factor 4GI (eIF4GI), ledit acide nucléotidique étant représenté par la séquence SEQ ID NO : 9 ou étant un acide nucléotidique ayant au moins 70% d'identité de séquence avec la séquence SEQ ID NO : 9. L'acide nucléotidique obtenu est désigné par U2U3.

Le susdit acide nucléotidique situé en aval de la région promotrice et en amont du site d'initiation de la traduction peut aussi comprendre trois régions 5'UTR, à savoir la région R du Long Terminal Repeat (LTR) du virus HTLV-1, la région 5' UTR du gène NF-κB Repressing Factor (NRF) et la région 5' UTR du gène eukaryotic Initiation Factor 4GI (eIF4GI), ledit acide nucléotidique étant représenté par la séquence SEQ ID NO : 10 ou étant un acide nucléotidique ayant au moins 70% d'identité de séquence avec la séquence SEQ ID NO : 10. L'acide nucléotidique obtenu est désigné par U1U2U3.

La présente description concerne également une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,

(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice, et

(iii) la région R du Long Terminal Repeat (LTR) du virus HTLV-1 représentée par la séquence nucléotidique SEQ ID NO : 4, ou un acide nucléotidique ayant au moins 70% d'identité de séquence avec la séquence SEQ ID NO : 4,

ladite région 5' UTR étant située en aval de la région promotrice et en amont du site d'initiation de la traduction.

La présente description divulgue en particulier une unité de transcription constituée d'un polynucléotide comprenant un acide nucléotidique représenté par la séquence SEQ ID NO : 14 et constitué de :

(i) l'enhancer du virus hCMVie représenté par la séquence SEQ ID NO : 1,

(ii) la région promotrice du gène CDK9 représentée par la séquence SEQ ID NO : 2, et

(iii) la région 5'UTR du LTR du virus HTLV-1, représentée par la séquence SEQ ID NO : 4,

ou d'un acide nucléotidique ayant au moins 70% d'identité de séquence avec la séquence SEQ ID NO : 14.
L'unité de transcription obtenue est désignée par CMV-CDK9-U1, autrement appelée E2-CDK9-U1.

La présente description divulgue en particulier une unité de transcription constituée d'un polynucléotide comprenant les éléments régulateurs suivants :

(i) l'enhancer du virus hCMVie représentée par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,

(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice, et

(iii) la région 5' UTR du gène NF-κB Repressing Factor (NRF) représentée par la séquence nucléotidique SEQ ID NO : 5, ou un acide nucléotidique ayant au moins 70% d'identité de séquence avec la séquence SEQ ID NO : 5,

ladite région 5' UTR étant située en aval de la région promotrice et en amont du site d'initiation de la traduction.

La présente description divulgue en particulier une unité de transcription constituée d'un polynucléotide comprenant un acide nucléotidique représenté par la séquence SEQ ID NO : 15 et constitué de :

(i) l'enhancer du virus hCMVie représenté par la séquence SEQ ID NO : 1,

(ii) la région promotrice du gène CDK9 représentée par la séquence SEQ ID NO : 2, et

(iii) la région 5' UTR du gène NRF, représentée par la séquence SEQ ID NO : 5, ou un acide nucléotidique ayant au moins 70% d'identité de séquence avec la séquence SEQ ID NO : 15.

L'unité de transcription obtenue est désignée par CMV-CDK9-U2, autrement appelée E2-CDK9-U2.

La présente description divulgue en particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,

(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice, et

(iii) la région 5' UTR du gène eukaryotic Initiation Factor 4GI (eIF4GI) représentée par la séquence nucléotidique SEQ ID NO : 6, ou un acide nucléotidique ayant au moins 70% d'identité de séquence avec la séquence SEQ ID NO : 6,

ladite région 5' UTR étant située en aval de la région promotrice et en amont du site d'initiation de la traduction.

La présente description concerne en particulier une unité de transcription constituée d'un polynucléotide comprenant un acide nucléotidique représenté par la séquence SEQ ID NO : 16 et constitué de :

(i) l'enhancer du virus hCMVie représenté par la séquence SEQ ID NO : 1,

(ii) la région promotrice du gène CDK9 représentée par la séquence SEQ ID NO : 2, et
(iii) la région 5' UTR du gène eIF4GI représentée par la séquence SEQ ID NO : 6,

ou d'un acide nucléotidique ayant au moins 70% d'identité de séquence avec la séquence SEQ ID NO : 16.

L'unité de transcription obtenue est désignée par CMV-CDK9-U3 autrement appelée E2-CDK9-U3.

La présente description concerné également une unité de transcription comprenant deux régions 5'UTR. Une telle unité de transcription est constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice,
(iii) la région R du Long Terminal Repeat (LTR) du virus HTLV-1 représentée par la séquence nucléotidique SEQ ID NO : 4, ou un acide nucléotidique ayant au moins 70% d'identité de séquence avec la séquence SEQ ID NO : 4, et
(iv) la région 5' UTR du gène NF-κB Repressing Factor (NRF) représentée par la séquence nucléotidique SEQ ID NO : 5, ou un acide nucléotidique ayant au moins 70% d'identité de séquence avec la séquence SEQ ID NO : 5,

les régions 5' UTR étant situées en aval de la région promotrice et en amont du site d'initiation de la traduction.

La présente description divulgue en particulier une unité de transcription constituée d'un polynucléotide comprenant un acide nucléotidique représenté par la séquence SEQ ID NO : 17 et constitué de :

(i) l'enhancer du virus hCMVie représenté par la séquence SEQ ID NO : 1,
(ii) la région promotrice du gène CDK9 représentée par la séquence SEQ ID NO : 2, et
(iii) la région 5' UTR représentée par la séquence SEQ ID NO : 7,

ou d'un acide nucléotidique ayant au moins 70% d'identité de séquence avec la séquence SEQ ID NO : 17.

L'unité de transcription obtenue est désignée par CMV-CDK9-U1U2 autrement appelée E2-CDK9-U1U2.

La présente description divulgue en particulier une unité de transcription comprenant deux régions 5'UTR. Une telle unité de transcription est constituée d'un polynucléotide comprenant les éléments régulateurs suivants :

(i) l'enhancer du virus hCMVie représentée par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice,
(iii) la région R du Long Terminal Repeat (LTR) du virus HTLV-1 ayant la séquence nucléotidique SEQ ID NO : 4, ou un acide nucléotidique ayant au moins 70% d'identité de séquence avec la séquence SEQ ID NO : 4, et
(iv) la région 5' UTR du gène eukaryotic Initiation Factor 4GI (eIF4GI) ayant la séquence nucléotidique SEQ ID NO : 6, ou un acide nucléotidique ayant au moins 70% d'identité de séquence avec la séquence SEQ ID NO : 6,

les régions 5' UTR étant situées en aval de la région promotrice et en amont du site d'initiation de la traduction.

La présente description divulgue en particulier une unité de transcription constituée d'un polynucléotide comprenant un acide nucléotidique représenté par la séquence SEQ ID NO : 18 et constitué de :

(i) l'enhancer du virus hCMVie représenté par la séquence SEQ ID NO : 1,
(ii) la région promotrice du gène CDK9 représentée par la séquence SEQ ID NO : 2, et
(iii) la région 5' UTR représentée par la séquence SEQ ID NO : 8,

ou d'un acide nucléotidique ayant au moins 70% d'identité de séquence avec la séquence SEQ ID NO : 18.

L'unité de transcription obtenue est désignée par CMV-CDK9-U1U3 autrement appelée E2-CDK9-U1U3.

La présente description divulgue en particulier une unité de transcription comprenant deux régions 5'UTR. Une telle unité de transcription est constituée d'un polynucléotide comprenant les éléments régulateurs suivants :

(i) l'enhancer du virus hCMVie représentée par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,

(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice,

(iii) la région 5' UTR du gène NF-κB Repressing Factor (NRF) ayant la séquence nucléotidique SEQ ID NO : 5, ou un acide nucléotidique ayant au moins 70% d'identité de séquence avec la séquence SEQ ID NO : 5, et

(iv) la région 5' UTR du gène eukaryotic Initiation Factor 4GI (eIF4GI) ayant la séquence nucléotidique SEQ ID NO : 6, ou un acide nucléotidique ayant au moins 70% d'identité de séquence avec la séquence SEQ ID NO : 6,

les régions 5' UTR étant situées en aval de la région promotrice et en amont du site d'initiation de la traduction.

La présente description divulgue en particulier une unité de transcription constituée d'un polynucléotide comprenant un acide nucléotidique représenté par la séquence SEQ ID NO : 19 et constitué de :

(i) l'enhancer du virus hCMVie représenté par la séquence SEQ ID NO : 1,

(ii) la région promotrice du gène CDK9 représentée par la séquence SEQ ID NO : 2, et

(iii) la région 5' UTR représentée par la séquence SEQ ID NO : 9,

ou d'un acide nucléotidique ayant au moins 70% d'identité de séquence avec la séquence SEQ ID NO : 19.

L'unité de transcription obtenue est désignée par CMV-CDK9-U2U3 autrement appelée E2-CDK9-U2U3.

La présente description divulgue en particulier une unité de transcription comprenant trois régions 5'UTR. Une telle unité de transcription est constituée d'un polynucléotide comprenant les éléments régulateurs suivants :

(i) l'enhancer du virus hCMVie représentée par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,

(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice,

(iii) la région R du Long Terminal Repeat (LTR) du virus HTLV-1 ayant la séquence nucléotidique SEQ ID NO : 4, ou un acide nucléotidique ayant au moins 70% d'identité de séquence avec la séquence SEQ ID NO : 4,

(iv) la région 5' UTR du gène NF-κB Repressing Factor (NRF) ayant la séquence nucléotidique SEQ ID NO : 5, ou un acide nucléotidique ayant au moins 70% d'identité de séquence avec la séquence SEQ ID NO : 5, et

(v) la région 5' UTR du gène eukaryotic Initiation Factor 4GI (eIF4GI) ayant la séquence nucléotidique SEQ ID NO : 6, ou un acide nucléotidique ayant au moins 70% d'identité de séquence avec la séquence SEQ ID NO : 6,

les régions 5' UTR étant situées en aval de la région promotrice et en amont du site d'initiation de la traduction.

La présente description divulgue en particulier une unité de transcription constituée d'un polynucléotide comprenant un acide nucléotidique représenté par la séquence SEQ ID NO : 20 et constitué de :

(i) l'enhancer du virus hCMVie représenté par la séquence SEQ ID NO : 1,

(ii) la région promotrice du gène CDK9 représentée par la séquence SEQ ID NO : 2, et

(iii) la région 5' UTR représentée par la séquence SEQ ID NO : 10,

ou d'un acide nucléotidique ayant au moins 70% d'identité de séquence avec la séquence SEQ ID NO : 20.

L'unité de transcription obtenue est désignée par CMV-CDK9-U1U2U3, autrement appelée E2-CDK9-U1U2U3.

La présente description concerne également selon un mode de réalisation particulier, une unité de transcription constituée d'un polynucléotide comprenant les éléments régulateurs suivants :

(i) l'enhancer du virus hCMVie représentée par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,

(ii) la région promotrice de la β-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice, et

(iii) la région R du Long Terminal Repeat (LTR) du virus HTLV-1 ayant la séquence nucléotidique SEQ ID NO : 4, ou un acide nucléotidique ayant au moins 70% d'identité de séquence avec la séquence SEQ ID NO : 4,

ladite région 5' UTR étant située en aval de la région promotrice et en amont du site d'initiation de la traduction.

La présente description divulgue également un mode de réalisation, dans lequel une unité de transcription est constituée d'un polynucléotide comprenant un acide nucléotidique représenté par la séquence SEQ ID NO : 21 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3, et
(iii) la région R du LTR du virus HTLV-1 représentée par la séquence nucléotidique SEQ ID NO : 4,

ou d'un acide nucléotidique ayant au moins 70% d'identité de séquence avec la séquence SEQ ID NO : 21.
L'unité de transcription obtenue est désignée par CMV-βactine-U1, autrement appelée E2-bActine-U1.
La présente description divulgue également un autre mode de réalisation dans lequel l'unité de transcription est constituée d'un polynucléotide comprenant les éléments régulateurs suivants :

(i) l'enhancer du virus hCMVie représentée par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la β-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice, et
(iii) la région 5' UTR du gène NF-κB Repressing Factor (NRF) ayant la séquence nucléotidique SEQ ID NO : 5, ou un acide nucléotidique ayant au moins 70% d'identité de séquence avec la séquence SEQ ID NO : 5,

ladite région 5' UTR étant située en aval de la région promotrice et en amont du site d'initiation de la traduction.
La présente description divulgue également un mode de réalisation particulier, dans lequel l'une unité de transcription est constituée d'un polynucléotide comprenant un acide nucléotidique représenté par la séquence SEQ ID NO : 22 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3, et
(iii) la région 5' UTR du gène NRF ayant la séquence nucléotidique SEQ ID NO : 5,

ou d'un acide nucléotidique ayant au moins 70% d'identité de séquence avec la séquence SEQ ID NO : 22.
L'unité de transcription obtenue est désignée par CMV-βactine-U2, autrement appelée E2-bActine-U2.
Dans un autre mode de réalisation, une unité de transcription selon la présente description est constituée d'un polynucléotide comprenant les éléments régulateurs suivants :

(i) l'enhancer du virus hCMVie représentée par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la β-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice, et
(iii) la région 5' UTR du gène eukaryotic Initiation Factor 4GI (eIF4GI) ayant la séquence nucléotidique SEQ ID NO : 6, ou un acide nucléotidique présentant au moins 70% d'identité de séquence avec la séquence SEQ ID NO : 6,

ladite région 5' UTR étant située en aval de la région promotrice et en amont du site d'initiation de la traduction.
Dans un mode de réalisation particulier, une unité de transcription selon la présente description est constituée d'un polynucléotide comprenant un acide nucléotidique représenté par la séquence SEQ ID NO : 23 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3, et
(iii) la région 5' UTR du gène eIF4GI ayant la séquence nucléotidique SEQ ID NO : 6,

ou d'un acide nucléotidique ayant au moins 70% d'identité de séquence avec la séquence SEQ ID NO : 23.
L'unité de transcription obtenue est désignée par CMV-βactine-U3, autrement appelée E2-bActine-U3.
La présente description divulgue également un autre mode de réalisation particulier, dans lequel l'une unité de transcription est constituée d'un polynucléotide comprenant les éléments régulateurs suivants :

(i) l'enhancer du virus hCMVie représentée par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la β-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice,
(iii) la région R du Long Terminal Repeat (LTR) du virus HTLV-1 ayant la séquence nucléotidique SEQ ID NO : 4, ou un acide nucléotidique ayant au moins 70% d'identité de séquence avec la séquence SEQ ID NO : 4, et
(iv) la région 5' UTR du gène NF-κB Repressing Factor (NRF) ayant la séquence nucléotidique SEQ ID NO : 5, ou un acide nucléotidique ayant au moins 70% d'identité de séquence avec la séquence SEQ ID NO : 5,

les régions 5' UTR étant situées en aval de la région promotrice et en amont du site d'initiation de la traduction.
La présente description divulgue également un mode de réalisation particulier dans lequel l'une unité de transcription est constituée d'un polynucléotide comprenant un acide nucléotidique représenté par la séquence SEQ ID NO : 24 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3, et
(iii) la région 5' UTR représentée par la séquence SEQ ID NO : 7,

ou d'un acide nucléotidique ayant au moins 70% d'identité de séquence avec la séquence SEQ ID NO : 24.
L'unité de transcription obtenue est désignée par CMV-βactine-U1U2, autrement appelée E2-bActine-U1U2.
La présente invention concerne une unité de transcription constituée d'un polynucléotide comprenant les éléments régulateurs suivants :

(i) l'enhancer du virus hCMVie représentée par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la β-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice,
(iii) la région R du Long Terminal Repeat (LTR) du virus HTLV-1 ayant la séquence nucléotidique SEQ ID NO : 4, ou un acide nucléotidique ayant au moins 70% d'identité de séquence avec la séquence SEQ ID NO : 4, et
(iv) la région 5' UTR du gène eukaryotic Initiation Factor 4GI (eIF4GI) ayant la séquence nucléotidique SEQ ID NO : 6, ou un acide nucléotidique présentant au moins 70% d'identité de séquence avec la séquence SEQ ID NO : 6,

les régions 5' UTR étant situées en aval de la région promotrice et en amont du site d'initiation de la traduction.
Dans un mode de réalisation particulier de l'invention, une unité de transcription selon la présente invention est constituée d'un polynucléotide comprenant un acide nucléotidique représenté par la séquence SEQ ID NO : 25 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3, et
(iii) la région 5' UTR représentée par la séquence SEQ ID NO : 8,

ou d'un acide nucléotidique ayant au moins 70% d'identité de séquence avec la séquence SEQ ID NO : 25.
L'unité de transcription obtenue est désignée par CMV-βactine-U1U3, autrement appelée E2-bActine-U1U 3.
La présente description divulgue également un autre mode de réalisation particulier, dans lequel l'une unité de transcription est constituée d'un polynucléotide comprenant les éléments régulateurs suivants :

(i) l'enhancer du virus hCMVie représentée par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la β-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice,
(iii) la région 5' UTR du gène NF-κB Repressing Factor (NRF) ayant la séquence nucléotidique SEQ ID NO : 5, ou un acide nucléotidique ayant au moins 70% d'identité de séquence avec la séquence SEQ ID NO : 5, et

(iv) la région 5' UTR du gène eukaryotic Initiation Factor 4GI (eIF4GI) ayant la séquence nucléotidique SEQ ID NO : 6, ou un acide nucléotidique présentant au moins 70% d'identité de séquence avec la séquence SEQ ID NO : 6,

les régions 5' UTR étant situées en aval de la région promotrice et en amont du site d'initiation de la traduction.

La présente description divulgue également un mode de réalisation particulier dans lequel l'une unité de transcription est constituée d'un polynucléotide comprenant un acide nucléotidique représenté par la séquence SEQ ID NO : 26 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3, et
(iii) la région 5' UTR représentée par la séquence SEQ ID NO : 9,

ou d'un acide nucléotidique ayant au moins 70% d'identité de séquence avec la séquence SEQ ID NO : 26.

L'unité de transcription obtenue est désignée par CMV-βactine-U2U3, autrement appelée E2-bActine-U2U3.

La présente description divulgue également un autre mode de réalisation particulier , dans lequel l'une unité de transcription peut comprendre trois régions 5'UTR. Une telle unité de transcription est constituée d'un polynucléotide comprenant les éléments régulateurs suivants :

(i) l'enhancer du virus hCMVie représentée par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la β-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice,
(iii) la région R du Long Terminal Repeat (LTR) du virus HTLV-1 ayant la séquence nucléotidique SEQ ID NO : 4, ou un acide nucléotidique ayant au moins 70% d'identité de séquence avec la séquence SEQ ID NO : 4,
(iv) la région 5' UTR du gène NF-κB Repressing Factor (NRF) ayant la séquence nucléotidique SEQ ID NO : 5, ou un acide nucléotidique ayant au moins 70% d'identité de séquence avec la séquence SEQ ID NO : 5, et
(v) la région 5' UTR du gène eukaryotic Initiation Factor 4GI (eIF4GI) ayant la séquence nucléotidique SEQ ID NO : 6, ou un acide nucléotidique ayant au moins 70% d'identité de séquence avec la séquence SEQ ID NO : 6,

les régions 5' UTR étant situées en aval de la région promotrice et en amont du site d'initiation de la traduction.

La présente description divulgue également dans un mode de réalisation plus particulier, une unité de transcription constituée d'un polynucléotide comprenant un acide nucléotidique représenté par la séquence SEQ ID NO : 27 et constitué de :

(i) l'enhancer du virus hCMVie représenté par la séquence SEQ ID NO : 1,
(ii) la région promotrice de la β-actine représentée par la séquence SEQ ID NO : 3, et
(iii) la région 5'UTR représentée par la séquence SEQ ID NO : 10,

ou d'un acide nucléotidique ayant au moins 70% d'identité de séquence avec la séquence SEQ ID NO : 27.

L'unité de transcription obtenue est désignée par CMV-βactine-U1U2U3, autrement appelée E2-bActine-U1U2U3.

Une unité de transcription selon la présente invention peut comprendre également un intron situé en aval de ladite région promotrice.

On entend par « **intron** », une partie non codante d'un gène. Un intron est situé souvent entre deux exons. Après la transcription, cette partie est excisée de l'ARN pour donner l'ARN messager. La présence d'un intron hétérologue permet d'optimiser l'expression des gènes exogènes dans une construction d'ADN.

Dans la construction d'une unité de transcription selon la présente invention, un intron peut être situé :

(i) en aval de la région 5' UTR et en amont du site d'initiation de la traduction, ou
(ii) en aval du promoteur et en amont de la région 5'UTR, ou
(iii) après le site d'initiation de la traduction et à l'intérieur d'une séquence codante, ou
(iv) entre le codon stop de la séquence codante et le signal de polyadénylation.

On entend par « un intron situé en aval de ladite région promotrice » un intron situé après 3' de l'ADN de la région promotrice.

Ledit intron peut être choisi parmi les suivants :

- l'intron du gène Elongation Factor 1α (EF1α) ayant la séquence nucléotidique SEQ ID NO : 11, ou un acide nucléotidique présentant au moins 70% d'identité de séquence avec la séquence SEQ ID NO : 11,
- l'intron murin ROSA ayant la séquence nucléotidique SEQ ID NO : 12, ou un acide nucléotidique ayant au moins 70% d'identité de séquence avec la séquence SEQ ID NO : 12,
- l'intron humain ROSA ayant la séquence nucléotidique SEQ ID NO : 13, ou un acide nucléotidique ayant au moins 70% d'identité de séquence avec la séquence SEQ ID NO : 13.

Ainsi, la présente description divulgue également une unité de transcription comprenant :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) une région promotrice choisie parmi :

- la cycline dépendante des kinases 9 (CDK9), ladite région promotrice ayant la séquence nucléotidique SEQ ID NO : 2, ou un acide nucléotidique ayant au moins 70% d'identité de séquence avec la séquence SEQ ID NO : 2 et possédant essentiellement une activité promotrice, ou
- la β-actine, ladite région promotrice ayant la séquence nucléotidique SEQ ID NO : 3, ou un acide nucléotidique ayant au moins 70% d'identité de séquence avec la séquence SEQ ID NO : 3 et possédant essentiellement une activité promotrice, et

(iii) un intron choisi parmi :

- l'intron du gène Elongation Factor la (EF1α ayant la séquence nucléotidique SEQ ID NO : 11, ou un acide nucléotidique présentant au moins 70% d'identité de séquence avec la séquence SEQ ID NO : 11,
- l'intron murin ROSA ayant la séquence nucléotidique SEQ ID NO : 12, ou un acide nucléotidique ayant au moins 70% d'identité de séquence avec la séquence SEQ ID NO : 12,
- l'intron humain ROSA ayant la séquence nucléotidique SEQ ID NO : 13, ou un acide nucléotidique ayant au moins 70% d'identité de séquence avec la séquence SEQ ID NO : 13.

ledit enhancer étant situé en 5' ou en 3' de l'unité de transcription, ou à l'intérieur de la séquence codante ou dans un intron ; ledit intron étant situé :

(i) en aval de la région 5' UTR et en amont du site d'initiation de la traduction, ou
(ii) en aval du promoteur et en amont de la région 5'UTR, ou
(iii) après le site d'initiation de la traduction et à l'intérieur de la séquence codante, ou
(iv) entre le codon stop de la séquence codante et le signal de polyadénylation.

La présente description divulgue également une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice, et
(iii) l'intron du gène Elongation Factor la (EF1α) ayant la séquence nucléotidique SEQ ID NO : 11, ou un acide nucléotidique présentant au moins 70% d'identité de séquence avec la séquence SEQ ID NO : 11.

La présente description divulgue également un mode de réalisation plus particulier dans lequel l'une unité de transcription est constituée d'un polynucléotide comprenant un acide nucléotidique représenté par la séquence SEQ ID NO : 28 et constitué de :

(i) l'enhancer du virus hCMVie représenté par la séquence nucléotidique SEQ ID NO : 1,
(ii) la région promotrice du gène CDK9 représentée par la séquence nucléotidique SEQ ID NO : 2, et
(iii) l'intron du gène EF1α représenté par la séquence nucléotidique SEQ ID NO : 11,

ou d'un acide nucléotidique ayant au moins 70% d'identité de séquence avec la séquence SEQ ID NO : 28.

L'unité de transcription obtenue est désignée par CMV-CDK9-EF1α, autrement appelée E2-CDK9-EF1a.

La présente description divulgue en particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice, et
(iii) l'intron murin ROSA ayant la séquence nucléotidique SEQ ID NO : 12, ou un acide nucléotidique ayant au moins 70% d'identité de séquence avec la séquence SEQ ID NO : 12.

La présente description divulgue en particulier une unité de transcription constituée d'un polynucléotide comprenant un acide nucléotidique représenté par la séquence SEQ ID NO : 29 et constitué de :

(i) l'enhancer du virus hCMVie représenté par la séquence nucléotidique SEQ ID NO : 1,
(ii) la région promotrice du gène CDK9 représentée par la séquence nucléotidique SEQ ID NO : 2, et
(iii) l'intron murin ROSA ayant la séquence nucléotidique SEQ ID NO : 12,

ou d'un acide nucléotidique ayant au moins 70% d'identité de séquence avec la séquence SEQ ID NO : 29.

L'unité de transcription obtenue est désignée par CMV-CDK9-mROSA, autrement appelée E2-CDK9-mROSA.

La présente description divulgue en particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice, et
(iii) l'intron humain ROSA ayant la séquence nucléotidique SEQ ID NO : 13, ou un acide nucléotidique ayant au moins 70% d'identité de séquence avec la séquence SEQ ID NO : 13.

La présente description divulgue en particulier une unité de transcription constituée d'un polynucléotide comprenant un acide nucléotidique représenté par la séquence SEQ ID NO : 30 et constitué de :

(i) l'enhancer du virus hCMVie représenté par la séquence nucléotidique SEQ ID NO : 1,
(ii) la région promotrice du gène CDK9 représentée par la séquence nucléotidique SEQ ID NO : 2, et
(iii) l'intron humain ROSA ayant la séquence nucléotidique SEQ ID NO : 12, ou d'un acide nucléotidique ayant au moins 70% d'identité de séquence avec la séquence SEQ ID NO : 30.

L'unité de transcription obtenue est désignée par CMV-CDK9-hROSA, autrement appelée E2-CDK9-hROSA.

La présente description divulgue également un mode de réalisation particulier concernant une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
(ii) la région promotrice de la β-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice, et
(iii) l'intron du gène Elongation Factor 1α (EF1α) ayant la séquence nucléotidique SEQ ID NO : 11, ou un acide nucléotidique présentant au moins 70% d'identité de séquence avec la séquence SEQ ID NO : 11.

La présente description divulgue également un mode de réalisation plus particulier dans lequel l'une unité de transcription est constituée d'un polynucléotide comprenant un acide nucléotidique représenté par la séquence SEQ ID NO : 31 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3, et
(iii) l'intron du gène EF1α représenté par la séquence nucléotidique SEQ ID NO : 11,

ou d'un acide nucléotidique ayant au moins 70% d'identité de séquence avec la séquence SEQ ID NO : 31.

L'unité de transcription obtenue est désignée par CMV-βactine-EF1α, autrement appelée E2-bActine-EF.

La présente description divulgue également un mode de réalisation particulier concernant une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la β-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice, et
(iii) l'intron murin ROSA ayant la séquence nucléotidique SEQ ID NO : 12, ou un acide nucléotidique ayant au moins 70% d'identité de séquence avec la séquence SEQ ID NO : 12.

La présente description divulgue également un mode de réalisation plus particulier dans lequel l'une unité de transcription est constituée d'un polynucléotide comprenant un acide nucléotidique représenté par la séquence SEQ ID NO : 32 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3, et
(iii) l'intron murin ROSA ayant la séquence nucléotidique SEQ ID NO : 12,

ou d'un acide nucléotidique ayant au moins 70% d'identité de séquence avec la séquence SEQ ID NO : 32.

L'unité de transcription obtenue est désignée par CMV-βactine-mROSA, autrement appelée E2-bActine-mROSA.

La présente description divulgue également un mode de réalisation particulier concernant une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la β-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice, et
(iii) l'intron humain ROSA ayant la séquence nucléotidique SEQ ID NO : 13, ou un acide nucléotidique ayant au moins 70% d'identité de séquence avec la séquence SEQ ID NO : 13.

La présente description divulgue également un mode de réalisation plus particulier dans lequel l'une unité de transcription est constituée d'un polynucléotide comprenant un acide nucléotidique représenté par la séquence SEQ ID NO : 33 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3, et
(iii) l'intron humain ROSA ayant la séquence nucléotidique SEQ ID NO : 13, ou d'un acide nucléotidique ayant au moins 70% d'identité de séquence avec la séquence SEQ ID NO : 33.

L'unité de transcription obtenue est désignée par CMV-βactine-hROSA, autrement appelée E2-bActine-hROSA.

La présente description divulgue également une unité de transcription comprenant :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) une région promotrice choisie parmi :

- la cycline dépendante des kinases 9 (CDK9), ladite région promotrice ayant la séquence nucléotidique SEQ ID NO : 2, ou un acide nucléotidique ayant au moins 70% d'identité de séquence avec la séquence SEQ ID NO : 2 et possédant essentiellement une activité promotrice, ou
- la β-actine, ladite région promotrice ayant la séquence nucléotidique SEQ ID NO : 3, ou un acide nucléotidique ayant au moins 70% d'identité de séquence avec la séquence SEQ ID NO : 3 et possédant essentiellement une activité promotrice,

(iii) au moins l'une des régions 5' non traduite (5' UTR) choisie parmi :

- la région R du Long Terminal Repeat (LTR) du virus HTLV-1 ayant la séquence nucléotidique SEQ ID NO : 4, ou un acide nucléotidique ayant au moins 70% d'identité de séquence avec la séquence SEQ ID NO : 4,
- la région 5' UTR du gène NF-κB Repressing Factor (NRF) ayant la séquence nucléotidique SEQ ID NO : 5, ou un acide nucléotidique ayant au moins 70% d'identité de séquence avec la séquence SEQ ID NO : 5,
- la région 5' UTR du gène eukaryotic Initiation Factor 4GI (eIF4GI) ayant la séquence nucléotidique SEQ ID NO : 6, ou un acide nucléotidique présentant au moins 70% d'identité de séquence avec la séquence SEQ ID NO : 6, et

(iv) un intron choisi parmi :

- l'intron du gène Elongation Factor 1α (EF1 a ayant la séquence nucléotidique SEQ ID NO : 11, ou un acide nucléotidique présentant au moins 70% d'identité de séquence avec la séquence SEQ ID NO : 11,
- l'intron murin ROSA ayant la séquence nucléotidique SEQ ID NO : 12, ou un acide nucléotidique ayant au moins 70% d'identité de séquence avec la séquence SEQ ID NO : 12,
- l'intron humain ROSA ayant la séquence nucléotidique SEQ ID NO : 13, ou un acide nucléotidique ayant au moins 70% d'identité de séquence avec la séquence SEQ ID NO : 13.

ledit enhancer étant situé en 5' ou en 3' de l'unité de transcription, ou à l'intérieur de la séquence codante ou dans un intron ; ladite région promotrice étant situé en amont de la région 5'UTR ; ledit intron étant situé :

(i) en aval de la région 5' UTR et en amont du site d'initiation de la traduction, ou
(ii) en aval du promoteur et en amont de la région 5'UTR, ou
(iii) après le site d'initiation de la traduction et à l'intérieur de la séquence codante, ou
(iv) entre le codon stop de la séquence codante et le signal de polyadénylation.

La présente description divulgue en particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région R du Long Terminal Repeat (LTR) du virus HTLV-1 ayant la séquence nucléotidique SEQ ID NO : 4, ou un acide nucléotidique ayant au moins 70% d'identité de séquence avec la séquence SEQ ID NO : 4, et
(iv) l'intron du gène Elongation Factor la (EF1α) ayant la séquence nucléotidique SEQ ID NO : 11, ou un acide nucléotidique présentant au moins 70% d'identité de séquence avec la séquence SEQ ID NO : 11.

La présente description divulgue en particulier une unité de transcription constituée d'un polynucléotide comprenant un acide nucléotidique représenté par la séquence SEQ ID NO : 34 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région R du LTR du virus HTLV-1 ayant la séquence nucléotidique SEQ ID NO : 4, et

(iv) l'intron du gène Elongation Factor 1α (EF1α) ayant la séquence nucléotidique SEQ ID NO : 11,

ou d'un acide nucléotidique ayant au moins 70% d'identité de séquence avec la séquence SEQ ID NO : 34.
L'unité de transcription obtenue est désignée par CMV-CDK9-U1-EF1α, autrement appelée E2-CDK9-U1-EF.
La présente description divulgue en particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région R du Long Terminal Repeat (LTR) du virus HTLV-1 ayant la séquence nucléotidique SEQ ID NO : 4, ou un acide nucléotidique ayant au moins 70% d'identité de séquence avec la séquence SEQ ID NO : 4, et
(iv) l'intron murin ROSA ayant la séquence nucléotidique SEQ ID NO : 12, ou un acide nucléotidique ayant au moins 70% d'identité de séquence avec la séquence SEQ ID NO : 12.

La présente description divulgue en particulier une unité de transcription constituée d'un polynucléotide comprenant un acide nucléotidique représenté par la séquence SEQ ID NO : 35 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région R du LTR du virus HTLV-1 ayant la séquence nucléotidique SEQ ID NO : 4, et
(iv) l'intron murin ROSA ayant la séquence nucléotidique SEQ ID NO : 12, ou d'un acide nucléotidique ayant au moins 70% d'identité de séquence avec la séquence SEQ ID NO : 35.

L'unité de transcription obtenue est désignée par CMV-CDK9-U1-mROSA, autrement appelée E2-CDK9-U1-mROSA.
La présente description divulgue en particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice,
(iii) la région R du Long Terminal Repeat (LTR) du virus HTLV-1 ayant la séquence nucléotidique SEQ ID NO : 4, ou un acide nucléotidique ayant au moins 70% d'identité de séquence avec la séquence SEQ ID NO : 4, et
(iv) l'intron humain ROSA ayant la séquence nucléotidique SEQ ID NO : 13, ou un acide nucléotidique ayant au moins 70% d'identité de séquence avec la séquence SEQ ID NO : 13.

La présente description divulgue également une unité de transcription constituée d'un polynucléotide comprenant un acide nucléotidique représenté par la séquence SEQ ID NO : 36 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région R du LTR du virus HTLV-1 ayant la séquence nucléotidique SEQ ID NO : 4, et
(iv) l'intron humain ROSA ayant la séquence nucléotidique SEQ ID NO : 13, ou d'un acide nucléotidique ayant au moins 70% d'identité de séquence avec la séquence SEQ ID NO : 36.

L'unité de transcription obtenue est désignée par CMV-CDK9-U1-hROSA, autrement appelée E2-CDK9-U1-hROSA.
La présente description divulgue en particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice,
(iii) la région 5' UTR du gène NF-κB Repressing Factor (NRF) ayant la séquence nucléotidique SEQ ID NO : 5, ou un acide nucléotidique ayant au moins 70% d'identité de séquence avec la séquence SEQ ID NO : 5, et
(iv) l'intron du gène Elongation Factor 1α (EF1α) ayant la séquence nucléotidique SEQ ID NO : 11, ou un acide nucléotidique présentant au moins 70% d'identité de séquence avec la séquence SEQ ID NO : 11.

La présente description divulgue en particulier une unité de transcription constituée d'un polynucléotide comprenant un acide nucléotidique représenté par la séquence SEQ ID NO : 37 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR du gène NRF ayant la séquence nucléotidique SEQ ID NO : 5, et
(iv) l'intron du gène Elongation Factor 1α (EF1α) ayant la séquence nucléotidique SEQ ID NO : 11,

ou d'un acide nucléotidique ayant au moins 70% d'identité de séquence avec la séquence SEQ ID NO : 37.
L'unité de transcription obtenue est désignée par CMV-CDK9-U2-EF1α, autrement appelée E2-CDK9-U2-EF.
La présente description divulgue en particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR du gène NF-κB Repressing Factor (NRF) ayant la séquence nucléotidique SEQ ID NO : 5, ou un acide nucléotidique ayant au moins 70% d'identité de séquence avec la séquence SEQ ID NO : 5, et
(iv) l'intron murin ROSA ayant la séquence nucléotidique SEQ ID NO : 12, ou un acide nucléotidique ayant au moins 70% d'identité de séquence avec la séquence SEQ ID NO : 12.

La présente description divulgue en particulier une unité de transcription constituée d'un polynucléotide comprenant un acide nucléotidique représenté par la séquence SEQ ID NO : 38 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR du gène NRF ayant la séquence nucléotidique SEQ ID NO : 5, et
(iv) l'intron murin ROSA ayant la séquence nucléotidique SEQ ID NO : 12,

ou d'un acide nucléotidique ayant au moins 70% d'identité de séquence avec la séquence SEQ ID NO : 38.
L'unité de transcription obtenue est désignée par CMV-CDK9-U2-mROSA, autrement appelée E2-CDK9-U2-mROSA.
La présente description divulgue en particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR du gène NF-κB Repressing Factor (NRF) ayant la séquence nucléotidique SEQ ID NO : 5, ou un acide nucléotidique ayant au moins 70% d'identité de séquence avec la séquence SEQ ID NO : 5, et
(iv) l'intron humain ROSA ayant la séquence nucléotidique SEQ ID NO : 13, ou un acide nucléotidique ayant au moins 70% d'identité de séquence avec la séquence SEQ ID NO : 13.

La présente description divulgue en particulier une unité de transcription constituée d'un polynucléotide comprenant un acide nucléotidique représenté par la séquence SEQ ID NO : 39 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR du gène NRF ayant la séquence nucléotidique SEQ ID NO : 5, et
(iv) l'intron humain ROSA ayant la séquence nucléotidique SEQ ID NO : 13, ou d'un acide nucléotidique ayant au moins 70% d'identité de séquence avec la séquence SEQ ID NO : 39.

L'unité de transcription obtenue est désignée par CMV-CDK9-U2-hROSA, autrement appelée E2-CDK9-U2-hROSA.
La présente description divulgue en particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice,
(iii) la région 5' UTR du gène eukaryotic Initiation Factor 4GI (eIF4GI) ayant la séquence nucléotidique SEQ ID NO : 6, ou un acide nucléotidique présentant au moins 70% d'identité de séquence avec la séquence SEQ ID NO : 6, et
(iv) l'intron du gène Elongation Factor la (EF1$\alpha$) ayant la séquence nucléotidique SEQ ID NO : 11, ou un acide nucléotidique présentant au moins 70% d'identité de séquence avec la séquence SEQ ID NO : 11.

La présente description divulgue en particulier une unité de transcription constituée d'un polynucléotide comprenant un acide nucléotidique représenté par la séquence SEQ ID NO : 40 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR du gène eIF4GI ayant la séquence nucléotidique SEQ ID NO : 6, et
(iv) l'intron du gène Elongation Factor 1$\alpha$ (EF1$\alpha$) ayant la séquence nucléotidique SEQ ID NO : 11,

ou d'un acide nucléotidique ayant au moins 70% d'identité de séquence avec la séquence SEQ ID NO : 40, et possédant essentiellement des propriétés d'activation de la transcription supérieures à celles de l'enhancer CMV associé à la région promotrice de CDK9.
L'unité de transcription obtenue est désignée par CMV-CDK9-U3-EF1$\alpha$, autrement appelée E2-CDK9-U3-EF.
La présente description divulgue en particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice,
(iii) la région 5' UTR du gène eukaryotic Initiation Factor 4GI (eIF4GI) ayant la séquence nucléotidique SEQ ID NO : 6, ou un acide nucléotidique présentant au moins 70% d'identité de séquence avec la séquence SEQ ID NO : 6, et
(iv) l'intron murin ROSA ayant la séquence nucléotidique SEQ ID NO : 12, ou un acide nucléotidique ayant au moins 70% d'identité de séquence avec la séquence SEQ ID NO : 12,

La présente description divulgue en particulier une unité de transcription constituée d'un polynucléotide comprenant un acide nucléotidique représenté par la séquence SEQ ID NO : 41 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR du gène eIF4GI ayant la séquence nucléotidique SEQ ID NO : 6, et
(iv) l'intron murin ROSA ayant la séquence nucléotidique SEQ ID NO : 12, ou d'un acide nucléotidique ayant au moins 70% d'identité de séquence avec la séquence SEQ ID NO: 41.

L'unité de transcription obtenue est désignée par CMV-CDK9-U3-mROSA, autrement appelée E2-CDK9-U3-mROSA.
La présente description divulgue en particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice,
(iii) la région 5' UTR du gène eukaryotic Initiation Factor 4GI (eIF4GI) ayant la séquence nucléotidique SEQ ID NO : 6, ou un acide nucléotidique présentant au moins 70% d'identité de séquence avec la séquence SEQ ID NO : 6, et
(iv) l'intron humain ROSA ayant la séquence nucléotidique SEQ ID NO : 13, ou un acide nucléotidique ayant au moins 70% d'identité de séquence avec la séquence SEQ ID NO : 13,

La présente description divulgue en particulier une unité de transcription constituée d'un polynucléotide comprenant un acide nucléotidique représenté par la séquence SEQ ID NO : 42 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR du gène eIF4GI ayant la séquence nucléotidique SEQ ID NO : 6, et
(iv) l'intron humain ROSA ayant la séquence nucléotidique SEQ ID NO : 13, ou d'un acide nucléotidique ayant au moins 70% d'identité de séquence avec la séquence SEQ ID NO : 42.

L'unité de transcription obtenue est désignée par CMV-CDK9-U3-hROSA, autrement appelée E2-CDK9-U3-hROSA.
La présente description divulgue en particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 7, ou un acide nucléotidique présentant au moins 70% d'identité de séquence avec la séquence SEQ ID NO: 7,
(iv) l'intron du gène Elongation Factor 1$\alpha$ (EF1$\alpha$) ayant la séquence nucléotidique SEQ ID NO : 11, ou un acide nucléotidique présentant au moins 70% d'identité de séquence avec la séquence SEQ ID NO : 11.

La présente description divulgue en particulier une unité de transcription constituée d'un polynucléotide comprenant un acide nucléotidique représenté par la séquence SEQ ID NO : 43 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR représentée par la séquence nucléotidique SEQ ID NO : 7, et

(iv) l'intron du gène Elongation Factor 1α (EF1α) ayant la séquence nucléotidique SEQ ID NO : 11,

ou d'un acide nucléotidique ayant au moins 70% d'identité de séquence avec la séquence SEQ ID NO : 43.

L'unité de transcription obtenue est désignée par CMV-CDK9-U1U2-EF1α, autrement appelée E2-CDK9-U1U2-EF.

La présente description divulgue en particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 7, ou un acide nucléotidique présentant au moins 70% d'identité de séquence avec la séquence SEQ ID NO : 7, et
(iv) l'intron murin ROSA ayant la séquence nucléotidique SEQ ID NO : 12, ou un acide nucléotidique ayant au moins 70% d'identité de séquence avec la séquence SEQ ID NO : 12.

La présente description divulgue en particulier une unité de transcription constituée d'un polynucléotide comprenant un acide nucléotidique représenté par la séquence SEQ ID NO : 44 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR représentée par la séquence nucléotidique SEQ ID NO : 7, et
(iv) l'intron murin ROSA ayant la séquence nucléotidique SEQ ID NO : 12,

ou d'un acide nucléotidique ayant au moins 70% d'identité de séquence avec la séquence SEQ ID NO : 44.

L'unité de transcription obtenue est désignée par CMV-CDK9-U1U2-mROSA, autrement appelée E2-CDK9-U1U2-mROSA.

La présente description divulgue en particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice,
(iii) la région 5' UTR représentée par la séquence SEQ ID NO : 7, ou un acide nucléotidique présentant au moins 70% d'identité de séquence avec la séquence SEQ ID NO : 7, et
(iv) l'intron humain ROSA ayant la séquence nucléotidique SEQ ID NO : 13, ou un acide nucléotidique ayant au moins 70% d'identité de séquence avec la séquence SEQ ID NO : 13.

La présente description divulgue en particulier une unité de transcription constituée d'un polynucléotide comprenant un acide nucléotidique représenté par la séquence SEQ ID NO : 45 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR représentée par la séquence nucléotidique SEQ ID NO : 7, et
(iv) l'intron humain ROSA ayant la séquence nucléotidique SEQ ID NO : 13, ou d'un acide nucléotidique ayant au moins 70% d'identité de séquence avec la séquence SEQ ID NO : 45.

L'unité de transcription obtenue est désignée par CMV-CDK9-U1U2-hROSA, autrement appelée E2-CDK9-U1U2-hROSA.

La présente description divulgue en particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice,
(iii) la région 5' UTR représentée par la séquence SEQ ID NO : 8, ou un acide nucléotidique présentant au moins 70% d'identité de séquence avec la séquence SEQ ID NO : 8, et
(iv) l'intron du gène Elongation Factor 1$\alpha$ (EF1$\alpha$) ayant la séquence nucléotidique SEQ ID NO : 11, ou un acide nucléotidique présentant au moins 70% d'identité de séquence avec la séquence SEQ ID NO : 11.

La présente description divulgue en particulier une unité de transcription constituée d'un polynucléotide comprenant un acide nucléotidique représenté par la séquence SEQ ID NO : 46 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR représentée par la séquence nucléotidique SEQ ID NO : 8, et
(iv) l'intron du gène Elongation Factor 1$\alpha$ (EF1$\alpha$) ayant la séquence nucléotidique SEQ ID NO : 11,

ou d'un acide nucléotidique ayant au moins 70% d'identité de séquence avec la séquence SEQ ID NO : 46.
L'unité de transcription obtenue est désignée par CMV-CDK9-U1U3-EF1$\alpha$, autrement appelée E2-CDK9-U1U3-EF.
La présente description divulgue en particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 8, ou un acide nucléotidique présentant au moins 70% d'identité de séquence avec la séquence SEQ ID NO : 8, et
(iv) l'intron murin ROSA ayant la séquence nucléotidique SEQ ID NO : 12, ou un acide nucléotidique ayant au moins 70% d'identité de séquence avec la séquence SEQ ID NO : 12.

La présente description divulgue en particulier une unité de transcription constituée d'un polynucléotide comprenant un acide nucléotidique représenté par la séquence SEQ ID NO : 47 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR représentée par la séquence nucléotidique SEQ ID NO : 8, et
(iv) l'intron murin ROSA ayant la séquence nucléotidique SEQ ID NO : 12,

ou d'un acide nucléotidique ayant au moins 70% d'identité de séquence avec la séquence SEQ ID NO : 47.
L'unité de transcription obtenue est désignée par CMV-CDK9-U1U3-mROSA, autrement appelée E2-CDK9-U1U3-mROSA.
La présente description divulgue en particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice,
(iii) la région 5' UTR représentée par la séquence SEQ ID NO : 9, ou un acide nucléotidique présentant au moins 70% d'identité de séquence avec la séquence SEQ ID NO : 9, et
(iv) l'intron humain ROSA ayant la séquence nucléotidique SEQ ID NO : 13, ou un acide nucléotidique ayant au moins 70% d'identité de séquence avec la séquence SEQ ID NO : 13.

La présente description divulgue en particulier une unité de transcription constituée d'un polynucléotide comprenant un acide nucléotidique représenté par la séquence SEQ ID NO : 48 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR représentée par la séquence nucléotidique SEQ ID NO : 8, et
(iv) l'intron humain ROSA ayant la séquence nucléotidique SEQ ID NO : 13, ou d'un acide nucléotidique ayant au moins 70% d'identité de séquence avec la séquence SEQ ID NO : 48.

L'unité de transcription obtenue est désignée par CMV-CDK9-U1U3-hROSA, autrement appelée E2-CDK9-U1U3-hROSA.

La présente description divulgue en particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 9, ou un acide nucléotidique présentant au moins 70% d'identité de séquence avec la séquence SEQ ID NO : 9, et
(iv) l'intron du gène Elongation Factor 1$\alpha$ (EF1$\alpha$) ayant la séquence nucléotidique SEQ ID NO : 11, ou un acide nucléotidique présentant au moins 70% d'identité de séquence avec la séquence SEQ ID NO : 11.

La présente description divulgue en particulier une unité de transcription constituée d'un polynucléotide comprenant un acide nucléotidique représenté par la séquence SEQ ID NO : 49 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR représentée par la séquence nucléotidique SEQ ID NO : 9, et
(iv) l'intron du gène Elongation Factor 1$\alpha$ (EF1$\alpha$) ayant la séquence nucléotidique SEQ ID NO : 11,

ou d'un acide nucléotidique ayant au moins 70% d'identité de séquence avec la séquence SEQ ID NO : 49.

L'unité de transcription obtenue est désignée par CMV-CDK9-U2U3-EF1$\alpha$, autrement appelée E2-CDK9-U2U3-EF.

La présente description divulgue en particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 9, ou un acide nucléotidique présentant au moins 70% d'identité de séquence avec la séquence SEQ ID NO : 9,
(iv) l'intron murin ROSA ayant la séquence nucléotidique SEQ ID NO : 12, ou un acide nucléotidique ayant au moins 70% d'identité de séquence avec la séquence SEQ ID NO : 12.

La présente description divulgue en particulier une unité de transcription constituée d'un polynucléotide comprenant un acide nucléotidique représenté par la séquence SEQ ID NO : 50 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR représentée par la séquence nucléotidique SEQ ID NO : 9, et
(iv) l'intron murin ROSA ayant la séquence nucléotidique SEQ ID NO : 12,

ou d'un acide nucléotidique ayant au moins 70% d'identité de séquence avec la séquence SEQ ID NO : 50.

L'unité de transcription obtenue est désignée par CMV-CDK9-U2U3-mROSA, autrement appelée E2-CDK9-UU2U3-mROSA.

La présente description divulgue en particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 9, ou un acide nucléotidique présentant au moins 70% d'identité de séquence avec la séquence SEQ ID NO : 9, et
(iv) l'intron humain ROSA ayant la séquence nucléotidique SEQ ID NO : 13, ou un acide nucléotidique ayant au moins 70% d'identité de séquence avec la séquence SEQ ID NO : 13.

La présente description divulgue en particulier une unité de transcription constituée d'un polynucléotide comprenant un acide nucléotidique représenté par la séquence SEQ ID NO : 51 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR représentée par la séquence nucléotidique SEQ ID NO : 9, et
(iv) l'intron humain ROSA ayant la séquence nucléotidique SEQ ID NO : 13, ou d'un acide nucléotidique ayant au moins 70% d'identité de séquence avec la séquence SEQ ID NO : 51.

L'unité de transcription obtenue est désignée par CMV-CDK9-U2U3-hROSA, autrement appelée E2-CDK9-U2U3-hROSA.

La présente description divulgue en particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 10, ou un acide nucléotidique présentant au moins 70% d'identité de séquence avec la séquence SEQ ID NO : 10, et
(iv) l'intron du gène Elongation Factor 1$\alpha$ (EF1$\alpha$) ayant la séquence nucléotidique SEQ ID NO : 11, ou un acide nucléotidique présentant au moins 70% d'identité de séquence avec la séquence SEQ ID NO : 11.

La présente description divulgue en particulier une unité de transcription constituée d'un polynucléotide comprenant un acide nucléotidique représenté par la séquence SEQ ID NO : 52 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,

(iii) la région 5' UTR représentée par la séquence nucléotidique SEQ ID NO : 10, et
(iv) l'intron du gène Elongation Factor 1α (EF1α) ayant la séquence nucléotidique SEQ ID NO : 11,

ou d'un acide nucléotidique ayant au moins 70% d'identité de séquence avec la séquence SEQ ID NO : 52.

L'unité de transcription obtenue est désignée par CMV-CDK9-U1U2U3-EF1α, autrement appelée E2-CDK9-U1U2U3-EF.

La présente description divulgue en particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 10, ou un acide nucléotidique présentant au moins 70% d'identité de séquence avec la séquence SEQ ID NO : 10, et
(iv) l'intron murin ROSA ayant la séquence nucléotidique SEQ ID NO : 12, ou un acide nucléotidique ayant au moins 70% d'identité de séquence avec la séquence SEQ ID NO : 12.

La présente description divulgue en particulier une unité de transcription constituée d'un polynucléotide comprenant un acide nucléotidique représenté par la séquence SEQ ID NO : 53 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR représentée par la séquence nucléotidique SEQ ID NO : 10, et
(iv) l'intron murin ROSA ayant la séquence nucléotidique SEQ ID NO : 12,

ou d'un acide nucléotidique ayant au moins 70% d'identité de séquence avec la séquence SEQ ID NO : 53.

L'unité de transcription obtenue est désignée par CMV-CDK9-U1U2U3-mROSA, autrement appelée E2-CDK9-U1U2U3-mROSA.

La présente description divulgue en particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice,
(iii) la région 5' UTR représentée par la séquence SEQ ID NO : 10, ou un acide nucléotidique présentant au moins 70% d'identité de séquence avec la séquence SEQ ID NO : 10, et
(iv) l'intron humain ROSA ayant la séquence nucléotidique SEQ ID NO : 13, ou un acide nucléotidique ayant au moins 70% d'identité de séquence avec la séquence SEQ ID NO : 13.

La présente description divulgue en particulier une unité de transcription constituée d'un polynucléotide comprenant un acide nucléotidique représenté par la séquence SEQ ID NO : 54 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR représentée par la séquence nucléotidique SEQ ID NO : 10, et
(iv) l'intron humain ROSA ayant la séquence nucléotidique SEQ ID NO : 13, ou d'un acide nucléotidique ayant au moins 70% d'identité de séquence avec la séquence SEQ ID NO : 54.

L'unité de transcription obtenue est désignée par CMV-CDK9-U1U2U3-hROSA, autrement appelée E2-CDK9-U1U2U3-hROSA.

La présente description concerne selon un mode de réalisation particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la β-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice,
(iii) la région R du Long Terminal Repeat (LTR) du virus HTLV-1 ayant la séquence nucléotidique SEQ ID NO : 4, ou un acide nucléotidique ayant au moins 70% d'identité de séquence avec la séquence SEQ ID NO : 4, et
(iv) l'intron du gène Elongation Factor 1α (EF1α) ayant la séquence nucléotidique SEQ ID NO : 11, ou un acide nucléotidique présentant au moins 70% d'identité de séquence avec la séquence SEQ ID NO : 11.

Dans un mode de réalisation plus particulier La présente description concerne , une unité de transcription constituée d'un polynucléotide comprenant un acide nucléotidique représenté par la séquence SEQ ID NO : 55 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3,
(iii) la région R du LTR du virus HTLV-1 ayant la séquence nucléotidique SEQ ID NO : 4,
(iv) l'intron du gène Elongation Factor 1α (EF1α) ayant la séquence nucléotidique SEQ ID NO : 11,

ou d'un acide nucléotidique ayant au moins 70% d'identité de séquence avec la séquence SEQ ID NO : 55.
L'unité de transcription obtenue est désignée par CMV-βactine-U1-EF1α, autrement appelée E2-bActine-U1-EF.
La présente description concerne selon un mode de réalisation particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la β-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice,
(iii) la région R du Long Terminal Repeat (LTR) du virus HTLV-1 ayant la séquence nucléotidique SEQ ID NO : 4, ou un acide nucléotidique ayant au moins 70% d'identité de séquence avec la séquence SEQ ID NO : 4, et
(iv) l'intron murin ROSA ayant la séquence nucléotidique SEQ ID NO : 12, ou un acide nucléotidique ayant au moins 70% d'identité de séquence avec la séquence SEQ ID NO : 12.

La présente description concerne selon un mode de réalisation plus particulier, une unité de transcription constituée d'un polynucléotide comprenant un acide nucléotidique représenté par la séquence SEQ ID NO : 56 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3,
(iii) la région R du LTR du virus HTLV-1 ayant la séquence nucléotidique SEQ ID NO : 4, et
(iv) l'intron murin ROSA ayant la séquence nucléotidique SEQ ID NO : 12,

ou d'un acide nucléotidique ayant au moins 70% d'identité de séquence avec la séquence SEQ ID NO : 56.
L'unité de transcription obtenue est désignée par CMV-βactine-U1-mROSA, autrement appelée E2-bActine-U1-mROSA.
La présente description concerne selon un mode de réalisation particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,

(ii) la région promotrice de la β-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice,
(iii) la région R du Long Terminal Repeat (LTR) du virus HTLV-1 ayant la séquence nucléotidique SEQ ID NO : 4, ou un acide nucléotidique ayant au moins 70% d'identité de séquence avec la séquence SEQ ID NO : 4, et
(iv) l'intron humain ROSA ayant la séquence nucléotidique SEQ ID NO : 13, ou un acide nucléotidique ayant au moins 70% d'identité de séquence avec la séquence SEQ ID NO : 13.

Dans un mode de réalisation plus particulier, l'unité de transcription est constituée d'un polynucléotide comprenant un acide nucléotidique représenté par la séquence SEQ ID NO : 57 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3,
(iii) la région R du LTR du virus HTLV-1 ayant la séquence nucléotidique SEQ ID NO : 4, et
(iv) l'intron humain ROSA ayant la séquence nucléotidique SEQ ID NO : 13, ou d'un acide nucléotidique ayant au moins 70% d'identité de séquence avec la séquence SEQ ID NO : 57.

L'unité de transcription obtenue est désignée par CMV-βactine-U1-hROSA, autrement appelée E2-bActine-U1-hROSA.

La présente description concerne selon un mode de réalisation particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la β-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice,
(iii) la région 5' UTR du gène NF-κB Repressing Factor (NRF) ayant la séquence nucléotidique SEQ ID NO : 5, ou un acide nucléotidique ayant au moins 70% d'identité de séquence avec la séquence SEQ ID NO : 5, et
(iv) l'intron du gène Elongation Factor 1α (EF1α) ayant la séquence nucléotidique SEQ ID NO : 11, ou un acide nucléotidique présentant au moins 70% d'identité de séquence avec la séquence SEQ ID NO : 11.

La présente description concerne selon un mode de réalisation plus particulier, une unité de transcription constituée d'un polynucléotide comprenant un acide nucléotidique représenté par la séquence SEQ ID NO : 58 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3,
(iii) la région 5' UTR du gène NRF ayant la séquence nucléotidique SEQ ID NO : 5, et
(iv) l'intron du gène Elongation Factor 1α (EF1α) ayant la séquence nucléotidique SEQ ID NO : 11,

ou d'un acide nucléotidique ayant au moins 70% d'identité de séquence avec la séquence SEQ ID NO : 58.

L'unité de transcription obtenue est désignée par CMV-βactine-U2-EF1α, autrement appelée E2-bActine-U2-EF.

La présente description concerne selon un mode de réalisation particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la β-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice,
(iii) la région 5' UTR du gène NF-κB Repressing Factor (NRF) ayant la séquence nucléotidique SEQ ID NO : 5, ou un acide nucléotidique ayant au moins 70% d'identité de séquence avec la séquence SEQ ID NO : 5, et
(iv) l'intron murin ROSA ayant la séquence nucléotidique SEQ ID NO : 12, ou un acide nucléotidique ayant au moins 70% d'identité de séquence avec la séquence SEQ ID NO : 12.

La présente description concerne selon un mode de réalisation plus particulier, une unité de transcription constituée d'un polynucléotide comprenant un acide nucléotidique représenté par la séquence SEQ ID NO : 59 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3,
(iii) la région 5' UTR du gène NRF ayant la séquence nucléotidique SEQ ID NO : 5, et
(iv) l'intron murin ROSA ayant la séquence nucléotidique SEQ ID NO : 12,

ou d'un acide nucléotidique ayant au moins 70% d'identité de séquence avec la séquence SEQ ID NO : 59.

L'unité de transcription obtenue est désignée par CMV-βactine-U2-mROSA, autrement appelée E2-bActine-U2-mROSA.

La présente description concerne selon un mode de réalisation particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la β-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice,
(iii) la région 5' UTR du gène NF-κB Repressing Factor (NRF) ayant la séquence nucléotidique SEQ ID NO : 5, ou un acide nucléotidique ayant au moins 70% d'identité de séquence avec la séquence SEQ ID NO : 5, et
(iv) l'intron humain ROSA ayant la séquence nucléotidique SEQ ID NO : 13, ou un acide nucléotidique ayant au moins 70% d'identité de séquence avec la séquence SEQ ID NO : 13.

La présente description concerne selon un mode de réalisation plus particulier, une unité de transcription constituée d'un polynucléotide comprenant un acide nucléotidique représenté par la séquence SEQ ID NO : 60 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3,
(iii) la région 5' UTR du gène NRF ayant la séquence nucléotidique SEQ ID NO : 5, et
(iv) l'intron humain ROSA ayant la séquence nucléotidique SEQ ID NO : 13, ou d'un acide nucléotidique ayant au moins 70% d'identité de séquence avec la séquence SEQ ID NO : 60.

L'unité de transcription obtenue est désignée par CMV-βactine-U2-hROSA, autrement appelée E2-bActine-U2-hROSA.

La présente description concerne selon un mode de réalisation particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la β-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice,
(iii) la région 5' UTR du gène eukaryotic Initiation Factor 4GI (eIF4GI) ayant la séquence nucléotidique SEQ ID NO : 6, ou un acide nucléotidique présentant au moins 70% d'identité de séquence avec la séquence SEQ ID NO : 6, et
(iv) l'intron du gène Elongation Factor 1α (EF1α) ayant la séquence nucléotidique SEQ ID NO : 11, ou un acide nucléotidique présentant au moins 70% d'identité de séquence avec la séquence SEQ ID NO : 11.

La présente description concerne selon un mode de réalisation plus particulier, une unité de transcription constituée d'un polynucléotide comprenant un acide nucléotidique représenté par la séquence SEQ ID NO : 61 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3,

(iii) la région 5' UTR du gène eIF4GI ayant la séquence nucléotidique SEQ ID NO : 6, et
(iv) l'intron du gène Elongation Factor 1$\alpha$ (EF1$\alpha$) ayant la séquence nucléotidique SEQ ID NO : 11,

ou d'un acide nucléotidique ayant au moins 70% d'identité de séquence avec la séquence SEQ ID NO : 61.

L'unité de transcription obtenue est désignée par CMV-$\beta$actine-U3-EF1$\alpha$, autrement appelée E2-bActine-U3-EF.

La présente description concerne selon un mode de réalisation particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la $\beta$-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice,
(iii) la région 5' UTR du gène eukaryotic Initiation Factor 4GI (eIF4GI) ayant la séquence nucléotidique SEQ ID NO : 6, ou un acide nucléotidique présentant au moins 70% d'identité de séquence avec la séquence SEQ ID NO : 6, et
(iv) l'intron murin ROSA ayant la séquence nucléotidique SEQ ID NO : 12, ou un acide nucléotidique ayant au moins 70% d'identité de séquence avec la séquence SEQ ID NO : 12,

La présente description concerne selon un mode de réalisation plus particulier une unité de transcription constituée d'un polynucléotide comprenant un acide nucléotidique représenté par la séquence SEQ ID NO : 62 et constitué de :

(i) l'enhancer du virus hCMVie représenté par la séquence nucléotidique SEQ ID NO : 1,
(ii) la région promotrice de la $\beta$-actine représentée par la séquence nucléotidique SEQ ID NO : 3,
(iii) la région 5' UTR du gène eIF4GI ayant la séquence nucléotidique SEQ ID NO : 6, et
(iv) l'intron murin ROSA ayant la séquence nucléotidique SEQ ID NO : 12,

ou d'un acide nucléotidique ayant au moins 70% d'identité de séquence avec la séquence SEQ ID NO : 62.

L'unité de transcription obtenue est désignée par CMV-$\beta$actine-U3-mROSA, autrement appelée E2-bActine-U3-mROSA.

La présente description concerne selon un mode de réalisation particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la $\beta$-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice,
(iii) la région 5' UTR du gène eukaryotic Initiation Factor 4GI (eIF4GI) ayant la séquence nucléotidique SEQ ID NO : 6, ou un acide nucléotidique présentant au moins 70% d'identité de séquence avec la séquence SEQ ID NO : 6, et
(iv) l'intron humain ROSA ayant la séquence nucléotidique SEQ ID NO : 13, ou un acide nucléotidique ayant au moins 70% d'identité de séquence avec la séquence SEQ ID NO : 13,

La présente description concerne selon un mode de réalisation plus particulier, une unité de transcription constituée d'un polynucléotide comprenant un acide nucléotidique représenté par la séquence SEQ ID NO : 63 et constitué de :

(i) l'enhancer du virus hCMVie représenté par la séquence nucléotidique SEQ ID NO : 1,
(ii) la région promotrice de la $\beta$-actine représentée par la séquence nucléotidique SEQ ID NO : 3,
(iii) la région 5' UTR du gène eIF4GI ayant la séquence nucléotidique SEQ ID NO : 6, et
(iv) l'intron humain ROSA ayant la séquence nucléotidique SEQ ID NO : 13, ou d'un acide nucléotidique ayant au moins 70% d'identité de séquence avec la séquence SEQ ID NO : 63.

L'unité de transcription obtenue est désignée par CMV-$\beta$actine-U3-hROSA, autrement appelée E2-bActine-U3-hROSA.

La présente description concerne selon un mode de réalisation particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la β-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice,
(iii) la région 5' UTR représentée par la séquence SEQ ID NO : 7, ou un acide nucléotidique présentant au moins 70% d'identité de séquence avec la séquence SEQ ID NO : 7, et
(iv) l'intron du gène Elongation Factor 1α (EF1α) ayant la séquence nucléotidique SEQ ID NO : 11, ou un acide nucléotidique présentant au moins 70% d'identité de séquence avec la séquence SEQ ID NO : 11.

La présente description concerne selon un mode de réalisation plus particulier une unité de transcription constituée d'un polynucléotide comprenant un acide nucléotidique représenté par la séquence SEQ ID NO : 64 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3,
(iii) la région 5' UTR représentée par la séquence nucléotidique SEQ ID NO : 7, et
(iv) l'intron du gène Elongation Factor 1α (EF1α) ayant la séquence nucléotidique SEQ ID NO : 11,

ou d'un acide nucléotidique ayant au moins 70% d'identité de séquence avec la séquence SEQ ID NO : 64.
L'unité de transcription obtenue est désignée par CMV-βactine-U1U2-EF1α, autrement appelée E2-bActine-U1U2-EF.
La présente description concerne selon un mode de réalisation particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la β-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice,
(iii) la région 5' UTR représentée par la séquence SEQ ID NO : 7, ou un acide nucléotidique présentant au moins 70% d'identité de séquence avec la séquence SEQ ID NO : 7, et
(iv) l'intron murin ROSA ayant la séquence nucléotidique SEQ ID NO : 12, ou un acide nucléotidique ayant au moins 70% d'identité de séquence avec la séquence SEQ ID NO : 12.

La présente description concerne selon unmode de réalisation plus particulier, une unité de transcription constituée d'un polynucléotide comprenant un acide nucléotidique représenté par la séquence SEQ ID NO : 65 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3,
(iii) la région 5' UTR représentée par la séquence nucléotidique SEQ ID NO : 7, et
(iv) l'intron murin ROSA ayant la séquence nucléotidique SEQ ID NO : 12,

ou d'un acide nucléotidique ayant au moins 70% d'identité de séquence avec la séquence SEQ ID NO : 65.
L'unité de transcription obtenue est désignée par CMV-βactine-U1U2-mROSA, autrement appelée E2-bActine-U1U2-mROSA.
La présente description concerne selon un mode de réalisation particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la β-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice,

(iii) la région 5' UTR représentée par la séquence SEQ ID NO : 7, ou un acide nucléotidique présentant au moins 70% d'identité de séquence avec la séquence SEQ ID NO : 7, et
(iv) l'intron humain ROSA ayant la séquence nucléotidique SEQ ID NO : 13, ou un acide nucléotidique ayant au moins 70% d'identité de séquence avec la séquence SEQ ID NO : 13.

La présente description concerne selon unmode de réalisation plus particulier, une unité de transcription constituée d'un polynucléotide comprenant un acide nucléotidique représenté par la séquence SEQ ID NO : 66 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3,
(iii) la région 5' UTR représentée par la séquence nucléotidique SEQ ID NO : 7, et
(iv) l'intron humain ROSA ayant la séquence nucléotidique SEQ ID NO : 13, ou d'un acide nucléotidique ayant au moins 70% d'identité de séquence avec la séquence SEQ ID NO : 66.

L'unité de transcription obtenue est désignée par CMV-βactine-U1U2-hROSA, autrement appelée E2-bActine-U1U2-hROSA.

L'invention concerne une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la β-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice,
(iii) la région 5' UTR représentée par la séquence SEQ ID NO : 8, ou un acide nucléotidique présentant au moins 70% d'identité de séquence avec la séquence SEQ ID NO : 8, et
(iv) l'intron du gène Elongation Factor 1α (EF1α) ayant la séquence nucléotidique SEQ ID NO : 11, ou un acide nucléotidique présentant au moins 70% d'identité de séquence avec la séquence SEQ ID NO : 11.

Dans un mode de réalisation plus particulier de l'invention, une unité de transcription selon l'invention est constituée d'un polynucléotide comprenant un acide nucléotidique représenté par la séquence SEQ ID NO : 67 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3,
(iii) la région 5' UTR représentée par la séquence nucléotidique SEQ ID NO : 8, et
(iv) l'intron du gène Elongation Factor 1α (EF1α) ayant la séquence nucléotidique SEQ ID NO : 11,

ou d'un acide nucléotidique ayant au moins 70% d'identité de séquence avec la séquence SEQ ID NO : 67.

L'unité de transcription obtenue est désignée par CMV-pactine-U1U3-EF1α, autrement appelée E2-bActine-U1U3-EF.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la β-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice,
(iii) la région 5' UTR représentée par la séquence SEQ ID NO : 8, ou un acide nucléotidique présentant au moins 70% d'identité de séquence avec la séquence SEQ ID NO : 8, et
(iv) l'intron murin ROSA ayant la séquence nucléotidique SEQ ID NO : 12, ou un acide nucléotidique ayant au moins 70% d'identité de séquence avec la séquence SEQ ID NO : 12.

Dans un mode de réalisation plus particulier de l'invention, une unité de transcription selon l'invention est constituée d'un polynucléotide comprenant un acide nucléotidique représenté par la séquence SEQ ID NO : 68 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3,
(iii) la région 5' UTR représentée par la séquence nucléotidique SEQ ID NO : 8, et
(iv) l'intron murin ROSA ayant la séquence nucléotidique SEQ ID NO : 12,

ou d'un acide nucléotidique ayant au moins 70% d'identité de séquence avec la séquence SEQ ID NO : 68.

L'unité de transcription obtenue est désignée par CMV-βactine-U1U3-mROSA, autrement appelée E2-bActine-U1U3-mROSA.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la β-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice,
(iii) la région 5' UTR représentée par la séquence SEQ ID NO : 8, ou un acide nucléotidique présentant au moins 70% d'identité de séquence avec la séquence SEQ ID NO : 9, et
(iv) l'intron humain ROSA ayant la séquence nucléotidique SEQ ID NO : 13, ou un acide nucléotidique ayant au moins 70% d'identité de séquence avec la séquence SEQ ID NO : 13.

Dans un mode de réalisation plus particulier de l'invention, une unité de transcription selon l'invention est constituée d'un polynucléotide comprenant un acide nucléotidique représenté par la séquence SEQ ID NO : 69 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3,
(iii) la région 5' UTR représentée par la séquence nucléotidique SEQ ID NO : 8, et
(iv) l'intron humain ROSA ayant la séquence nucléotidique SEQ ID NO : 13, ou d'un acide nucléotidique ayant au moins 70% d'identité de séquence avec la séquence SEQ ID NO : 69.

L'unité de transcription obtenue est désignée par CMV-βactine-U1U3-hROSA, autrement appelée E2-bActine-U1U3-hROSA.

La présente description concerne selon un mode de réalisation particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la β-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice,
(iii) la région 5' UTR représentée par la séquence SEQ ID NO : 9, ou un acide nucléotidique présentant au moins 70% d'identité de séquence avec la séquence SEQ ID NO : 9, et
(iv) l'intron du gène Elongation Factor 1α (EF1α) ayant la séquence nucléotidique SEQ ID NO : 11, ou un acide nucléotidique présentant au moins 70% d'identité de séquence avec la séquence SEQ ID NO : 11.

La présente description concerne selon unmode de réalisation plus particulier, une unité de transcription constituée d'un polynucléotide comprenant un acide nucléotidique représenté par la séquence SEQ ID NO : 70 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3,
(iii) la région 5' UTR représentée par la séquence nucléotidique SEQ ID NO : 9, et
(iv) l'intron du gène Elongation Factor 1α (EF1α) ayant la séquence nucléotidique SEQ ID NO : 11,

ou d'un acide nucléotidique ayant au moins 70% d'identité de séquence avec la séquence SEQ ID NO : 70.

L'unité de transcription obtenue est désignée par CMV-βactine-U2U3-EF1α, autrement appelée E2-bActine-U2U3-EF.

La présente description concerne selon un mode de réalisation particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la β-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice,
(iii) la région 5' UTR représentée par la séquence SEQ ID NO : 9, ou un acide nucléotidique présentant au moins 70% d'identité de séquence avec la séquence SEQ ID NO : 9, et
(iv) l'intron murin ROSA ayant la séquence nucléotidique SEQ ID NO : 12, ou un acide nucléotidique ayant au moins 70% d'identité de séquence avec la séquence SEQ ID NO : 12.

La présente description concerne selon unmode de réalisation plus particulier une unité de transcription constituée d'un polynucléotide comprenant un acide nucléotidique représenté par la séquence SEQ ID NO : 71 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3,
(iii) la région 5' UTR représentée par la séquence nucléotidique SEQ ID NO : 9, et
(iv) l'intron murin ROSA ayant la séquence nucléotidique SEQ ID NO : 12,

ou d'un acide nucléotidique ayant au moins 70% d'identité de séquence avec la séquence SEQ ID NO: 71.

L'unité de transcription obtenue est désignée par CMV-βactine-U2U3-mROSA, autrement appelée E2-bActine-U2U3-mROSA.

La présente description concerne selon un mode de réalisation particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la β-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice,
(iii) la région 5' UTR représentée par la séquence SEQ ID NO : 9, ou un acide nucléotidique présentant au moins 70% d'identité de séquence avec la séquence SEQ ID NO : 9, et
(iv) l'intron humain ROSA ayant la séquence nucléotidique SEQ ID NO : 13, ou un acide nucléotidique ayant au moins 70% d'identité de séquence avec la séquence SEQ ID NO : 13.

La présente description concerne selon unmode de réalisation plus particulier, une unité de transcription constituée d'un polynucléotide comprenant un acide nucléotidique représenté par la séquence SEQ ID NO : 72 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3,
(iii) la région 5' UTR représentée par la séquence nucléotidique SEQ ID NO : 9,
(iv) l'intron humain ROSA ayant la séquence nucléotidique SEQ ID NO : 13, et ou d'un acide nucléotidique ayant au moins 70% d'identité de séquence avec la séquence SEQ ID NO : 72.

L'unité de transcription obtenue est désignée par CMV-βactine-U2U3-hROSA, autrement appelée E2-bActine-U2U3-hROSA.

La présente description concerne selon un mode de réalisation particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,

(ii) la région promotrice de la β-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice,

(iii) la région 5' UTR représentée par la séquence SEQ ID NO : 10, ou un acide nucléotidique présentant au moins 70% d'identité de séquence avec la séquence SEQ ID NO : 10, et

(iv) l'intron du gène Elongation Factor 1α (EF1α) ayant la séquence nucléotidique SEQ ID NO : 11, ou un acide nucléotidique présentant au moins 70% d'identité de séquence avec la séquence SEQ ID NO : 11.

La présente description concerne selon unmode de réalisation plus particulier, une unité de transcription constituée d'un polynucléotide comprenant un acide nucléotidique représenté par la séquence SEQ ID NO : 73 et constitué de :

(i) l'enhancer du virus hCMVie représenté par la séquence nucléotidique SEQ ID NO : 1,

(ii) la région promotrice de la β-actine représentée par la séquence nucléotidique SEQ ID NO : 3,

(iii) la région 5' UTR représentée par la séquence nucléotidique SEQ ID NO : 10, et

(iv) l'intron du gène Elongation Factor 1α (EF1α) ayant la séquence nucléotidique SEQ ID NO : 11,

ou d'un acide nucléotidique ayant au moins 70% d'identité de séquence avec la séquence SEQ ID NO : 73.

L'unité de transcription obtenue est désignée par CMV-βactine-U1U2U3-EF1α, autrement appelée E2-bActine-U1U2U3-EF.

La présente description concerne selon un mode de réalisation particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,

(ii) la région promotrice de la β-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice,

(iii) la région 5' UTR représentée par la séquence SEQ ID NO : 10, ou un acide nucléotidique présentant au moins 70% d'identité de séquence avec la séquence SEQ ID NO : 10, et

(iv) l'intron murin ROSA ayant la séquence nucléotidique SEQ ID NO : 12, ou un acide nucléotidique ayant au moins 70% d'identité de séquence avec la séquence SEQ ID NO : 12.

La présente description concerne selon unmode de réalisation plus particulier une unité de transcription constituée d'un polynucléotide comprenant un acide nucléotidique représenté par la séquence SEQ ID NO : 74 et constitué de :

(i) l'enhancer du virus hCMVie représenté par la séquence nucléotidique SEQ ID NO : 1,

(ii) la région promotrice de la β-actine représentée par la séquence nucléotidique SEQ ID NO : 3,

(iii) la région 5' UTR représentée par la séquence nucléotidique SEQ ID NO : 10, et

(iv) l'intron murin ROSA ayant la séquence nucléotidique SEQ ID NO : 12,

ou d'un acide nucléotidique ayant au moins 70% d'identité de séquence avec la séquence SEQ ID NO : 74.

L'unité de transcription obtenue est désignée par CMV-βactine-U1U2U3-mROSA, autrement appelée E2-bActine-U1U2U3-mROSA.

La présente description concerne selon un mode de réalisation particulier une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,

(ii) la région promotrice de la β-actine représentée par la séquence nucléotidique SEQ ID NO : 3, ou un acide nucléotidique présentant au moins 70% d'identité de séquence avec la séquence SEQ ID NO : 3 et possédant essentiellement une activité promotrice,

(iii) la région 5' UTR représentée par la séquence SEQ ID NO : 10, ou un acide nucléotidique présentant au moins 70% d'identité de séquence avec la séquence SEQ ID NO : 10, et

(iv) l'intron humain ROSA ayant la séquence nucléotidique SEQ ID NO : 13, ou un acide nucléotidique ayant au moins 70% d'identité de séquence avec la séquence SEQ ID NO : 13.

La présente description concerne selon unmode de réalisation plus particulier, une unité de transcription constituée d'un polynucléotide comprenant un acide nucléotidique représenté par la séquence SEQ ID NO : 75 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3,
(iii) la région 5' UTR représentée par la séquence nucléotidique SEQ ID NO : 10, et
(iv) l'intron humain ROSA ayant la séquence nucléotidique SEQ ID NO : 13, ou d'un acide nucléotidique ayant au moins 70% d'identité de séquence avec la séquence SEQ ID NO : 75.

L'unité de transcription obtenue est désignée par CMV-βactine-U1U2U3-hROSA, autrement appelée E2-bActine-U1U2U3-hROSA.

La présente invention concerne, dans un mode de réalisation avantageux, une unité de transcription, ladite unité de transcription comprenant l'enhancer du virus hCMVie, la région promotrice de la β-actine, la région 5'UTR du virus HTLV-1 (U1) et la région 5' UTR est celle du gène eIF4GI (U3), ladite unité de transcription ayant la séquence nucléotidique SEQ ID NO : 25, ou une séquence nucléotidique présentant au moins 70% d'identité avec la SEQ ID NO : 25 et permettant une production volumique d'une protéine d'intérêt supérieure à celle obtenue avec la combinaison de l'enhancer CMV associé à la région promotrice de la β-actine.

On entend par une « production volumique », une quantité de protéine exprimée en masse par unité de volume (g/L) aussi appelée titre en protéine ou concentration de la protéine d'intérêt.

La présente invention concerne également un vecteur d'expression comprenant au moins une unité de transcription telle que définie ci-dessus et au moins un site de clonage permettant l'intégration d'un acide nucléotidique codant pour une protéine d'intérêt.

Ledit acide nucléotidique peut être d'ADN génomique ou d'ADNc.

Par « **site de clonage** », on entend un segment court d'ADN qui comporte plusieurs sites de restriction, reconnus respectivement par différentes enzymes de restriction.

La présente invention concerne également un vecteur d'expression comprenant au moins une unité de transcription telle que définie ci-dessus et au moins un site pour la recombinaison site spécifique permettant l'intégration d'un acide nucléotidique codant pour une protéine d'intérêt.

Ledit acide nucléotidique peut être d'ADN génomique ou d'ADNc.

Par « **site pour la recombinaison site spécifique** », on entend un segment court d'ADN qui est reconnu par une recombinase, tel que le site *loxP* qui est reconnu par la recombinase Cre, le site *xis* qui est reconnu par l'intégrase Int, le site FRT qui est reconnu par la recombinase FLP.

Un vecteur d'expression selon la présente invention peut comprendre en outre un gène de résistance eucaryote, un gène de résistance bactérien, une origine de réplication bactérienne et une unité dédiée à l'amplification génique.

Un gène de résistance eucaryote peut être un gène de résistance à la Généticine (G418), Blasticidine, zéocine.

Un gène de résistance bactérien peut être un gène de résistance à l'ampicilline, Kanamycine, Puromycine, Blasticidine, Zéocine.

Une origine de réplication (Ori) bactérienne est une séquence d'ADN particulier d'origine bactérienne permettant l'initiation de la réplication du matériel génétique comme un vecteur d'expression et de conditionner chez la bactérie le nombre de copies de vecteur par bactérie. Une telle origine de réplication peut être choisie parmi Ori-P, Ori-C, Ori-fl, ColE1, pSC101 Ori, p15A Ori, pACYC Ori, SV40 Ori, Pmb1 Ori, Puc Ori.

Par « **une unité dédiée à l'amplification génique** », on entend toute unité permettent de réaliser une amplification génique et/ou un enrichissement en forts expresseurs. Le plus souvent, cette unité permet l'expression d'un gène de résistance à un unhibiteur agissant de manière dose-dépendante ; par augmentation de la dose d'inhibiteur, il y a sélection de variants exprimant plus fortement le gène de résistance, en particulier suite à une amplification génique ou intégration dans un site de forte expression. Le plus souvent, les gènes proches de cette unité sont également amplifiés géniquement etou une expression augmentée. Une telle unité peut être le gène dhfr (dihydrofolate réductase) dont l'inhibiteur est le méthotrexate ou le gène glutamine synthétase dont l'inhibiteur est le méthionyl sulfoximine, un système d'amplification de fragments géniques qui repose sur la sélection de transformants résistants au méthotrexate (MTX). Il nécessite l'introduction préalable d'une unité de transcription comprenant l'acide nucléique codant pour 'enzyme DHFR (dihydrofolate réductase) dans le vecteur d'expression pour la production de la molécule recombinante d'intérêt (SHITARI et al, 1994).

Une protéine d'intérêt susceptible d'être produit par un vecteur selon l'invention est une protéine choisie parmi le groupe constitué des protéines participant de la coagulation ou une immunoglobuline, des cytokines, des hormones, des facteurs de croissance ou facteurs du complément et de toute protéine de fusion.

Dans un mode de réalisation plus particulier de l'invention, le vecteur d'expression selon la description comporte une unité de transcription comprenant un acide nucléotidique représenté par la séquence SEQ ID NO : 21 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3, et
(iii) la région R du LTR du virus HTLV-1 représentée par la séquence nucléotidique SEQ ID NO : 4.

La présente description concerne selon un mode de réalisation plus particulier un vecteur d'expression comportant une unité de transcription comprenant un acide nucléotidique représenté par la séquence SEQ ID NO : 22 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3, et
(iii) la région 5' UTR du gène NF-κB Repressing Factor (NRF) ayant la séquence nucléotidique SEQ ID NO : 5.

La présente description concerne selon un mode de réalisation plus particulier, un vecteur d'expression comportant une unité de transcription comprenant un acide nucléotidique représenté par la séquence SEQ ID NO : 23 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3, et
(iii) la région 5' UTR du gène eukaryotic Initiation Factor 4GI (eIF4GI) ayant la séquence nucléotidique SEQ ID NO : 6.

Dans un mode de réalisation plus particulier de l'invention, le vecteur d'expression selon l'invention comporte une unité de transcription comprenant un acide nucléotidique représenté par la séquence SEQ ID NO : 25 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3, et
(iii) la région 5'UTR représentée par la séquence nucléotidique SEQ ID NO : 8.

La présente description concerne selon unmode de réalisation plus particulierun vecteur d'expression comportant une unité de transcription comprenant un acide nucléotidique représenté par la séquence SEQ ID NO : 26 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3, et
(iii) la région 5'UTR représentée par la séquence nucléotidique SEQ ID NO : 9.

La présente description concerne selon unmode de réalisation plus particulier un vecteur d'expression comportant une unité de transcription comprenant un acide nucléotidique représenté par la séquence SEQ ID NO : 27 et constitué de :

(i) l'enhancer du virus hCMVie représenté par la séquence nucléotidique SEQ ID NO : 1,
(ii) la région promotrice de la β-actine représentée par la séquence nucléotidique SEQ ID NO : 3, et
(iii) la région 5'UTR représentée par la séquence nucléotidique SEQ ID NO : 10.

La présente description concerne également un vecteur d'expression comportant une unité de transcription comprenant un acide nucléotidique représenté par la séquence SEQ ID NO : 14 et constitué de :

(i) l'enhancer du virus hCMVie représenté par la séquence nucléotidique SEQ ID NO : 1,
(ii) la région promotrice du gène CDK9 représentée par la séquence nucléotidique SEQ ID NO : 2, et
(iii) la région R du LTR du virus HTLV-1 représentée par la séquence nucléotidique SEQ ID NO : 4.

La présente description concerne également un vecteur d'expression comportant une unité de transcription comprenant un acide nucléotidique représenté par la séquence SEQ ID NO : 15 et constitué de :

(i) l'enhancer du virus hCMVie représenté par la séquence nucléotidique SEQ ID NO : 1,
(ii) la région promotrice du gène CDK9 représentée par la séquence nucléotidique SEQ ID NO : 2, et
(iii) la région 5' UTR du gène NF-κB Repressing Factor (NRF) ayant la séquence nucléotidique SEQ ID NO : 5.

La présente description concerne également un vecteur d'expression comportant une unité de transcription comprenant un acide nucléotidique représenté par la séquence SEQ ID NO : 16 et constitué de :

(i) l'enhancer du virus hCMVie représenté par la séquence nucléotidique SEQ ID NO : 1,
(ii) la région promotrice du gène CDK9 représentée par la séquence nucléotidique SEQ ID NO : 2, et
(iii) la région 5' UTR du gène eukaryotic Initiation Factor 4GI (eIF4GI) ayant la séquence nucléotidique SEQ ID NO : 6.

La présente description concerne également un vecteur d'expression comportant une unité de transcription comprenant un acide nucléotidique représenté par la séquence SEQ ID NO : 18 et constitué de :

(i) l'enhancer du virus hCMVie représenté par la séquence nucléotidique SEQ ID NO : 1,
(ii) la région promotrice du gène CDK9 représentée par la séquence nucléotidique SEQ ID NO : 2, et
(iii) la région 5'UTR représentée par la séquence nucléotidique SEQ ID NO : 8.

La présente description concerne également un vecteur d'expression comportant une unité de transcription comprenant un acide nucléotidique représenté par la séquence SEQ ID NO : 19 et constitué de :

(i) l'enhancer du virus hCMVie représenté par la séquence nucléotidique SEQ ID NO : 1,
(ii) la région promotrice du gène CDK9 représentée par la séquence nucléotidique SEQ ID NO : 2, et
(iii) la région 5'UTR représentée par la séquence nucléotidique SEQ ID NO : 9.

La présente description concerne également un vecteur d'expression comportant une unité de transcription comprenant un acide nucléotidique représenté par la séquence SEQ ID NO : 20 et constitué de :

(i) l'enhancer du virus hCMVie représenté par la séquence nucléotidique SEQ ID NO : 1,
(ii) la région promotrice du gène CDK9 représentée par la séquence nucléotidique SEQ ID NO : 2, et
(iii) la région 5'UTR représentée par la séquence nucléotidique SEQ ID NO : 10.

La présente description concerne également un vecteur d'expression comportant une unité de transcription comprenant un acide nucléotidique représenté par la séquence SEQ ID NO : 40 et constitué de :

(i) l'enhancer du virus hCMVie représenté par la séquence nucléotidique SEQ ID NO : 1,
(ii) la région promotrice du gène CDK9 représentée par la séquence nucléotidique SEQ ID NO : 2,
(iii) la région 5' UTR du gène eukaryotic Initiation Factor 4GI (eIF4GI) ayant la séquence nucléotidique SEQ ID NO : 6, et
(iv) l'intron du gène EF1$\alpha$ représenté par la séquence nucléotidique SEQ ID NO : 11.

La présente description concerne également un vecteur d'expression comportant une unité de transcription comprenant un acide nucléotidique représenté par la séquence SEQ ID NO : 41 et constitué de :

(i) l'enhancer du virus hCMVie représenté par la séquence nucléotidique SEQ ID NO : 1,
(ii) la région promotrice du gène CDK9 représentée par la séquence nucléotidique SEQ ID NO : 2,
(iii) la région 5' UTR du gène eukaryotic Initiation Factor 4GI (eIF4GI) ayant la séquence nucléotidique SEQ ID NO : 6, et
(iv) l'intron murin ROSA ayant la séquence nucléotidique SEQ ID NO : 12.

La présente description concerne également un vecteur d'expression comportant une unité de transcription comprenant un acide nucléotidique représenté par la séquence SEQ ID NO : 42 et constitué de :

(i) l'enhancer du virus hCMVie représenté par la séquence nucléotidique SEQ ID NO : 1,
(ii) la région promotrice du gène CDK9 représentée par la séquence nucléotidique SEQ ID NO : 2,
(iii) la région 5' UTR du gène eukaryotic Initiation Factor 4GI (eIF4GI) ayant la séquence nucléotidique SEQ ID NO : 6, et
(iv) l'intron humain ROSA ayant la séquence nucléotidique SEQ ID NO : 13.

La présente description concerne également un vecteur d'expression dérivé de pcDNA3.1 (invitrogen) comportant une unité de transcription comprenant un acide nucléotidique représenté par la séquence SEQ ID NO : 27 et constitué de :

(i) l'enhancer du virus hCMVie représenté par la séquence SEQ ID NO : 1,
(ii) la région promotrice de la β-actine représentée par la séquence SEQ ID NO : 3,
(iii) la région 5'UTR représentée par la séquence SEQ ID NO : 10.

L'invention concerne dans un mode de réalisation particulier, un vecteur d'expression dérivé de pcDNA3.1 (invitrogen) comportant une unité de transcription comprenant un acide nucléotidique représenté par la séquence SEQ ID NO : 25 et constitué de :

(i) l'enhancer du virus hCMVie représenté par la séquence SEQ ID NO : 1,
(ii) la région promotrice de la β-actine représentée par la séquence SEQ ID NO : 3,
(iii) la région 5'UTR représentée par la séquence SEQ ID NO : 8.

La présente description divulgue également un vecteur dérivé de pREP4 comportant une unité de transcription comprenant un acide nucléotidique représenté par la séquence SEQ ID NO : 21 et constitué de :

(i) l'enhancer du virus hCMVie représenté par la séquence SEQ ID NO : 1,
(ii) la région promotrice de la β-actine représentée par la séquence SEQ ID NO : 3,
(iii) la région R du LTR du virus HTLV-1 représentée par la séquence nucléotidique SEQ ID NO : 4.

La présente description divulgue également un vecteur dérivé de pREP4 comportant une unité de transcription comprenant un acide nucléotidique représenté par la séquence SEQ ID NO : 23 et constitué de :

(i) l'enhancer du virus hCMVie représenté par la séquence SEQ ID NO : 1,
(ii) la région promotrice de la β-actine représentée par la séquence SEQ ID NO : 3,
(iii) la région 5' UTR du gène eukaryotic Initiation Factor 4GI (eIF4GI) ayant la séquence nucléotidique SEQ ID NO : 6.

Dans un mode de réalisation plus particulier de l'invention, le vecteur d'expression selon l'invention est un vecteur dérivé de pREP4 comportant une unité de transcription comprenant un acide nucléotidique représenté par la séquence SEQ ID NO : 25 et constitué de :

(i) l'enhancer du virus hCMVie représenté par la séquence SEQ ID NO : 1,
(ii) la région promotrice de la β-actine représentée par la séquence SEQ ID NO : 3,
(iii) la région 5'UTR représentée par la séquence nucléotidique SEQ ID NO : 8.

La présente description divulgue également est un vecteur dérivé de pREP4 comportant une unité de transcription comprenant un acide nucléotidique représenté par la séquence SEQ ID NO : 16 et constitué de :

(i) l'enhancer du virus hCMVie représenté par la séquence SEQ ID NO : 1,
(ii) la région promotrice du gène CDK9 représentée par la séquence SEQ ID NO : 2,
(iii) la région 5' UTR du gène eukaryotic Initiation Factor 4GI (eIF4GI) ayant la séquence nucléotidique SEQ ID NO : 6.

La présente description divulgue également un vecteur dérivé de pREP4 comportant une unité de transcription comprenant un acide nucléotidique représenté par la séquence SEQ ID NO : 18 et constitué de :

(i) l'enhancer du virus hCMVie représenté par la séquence SEQ ID NO : 1,
(ii) la région promotrice du gène CDK9 représentée par la séquence SEQ ID NO : 2,
(iii) la région 5' UTR représentée par la séquence nucléotidique SEQ ID NO : 8.

Dans un mode de réalisation plus particulier de l'invention, le vecteur d'expression selon l'invention est un vecteur dérivé de pCEP4 comportant une unité de transcription comprenant un acide nucléotidique représenté par la séquence SEQ ID NO : 25 et constitué de :

(i) l'enhancer du virus hCMVie représenté par la séquence SEQ ID NO : 1,
(ii) la région promotrice de la β-actine représentée par la séquence SEQ ID NO : 3,
(iii) la région 5' UTR représentée par la séquence nucléotidique SEQ ID NO : 8.

Dans un mode de réalisation plus particulier de l'invention, le vecteur d'expression selon l'invention est un vecteur dérivé de pCEP4 comportant une unité de transcription comprenant un acide nucléotidique représenté par la séquence SEQ ID NO : 25 et constitué de : (E2-bActine-U1U3)

(i) l'enhancer du virus hCMVie représenté par la séquence SEQ ID NO : 1,
(ii) la région promotrice de la β-actine représentée par la séquence SEQ ID NO : 3,
(iii) la région 5' UTR représentée par la séquence nucléotidique SEQ ID NO : 8.

La présente invention a également pour objectif de fournir les cellules hôtes comprenant un vecteur d'expression tel que décrit dans la présente invention.

Lesdites cellules hôtes peuvent être une lignée cellulaire choisie parmi CHO-S, CHO, ou HEK.

La présente description concerne également l'utilisation d'une unité de transcription constituée d'un polynucléotide comprenant les éléments régulateurs suivant :

- l'enhancer du virus hCMVie (E2), ledit enhancer ayant la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
- la région promotrice de :
  la cycline dépendante des kinases 9 (CDK9), ladite région promotrice ayant la séquence nucléotidique SEQ ID NO : 2, ou un acide nucléotidique ayant au moins 70% d'identité de séquence avec la SEQ ID NO : 2 et possédant essentiellement une activité promotrice

pour la préparation de vecteurs d'expressions utilisés lors de la transfection d'une cellule hôte de la lignée cellulaire CHO.

La présente description concerne également l'utilisation d'une unité de transcription telle que décrite ci-dessus pour la préparation de vecteurs d'expressions utilisés lors de la transfection d'une cellule hôte de la lignée cellulaire CHO, dans laquelle ledit polynucléotide comprend également un acide nucléotidique situé en aval de ladite région promotrice et en amont du site d'initiation de la traduction, ledit acide nucléotidique comprenant au moins l'une des régions 5' non traduite (5' UTR) choisie parmi les suivantes :

- la région régulatrice R du Long Terminal Repeat (LTR) du virus HTLV-1 ayant la séquence nucléotidique SEQ ID NO : 4, ou un acide nucléotidique ayant au moins 70% d'identité de séquence avec la SEQ ID NO : 4,
- la région 5' UTR du gène NF-κB Repressing Factor (NRF) ayant la séquence nucléotidique SEQ ID NO : 5, ou un acide nucléotidique ayant au moins 70% d'identité de séquence avec la SEQ ID NO : 5,
- la région 5' UTR du gène eukaryotic Initiation Factor 4GI (eIF4GI) ayant la séquence nucléotidique SEQ ID NO : 6, ou un acide nucléotidique présentant au moins 70% d'identité de séquence avec la SEQ ID NO : 6,

les susdits acides nucléotidiques ayant au moins 70% d'identité de séquence avec l'une des séquences représentées par SEQ ID NO : 4, SEQ ID NO : 5 ou SEQ ID NO : 6 ayant essentiellement des propriétés de stabilisation des ARNm et de facilitateur de la traduction.

La présente description concerne également selon un mode de réalisation particulier, l'utilisation d'une unité de transcription telle que décrite ci-dessus, ladite unité de transcription étant constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice, et
(iii) la région R du Long Terminal Repeat (LTR) du virus HTLV-1 représentée par la séquence nucléotidique SEQ ID NO : 4, ou un acide nucléotidique ayant au moins 70% d'identité de séquence avec la séquence SEQ ID NO : 4,

ladite région 5' UTR étant située en aval de la région promotrice et en amont du site d'initiation de la traduction.

La présente description concerne également selon un mode de réalisation plus particulier, l'utilisation d'une unité de transcription telle que décrite ci-dessus, ladite unité de transcription étant constituée d'un polynucléotide comprenant un acide nucléotidique représenté par la séquence SEQ ID NO : 14 et constitué de :

(i) l'enhancer du virus hCMVie représenté par la séquence SEQ ID NO : 1,
(ii) la région promotrice du gène CDK9 représentée par la séquence SEQ ID NO : 2, et
(iii) la région 5'UTR du LTR du virus HTLV-1, représentée par la séquence SEQ ID NO : 4,

ou d'un acide nucléotidique ayant au moins 70% d'identité de séquence avec la séquence SEQ ID NO : 14.
L'unité de transcription obtenue est désignée par CMV-CDK9-U1, autrement appelée E2-CDK9-U1.

La présente description concerne également selon un autre mode de réalisation l'utilisation d'une unité de transcription telle que décrite ci-dessus, ladite unité de transcription étant constituée d'un polynucléotide comprenant les éléments régulateurs suivants :

(i) l'enhancer du virus hCMVie représentée par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice, et
(iii) la région 5' UTR du gène NF-κB Repressing Factor (NRF) représentée par la séquence nucléotidique SEQ ID NO : 5, ou un acide nucléotidique ayant au moins 70% d'identité de séquence avec la séquence SEQ ID NO : 5,

ladite région 5' UTR étant située en aval de la région promotrice et en amont du site d'initiation de la traduction.

La présente description concerne également selon un mode de réalisation plus particulier, l'utilisation d'une unité de transcription telle que décrite ci-dessus, ladite unité de transcription étant constituée d'un polynucléotide comprenant un acide nucléotidique représenté par la séquence SEQ ID NO : 15 et constitué de :

(i) l'enhancer du virus hCMVie représenté par la séquence SEQ ID NO : 1,
(ii) la région promotrice du gène CDK9 représentée par la séquence SEQ ID NO : 2, et
(iii) la région 5' UTR du gène NRF, représentée par la séquence SEQ ID NO : 5, ou un acide nucléotidique ayant au moins 70% d'identité de séquence avec la séquence SEQ ID NO : 15.

L'unité de transcription obtenue est désignée par CMV-CDK9-U2, autrement appelée E2-CDK9-U2.

Dans un autre mode de réalisation particulier, La présente description concerne également l'utilisation d'une unité de transcription telle que décrite ci-dessus, ladite unité de transcription étant d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice, et
(iii) la région 5' UTR du gène eukaryotic Initiation Factor 4GI (eIF4GI) représentée par la séquence nucléotidique SEQ ID NO : 6, ou un acide nucléotidique ayant au moins 70% d'identité de séquence avec la séquence SEQ ID NO : 6,

ladite région 5' UTR étant située en aval de la région promotrice et en amont du site d'initiation de la traduction.

La présente description concerne également selon un mode de réalisation plus particulier, l'utilisation d'une unité de transcription telle que décrite ci-dessus, ladite unité de transcription étant constituée d'un polynucléotide comprenant un acide nucléotidique représenté par la séquence SEQ ID NO : 16 et constitué de :

(i) l'enhancer du virus hCMVie représenté par la séquence SEQ ID NO : 1,
(ii) la région promotrice du gène CDK9 représentée par la séquence SEQ ID NO : 2, et
(iii) la région 5' UTR du gène eIF4GI représentée par la séquence SEQ ID NO : 6,

ou d'un acide nucléotidique ayant au moins 70% d'identité de séquence avec la séquence SEQ ID NO : 16.

L'unité de transcription obtenue est désignée par CMV-CDK9-U3, autrement appelée E2-CDK9-U3.

La présente description concerne également selon un autre mode de réalisation l'utilisation d'une unité de transcription comprenant deux régions 5'UTR. Une telle unité de transcription est constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,

(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice,
(iii) la région R du Long Terminal Repeat (LTR) du virus HTLV-1 représentée par la séquence nucléotidique SEQ ID NO : 4, ou un acide nucléotidique ayant au moins 70% d'identité de séquence avec la séquence SEQ ID NO : 4, et
(iv) la région 5' UTR du gène NF-κB Repressing Factor (NRF) représentée par la séquence nucléotidique SEQ ID NO : 5, ou un acide nucléotidique ayant au moins 70% d'identité de séquence avec la séquence SEQ ID NO : 5,

les régions 5' UTR étant situées en aval de la région promotrice et en amont du site d'initiation de la traduction.

La présente description concerne également selon un mode de réalisation plus particulier, l'utilisation d'une unité de transcription telle que décrite ci-dessus, ladite unité de transcription étant constituée d'un polynucléotide comprenant un acide nucléotidique représenté par la séquence SEQ ID NO : 17 et constitué de :

(i) l'enhancer du virus hCMVie représenté par la séquence SEQ ID NO : 1,
(ii) la région promotrice du gène CDK9 représentée par la séquence SEQ ID NO : 2, et
(iii) la région 5' UTR représentée par la séquence SEQ ID NO : 7,

ou d'un acide nucléotidique ayant au moins 70% d'identité de séquence avec la séquence SEQ ID NO : 17.

L'unité de transcription obtenue est désignée par CMV-CDK9-U1U2, autrement appelée E2-CDK9-U1U2.

La présente description concerne également selon un autre mode de réalisation particulier, l'utilisation d'une unité de transcription comprenant deux régions 5'UTR. Une telle unité de transcription est constituée d'un polynucléotide comprenant les éléments régulateurs suivants :

(i) l'enhancer du virus hCMVie représentée par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice,
(iii) la région R du Long Terminal Repeat (LTR) du virus HTLV-1 ayant la séquence nucléotidique SEQ ID NO : 4, ou un acide nucléotidique ayant au moins 70% d'identité de séquence avec la séquence SEQ ID NO : 4, et
(iv) la région 5' UTR du gène eukaryotic Initiation Factor 4GI (eIF4GI) ayant la séquence nucléotidique SEQ ID NO : 6, ou un acide nucléotidique ayant au moins 70% d'identité de séquence avec la séquence SEQ ID NO : 6,

les régions 5' UTR étant situées en aval de la région promotrice et en amont du site d'initiation de la traduction.

La présente description concerne également selon un mode de réalisation plus particulier, l'utilisation d'une unité de transcription telle que décrite ci-dessus, ladite unité de transcription étant constituée d'un polynucléotide comprenant un acide nucléotidique représenté par la séquence SEQ ID NO : 18 et constitué de :

(i) l'enhancer du virus hCMVie représenté par la séquence SEQ ID NO : 1,
(ii) la région promotrice du gène CDK9 représentée par la séquence SEQ ID NO : 2, et
(iii) la région 5' UTR représentée par la séquence SEQ ID NO : 8,

ou d'un acide nucléotidique ayant au moins 70% d'identité de séquence avec la séquence SEQ ID NO : 18.

L'unité de transcription obtenue est désignée par CMV-CDK9-U1U3, autrement appelée E2-CDK9-U1U3.

La présente description concerne également selon un autre mode de réalisation l'utilisation d'une unité de transcription comprenant deux régions 5'UTR. Une telle unité de transcription est constituée d'un polynucléotide comprenant les éléments régulateurs suivants :

(i) l'enhancer du virus hCMVie représentée par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice,
(iii) la région 5' UTR du gène NF-κB Repressing Factor (NRF) ayant la séquence nucléotidique SEQ ID NO : 5, ou un acide nucléotidique ayant au moins 70% d'identité de séquence avec la séquence SEQ ID NO : 5, et
(iv) la région 5' UTR du gène eukaryotic Initiation Factor 4GI (eIF4GI) ayant la séquence nucléotidique SEQ ID NO :

6, ou un acide nucléotidique ayant au moins 70% d'identité de séquence avec la séquence SEQ ID NO : 6,

les régions 5' UTR étant situées en aval de la région promotrice et en amont du site d'initiation de la traduction.

La présente description concerne également selon un mode de réalisation plus particulier, l'utilisation d'une unité de transcription telle que décrite ci-dessus, ladite unité de transcription étant constituée d'un polynucléotide comprenant un acide nucléotidique représenté par la séquence SEQ ID NO : 19 et constitué de :

(i) l'enhancer du virus hCMVie représenté par la séquence SEQ ID NO : 1,
(ii) la région promotrice du gène CDK9 représentée par la séquence SEQ ID NO : 2, et
(iii) la région 5' UTR représentée par la séquence SEQ ID NO : 9,

ou d'un acide nucléotidique ayant au moins 70% d'identité de séquence avec la séquence SEQ ID NO : 19.

L'unité de transcription obtenue est désignée par CMV-CDK9-U2U3, autrement appelée E2-CDK9-U2U3.

La présente description concerne également selon un autre mode de réalisation particulier, l'utilisation d'une unité de transcription comprenant trois régions 5'UTR. Une telle unité de transcription est constituée d'un polynucléotide comprenant les éléments régulateurs suivants :

(i) l'enhancer du virus hCMVie représentée par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région R du Long Terminal Repeat (LTR) du virus HTLV-1 ayant la séquence nucléotidique SEQ ID NO : 4, ou un acide nucléotidique ayant au moins 70% d'identité de séquence avec la séquence SEQ ID NO : 4,
(iv) la région 5' UTR du gène NF-κB Repressing Factor (NRF) ayant la séquence nucléotidique SEQ ID NO : 5, ou un acide nucléotidique ayant au moins 70% d'identité de séquence avec la séquence SEQ ID NO : 5, et
(v) la région 5' UTR du gène eukaryotic Initiation Factor 4GI (eIF4GI) ayant la séquence nucléotidique SEQ ID NO : 6, ou un acide nucléotidique ayant au moins 70% d'identité de séquence avec la séquence SEQ ID NO : 6,

les régions 5' UTR étant situées en aval de la région promotrice et en amont du site d'initiation de la traduction.

La présente description concerne également selon un mode de réalisation plus particulier, l'utilisation d'une unité de transcription telle que décrite ci-dessus, ladite unité de transcription étant constituée d'un polynucléotide comprenant un acide nucléotidique représenté par la séquence SEQ ID NO : 20 et constitué de :

(i) l'enhancer du virus hCMVie représenté par la séquence SEQ ID NO : 1,
(ii) la région promotrice du gène CDK9 représentée par la séquence SEQ ID NO : 2, et
(iii) la région 5' UTR représentée par la séquence SEQ ID NO : 10,

ou d'un acide nucléotidique ayant au moins 70% d'identité de séquence avec la séquence SEQ ID NO : 20.

L'unité de transcription obtenue est désignée par CMV-CDK9-U1U2U3, autrement appelée E2-CDK9-U1U2U3.

La présente description concerne également l'utilisation d'une unité de transcription pour la préparation de vecteurs d'expressions utilisés lors de la transfection d'une cellule hôte de la lignée cellulaire CHO, ladite unité de transcription comprenant :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) une région promotrice de la cycline dépendante des kinases 9 (CDK9), ladite région promotrice ayant la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) un intron choisi parmi :

- l'intron du gène Elongation Factor 1α (EF1α ayant la séquence nucléotidique SEQ ID NO : 11, ou un acide nucléotidique présentant au moins 70% d'identité de séquence avec la séquence SEQ ID NO : 11,
- l'intron murin ROSA ayant la séquence nucléotidique SEQ ID NO : 12, ou un acide nucléotidique ayant au moins 70% d'identité de séquence avec la séquence SEQ ID NO : 12,
- l'intron humain ROSA ayant la séquence nucléotidique SEQ ID NO : 13, ou un acide nucléotidique ayant au moins 70% d'identité de séquence avec la séquence SEQ ID NO : 13.

ledit enhancer étant situé en 5' ou en 3' de l'unité de transcription, ou à l'intérieur de la séquence codante ou dans un intron ;
ledit intron étant situé :

(i) en aval de la région 5' UTR et en amont du site d'initiation de la traduction, ou
(ii) en aval du promoteur et en amont de la région 5'UTR, ou
(iii) après le site d'initiation de la traduction et à l'intérieur de la séquence codante, ou
(iv) entre le codon stop de la séquence codante et le signal de polyadénylation.

La présente description concerne également selon un mode de réalisation particulier, l'utilisation d'une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice, et
(iii) l'intron du gène Elongation Factor 1$\alpha$ (EF1$\alpha$) ayant la séquence nucléotidique SEQ ID NO : 11, ou un acide nucléotidique présentant au moins 70% d'identité de séquence avec la séquence SEQ ID NO : 11.

La présente description concerne également selon un mode de réalisation plus particulier, l'utilisation d'une unité de transcription constituée d'un polynucléotide comprenant un acide nucléotidique représenté par la séquence SEQ ID NO : 28 et constitué de :

(i) l'enhancer du virus hCMVie représenté par la séquence nucléotidique SEQ ID NO : 1,
(ii) la région promotrice du gène CDK9 représentée par la séquence nucléotidique SEQ ID NO : 2, et
(iii) l'intron du gène EF1$\alpha$ représenté par la séquence nucléotidique SEQ ID NO : 11,

ou d'un acide nucléotidique ayant au moins 70% d'identité de séquence avec la séquence SEQ ID NO : 28.
L'unité de transcription obtenue est désignée par CMV-CDK9-EF1$\alpha$, autrement appelée E2-CDK9-EF.
La présente description concerne également selon un mode de réalisation particulier l'utilisation d'une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice, et
(iii) l'intron murin ROSA ayant la séquence nucléotidique SEQ ID NO : 12, ou un acide nucléotidique ayant au moins 70% d'identité de séquence avec la séquence SEQ ID NO : 12.

La présente description concerne également selon un mode de réalisation plus particulier, l'utilisation d'une unité de transcription constituée d'un polynucléotide comprenant un acide nucléotidique représenté par la séquence SEQ ID NO : 29 et constitué de :

(i) l'enhancer du virus hCMVie représenté par la séquence nucléotidique SEQ ID NO : 1,
(ii) la région promotrice du gène CDK9 représentée par la séquence nucléotidique SEQ ID NO : 2, et
(iii) l'intron murin ROSA ayant la séquence nucléotidique SEQ ID NO : 12,

ou d'un acide nucléotidique ayant au moins 70% d'identité de séquence avec la séquence SEQ ID NO : 29.
L'unité de transcription obtenue est désignée par CMV-CDK9-mROSA, autrement appelée E2-CDK9-mROSA.
La présente description concerne également selon un mode de réalisation particulier l'utilisation d'une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice, et
(iii) l'intron humain ROSA ayant la séquence nucléotidique SEQ ID NO : 13, ou un acide nucléotidique ayant au moins 70% d'identité de séquence avec la séquence SEQ ID NO : 13.

Dans un mode de réalisation plus particulier, La présente description concerne l'utilisation d'une unité de transcription constituée d'un polynucléotide comprenant un acide nucléotidique représenté par la séquence SEQ ID NO : 30 et constitué de :

(i) l'enhancer du virus hCMVie représenté par la séquence nucléotidique SEQ ID NO : 1,
(ii) la région promotrice du gène CDK9 représentée par la séquence nucléotidique SEQ ID NO : 2, et
(iii) l'intron humain ROSA ayant la séquence nucléotidique SEQ ID NO : 12, ou d'un acide nucléotidique ayant au moins 70% d'identité de séquence avec la séquence SEQ ID NO : 30.

L'unité de transcription obtenue est désignée par CMV-CDK9-hROSA, autrement appelée E2-CDK9-hROSA.
La présente description concerne également l'utilisation d'une unité de transcription pour la transfection d'une cellule hôte de la lignée cellulaire CHO, ladite unité de transcription comprenant :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) une région promotrice de la cycline dépendante des kinases 9 (CDK9), ladite région promotrice ayant la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) au moins l'une des régions 5' non traduite (5' UTR) choisie parmi :

- la région R du Long Terminal Repeat (LTR) du virus HTLV-1 ayant la séquence nucléotidique SEQ ID NO : 4, ou un acide nucléotidique ayant au moins 70% d'identité de séquence avec la séquence SEQ ID NO : 4,
- la région 5' UTR du gène NF-κB Repressing Factor (NRF) ayant la séquence nucléotidique SEQ ID NO : 5, ou un acide nucléotidique ayant au moins 70% d'identité de séquence avec la séquence SEQ ID NO : 5,
- la région 5' UTR du gène eukaryotic Initiation Factor 4GI (eIF4GI) ayant la séquence nucléotidique SEQ ID NO : 6, ou un acide nucléotidique présentant au moins 70% d'identité de séquence avec la séquence SEQ ID NO : 6, et

(iv) un intron choisi parmi :

- l'intron du gène Elongation Factor 1$\alpha$ (EF1 $\alpha$□ ayant la séquence nucléotidique SEQ ID NO : 11, ou un acide nucléotidique présentant au moins 70% d'identité de séquence avec la séquence SEQ ID NO : 11,
- l'intron murin ROSA ayant la séquence nucléotidique SEQ ID NO : 12, ou un acide nucléotidique ayant au moins 70% d'identité de séquence avec la séquence SEQ ID NO : 12,
- l'intron humain ROSA ayant la séquence nucléotidique SEQ ID NO : 13, ou un acide nucléotidique ayant au moins 70% d'identité de séquence avec la séquence SEQ ID NO : 13.

ledit enhancer étant situé en 5' ou en 3' de l'unité de transcription, ou à l'intérieur de la séquence codante ou dans un intron ;
ladite région promotrice étant situé en amont de la région 5'UTR ;
ledit intron étant situé :

(i) en aval de la région 5' UTR et en amont du site d'initiation de la traduction, ou
(ii) en aval du promoteur et en amont de la région 5'UTR, ou
(iii) après le site d'initiation de la traduction et à l'intérieur de la séquence codante, ou
(iv) entre le codon stop de la séquence codante et le signal de polyadénylation.

La présente description concerne également selon un mode de réalisation particulier l'utilisation d'une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice,
(iii) la région R du Long Terminal Repeat (LTR) du virus HTLV-1 ayant la séquence nucléotidique SEQ ID NO : 4, ou un acide nucléotidique ayant au moins 70% d'identité de séquence avec la séquence SEQ ID NO : 4, et
(iv) l'intron du gène Elongation Factor 1$\alpha$ (EF1$\alpha$) ayant la séquence nucléotidique SEQ ID NO : 11, ou un acide nucléotidique présentant au moins 70% d'identité de séquence avec la séquence SEQ ID NO : 11.

La présente description concerne également selon un mode de réalisation plus particulier, l'utilisation d'une unité de transcription constituée d'un polynucléotide comprenant un acide nucléotidique représenté par la séquence SEQ ID NO : 34 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région R du LTR du virus HTLV-1 ayant la séquence nucléotidique SEQ ID NO : 4, et
(iv) l'intron du gène Elongation Factor 1$\alpha$ (EF1$\alpha$) ayant la séquence nucléotidique SEQ ID NO : 11,

ou d'un acide nucléotidique ayant au moins 70% d'identité de séquence avec la séquence SEQ ID NO : 34.
L'unité de transcription obtenue est désignée par CMV-CDK9-U1-EF1$\alpha$, autrement appelée E2-CDK9-U1-EF.
La présente description concerne également selon un mode de réalisation particulier l'utilisation d'une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice,
(iii) la région R du Long Terminal Repeat (LTR) du virus HTLV-1 ayant la séquence nucléotidique SEQ ID NO : 4, ou un acide nucléotidique ayant au moins 70% d'identité de séquence avec la séquence SEQ ID NO : 4, et
(iv) l'intron murin ROSA ayant la séquence nucléotidique SEQ ID NO : 12, ou un acide nucléotidique ayant au moins 70% d'identité de séquence avec la séquence SEQ ID NO : 12.

La présente description concerne également selon un mode de réalisation plus particulier, l'utilisation d'une unité de transcription constituée d'un polynucléotide comprenant un acide nucléotidique représenté par la séquence SEQ ID NO : 35 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région R du LTR du virus HTLV-1 ayant la séquence nucléotidique SEQ ID NO : 4, et
(iv) l'intron murin ROSA ayant la séquence nucléotidique SEQ ID NO : 12,

ou d'un acide nucléotidique ayant au moins 70% d'identité de séquence avec la séquence SEQ ID NO : 35.
L'unité de transcription obtenue est désignée par CMV-CDK9-U1-mROSA, autrement appelée E2-CDK9-U1-mROSA.
La présente description concerne également selon un mode de réalisation particulier l'utilisation d'une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID :

NO 2 et possédant essentiellement une activité promotrice,
(iii) la région R du Long Terminal Repeat (LTR) du virus HTLV-1 ayant la séquence nucléotidique SEQ ID NO : 4, ou un acide nucléotidique ayant au moins 70% d'identité de séquence avec la séquence SEQ ID NO : 4, et
(iv) l'intron humain ROSA ayant la séquence nucléotidique SEQ ID NO : 13, ou un acide nucléotidique ayant au moins 70% d'identité de séquence avec la séquence SEQ ID NO : 13.

La présente description concerne également selon un mode de réalisation plus particulier, l'utilisation d'une unité de transcription constituée d'un polynucléotide comprenant un acide nucléotidique représenté par la séquence SEQ ID NO : 36 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région R du LTR du virus HTLV-1 ayant la séquence nucléotidique SEQ ID NO : 4, et
(iv) l'intron humain ROSA ayant la séquence nucléotidique SEQ ID NO : 13,

ou d'un acide nucléotidique ayant au moins 70% d'identité de séquence avec la séquence SEQ ID NO : 36.
L'unité de transcription obtenue est désignée par CMV-CDK9-U1-hROSA, autrement appelée E2-CDK9-U1-hROSA.
La présente description concerne également selon un mode de réalisation particulier l'utilisation d'une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice,
(iii) la région 5' UTR du gène NF-κB Repressing Factor (NRF) ayant la séquence nucléotidique SEQ ID NO : 5, ou un acide nucléotidique ayant au moins 70% d'identité de séquence avec la séquence SEQ ID NO : 5, et
(iv) l'intron du gène Elongation Factor 1$\alpha$ (EF1$\alpha$) ayant la séquence nucléotidique SEQ ID NO : 11, ou un acide nucléotidique présentant au moins 70% d'identité de séquence avec la séquence SEQ ID NO : 11.

La présente description concerne également selon un mode de réalisation plus particulier, l'utilisation d'une unité de transcription constituée d'un polynucléotide comprenant un acide nucléotidique représenté par la séquence SEQ ID NO : 37 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR du gène NRF ayant la séquence nucléotidique SEQ ID NO : 5, et
(iv) l'intron du gène Elongation Factor 1$\alpha$ (EF1$\alpha$) ayant la séquence nucléotidique SEQ ID NO : 11,

ou d'un acide nucléotidique ayant au moins 70% d'identité de séquence avec la séquence SEQ ID NO : 37.
L'unité de transcription obtenue est désignée par CMV-CDK9-U2-EF1$\alpha$, autrement appelée E2-CDK9-U2-EF.
La présente description concerne également selon un mode de réalisation particulier l'utilisation d'une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice,
(iii) la région 5' UTR du gène NF-κB Repressing Factor (NRF) ayant la séquence nucléotidique SEQ ID NO : 5, ou un acide nucléotidique ayant au moins 70% d'identité de séquence avec la séquence SEQ ID NO : 5, et
(iv) l'intron murin ROSA ayant la séquence nucléotidique SEQ ID NO : 12, ou un acide nucléotidique ayant au moins 70% d'identité de séquence avec la séquence SEQ ID NO : 12.

La présente description concerne également selon un mode de réalisation plus particulier, l'utilisation d'une unité de transcription constituée d'un polynucléotide comprenant un acide nucléotidique représenté par la séquence SEQ ID NO : 38 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR du gène NRF ayant la séquence nucléotidique SEQ ID NO : 5, et
(iv) l'intron murin ROSA ayant la séquence nucléotidique SEQ ID NO : 12,

ou d'un acide nucléotidique ayant au moins 70% d'identité de séquence avec la séquence SEQ ID NO : 38.
L'unité de transcription obtenue est désignée par CMV-CDK9-U2-mROSA, autrement appelée E2-CDK9-U2-mROSA.
La présente description concerne également selon un mode de réalisation particulier l'utilisation d'une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice,
(iii) la région 5' UTR du gène NF-κB Repressing Factor (NRF) ayant la séquence nucléotidique SEQ ID NO : 5, ou un acide nucléotidique ayant au moins 70% d'identité de séquence avec la séquence SEQ ID NO : 5, et
(iv) l'intron humain ROSA ayant la séquence nucléotidique SEQ ID NO : 13, ou un acide nucléotidique ayant au moins 70% d'identité de séquence avec la séquence SEQ ID NO : 13.

La présente description concerne également selon un mode de réalisation plus particulier, l'utilisation d'une unité de transcription constituée d'un polynucléotide comprenant un acide nucléotidique représenté par la séquence SEQ ID NO : 39 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR du gène NRF ayant la séquence nucléotidique SEQ ID NO : 5, et
(iv) l'intron humain ROSA ayant la séquence nucléotidique SEQ ID NO : 13, ou d'un acide nucléotidique ayant au moins 70% d'identité de séquence avec la séquence SEQ ID NO : 39.

L'unité de transcription obtenue est désignée par CMV-CDK9-U2-hROSA, autrement appelée E2-CDK9-U2-hROSA.
La présente description concerne également selon un mode de réalisation particulier l'utilisation d'une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice,
(iii) la région 5' UTR du gène eukaryotic Initiation Factor 4GI (eIF4GI) ayant la séquence nucléotidique SEQ ID NO : 6, ou un acide nucléotidique présentant au moins 70% d'identité de séquence avec la séquence SEQ ID NO : 6, et
(iv) l'intron du gène Elongation Factor 1α (EF1α) ayant la séquence nucléotidique SEQ ID NO : 11, ou un acide nucléotidique présentant au moins 70% d'identité de séquence avec la séquence SEQ ID NO : 11.

La présente description concerne également selon un mode de réalisation plus particulier, l'utilisation d'une unité de transcription constituée d'un polynucléotide comprenant un acide nucléotidique représenté par la séquence SEQ ID NO : 40 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR du gène eIF4GI ayant la séquence nucléotidique SEQ ID NO : 6, et
(iv) l'intron du gène Elongation Factor 1$\alpha$ (EF1$\alpha$) ayant la séquence nucléotidique SEQ ID NO : 11,

ou d'un acide nucléotidique ayant au moins 70% d'identité de séquence avec la séquence SEQ ID NO : 40, et possédant essentiellement des propriétés d'activation de la transcription supérieures à celles de l'enhancer CMV associé à la région promotrice de CDK9.

L'unité de transcription obtenue est désignée par CMV-CDK9-U3-EF1$\alpha$, autrement appelée E2-CDK9-U3-EF.

La présente description concerne également selon un mode de réalisation particulier l'utilisation d'une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR du gène eukaryotic Initiation Factor 4GI (eIF4GI) ayant la séquence nucléotidique SEQ ID NO : 6, ou un acide nucléotidique présentant au moins 70% d'identité de séquence avec la séquence SEQ ID NO : 6, et
(iv) l'intron murin ROSA ayant la séquence nucléotidique SEQ ID NO : 12, ou un acide nucléotidique ayant au moins 70% d'identité de séquence avec la séquence SEQ ID NO : 12,

La présente description concerne également selon un mode de réalisation plus particulier, l'utilisation d'une unité de transcription constituée d'un polynucléotide comprenant un acide nucléotidique représenté par la séquence SEQ ID NO : 41 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR du gène eIF4GI ayant la séquence nucléotidique SEQ ID NO : 6, et
(iv) l'intron murin ROSA ayant la séquence nucléotidique SEQ ID NO : 12,

ou d'un acide nucléotidique ayant au moins 70% d'identité de séquence avec la séquence SEQ ID NO: 41.

L'unité de transcription obtenue est désignée par CMV-CDK9-U3-mROSA, autrement appelée E2-CDK9-U3-mROSA.

La présente description concerne également selon un mode de réalisation particulier l'utilisation d'une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice,
(iii) la région 5' UTR du gène eukaryotic Initiation Factor 4GI (eIF4GI) ayant la séquence nucléotidique SEQ ID NO : 6, ou un acide nucléotidique présentant au moins 70% d'identité de séquence avec la séquence SEQ ID NO : 6, et
(iv) l'intron humain ROSA ayant la séquence nucléotidique SEQ ID NO : 13, ou un acide nucléotidique ayant au moins 70% d'identité de séquence avec la séquence SEQ ID NO : 13,

La présente description concerne également selon un mode de réalisation plus particulier, l'utilisation d'une unité de transcription constituée d'un polynucléotide comprenant un acide nucléotidique représenté par la séquence SEQ ID NO : 42 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,

(iii) la région 5' UTR du gène eIF4GI ayant la séquence nucléotidique SEQ ID NO : 6, et
(iv) l'intron humain ROSA ayant la séquence nucléotidique SEQ ID NO : 13, ou d'un acide nucléotidique ayant au moins 70% d'identité de séquence avec la séquence SEQ ID NO : 42.

L'unité de transcription obtenue est désignée par CMV-CDK9-U3-hROSA, autrement appelée E2-CDK9-U3-hROSA.

La présente description concerne également selon un mode de réalisation particulier l'utilisation d'une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice,
(iii) la région 5' UTR représentée par la séquence SEQ ID NO : 7, ou un acide nucléotidique présentant au moins 70% d'identité de séquence avec la séquence SEQ ID NO : 7,
(iv) l'intron du gène Elongation Factor 1$\alpha$ (EF1$\alpha$) ayant la séquence nucléotidique SEQ ID NO : 11, ou un acide nucléotidique présentant au moins 70% d'identité de séquence avec la séquence SEQ ID NO : 11.

La présente description concerne également selon un mode de réalisation plus particulier, l'utilisation d'une unité de transcription constituée d'un polynucléotide comprenant un acide nucléotidique représenté par la séquence SEQ ID NO : 43 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR représentée par la séquence nucléotidique SEQ ID NO : 7, et
(iv) l'intron du gène Elongation Factor 1$\alpha$ (EF1$\alpha$) ayant la séquence nucléotidique SEQ ID NO : 11,

ou d'un acide nucléotidique ayant au moins 70% d'identité de séquence avec la séquence SEQ ID NO : 43.

L'unité de transcription obtenue est désignée par CMV-CDK9-UIU2-EF1$\alpha$, autrement appelée E2-CDK9-U1U2-EF.

La présente description concerne également selon un mode de réalisation particulier l'utilisation d'une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice,
(iii) la région 5' UTR représentée par la séquence SEQ ID NO : 7, ou un acide nucléotidique présentant au moins 70% d'identité de séquence avec la séquence SEQ ID NO : 7, et
(iv) l'intron murin ROSA ayant la séquence nucléotidique SEQ ID NO : 12, ou un acide nucléotidique ayant au moins 70% d'identité de séquence avec la séquence SEQ ID NO : 12.

La présente description concerne également selon un mode de réalisation plus particulier, l'utilisation d'une unité de transcription constituée d'un polynucléotide comprenant un acide nucléotidique représenté par la séquence SEQ ID NO : 44 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR représentée par la séquence nucléotidique SEQ ID NO : 7, et
(iv) l'intron murin ROSA ayant la séquence nucléotidique SEQ ID NO : 12,

ou d'un acide nucléotidique ayant au moins 70% d'identité de séquence avec la séquence SEQ ID NO : 44.

L'unité de transcription obtenue est désignée par CMV-CDK9-U1U2-mROSA, autrement appelée E2-CDK9-U1U2-mROSA.

La présente description concerne également selon un mode de réalisation particulier l'utilisation d'une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice,
(iii) la région 5' UTR représentée par la séquence SEQ ID NO : 7, ou un acide nucléotidique présentant au moins 70% d'identité de séquence avec la séquence SEQ ID NO : 7, et
(iv) l'intron humain ROSA ayant la séquence nucléotidique SEQ ID NO : 13, ou un acide nucléotidique ayant au moins 70% d'identité de séquence avec la séquence SEQ ID NO : 13.

La présente description concerne également selon un mode de réalisation plus particulier, l'utilisation d'une unité de transcription constituée d'un polynucléotide comprenant un acide nucléotidique représenté par la séquence SEQ ID NO : 45 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR représentée par la séquence nucléotidique SEQ ID NO : 7, et
(iv) l'intron humain ROSA ayant la séquence nucléotidique SEQ ID NO : 13, ou d'un acide nucléotidique ayant au moins 70% d'identité de séquence avec la séquence SEQ ID NO : 45.

L'unité de transcription obtenue est désignée par CMV-CDK9-U1U2-hROSA, autrement appelée E2-CDK9-U1U2-hROSA.

La présente description concerne également selon un mode de réalisation particulier l'utilisation d'une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice,
(iii) la région 5' UTR représentée par la séquence SEQ ID NO : 8, ou un acide nucléotidique présentant au moins 70% d'identité de séquence avec la séquence SEQ ID NO : 8, et
(iv) l'intron du gène Elongation Factor 1$\alpha$ (EF1$\alpha$)□ ayant la séquence nucléotidique SEQ ID NO : 11, ou un acide nucléotidique présentant au moins 70% d'identité de séquence avec la séquence SEQ ID NO : 11.

La présente description concerne également selon un mode de réalisation plus particulier, l'utilisation d'une unité de transcription constituée d'un polynucléotide comprenant un acide nucléotidique représenté par la séquence SEQ ID NO : 46 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR représentée par la séquence nucléotidique SEQ ID NO : 8, et
(iv) l'intron du gène Elongation Factor 1$\alpha$ (EF1$\alpha$)□ ayant la séquence nucléotidique SEQ ID NO : 11,

ou d'un acide nucléotidique ayant au moins 70% d'identité de séquence avec la séquence SEQ ID NO : 46.

L'unité de transcription obtenue est désignée par CMV-CDK9-U1U3-EF1$\alpha$, autrement appelée E2-CDK9-U1U3-EF.

La présente description concerne également selon un mode de réalisation particulier l'utilisation d'une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 8, ou un acide nucléotidique présentant au moins 70% d'identité de séquence avec la séquence SEQ ID NO : 8, et
(iv) l'intron murin ROSA ayant la séquence nucléotidique SEQ ID NO : 12, ou un acide nucléotidique ayant au moins 70% d'identité de séquence avec la séquence SEQ ID NO : 12.

La présente description concerne également selon un mode de réalisation plus particulier, l'utilisation d'une unité de transcription constituée d'un polynucléotide comprenant un acide nucléotidique représenté par la séquence SEQ ID NO : 47 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR représentée par la séquence nucléotidique SEQ ID NO : 8, et
(iv) l'intron murin ROSA ayant la séquence nucléotidique SEQ ID NO : 12,

ou d'un acide nucléotidique ayant au moins 70% d'identité de séquence avec la séquence SEQ ID NO : 47.
L'unité de transcription obtenue est désignée par CMV-CDK9-U1U3-mROSA, autrement appelée E2-CDK9-U1U3-mROSA.
La présente description concerne également selon un mode de réalisation particulier l'utilisation d'une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 9, ou un acide nucléotidique présentant au moins 70% d'identité de séquence avec la séquence SEQ ID NO : 9, et
(iv) l'intron humain ROSA ayant la séquence nucléotidique SEQ ID NO : 13, ou un acide nucléotidique ayant au moins 70% d'identité de séquence avec la séquence SEQ ID NO : 13.

La présente description concerne également selon un mode de réalisation plus particulier, l'utilisation d'une unité de transcription constituée d'un polynucléotide comprenant un acide nucléotidique représenté par la séquence SEQ ID NO : 48 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR représentée par la séquence nucléotidique SEQ ID NO : 8, et
(iv) l'intron humain ROSA ayant la séquence nucléotidique SEQ ID NO : 13, ou d'un acide nucléotidique ayant au moins 70% d'identité de séquence avec la séquence SEQ ID NO : 48.

L'unité de transcription obtenue est désignée par CMV-CDK9-U1U3-hROSA, autrement appelée E2-CDK9-U1U3-hROSA.
La présente description concerne également selon un mode de réalisation particulier l'utilisation d'une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 9, ou un acide nucléotidique présentant au moins 70% d'identité de séquence avec la séquence SEQ ID NO : 9, et
(iv) l'intron du gène Elongation Factor 1α (EF1α) ayant la séquence nucléotidique SEQ ID NO : 11, ou un acide nucléotidique présentant au moins 70% d'identité de séquence avec la séquence SEQ ID NO : 11.

La présente description concerne également selon un mode de réalisation plus particulier, l'utilisation d'une unité de transcription constituée d'un polynucléotide comprenant un acide nucléotidique représenté par la séquence SEQ ID NO : 49 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR représentée par la séquence nucléotidique SEQ ID NO : 9, et
(iv) l'intron du gène Elongation Factor 1α (EF1α) ayant la séquence nucléotidique SEQ ID NO : 11,

ou d'un acide nucléotidique ayant au moins 70% d'identité de séquence avec la séquence SEQ ID NO : 49.
L'unité de transcription obtenue est désignée par CMV-CDK9-U2U3-EF1α, autrement appelée E2-CDK9-U2U3-EF.
La présente description concerne également selon un mode de réalisation particulier l'utilisation d'une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 9, ou un acide nucléotidique présentant au moins 70% d'identité de séquence avec la séquence SEQ ID NO : 9,
(iv) l'intron murin ROSA ayant la séquence nucléotidique SEQ ID NO : 12, ou un acide nucléotidique ayant au moins 70% d'identité de séquence avec la séquence SEQ ID NO : 12.

La présente description concerne également selon un mode de réalisation plus particulier, l'utilisation d'une unité de transcription constituée d'un polynucléotide comprenant un acide nucléotidique représenté par la séquence SEQ ID NO : 50 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR représentée par la séquence nucléotidique SEQ ID NO : 9, et
(iv) l'intron murin ROSA ayant la séquence nucléotidique SEQ ID NO : 12,

ou d'un acide nucléotidique ayant au moins 70% d'identité de séquence avec la séquence SEQ ID NO : 50.
L'unité de transcription obtenue est désignée par CMV-CDK9-U2U3-mROSA, autrement appelée E2-CDK9-U2U3-mROSA.
La présente description concerne également selon un mode de réalisation particulier l'utilisation d'une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 9, ou un acide nucléotidique présentant au moins 70% d'identité de séquence avec la séquence SEQ ID NO : 9, et

(iv) l'intron humain ROSA ayant la séquence nucléotidique SEQ ID NO : 13, ou un acide nucléotidique ayant au moins 70% d'identité de séquence avec la séquence SEQ ID NO : 13.

La présente description concerne également selon un mode de réalisation plus particulier, l'utilisation d'une unité de transcription constituée d'un polynucléotide comprenant un acide nucléotidique représenté par la séquence SEQ ID NO : 51 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR représentée par la séquence nucléotidique SEQ ID NO : 9, et
(iv) l'intron humain ROSA ayant la séquence nucléotidique SEQ ID NO : 13, ou d'un acide nucléotidique ayant au moins 70% d'identité de séquence avec la séquence SEQ ID NO : 51.

L'unité de transcription obtenue est désignée par CMV-CDK9-U2U3-hROSA, autrement appelée E2-CDK9-U2U3-hROSA.

La présente description concerne également selon un mode de réalisation particulier l'utilisation d'une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 10, ou un acide nucléotidique présentant au moins 70% d'identité de séquence avec la séquence SEQ ID NO : 10, et
(iv) l'intron du gène Elongation Factor 1$\alpha$ (EF1$\alpha$) ayant la séquence nucléotidique SEQ ID NO : 11, ou un acide nucléotidique présentant au moins 70% d'identité de séquence avec la séquence SEQ ID NO : 11.

La présente description concerne également selon un mode de réalisation plus particulier, l'utilisation d'une unité de transcription constituée d'un polynucléotide comprenant un acide nucléotidique représenté par la séquence SEQ ID NO : 52 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR représentée par la séquence nucléotidique SEQ ID NO : 10, et
(iv) l'intron du gène Elongation Factor 1$\alpha$ (EF1$\alpha$) ayant la séquence nucléotidique SEQ ID NO : 11,

ou d'un acide nucléotidique ayant au moins 70% d'identité de séquence avec la séquence SEQ ID NO : 52.

L'unité de transcription obtenue est désignée par CMV-CDK9-U1U2U3-EF1$\alpha$, autrement appelée E2-CDK9-U1U2U3-EF.

La présente description concerne également selon un mode de réalisation particulier l'utilisation d'une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 10, ou un acide nucléotidique présentant au moins 70% d'identité de séquence avec la séquence SEQ ID NO : 10, et
(iv) l'intron murin ROSA ayant la séquence nucléotidique SEQ ID NO : 12, ou un acide nucléotidique ayant au moins 70% d'identité de séquence avec la séquence SEQ ID NO : 12.

La présente description concerne également selon un mode de réalisation plus particulier, l'utilisation d'une unité de transcription constituée d'un polynucléotide comprenant un acide nucléotidique représenté par la séquence SEQ ID NO : 53 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR représentée par la séquence nucléotidique SEQ ID NO : 10, et
(iv) l'intron murin ROSA ayant la séquence nucléotidique SEQ ID NO : 12,

ou d'un acide nucléotidique ayant au moins 70% d'identité de séquence avec la séquence SEQ ID NO : 53.

L'unité de transcription obtenue est désignée par CMV-CDK9-U1U2U3-mROSA, autrement appelée E2-CDK9-U1U2U3-.

La présente description concerne également selon un mode de réalisation particulier l'utilisation d'une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cycline dépendante des kinases 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 10, ou un acide nucléotidique présentant au moins 70% d'identité de séquence avec la séquence SEQ ID NO : 10, et
(iv) l'intron humain ROSA ayant la séquence nucléotidique SEQ ID NO : 13, ou un acide nucléotidique ayant au moins 70% d'identité de séquence avec la séquence SEQ ID NO : 13.

La présente description concerne également selon un mode de réalisation plus particulier, l'utilisation d'une unité de transcription constituée d'un polynucléotide comprenant un acide nucléotidique représenté par la séquence SEQ ID NO : 54 et constitué de :

(i) l'enhancer du virus hCMVie représenté par la séquence nucléotidique SEQ ID NO : 1,
(ii) la région promotrice de la cycline dépendante des kinases 9 (CDK9) représentée par la séquence nucléotidique SEQ ID NO : 2,
(iii) la région 5' UTR représentée par la séquence nucléotidique SEQ ID NO : 10, et
(iv) l'intron humain ROSA ayant la séquence nucléotidique SEQ ID NO : 13, ou d'un acide nucléotidique ayant au moins 70% d'identité de séquence avec la séquence SEQ ID NO : 54.

L'unité de transcription obtenue est désignée par CMV-CDK9-U1U2U3-hROSA, autrement appelée E2-CDK9-U1U2U3-hROSA.

Dans un mode de réalisation plus particulier, la présente description concerne également l'utilisation d'une unité de transcription contenant la région promotrice du gène CKD9, la région 5'UTR du gène eIF4GI (U3) et l'intron du gène EF1$\alpha$ pour la transfection d'une cellule hôte de la lignée cellulaire CHO choisie parmi CHO-S, CHO, ladite unité de transcription permettant une production volumique d'une protéine d'intérêt supérieure à celle obtenue avec la combinaison de l'enhancer CMV associé à la région promotrice de CDK9.

Dans un mode de réalisation encore plus particulier, la présente description concerne égalementl'utilisation d'une unité de transcription ayant la séquence nucléotidique SEQ ID NO : 40, ou une séquence nucléotidique présentant au moins 70% d'identité avec la SEQ ID NO : 40 pour la transfection d'une cellule hôte de la lignée cellulaire CHO choisie parmi CHO-S, CHO, ladite unité de transcription permettant une production volumique d'une protéine d'intérêt supérieure à celle obtenue avec la combinaison de l'enhancer CMV associé à la région promotrice de CDK9.

La lignée cellulaire CHO utilisée dans la présente invention peut être choisie parmi CHO-S, CHO.

La présente invention concerne également l'utilisation d'un vecteur d'expression décrit ci-dessus pour transfecter une cellule hôte.

Un autre objectif de la présente invention est de mettre à disposition d'un système d'expression comprenant un vecteur d'expression selon la présente invention et une cellule hôte telle que décrite ci-dessus, permettant l'expression d'une protéine d'intérêt codée par un acide nucléotidique.

La présente invention concerne également l'utilisation d'un vecteur d'expression comprenant au moins une unité de transcription selon la présente invention dans une cellule hôte telle que décrite ci-dessus pour produire une protéine codée par un acide nucléotidique, ladite protéine étant produite avec un titre plus élevé que dans le vecteur d'expression de référence comprenant au moins un promoteur RSV, un intron pCInéo, une séquence de polyadénylation, un gène de résistance eucaryote, un gène de résistance bactérien, une origine de réplication bactérienne et une unité dédiée à l'amplification génique, ledit vecteur de référence comportant la même séquence nucléotidique.

La présente invention a également pour objet un procédé de production *in vitro* d'une protéine recombinante d'intérêt comprenant les étapes de :

- introduction du vecteur d'expression comprenant au moins une unité de transcription selon la présente invention et un ADNc codant pour une protéine d'intérêt dans une cellule hôte,
- sélection et identification des cellules hôtes obtenues à l'étape précédente exprimant de manière stable ladite protéine d'intérêt,
- extraction et purification de ladite protéine d'intérêt.

Une telle protéine recombinante peut être une protéine participant de la coagulation, une immunoglobuline, des cytokines, des hormones, des facteurs de croissance ou facteurs du complément et de toute protéine de fusion.

Un procédé selon la présente invention peut comprendre en outre une étape de sélection et identification des cellules hôtes obtenues exprimant de manière stable ladite protéine d'intérêt.

La présente invention est illustrée par les figures et les exemples ci-après. Cependant, la présente invention n'est limitée en aucun cas aux figures et exemples ci-après.

## Figures

Figure 1 illustre le vecteur E2-bActin-U1 comportant une unité de transcription comprenant l'enhancer de hCMVie, la région promotrice de la β-actine et la région R du LTR du virus HTLV-1.

Figure 2 illustre le vecteur E2-bActin-U2 comportant une unité de transcription comprenant l'enhancer de hCMVie, la région promotrice de la β-actine et la région 5'UTR du gène NRF.

Figure 3 illustre le vecteur E2-bActin-U3 comportant une unité de transcription comprenant l'enhancer de hCMVie, la région promotrice de la β-actine et la région 5'UTR du gène eIF4G1.

Figure 4 illustre le vecteur E2-bActin-U1U3 comportant une unité de transcription comprenant l'enhancer de hCMVie, la région promotrice de la β-actine, la région R du LTR du virus HTLV-1 et la région 5'UTR du gène eIF4G1.

Figure 5 illustre le vecteur E2-bActin-U2U3 comportant une unité de transcription comprenant l'enhancer de hCMVie, la région promotrice de la β-actine, la région 5'UTR du gène NRF et la région 5'UTR du gène eIF4G1.

Figure 6 illustre le vecteur E2-bActin-U1U2U3 comportant une unité de transcription comprenant l'enhancer de hCMVie, la région promotrice de la β-actine, la région R du LTR du virus HTLV-1, la région 5'UTR du gène NRF et la région 5'UTR du gène eIF4G1.

Figure 7 illustre le vecteur E2-CDK9-U1 comportant une unité de transcription comprenant l'enhancer de hCMVie, la région promotrice du gène CDK9 et la région R du LTR du virus HTLV-1.

Figure 8 illustre le vecteur E2-CDK9-U2 comportant une unité de transcription comprenant l'enhancer de hCMVie, la région promotrice du gène CDK9 et la région 5'UTR du gène NRF.

Figure 9 illustre le vecteur E2-CDK9-U3 comportant une unité de transcription comprenant l'enhancer de hCMVie, la région promotrice du gène CDK9 et la région 5'UTR du gène eIF4G1.

Figure 10 illustre le vecteur E2-CDK9-U1U3 comportant une unité de transcription comprenant l'enhancer de hCMVie, la région promotrice du gène CDK9, la région R du LTR du virus HTLV-1 et la région 5'UTR du gène eIF4G1.

Figure 11 illustre le vecteur E2-CDK9-U2U3 comportant une unité de transcription comprenant l'enhancer de hCMVie, la région promotrice du gène CDK9, la région 5'UTR du gène NRF et la région 5'UTR du gène eIF4G1.

Figure 12 illustre le vecteur E2-CDK9-U1U2U3 comportant une unité de transcription comprenant l'enhancer de hCMVie, la région promotrice du gène CDK9, la région R du LTR du virus HTLV-1, la région 5'UTR du gène NRF et la région 5'UTR du gène eIF4G1.

Figure 13 illustre le vecteur pcDNA3.1-E2-bActin-U1U2U3 issu du vecteur pcDNA3.1 invitrogen, comportant une unité de transcription comprenant l'enhancer de hCMVie, la région promotrice du gène CDK9, la région R du LTR du virus HTLV-1, la région 5'UTR du gène NRF et la région 5'UTR du gène eIF4G1.

Figure 14 illustre le vecteur pcDNA3.1-E2-bActin-U1U3 issu du vecteur pcDNA3.1 invitrogen, comportant une unité de transcription comprenant l'enhancer de hCMVie, la région promotrice du gène CDK9, la région R du LTR du virus HTLV-1 et la région 5'UTR du gène eIF4G1.

Figure 15 illustre le vecteur pREP4-E2-bActin-U1 issu du vecteur pREP4, comportant une unité de transcription comprenant l'enhancer de hCMVie, la région promotrice de la β-actine et la région R du LTR du virus HTLV-1.

Figure 16 illustre le vecteur pREP4-E2-bActin-U3 issu du vecteur pREP4, comportant une unité de transcription comprenant l'enhancer de hCMVie, la région promotrice de la β-actine et la région 5'UTR du gène NRF.

Figure 17 illustre le vecteur pREP4-E2-bActin-U1U3 issu du vecteur pREP4, comportant une unité de transcription comprenant l'enhancer de hCMVie, la région promotrice de la β-actine, la région R du LTR du virus HTLV-1 et la région 5'UTR du gène eIF4G1.

Figure 18 illustre le vecteur pREP4-E2-CDK9-U3 issu du vecteur pREP4, comportant une unité de transcription comprenant l'enhancer de hCMVie, la région promotrice du gène CDK9 et la région 5'UTR du gène NRF.

Figure 19 illustre le vecteur pREP4-E2-CDK9-U1U3 issu du vecteur pREP4, comportant une unité de transcription comprenant l'enhancer de hCMVie, la région promotrice du gène CDK9, la région R du LTR du virus HTLV-1 et la région 5'UTR du gène eIF4G1.

Figure 20 illustre le vecteur pCEP4-E2-bActin-U1U3 issu du vecteur pCEP4, comportant une unité de transcription comprenant l'enhancer de hCMVie, la région promotrice de la β-actine, la région R du LTR du virus HTLV-1 et la région 5'UTR du gène eIF4G1.

Figure 21 illustre le vecteur pCEP4-E2-bActin-U1U2U3 issu du vecteur pCEP4, comportant une unité de transcription comprenant l'enhancer de hCMVie, la région promotrice de la β-actine, la région R du LTR du virus HTLV-1, la région 5'UTR du gène NRF et la région 5'UTR du gène eIF4G1.

Figure 22 illustre le vecteur E2-CDK9-EF1α comportant une unité de transcription comprenant l'enhancer de hCMVie, la région promotrice du gène CDK9 et le premier intron du gène EF1α.

Figure 23 illustre le vecteur E2-CDK9-EF1α-U1U2U3 comportant une unité de transcription comprenant l'enhancer de hCMVie, la région promotrice du gène CDK9, le premier intron du gène EF1α, la région R du LTR du virus HTLV-1, la région 5'UTR du gène NRF et la région 5'UTR du gène eIF4G1.

Figure 24 illustre le vecteur E2-CDK9-EF1α-U1U3 comportant une unité de transcription comprenant l'enhancer de hCMVie, la région promotrice du gène CDK9, le premier intron du gène EF1α, la région R du LTR du virus HTLV-1 et la région 5'UTR du gène eIF4G1.

Figure 25 illustre le vecteur E2-CDK9-EF1α-U2U3 comportant une unité de transcription comprenant l'enhancer de hCMVie, la région promotrice du gène CDK9, le premier intron du gène EF1α, la région 5'UTR du gène NRF et la région 5'UTR du gène eIF4G1.

Figure 26 illustre le vecteur E2-CDK9-EF1α-U2 comportant une unité de transcription comprenant l'enhancer de hCMVie, la région promotrice du gène CDK9, le premier intron du gène EF1α et la région 5'UTR du gène NRF.

Figure 27 illustre le vecteur E2-CDK9-EF1α-U1 comportant une unité de transcription comprenant l'enhancer de hCMVie, la région promotrice du gène CDK9, le premier intron du gène EF1α et la région R du LTR du virus HTLV-1.

Figure 28 illustre le vecteur E2-CDK9-EF1α-U1U2 comportant une unité de transcription comprenant l'enhancer de hCMVie, la région promotrice du gène CDK9, le premier intron du gène EF1α, la région R du LTR du virus HTLV-1 et la région 5'UTR du gène NRF.

Figure 29 illustre la comparaison de l'effet de différents introns en association avec le LTR RSV sur l'expression de la chaîne kappa libre de l'anticorps anti-Rh(D) T125 dans la lignée CHO-S évaluée en transfection transitoire. Les colonnes de points, de gauche à droite, représentent respectivement le taux d'expression de la chaîne kappa libre sous le contrôle des introns : β-actine (Bact), EF, mROSA, hROSA, HTLV, ubiquitine (ubc). Les vecteurs de référence sont RSV_int_KT125_2STP et RSV_T125_K2. L'axe des ordonnées représente la concentration en chaînes kappa libres dans le milieu de culture.

Figure 30 illustre la comparaison de l'effet de différents introns en association avec l'unité de transcription E-2CDK9-U3 ou le LTR RSV sur l'expression de la chaîne kappa libre de l'anticorps anti-Rh(D) T125 dans la lignée CHO-S évaluée en transfection transitoire. Les colonnes de points, de gauche à droite, représentent respectivement le taux d'expression de la chaîne kappa libre sous le contrôle des associations : E2-CDK9-U3 sans intron, E2-CDK9-U3 avec intron hROSA, E2-CDK9-U3 avec intron mROSA, LTR RSV avec intron EF, LTR RSV avec intron mROSA, E2-CDK9-U3 avec intron EF, LTR RSV avec intron hROSA. Les vecteurs de référence sont RSV_T125_K2 et pRep4KT125. L'axe des ordonnées représente la concentration en chaînes kappa libres dans le milieu de culture.

Figure 31 illustre la comparaison de l'expression en pools stables de transfectants exprimant l'IgG anti-D en fonction du vecteur (E2CDK9U3 / LTR RSV intron pCI neo) et plus précisément la productivité en pools stables de l'anticorps entier anti-Rh(D) T125 avec le vecteur avec unité de transcription E2-CDK9-U3 (HK E2 CDK9 U3) en comparaison avec la référence avec LTR RSV intron pCI neo (HK463-18).

Figure 32 est un diagramme de répartition des transfectants exprimant l'IgG anti-D en fonction du vecteur (E2CDK9U3 / LTR RSV intron pCI neo). Ce diagramme illustre la productivité de clones produisant l'anticorps entier anti-Rh(D) T125 avec le vecteur avec unité de transcription E2-CDK9-U3 (HK E2 CDK9 U3) en comparaison avec la référence avec LTR RSV intron pCI neo (HK463-18).

Figure 33 illustre les titres moyens en chaines kappa T125 obtenus dans la lignée CHO-S transfectée par un vecteur contenant une unité de transcription ci-après respective: E2-bActine-Témoin, E2-bActine-U1, E2-bActine-U3, E2-bActine-U1U2U3. Le résultat est évalué sur la base de trois expériences indépendantes de transfection transitoire en triplicats. L'axe des ordonnées représente la concentration en chaînes kappa libres dans le milieu de culture. p-value<0.05, n= 10 à 13.

Figure 34 illustre les titres moyens en chaines kappa T125 obtenus dans la lignée HEK transfectée par un vecteur contenant une unité de transcription ci-après respective : E2-bActine-Témoin, E2-bActine-U1, E2-bActine-U1U2U3. Le résultat est évalué sur la base de trois expériences indépendantes de transfection transitoire en triplicats. L'axe des ordonnées représente la concentration en chaînes kappa libres dans le milieu de culture. p-value<0.05, n= 10 à 15.

Figure 35 illustré les titres moyens en chaines kappa T125 obtenus dans la lignée CHO-S transfectée respectivement par le vecteur pREP4-KT125, pREP4-E2-bActine-U3, pREP4-E2-U1U3. Le résultat est évalué sur la base de trois expériences indépendantes de transfection transitoire en triplicats. L'axe des ordonnées représente la concentration en chaînes kappa libres dans le milieu de culture. p-value<0.05, n= 6.

Figure 36 illustré les titres moyens en chaines kappa T125 obtenus dans la lignée HEK transfectée respectivement par le vecteur pCEP4-KT125, pCEP4-E2-bActine-U1U3. Le résultat est évalué sur la base de trois expériences indépendantes de transfection transitoire en triplicats. L'axe des ordonnées représente la concentration en chaînes kappa libres dans le milieu de culture. p-value<0.05, n= 6.

## Exemples :

### 1. Matériels et Méthodes

#### 1.1. Transfection transitoire

Dans CHO-S, les séquences à exprimer sont évaluées en transfection transitoire selon le protocole du kit FreeStyle (Invitrogen). Les cellules parentales sont ensemencées 24 h avant la transfection (J-1) en Erlenmeyer (VWR) à $6^E5$ cv/ml en FreeStyle CHO EM (Fisher Bioblock scientific) et incubées à 120 tr/min 37°C, 8 % $CO_2$. Le jour de la transfection un complexe FreeStyle MAX Reagent (Fisher Bioblock Scientific) /ADN, au ratio 1:1, est formé en Opti Pro SFM (Invitrogen). Le complexe est ensuite déposé sur les cellules en suspension préalablement centrifugées et reprises à $1^E6$ cv/ml en FreeStyle CHO EM dans un cultiflask (Sartorius) (5ml) et incubé à 200 tr/min à 37°C, 8 % de $CO_2$. Les surnageants sont récoltés à J+5 pour évaluation du taux de molécule sécrété dans le milieu.

#### 1.2. Transfection stable

Les évaluations sont réalisées sur des pools de transfectants (« transfection en pool stable ») afin de comparer les différentes constructions sur la base d'un niveau d'expression moyenné sur un grand nombre de transfectants (plusieurs milliers) ainsi que sur les meilleurs clones sélectionnés par ClonePixFL sur ces pools.

1.2.1. Obtention des pools et évaluations en pools

La lignée CHO-S est cultivée en milieu Freestyle CHO EM + 8 mM de glutamine, en flask à 37°C, 8% CO2, sous agitation à 135 rpm.

Les cellules sont repiquées la veille à $6x10^5$ cell/ ml.

Le jour de l'électroporation, les cellules sont électroporées par Gene Pulser Xcell (BioRad) avec un voltage de 300 V et une capacitance de 500 μF dans des cuvettes (Biorad) de 4 mm avec 5E6 cv (qsp 500 μl de tampon d'éléctroporation du kit electrobuffer (Ozyme) contenant l'ADN plasmidique linéarisé). Après électroporation les cellules sont reprises à 3E5 cv/ml en flacon de culture F75.

A J+3 : Mise en milieu sélectif pour obtenir les concentrations finales suivantes : Freestyle CHO EM + additifs LFB pour clonage cellulaire de faible densité LDCC + G418 1 mg/ml.

A J+10 : Dilution de moitié en Freestyle CHO EM + additifs LFB pour clonage cellulaire de faible densité LDCC + G418 1 mg/ml.

A partir de J+12 et 3 fois par semaine : si la densité cellulaire est supérieure à 6E5 cv/ml, repiquer les cellules à 3E5 cv/ml en F25.

A partir de J+17 repiquage en flacon F25 ou F75 en Freestyle CHO EM + G418 1 mg/ml.

A partir de J+25, réaliser une production en mode batch : inoculer les F25 à $3^E5$ cv/ml en Freestyle CHO EM + G418 1 mg/ml (production en pool).

Le surnageant est récolté à J+12 et dosé avec le kit Fast ELYSA (RD-biotech).

1.2.2. Obtention de clones et évaluations des clones

Les pools de cellules obtenus précédemment sont étalés en milieu semi-solide (CloneMedia CHO - Molecular Devices) en présence d'anticorps fluorescent de détection.

Les clones les plus forts producteurs de chaque pool sont sélectionnés tout d'abord en fonction de leur intensité de fluorescence (screening et picking par ClonePix$^{FL}$) puis en fonction de leur titre à saturation en P24.

Les meilleurs clones sont alors évalués en production en mode batch par inoculation de cultiflasks à $3^{E}5$ cv/ml en Freestyle CHO EM + G418 1 mg/ml et culture sous agitation à 250rpm.

Le surnageant est récolté lorsque la viabilité est inférieure à 50% et dosé avec le kit Fast ELYSA (RD-biotech).

**1.3. Evaluation du taux de protéine recombinante sécrétée**

L'évaluation du taux de chaine kappa libre de l'anticorps anti-Rh(D) T125 ainsi que la production d'IgG1 d'anti-CD20 ou d'anti- Rh(D) T125 sont déterminés par la technique Enzyme-linked immunosorbent assay (ELISA).

La chaine kappa libre présente dans le surnageant de culture est capturée pendant 2 h par un anticorps de chèvre anti-kappa humain (Caltag Lab) qui est adsorbé sur des plaques de 96 puits. L'anticorps capturé est ensuite révélé par un anti-kappa humain de chèvre biotinylé (Pierce) puis ajout de streptavidine couplée à la peroxydase (Pierce). Entre chaque étape 4 lavages sont effectués pour éliminer les protéines et réactifs n'entrant pas dans le complexe. La révélation est faite par ajout du substrat de l'enzyme, l'OPD (Sigma) et arrêt de la réaction par du HCl 1N. La lecture se fait au spectrophotomètre à 492nm. La concentration d'anticorps est déterminée en comparaison d'une gamme étalon.

Les IgG1 produites en transfections transitoires et stables sont évaluées par le kit Fast ELYSA (RD-biotech) selon les instructions du fournisseur. La lecture de la densité optique se fait au spectrophotomètre à 450 nm. La concentration d'anticorps est déterminée en comparaison d'une gamme étalon contenue dans le kit.

**1.4. Analyses statistiques**

Les résultats de production de chaine Kappa libre ou des immunoglobulines entières sont comparés sur des valeurs normalisées par les médianes d'une expérimentation à une autre. Les analyses statistiques sont faites à l'aide du logiciel STATGRAPHICS Centurion XV. Des tests étendus multiples sont appliqués aux données avec la méthode 95.0% LSD. Les paires de données ont des différences statistiquement significatives au niveau de confiance de 95,0%.

**Exemple 1 : Construction du vecteur E2-bActin-U1U2U3 (figure 6)**

- Digestion du vecteur E2- bActin par BamHI+NheI
- Récupération du fragment de 5560 élimination du fragment de 204 bases
- Digestion de l'insert synthétique par BamHI + NheI
- Récupération sur gel de l'insert de 1271 bases
- Ligation et obtention de E2-bActin-U1U2U3
- Screening par PCR avec les amorces appropriées qui donne un amplicon de 944 bases

**Exemple 2 : Construction du vecteur E2-bActin-U1 (Figure 1)**

- Digestion SpeI+NheI E2-bActin-U1U2U3
- Récupération sur gel du fragment à 5841 bases, élimination du fragment à 990 bases
- Ligation et obtention de E2-bActin-U1
- Screening par PCR avec les amorces appropriées qui donne un amplicon de 1118 bases

**Exemple 3 : Construction du vecteur E2-bActin-U3 (Figure 3)**

- Digestion HpaI+PmeI sur E2-bActin-U1U2U3
- Récupération sur gel du fragment à 5887 bases, élimination du fragment à 944 bases
- Ligation et obtention de E2-bActin-U3
- Screening par PCR avec les amorces appropriées qui donne un amplicon de 1164 bases

**Exemple 4 : Construction du vecteur E2-bActin-U2U3 (figure 5)**

- Digestion PmeI sur E2-bActin-UIU2U3
- Récupération du fragment 6550 bases élimination du fragment de 281 bases
- Ligation et obtention de E2-bActin-U2U3
- Screening par PCR avec les amorces appropriées qui donne un amplicon de 1468 bases

**Exemple 5 : Construction du vecteur E2-bActin-U2 (Figure 2)**

- Digestion SpeI+NheI de E2-bActin-U2U3
- Récupération sur gel du fragment à 6226 bases, élimination du fragment à 324 bases
- Ligation et obtention de E2-bActin-U2
- Screening par PCR avec les amorces appropriées qui donne un amplicon de 1468 bases

**Exemple 6 : Construction du vecteur E2-bActin-U1U3 (Figure 4)**

- Digestion SpeI sur E2-bActin-U1U2U3
- Récupération sur gel du fragment à 6165 bases, élimination du fragment à 666 bases
- Ligation et obtention de E2-bActin-U1U3
- Screening par PCR avec les amorces appropriées qui donne un amplicon de 578 bases

**Exemple 7 : Construction du Vecteur E2-CDK9-U1U2U3 (Figure 12)**

- Digestion du vecteur E2-CDK9 par BamHI et NheI
- Récupération du fragment de 5630 bases, élimination du fragment de 204 bases
- Digestion de l'insert synthétique par BamHI et NheI
- Récupération sur gel de l'insert de 1271 bases
- Ligation et obtention de E2-CDK9-U1U2U3
- Screening par PCR avec les amorces appropriées qui donne un amplicon de 1014 bases

**Exemple 8 : Construction du vecteur E2-CDK9-U2U3 (Figure 11)**

- Digestion PmeI sur E2-CDK9-U1U2U3
- Récupération du fragment 6620 bases élimination du fragment de 281 bases
- Ligation et obtention de E2-CDK9-U2U3
- Screening par PCR avec les amorces appropriées qui donne un amplicon de 1538 bases

**Exemple 9 : Construction du vecteur E2-CDK9-U2 (Figure 8)**

- Digestion SpeI+NheI de E2-CDK9-U2U3
- Récupération sur gel du fragment à 6296 bases, élimination du fragment à 324 bases
- Ligation et obtention de E2-CDK9-U2
- Screening par PCR avec les amorces appropriées qui donne un amplicon de 686 bases

**Exemple 10 : Construction du vecteur E2-CDK9-U1 (Figure 7)**

- Digestion SpeI+NheI sur E2-CDK9-U1U2U3
- Récupération sur gel du fragment à 5911 bases, élimination du fragment à 990 bases
- Ligation et obtention de E2-CDK9-U1
- Screening par PCR avec les amorces appropriées qui donne un amplicon de 254 bases

**Exemple 11 : Construction du vecteur E2-CDK9-U3 (Figure 9)**

- Digestion HpaI+PmeI sur E2-CDK9-U1U2U3
- Récupération sur gel du fragment à 5957 bases, élimination du fragment à 944 bases
- Ligation et obtention de E2-CDK9-U3
- Screening par PCR avec les amorces appropriées qui donne un amplicon de 1234 bases

**Exemple 12 : Construction du vecteur E2-CDK9-U1U3 (Figure 10)**

- Digestion SpeI sur E2-CDK9-U1U2U3
- Récupération sur gel du fragment à 6235 bases, élimination du fragment à 666 bases
- Ligation et obtention de E2-CDK9- U1 U3
- Screening par PCR avec les amorces appropriées qui donne un amplicon de 578 bases

**Exemple 13 : Construction du vecteur pcDNA3.1-E2-bActin-U1U2U3 (figure 13)**

- Digestion BglII+XbaI sur E2-bActin-U1U2U3

- Récupération sur gel du fragment à 2618 bases, élimination du fragment à 4213 bases

- Ligation en substitution de la région promotrice dans pcDNA3.1 contenant déjà la chaine légère KT125 et obtention de pcDNA3.1-E2-bActin-U1U2U3

- Screening par PCR avec les amorces appropriées qui donne un amplicon de 651 bases

**Exemple 14 : Construction du vecteur pcDNA3.1-E2-bActin-U1U3 (figure 14)**

- Digestion BglII+XbaI sur E2-bActin-U1U3
- Récupération sur gel du fragment à 1952 bases, élimination du fragment à 4213 bases
- Ligation en substitution de la région promotrice dans pcDNA3.1 contenant déjà la chaine légère KT125 et obtention de pcDNA3.1-E2-bActin-U1U3
- Screening par PCR avec les amorces appropriées qui donne un amplicon de 578 bases

**Exemple 15 : Construction du vecteur pREP4-E2-bActin-U1 (figure 15)**

- Digestion BglII+XbaI sur E2-bActin-U1
- Récupération sur gel du fragment à 1628 bases, élimination du fragment à 4213 bases
- Ligation en substitution de la région promotrice dans pREP4 contenant déjà la chaine légère KT125 et obtention de pREP4-E2-bActin-U1

- Screening par PCR avec les amorces appropriées qui donne un amplicon de 254 bases

**Exemple 16 : Construction du vecteur pREP4-E2-bActin-U3 (figure 16)**

- Digestion BglII+XbaI sur E2-bActin-U3
- Récupération sur gel du fragment à 1674 bases, élimination du fragment à 4213 bases
- Ligation en substitution de la région promotrice dans pREP4 contenant déjà la chaine légère KT125 et obtention de pREP4-E2bActine-U3
- Screening par PCR avec les amorces appropriées qui donne un amplicon de 752 bases

**Exemple 17 : Construction du vecteur pREP4-E2-bActin-U1U3 (figure 17)**

- Digestion SfuI+XbaI sur pCEP4-E2-bActin-U1U3
- Récupération sur gel du fragment à 4273 bases, élimination du fragment à 7188 bases
- Ligation en substitution de la région promotrice dans pREP4 contenant déjà la chaine légère KT125 et obtention de pREP4-E2-bActin-U1U3
- Screening par PCR avec les amorces appropriées qui donne un amplicon de 578 bases

**Exemple 18 : Construction du vecteur pREP4-E2-CDK9-U3 (figure 18)**

- Digestion BglII+XbaI sur E2-CDK9-U3
- Récupération sur gel du fragment à 1748 bases, élimination du fragment à 4213 bases
- Ligation en substitution de la région promotrice dans pREP4 contenant déjà la chaine légère KT125 et obtention de pREP4-E2-CDK9-U3
- Screening par PCR avec les amorces appropriées qui donne un amplicon de 752 bases

**Exemple 19 : Construction du vecteur pREP4-E2-CDK9-U1U3 (figure 19)**

- Digestion BglII+XbaI sur E2-CDK9-U1U3
- Récupération sur gel du fragment à 2026 bases, élimination du fragment à 4213 bases
- Ligation en substitution de la région promotrice dans pREP4 contenant déjà la chaine légère KT125 et obtention de pREP4-E2-CDK9-U1U3
- Screening par PCR avec les amorces appropriées qui donne un amplicon de 578bases

**Exemple 20 : Construction du vecteur pCEP4-E2-bActin-U1U3 (figure 20)**

- Digestion BglII+XbaI sur E2-bActin-U1U3
- Récupération sur gel du fragment à 1956 bases, élimination du fragment à 4213 bases
- Ligation en substitution de la région promotrice dans pCEP4 contenant déjà la chaine légère KT125 et obtention de pCEP4-E2-bActin-U1U3
- Screening par PCR avec les amorces appropriées qui donne un amplicon de 578 bases

**Exemple 21 : Construction du vecteur pCEP4-E2-bActin-U1U2U3 (figure 21)**

- Digestion BglII+XbaI sur E2-bActin-U1U2U3
- Récupération sur gel du fragment à 2622 bases, élimination du fragment à 4213 bases

- Ligation en substitution de la région promotrice dans pCEP4 contenant déjà la chaine légère KT125 et obtention de pCEP4-E2-bActin-U1U2U3
- Screening par PCR avec les amorces appropriées qui donne un amplicon de 1010 bases

**Exemple 22 : Construction du vecteur E2-CDK9-EF1α (figure 22)**

- Digestion SpeI+NheI de E2-CDK9
- Récupération sur gel du fragment à 5636 bases, élimination du fragment à 198 bases
- Digestion de l'insert synthétique par SpeI et NheI
- Récupération sur gel de l'insert de 1001 bases
- Ligation et obtention de E2-CDK9-EF1α
- Screening par PCR avec les amorces appropriées

**Exemple 23 : Construction du vecteur E2-CDK9-EF1α-U1U2U3 (figure 23)**

- Digestion SpeI+BamHI de E2-CDK9-EF1α
- Récupération sur gel du fragment à 6631 bases, élimination du fragment de 6 bases
- Digestion de l'insert synthétique par BamHI et NheI
- Récupération sur gel de l'insert de 1271 bases
- Ligation et obtention de E2-CDK9-EF1α-U1U2U3
- Screening par PCR avec les amorces appropriées

**Exemple 24 : Construction du vecteur E2-CDK9-EF1α-U1U3 (figure 24)**

- Digestion SpeI sur E2-CDK9-EF1α-U1U2U3
- Récupération du fragment à 7236 bases et élimination du fragment de 666 bases
- Ligation et obtention de E2-CDK9-EF1α-U1U3
- Screening par PCR avec les amorces appropriées

**Exemple 25 : Construction du vecteur E2-CDK9-EF1α-U2U3 (figure 25)**

- Digestion HpaI/PmeI sur E2-CDK9-EF1α-U1U2U3
- Récupération du fragment à 7230 bases, élimination du fragment de 672 bases
- Ligation et obtention de E2-CDK9-EF1α-U2U3
- Screening par PCR avec les amorces appropriées

**Exemple 26 : Construction du vecteur E2-CDK9-EF1α-U2 (figure 26)**

- Digestion SpeI de E2-CDK9-EF1α
- Récupération sur gel du fragment à 6637 bases,
- Digestion de l'insert synthétique par SpeI
- Récupération sur gel de l'insert de 666 bases
- Ligation et obtention de E2-CDK9-EF1α-U2
- Screening par PCR avec les amorces appropriées

**Exemple 27 : Construction du vecteur E2-CDK9-EF1α-U1 (figure 27)**

- Digestion BamHI+SpeI de E2-CDK9-EF1α
- Récupération sur gel du fragment à 6631 bases et élimination du fragment de 6 bases
- Digestion de l'insert synthétique par BamHI+SpeI
- Récupération sur gel de l'insert de 947 bases
- Ligation et obtention de E2-CDK9-EF1α-U1
- Screening par PCR avec les amorces appropriées

**Exemple 28 : Construction du vecteur E2-CDK9-EF1α-U1U2 (figure 28)**

- Digestion SpeI de E2-CDK9-EF1α-U1
- Récupération sur gel du fragment à 9612 bases
- Digestion de l'insert synthétique par SpeI
- Récupération sur gel de l'insert de 947 bases
- Ligation et obtention de E2-CDK9-EF1α-U1U2
- Screening par PCR avec les amorces appropriées

**Exemple 29 : Comparaison** des introns en association avec le LTR RSV

Les introns à tester (Bact (β-actine), EF, mROSA, hROSA, HTLV, ubc (ubiquitine) sont insérés dans le vecteur d'expression K622_37, comprenant le LTR RSV, pour produire la chaîne légère kappa de l'anticorps T125. Le gain de productivité des vecteurs ainsi construits est comparé avec celui des vecteurs de référence RSV_int_KT125_2STP et RSV_T125_K2.

Les résultats obtenus à partir de 3 transfections réalisées sur 3 semaines différentes sont illustrés sur la figure 29 et permettent d'observer des différences significatives entre les introns.

Une comparaison multiple est réalisée pour les moyennes (ng/mL) de production de chaîne légère d'Ig obtenues avec les différents introns dans la lignée CHO-S (Tableau 1). La méthode actuellement utilisée pour discriminer entre les moyennes est la procédure des différences significatives minimales de Fisher (LSD). Des tests des étendues multiples sont effectués avec la méthode 95.0% LSD. Ces paires ont des différences statistiquement significatives au niveau de confiance de 95,0%.

Tableau 1

| | *Effectif* | *Moyenne* | *Groupe homogène* |
|---|---|---|---|
| RSV_int_KT125_2STP | 18 | 25506,3 | X |
| K622_37_HTLV | 18 | 26511,3 | X |
| K622_37_Ubc | 18 | 28790,0 | XX |
| K622_37_Bact | 17 | 31992,3 | XX |
| K622_37_hROSA | 18 | 33561,0 | X |
| RSV_T125_K2 | 16 | 34362,8 | X |
| K622_37_mROSA | 15 | 38874,8 | X |
| K622_37_EF | 14 | 44104,4 | X |

Cinq groupes homogènes sont identifiés en utilisant des colonnes de X. L'intron EF est significativement le plus efficace. En deuxième position est situé l'intron mROSA. Les autres introns n'ont pas d'effet positif en association avec le LTR RSV.

**Exemple 30 : Comparaison des unités de transcription dans les contextes E2-CDK9-U3 et LTR RSV**

Les différentes unités de transcription à tester sont testées pour la production de la chaîne légère kappa de l'anticorps T125. Le gain de productivité des vecteurs ainsi construits est comparé avec celui des vecteurs de référence pRep4KT125 et RSV_T125_K2.

Les résultats obtenus à partir de 3 transfections réalisées sur 3 semaines différentes sont illustrés sur la figure 30 et permettent d'observer des différences significatives entre les associations testées.

Une comparaison multiple est réalisée pour les moyennes (ng/mL) de production de chaîne légère d'Ig obtenues avec les différentes associations dans la lignée CHO-S (Tableau 2). La méthode actuellement utilisée pour discriminer entre les moyennes est la procédure des différences significatives minimales de Fisher (LSD). Des tests des étendues multiples sont effectués avec la méthode 95.0% LSD. Ces paires ont des différences statistiquement significatives au niveau de confiance de 95,0%.

Tableau 2

| | *Effectif* | *Moyenne* | *Groupe homogène* |
|---|---|---|---|
| RSVT125K2 | 12 | 10940,2 | X |
| E2CDK9U3 hRosa | 12 | 15847,6 | X |
| K622_37 hRosa | 12 | 23340,0 | X |
| pRep4KT125 | 12 | 23843,2 | X |
| E2CDK9U3 | 12 | 31903,9 | X |
| K622_37 mRosa | 12 | 35041,1 | X |
| E2CDK9U3 mRosa | 12 | 40688,4 | X |
| K622_37 EF | 12 | 41708,2 | X |
| E2CDK9U3 EF | 12 | 51907,2 | X |

Cinq groupes homogènes sont identifiés en utilisant des colonnes de X.

L'association de E2-CDK9-U3 avec l'intron EF est significativement la plus efficace. Dans le contexte E2-CDK9-U3, l'intron EF apporte ainsi un gain de 63%.

Les associations LTR RSV avec intron EF et E2-CDK9-U3 avec intron mROSA sont également significativement très efficaces.

Dans une moindre mesure, les autres associations testées sont plus efficaces que la référence RSV T125 K2.

**Exemple 31 : Production de l'anticorps anti-Rh(D) entier (HK) par des vecteurs contenant E2CDK9U3**

Les anticorps anti-D entiers (HK) sont produits respectivement dans les cellules CHO-S transfectées par les vecteurs contenant une unité de transcription de structure E2-CDK9-U3 et dans les cellules CHO-S transfectées par les vecteur contenant une unité de transcription de structure RSV-intron pCineo (vecteur de référence).

Le tableau 3 ci-dessous montre les résultats de dosage des anticorps anti-D entiers produits par des pools de cellules transfectées par le vecteur HK463-18 ou par le vecteur HK E2-CDK9-U3. La figure 31 illustre ces résultats.

Tableau 3: F6-2= pool issu de transfection avec HK463-18, F11-2 = pool issu de transfection avec HK E2-CDK9-U3

| Pool | Milieu | Type de production batch | Dosage IgG ELISA en ng/ml | Gain E2CDK9U3 / RSV+intronpCI |
|---|---|---|---|---|
| F6-2 | Freestyle + G418 | J+12 F25 | 2 324 | |
| F11-2 | Freestyle + G418 | J+12 F25 | 14 193 | 6,1 |

L'unité de transcription E2CDK9U3 permet d'obtenir un gain de productivité de l'ordre de 6 fois plus élevé que celui obtenu avec le vecteur de référence.

Le tableau 4 ci-dessous montre les résultats de dosage des anticorps anti-D entiers produits par les meilleurs clones (issus du procédé de criblage décrit en matériels et méthodes, sur un nombre limité de colonies) issus des pools précédemment décrits, transfectés par le vecteur HK463-18 ou par le vecteur HK E2-CDK9-U3. La figure 32 illustre ces résultats.

Tableau 4

| | cultiflask | |
|---|---|---|
| nom du vecteur | Prod Max J -1 ELISA IgG en ng/ml | prod max ELISA IgG en ng/ml |
| | | |
| HK 463-18 | NA | <min |
| HK 463-18 | NA | 2 071 |
| HK 463-18 | NA | 2 732 |
| HK 463-18 | NA | 4 110 |
| HK 463-18 | NA | 16 937 |
| | | |
| | | |
| HK-E2-CDK9-U3 | NA | 4 061 |
| HK-E2-CDK9-U3 | NA | 10 585 |
| HK-E2-CDK9-U3 | 6 863 | 13 235 |
| HK-E2-CDK9-U3 | 13 389 | 14 221 |
| HK-E2-CDK9-U3 | 21 318 | 20 203 |
| HK-E2-CDK9-U3 | 29 860 | 33 069 |
| HK-E2-CDK9-U3 | 37 611 | 33 402 |
| HK-E2-CDK9-U3 | NA | 36 830 |
| HK-E2-CDK9-U3 | NA | 43 851 |
| HK-E2-CDK9-U3 | NA | 47 315 |
| HK-E2-CDK9-U3 | 58 007 | 58 007 |
| HK-E2-CDK9-U3 | 47 056 | 60 304 |
| HK-E2-CDK9-U3 | 61 902 | 74 233 |

L'unité de transcription E2-CDK9-U3 permet d'obtenir un gain de productivité conséquent par rapport au vecteur basé sur LTR RSV + intron pCI neo :

- la productivité maximale obtenue avec E2CDK9U3 est plus de 4 fois supérieure à celle obtenue avec LTR RSV+ intron pCI neo
- les productivités observées sur les clones obtenus avec E2-CDK9-U3 sont en moyenne plus élevées que ceux obtenus avec LTR RSV+ intron pCI neo : titre moyen de 60,0µg/ml sur les clones obtenus avec E2-CDK9-U3 à comparer avec un titre moyen de 6,5µg/ml sur les clones obtenus avec LTR RSV+ intron pCI neo , soit un titre moyen presque 10 fois supérieur avec E2-CDK9-U3

**Exemple 32 : Expression transitoire des chaînes kappa T125 dans la lignée CHO-S**

La chaîne kappa est produite dans les cellules CHO-S transfectées respectivement par les vecteurs contenant une unité de transcription de structure : E2-bActine-Témoin, E2-bActine-U1, E2-bActine-U3 ou E2-bActine-U1U2U3.
Le tableau 5 ci-après, ainsi que la figure 33, illustrent les résultats de dosage des chaînes kappa dans le milieu de la culture.
Ces résultats montrent qu'une unité de transcription selon la présente invention contenant au moins un 5'UTR peut augmenter la production de chaînes kappa T125 dans la lignée cellulaire CHO-S par rapport à celle d'un vecteur témoin ne contenant que l'enhanceur hCMVie et le promoteur de la β-actine.

Tableau 5

| | Effectif | Moyenne | Groupe homogène |
|---|---|---|---|
| E2_bActine_Témoin | 10 | 35847,2 | X |
| E2_bActine_U1 | 12 | 43788,0 | X |
| E2_bActine_U3 | 11 | 44314,6 | X |
| E2_bActine_U1U2U3 | 12 | 44804,5 | X |
| E2_CDK9_U3 | 12 | 45551,4 | X |

La chaîne kappa a été produite également dans les cellules CHO-S transfectées respectivement par les vecteurs pREP4-KT125, pREP4-E2-bActine-U3 ou pREP4-E2-bActine-U1U3.

Le tableau 6 ainsi que la figure 35 illustrent les résultats de dosage des chaînes kappa dans le milieu de la culture.

Tableau 6

| | Effectif | Moyenne | Groupe homogène |
|---|---|---|---|
| pREP4_KT125 | 6 | 14813,8 | XX |
| pREP4_E2_bActine_U3 | 6 | 20493,9 | XX |
| pREP4_E2_bActine_U1U3 | 6 | 24185,5 | XX |

Ces résultats confirment qu'une unité de transcription contenant l'enhanceur hCMVie, le promoteur de la β-actine et un 5'UTR permet d'augmenter la titration de protéines recombinantes.

**Exemple 33 : Expression transitoire des chaînes kappa T125 dans la lignée HEK**

La chaîne kappa est produite dans les cellules HEK transfectées respectivement par les vecteurs contenant une unité de transcription de structure : E2-bActine-Témoin, E2-bActine-U1 ou E2-bActine-U1U2U3.

Le tableau 7 ci-après, ainsi que la figure 34, illustrent les résultats de dosage des chaînes kappa dans le milieu de la culture.

Ces résultats montrent qu'une unité de transcription selon la présente invention contenant au moins un 5'UTR peut également augmenter la titration de chaînes kappa T125 dans la lignée cellulaire HEK par rapport à celle d'un vecteur témoin ne contenant que l'enhanceur hCMVie et le promoteur de la β-actine.

Tableau 7

| | Effectif | Moyenne | Groupe homogène |
|---|---|---|---|
| E2_bActine_Témoin | 11 | 13978,9 | X |
| E2_bActine_U1 | 5 | 37275,9 | X |
| E2_bActine_U1U2U3 | 11 | 76477,2 | X |

La chaîne kappa a été produite également dans les cellules HEK transfectées respectivement par les vecteurs pCEP4-KT125 et pCEPT4-E2-bActine-U1U3.

Les résultats illustrés dans la figure 36 confirment qu'une unité de transcription selon la présente invention permet d'augmenter la titration de protéines recombinantes.

## Revendications

**1.** Unité de transcription constituée d'un polynucléotide comprenant les éléments régulateurs suivant :

- l'enhancer du virus hCMVie, ledit enhancer ayant la séquence nucléotidique SEQ ID NO : 1, ou un acide nucléotidique ayant au moins 70% d'identité de séquence avec la séquence SEQ ID NO : 1 et possédant des propriétés d'activation de la transcription, et
- la région promotrice de :

    - la β-actine, ladite région promotrice ayant la séquence nucléotidique SEQ ID NO : 3, ou un acide nucléotidique ayant au moins 70% d'identité de séquence avec la séquence SEQ ID NO : 3 et possédant une activité promotrice, et

- un acide nucléotidique situé en aval de ladite région promotrice et en amont du site d'initiation de la traduction, ledit acide nucléotidique comprenant la région régulatrice R du 5' Long Terminal Repeat (LTR) du virus HTLV-1 et la région 5' UTR du gène eukaryotic Initiation Factor 4GI (eIF4GI), ledit acide nucléotidique ayant la séquence SEQ ID NO :8, , ou un acide nucléotidique ayant au moins 70% d'identité de séquence avec la SEQ ID NO :8 ayant des propriétés de stabilisation des ARNm et de facilitateur de la traduction.

**2.** Unité de transcription selon la revendication 1, dans laquelle ledit polynucléotide a la séquence SEQ ID NO :25.

**3.** Unité de transcription selon la revendication 1, ledit polynucléotide comprenant également un intron, ledit intron étant choisi parmi les suivants :

- intron du gène Elongation Factor 1α (EF1α) ayant la séquence nucléotidique SEQ ID NO : 11, ou un acide nucléotidique ayant au moins 70% d'identité de séquence avec la SEQ ID NO : 11,
- intron murin ROSA ayant la séquence nucléotidique SEQ ID NO : 12, ou un acide nucléotidique ayant au moins 70% d'identité de séquence avec la SEQ ID NO : 12,
- intron humain ROSA ayant la séquence nucléotidique SEQ ID NO : 13, ou un acide nucléotidique ayant au moins 70% d'identité de séquence avec la SEQ ID NO : 13,

ledit intron étant situé :

(i) en aval de la région 5' UTR et en amont du site d'initiation de la traduction, ou
(ii) en aval du promoteur et en amont de la région 5'UTR, ou
(iii) après le site d'initiation de la traduction et à l'intérieur d'une séquence codante, ou
(iv) entre le codon stop de la séquence codante et le signal de polyadénylation,

ledit polynucléotide ayant notamment l'une des séquences suivantes : SEQ ID NO :67, SEQ ID NO :68, SEQ ID NO :69.

**4.** Vecteur d'expression comprenant au moins une unité de transcription telle que définie selon l'une quelconque des revendications 1 à 3 et au moins un site de clonage permettant l'intégration d'un acide nucléotidique codant pour une protéine d'intérêt notamment choisie parmi le groupe constitué des protéines de la coagulation, des immunoglobulines, des cytokines, des hormones, des facteurs de croissance ou facteurs du complément et de toute protéine de fusion,
ledit vecteur d'expression comprenant notamment un gène de résistance eucaryote, un gène de résistance bactérien, une origine de réplication bactérienne et une unité dédiée à l'amplification génique.

**5.** Vecteur d'expression comprenant au moins une unité de transcription telle que définie selon l'une quelconque des revendications 1 à 3 et au moins un site pour la recombinaison site spécifique permettant l'intégration d'un acide nucléotidique codant pour une protéine d'intérêt notamment choisie parmi le groupe constitué des protéines de la coagulation, des immunoglobulines, des cytokines, des hormones, des facteurs de croissance ou facteurs du complément et de toute protéine de fusion,
ledit vecteur d'expression comprenant notamment un gène de résistance eucaryote, un gène de résistance bactérien, une origine de réplication bactérienne et une unité dédiée à l'amplification génique.

**6.** Cellule hôte comprenant un vecteur d'expression tel que défini dans l'une quelconque des revendications 4 à 5, ladite cellule hôte étant notamment une lignée cellulaire CHO choisie parmi CHO-S, CHO ou une lignée cellulaire HEK.

**7.** Utilisation *in vitro* d'un vecteur d'expression selon la revendication 3 pour transfecter une cellule hôte.

**8.** Système d'expression comprenant un vecteur d'expression tel que défini selon l'une quelconque des revendications 4 à 5 et une cellule hôte telle que définie selon la revendication 6 permettant l'expression d'une protéine d'intérêt codée par un acide nucléotidique.

**9.** Utilisation *in vitro* d'un vecteur d'expression comprenant au moins une unité de transcription selon l'une quelconque des revendications 1 à 3 dans une cellule hôte selon la revendication 5 pour produire une protéine d'intérêt codée par un acide nucléotidique, ladite protéine étant produite avec un titre plus élevé que dans un vecteur d'expression comprenant au moins un promoteur RSV, un intron pCInéo, une séquence de polyadénylation, un gène de résistance eucaryote, un gène de résistance bactérien, une origine de réplication bactérienne et une unité dédiée à l'amplification génique, ledit vecteur de référence comportant la même séquence nucléotidique.

**10.** Procédé de production *in vitro* d'une protéine recombinante d'intérêt comprenant les étapes de :

- introduction du vecteur d'expression comprenant au moins une unité de transcription selon l'une quelconque des revendications 1 à 3 et un ADNc codant pour une protéine d'intérêt dans une cellule hôte,
- extraction et purification de ladite protéine d'intérêt,
- optionnellement sélection et identification des cellules hôtes obtenues exprimant de manière stable ladite protéine d'intérêt.

**Patentansprüche**

1. Transkriptionseinheit, bestehend aus einem Polynukleotid, umfassend die folgenden regulatorischen Elemente:

   - den hCMVie-Virus-Enhancer, wobei der Enhancer die Nukleotidsequenz SEQ ID NO: 1 aufweist, oder eine Nukleinsäure, die mindestens 70 % Sequenzidentität mit der Sequenz SEQ ID NO: 1 aufweist und im Wesentlichen Transkriptionsaktivierungseigenschaften besitzt, und
   - die Promotorregion von:

     - β-Actin, wobei die Promotorregion die Nukleotidsequenz SEQ ID NO: 3 aufweist oder eine Nukleinsäure, die mindestens 70 % Sequenzidentität mit der Sequenz SEQ ID NO: 3 aufweist und eine Promotoraktivität besitzt, und

   - eine Nukleinsäure, die sich stromabwärts der Promotorregion und stromaufwärts der Translationsinitiationsstelle befindet, wobei die Nukleinsäure die regulatorische Region R des 5' Long Terminal Repeat (LTR) des HTLV-1-Virus und die 5'-UTR-Region des eukaryotischen Initiationsfaktor-4GI(eIF4GI)-Gens umfasst, wobei die Nukleotidsäure die Sequenz SEQ ID NO: 8 aufweist, oder eine Nukleotidsäure, die mindestens 70 % Sequenzidentität mit der SEQ ID NO: 8 aufweist, die mRNA-Stabilisierungs- und Translationsvermittler-Eigenschaften aufweist.

2. Transkriptionseinheit nach Anspruch 1, wobei das Polynukleotid die Sequenz SEQ ID NO: 25 aufweist.

3. Transkriptionseinheit nach Anspruch 1, wobei das Polynukleotid auch ein Intron umfasst, wobei das Intron aus Folgenden ausgewählt ist:

   - dem Intron des Elongation-Factor-1α(EF1α)-Gens, das die Nukleotidsequenz SEQ ID NO: 11 aufweist, oder eine Nukleotidsequenz, die mindestens 70 % Sequenzidentität mit der SEQ ID NO: 11 aufweist,
   - dem murinen ROSA-Intron, das die Nukleotidsequenz SEQ ID NO: 12 aufweist, oder eine Nukleotidsequenz, die mindestens 70 % Sequenzidentität mit der SEQ ID NO: 12 aufweist,
   - dem humanen ROSA-Intron, das die Nukleotidsequenz SEQ ID NO: 13 aufweist, oder eine Nukleotidsequenz, die mindestens 70 % Sequenzidentität mit der SEQ ID NO: 13 aufweist,

   wobei das Intron:

   (i) stromabwärts der 5'-UTR-Region und stromaufwärts der Translationsinitiationsstelle, oder
   (ii) stromabwärts des Promotors und stromaufwärts der 5'-UTR-Region oder
   (iii) nach der Translationsinitiationsstelle und innerhalb einer codierenden Sequenz oder
   (iv) zwischen dem Stoppcodon der codierenden Sequenz und dem Polyadenylierungssignal angeordnet ist,

   wobei das Polynukleotid insbesondere eine der folgenden Sequenzen aufweist: SEQ ID NO: 67, SEQ ID NO: 68, SEQ ID NO: 69.

4. Expressionsvektor, umfassend mindestens eine Transkriptionseinheit nach einem der Ansprüche 1 bis 3 und mindestens eine Klonierungsstelle, die die Integration einer Nukleotidsäure ermöglicht, die für ein interessierendes Protein kodiert, insbesondere ausgewählt aus der Gruppe bestehend aus Koagulationsproteinen, Immunglobulinen, Cytokinen, Hormonen, Wachstumsfaktoren oder Komplementfaktoren und einem beliebigen Fusionsprotein,
   wobei der Expressionsvektor insbesondere ein eukaryotisches Resistenzgen, ein bakterielles Resistenzgen, einen bakteriellen Replikationsursprung und eine Einheit zur Genamplifikation umfasst.

5. Expressionsvektor, umfassend mindestens eine Transkriptionseinheit nach einem der Ansprüche 1 bis 3 und mindestens eine Stelle für die ortsspezifische Rekombination, die die Integration einer Nukleotidsäure ermöglicht, die für ein interessierendes Protein kodiert, insbesondere ausgewählt aus der Gruppe bestehend aus Koagulationsproteinen, Immunglobulinen, Cytokinen, Hormonen, Wachstumsfaktoren oder Komplementfaktoren und einem beliebigen Fusionsprotein,
   wobei der Expressionsvektor insbesondere ein eukaryotisches Resistenzgen, ein bakterielles Resistenzgen, einen bakteriellen Replikationsursprung und eine Einheit zur Genamplifikation umfasst.

6. Wirtszelle, umfassend einen Expressionsvektor nach einem der Ansprüche 4 bis 5, wobei die Wirtszelle eine CHO-Zelllinie ist, die aus CHO-S, CHO oder einer HEK-Zelllinie ausgewählt ist.

**7.** In-vivo-Verwendung eines Expressionsvektors nach Anspruch 3 zum Transfizieren einer Wirtszelle.

**8.** Expressionssystem, umfassend einen Expressionsvektor nach einem der Ansprüche 4 bis 5 und eine Wirtszelle nach Anspruch 6, das die Expression eines interessierenden Proteins ermöglicht, das von einer Nukleotidsäure kodiert wird.

**9.** In-vitro-Verwendung eines Expressionsvektors, umfassend mindestens eine Transkriptionseinheit nach einem der Ansprüche 1 bis 3 in einer Wirtszelle nach Anspruch 5 zur Herstellung eines interessierenden Proteins, das von einer Nukleotidsäure kodiert wird, wobei das Protein mit einem höheren Titer produziert wird als in einem Expressionsvektor, umfassend mindestens einen RSV-Promotor, ein pCIneo-Intron, eine Polyadenylierungssequenz, ein eukaryotisches Resistenzgen, ein bakterielles Resistenzgen, einen bakteriellen Replikationsursprung und eine Einheit zur Genamplifikation, wobei der Referenzvektor die gleiche Nukleotidsequenz umfasst.

**10.** Verfahren zur *In*-vitro-Herstellung eines interessierenden rekombinanten Proteins, umfassend die Schritte:

- Einführen des Expressionsvektors, umfassend mindestens eine Transkriptionseinheit nach einem der Ansprüche 1 bis 3 und eine cDNA, die für ein interessierenden Protein in einer Wirtszelle kodiert,
- Extrahieren und Reinigen des interessierenden Proteins,
- gegebenenfalls Auswählen und Identifizieren der erhaltenen Wirtszellen, die das interessierende Protein stabil exprimieren.

## Claims

**1.** Transcription unit consisting of a polynucleotide comprising the following regulatory elements:

- the hCMVie virus enhancer, said enhancer having the nucleotide sequence SEQ ID NO: 1, or a nucleic acid having at least 70% sequence identity with the sequence SEQ ID NO: 1 and possessing properties of transcription activation, and
- promoter region of:

  - β-actin, said promoter region having the nucleotide sequence SEQ ID NO: 3, or a nucleic acid having at least 70% sequence identity with the sequence SEQ ID NO: 3 and possessing a promoter activity, and

- a nucleic acid located downstream of said promoter region and upstream of the translation initiation site, said nucleic acid comprising the regulatory region R of the 5' Long Terminal Repeat (LTR) of the HTLV-1 virus and the 5' UTR region of the eukaryotic Initiation Factor 4GI (eIF4GI) gene, said nucleic acid having the nucleotide sequence SEQ ID NO: 8, or a nucleic acid having at least 70% sequence identity with SEQ ID NO: 8 having properties of stabilization of the mRNA and the translation facilitator.

**2.** Transcription unit according to claim 1, wherein said polynucleotide has the sequence SEQ ID NO: 25.

**3.** Transcription unit according to claim 1, said polynucleotide also comprising an intron, said intron being selected from the following:

- the intron of the Elongation Factor 1α (EF1α) gene having the nucleotide sequence SEQ ID NO: 11, or a nucleic acid having at least 70% sequence identity with SEQ ID NO: 11,
- the murine ROSA intron having the nucleotide sequence SEQ ID NO: 12, or a nucleic acid having at least 70% sequence identity with SEQ ID NO: 12,
- the human ROSA intron having the nucleotide sequence SEQ ID NO: 13, or a nucleic acid having at least 70% sequence identity with SEQ ID NO: 13,

said intron being located:

(i) downstream of the 5' UTR region and upstream of the translation initiation site, or
(ii) downstream of the promoter and upstream of the 5' UTR region, or

(iii) after the translation initiation site and within a coding sequence, or
(iv) between the stop codon of the coding sequence and the polyadenylation signal,

said polynucleotide in particular having one of the following sequences: SEQ ID NO: 67, SEQ ID NO: 68, SEQ ID NO: 69.

**4.** Expression vector comprising at least one transcription unit as defined according to any one of claims 1 to 3 and at least one cloning site allowing integration of a nucleic acid coding for a protein of interest in particular selected from the group consisting of the proteins participating in coagulation, immunoglobulins, cytokines, hormones, growth factors or complement factors and any fusion protein,
said expression vector comprising in particular a eukaryotic resistance gene, a bacterial resistance gene, a bacterial replication origin and a unit dedicated to gene amplification.

**5.** Expression vector comprising at least one transcription unit as defined according to any one of claims 1 to 3 and at least one site for site-specific recombination allowing integration of a nucleic acid coding for a protein of interest in particular selected from the group consisting of the proteins participating in coagulation, immunoglobulins, cytokines, hormones, growth factors or complement factors and any fusion protein,
said expression vector comprising in particular a eukaryotic resistance gene, a bacterial resistance gene, a bacterial replication origin and a unit dedicated to gene amplification.

**6.** Host cell comprising an expression vector as defined in any one of claims 4 to 5, said host cell being in particular a CHO cell line selected from CHO-S, CHO or a HEK cell line.

**7.** *In vitro* use of an expression vector according to claim 3 for transfecting a host cell.

**8.** Expression system comprising an expression vector as defined according to any one of claims 4 to 5 and a host cell as defined according to claim 6 permitting expression of a protein of interest encoded by a nucleic acid.

**9.** *In vitro* use of an expression vector comprising at least one transcription unit according to any one of claims 1 to 3 in a host cell according to claim 5 for producing a protein of interest encoded by a nucleic acid, said protein being produced with a titre higher than in an expression vector comprising at least one RSV promoter, a pCIneo intron, a polyadenylation sequence, a eukaryotic resistance gene, a bacterial resistance gene, a bacterial replication origin and a unit dedicated to gene amplification, said reference vector comprising the same nucleotide sequence.

**10.** Method for *in vitro* production of a recombinant protein of interest comprising the steps of:

- introducing the expression vector comprising at least one transcription unit according to any one of claims 1 to 3 and a cDNA coding for a protein of interest into a host cell,
- extracting and purifying said protein of interest,
- optionally selecting and identifying the host cells obtained that stably express said protein of interest.

Figure 1

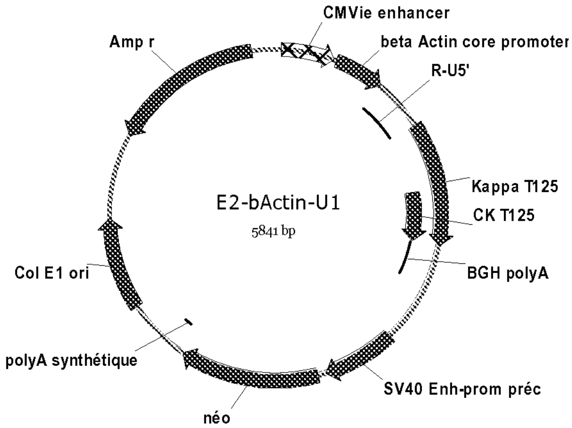


Figure 2

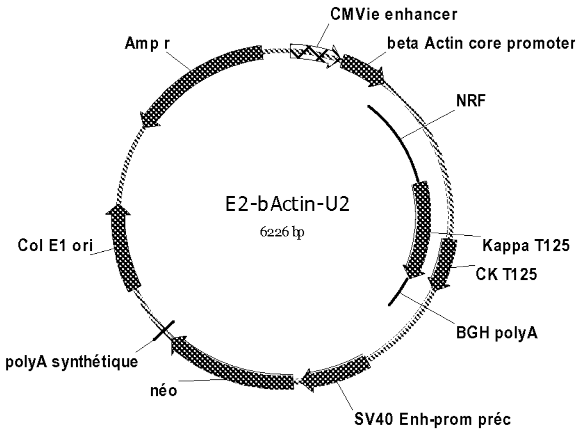


Figure 3

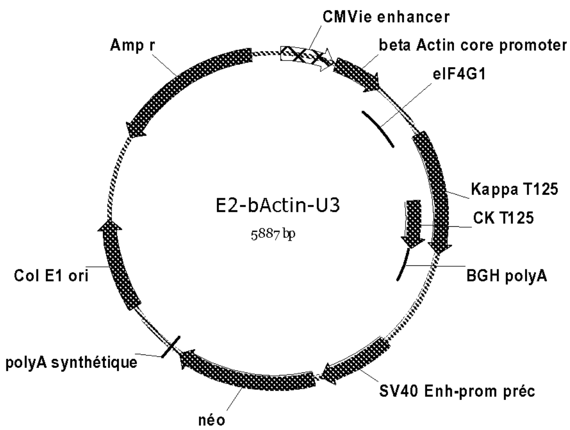

Figure 4

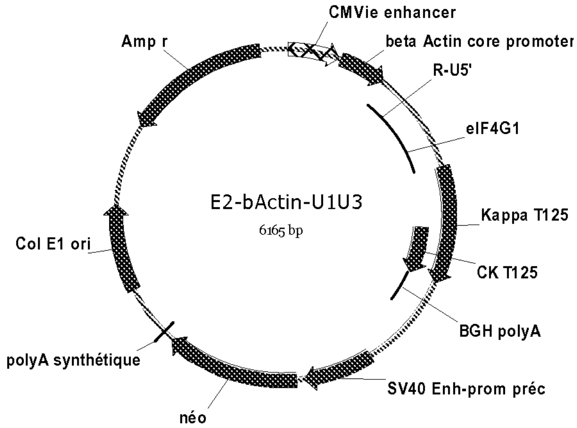


Figure 5

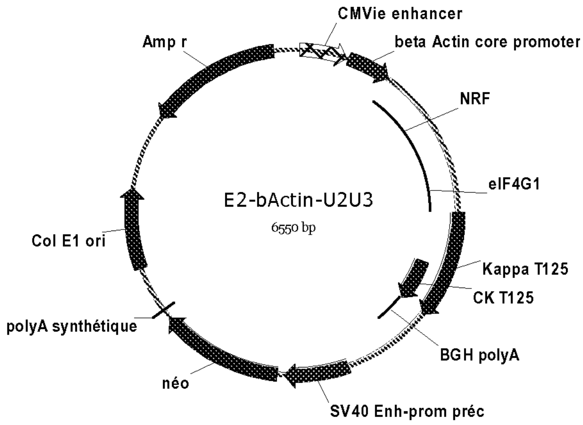


Figure 6

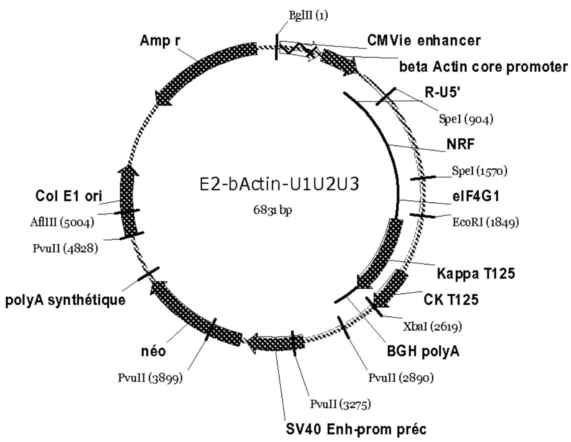

Figure 7

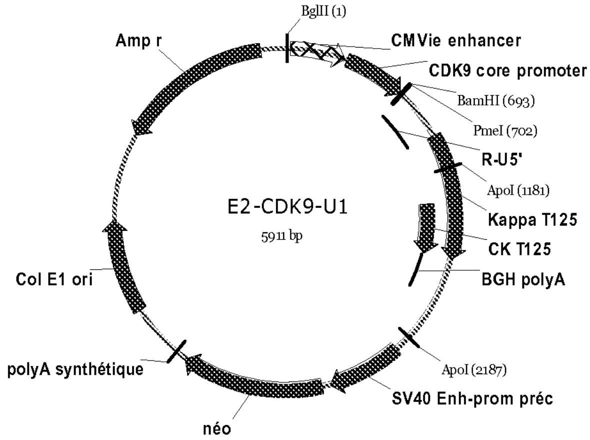


Figure 8

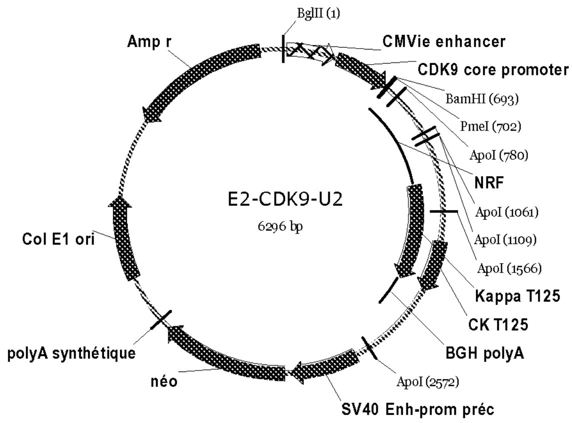


Figure 9

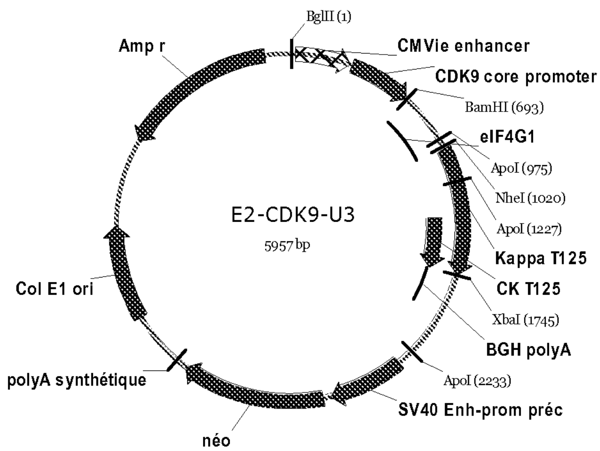

Figure 10

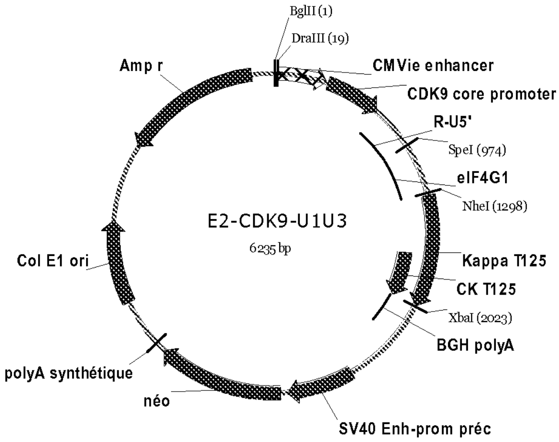


Figure 11

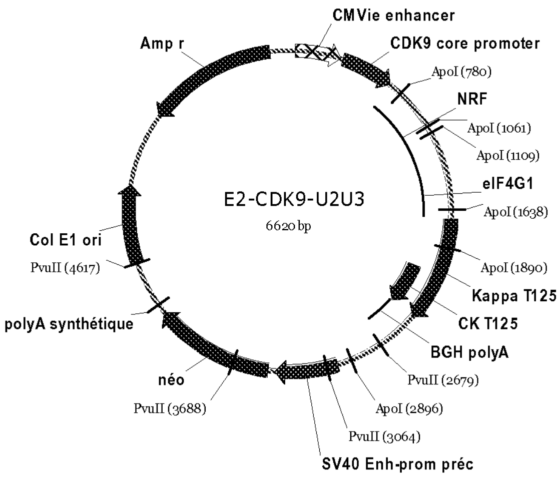


Figure 12

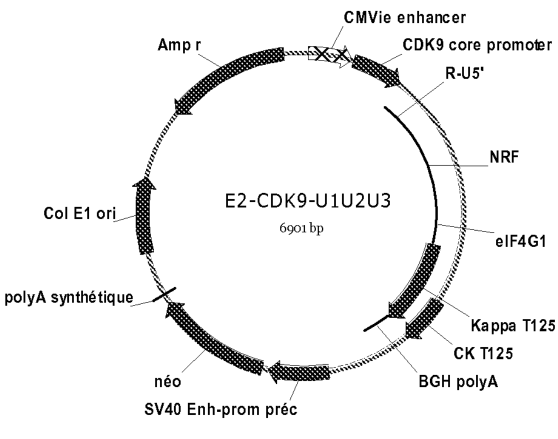

Figure 13

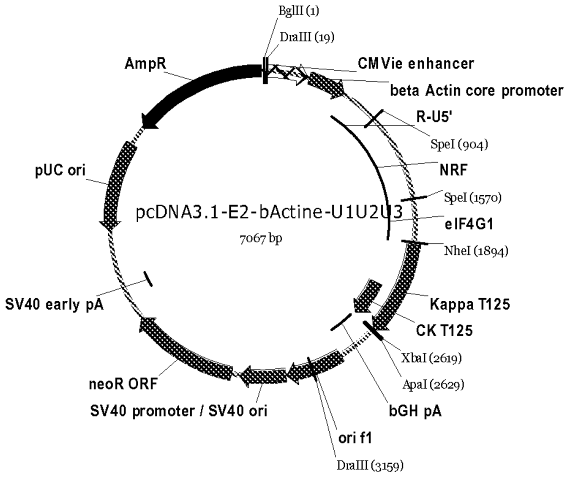


Figure 14

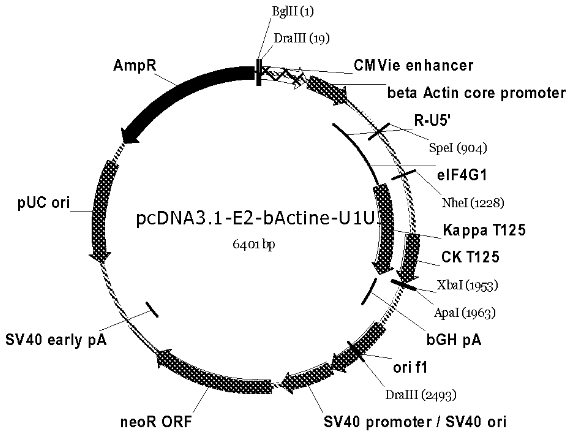


Figure 15

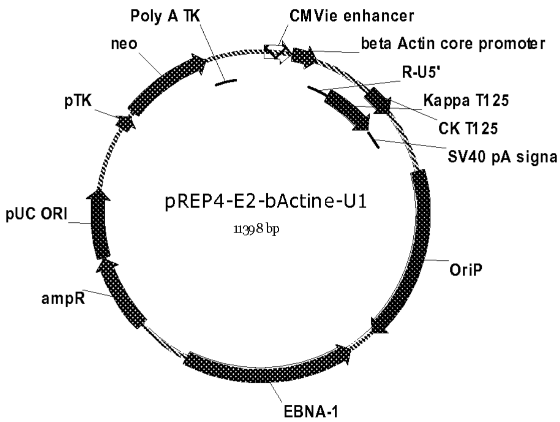

Figure 16

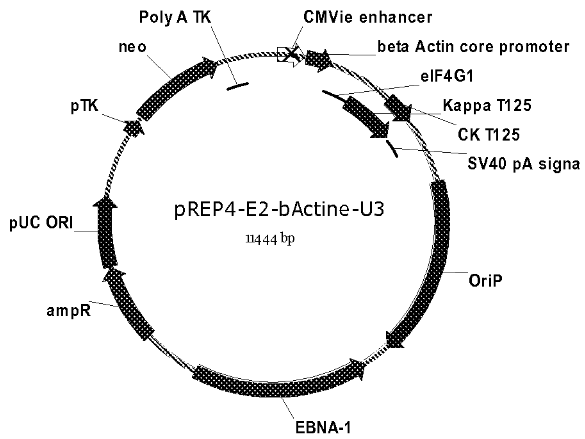


Figure 17

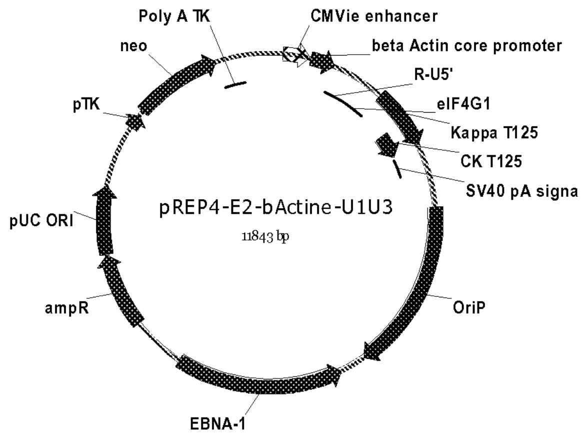


Figure 18

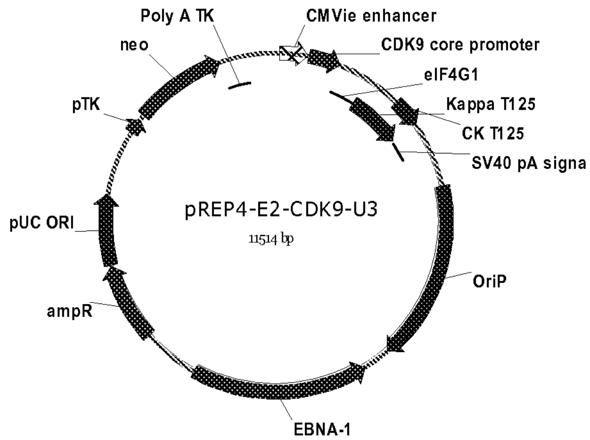

Figure 19

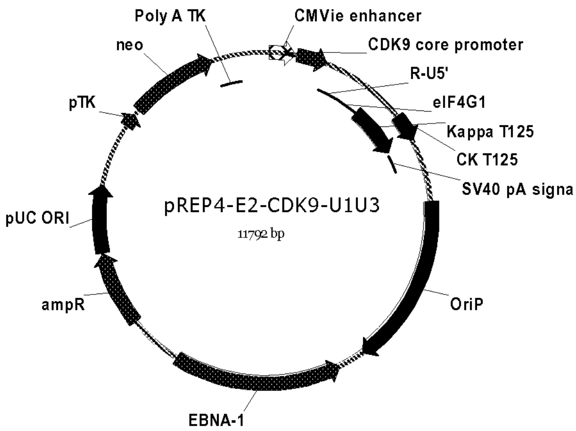


Figure 20

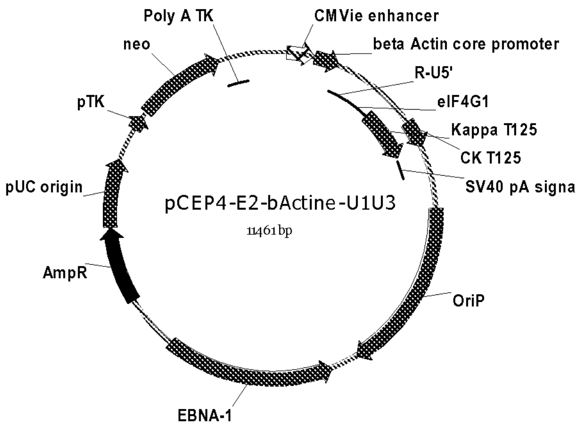


Figure 21

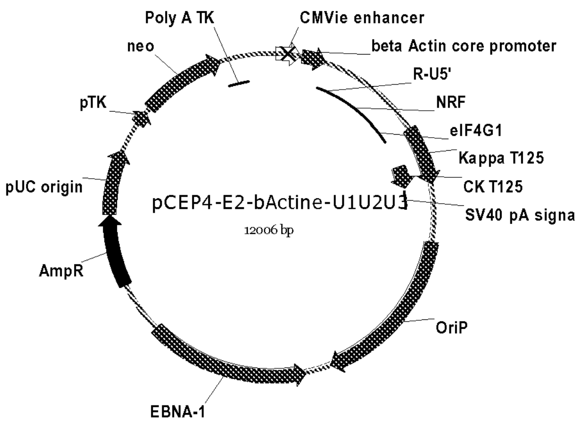

Figure 22

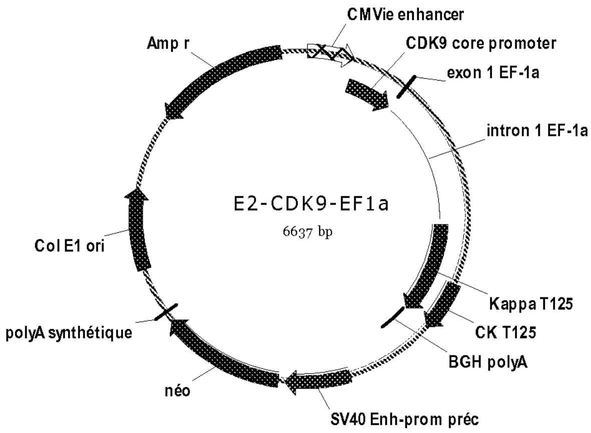


Figure 23

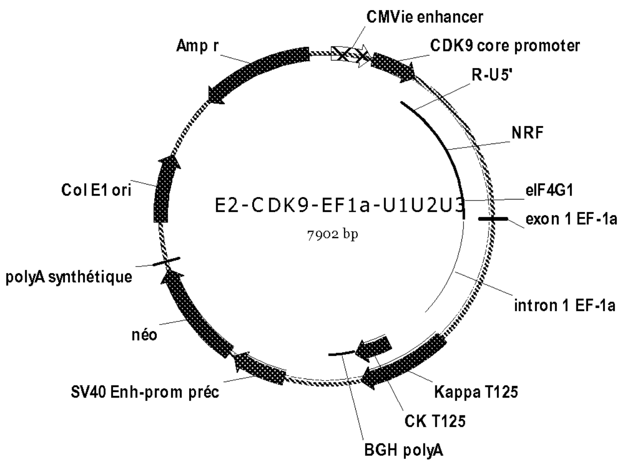


Figure 24

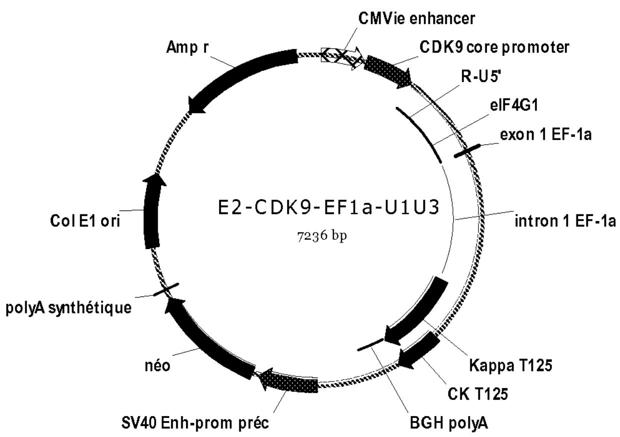

Figure 25

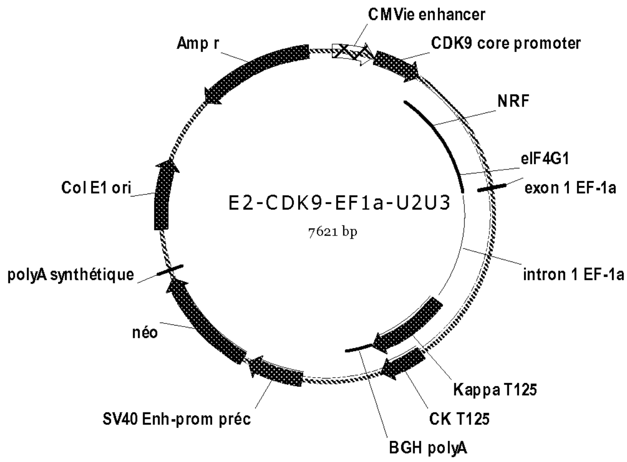


Figure 26

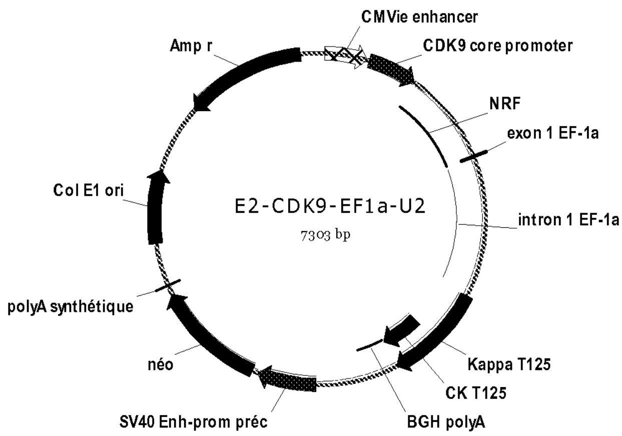


Figure 27

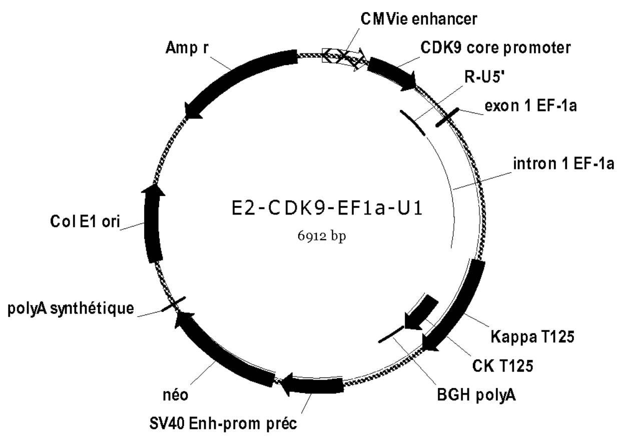

Figure 28

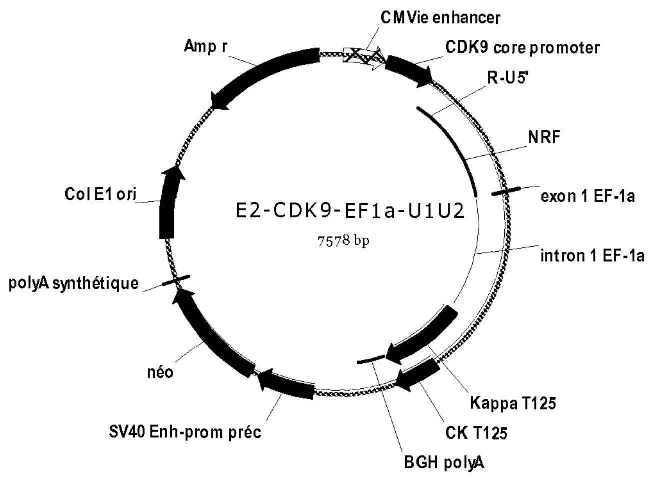


Figure 29

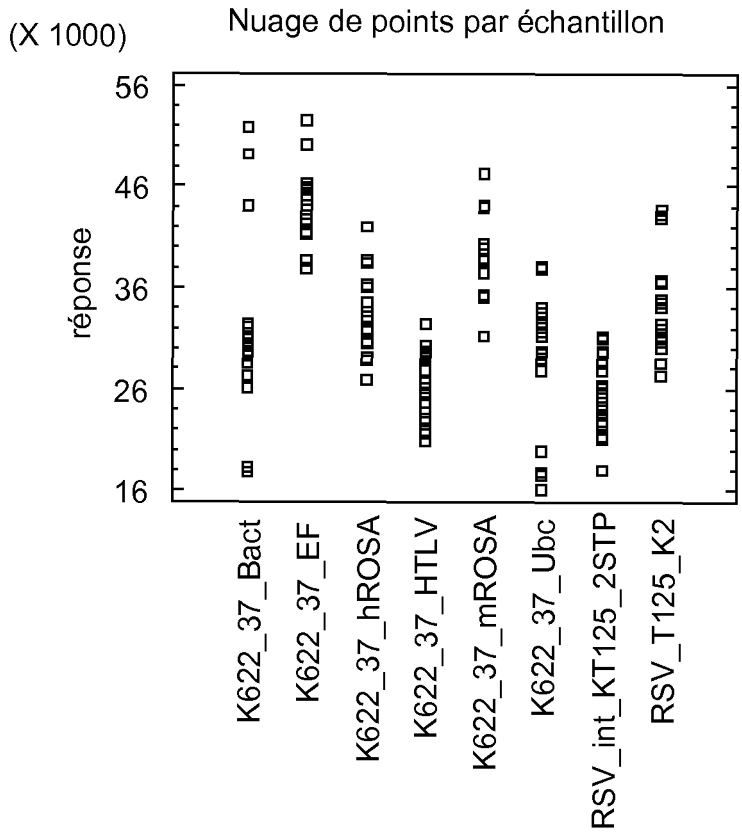

Figure 30

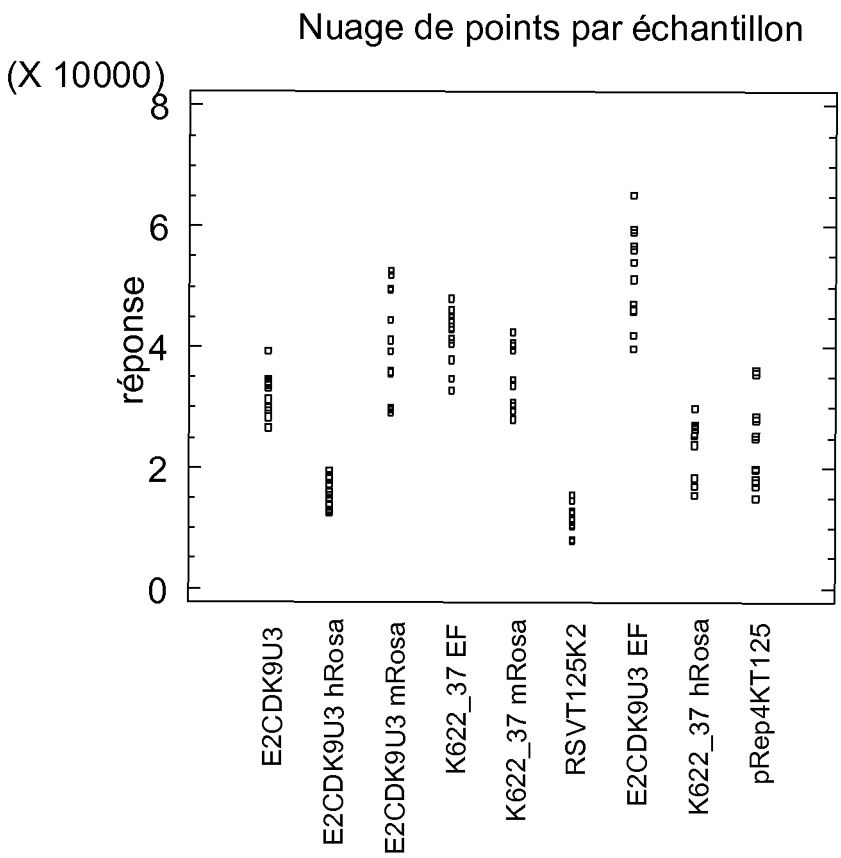
Nuage de points par échantillon
(X 10000)
8
6
4
2
0
réponse
E2CDK9U3
E2CDK9U3 hRosa
E2CDK9U3 mRosa
K622_37 EF
K622_37 mRosa
RSVT125K2
E2CDK9U3 EF
K622_37 hRosa
pRep4KT125

Figure 31

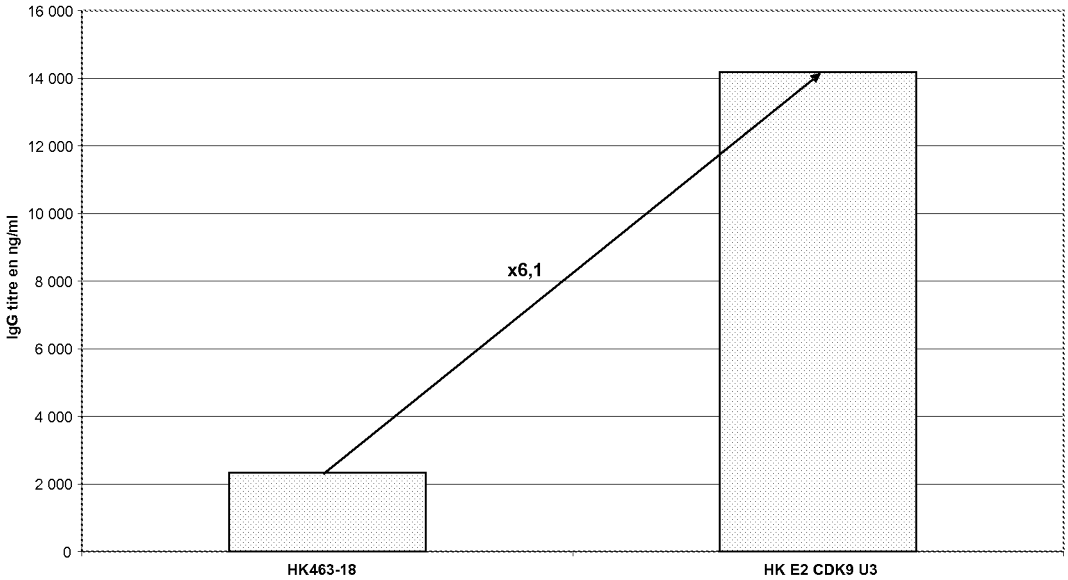
16 000
14 000
12 000
10 000
8 000
6 000
4 000
2 000
0
IgG titre en ng/ml
x6,1
HK463-18
HK E2 CDK9 U3

Figure 32

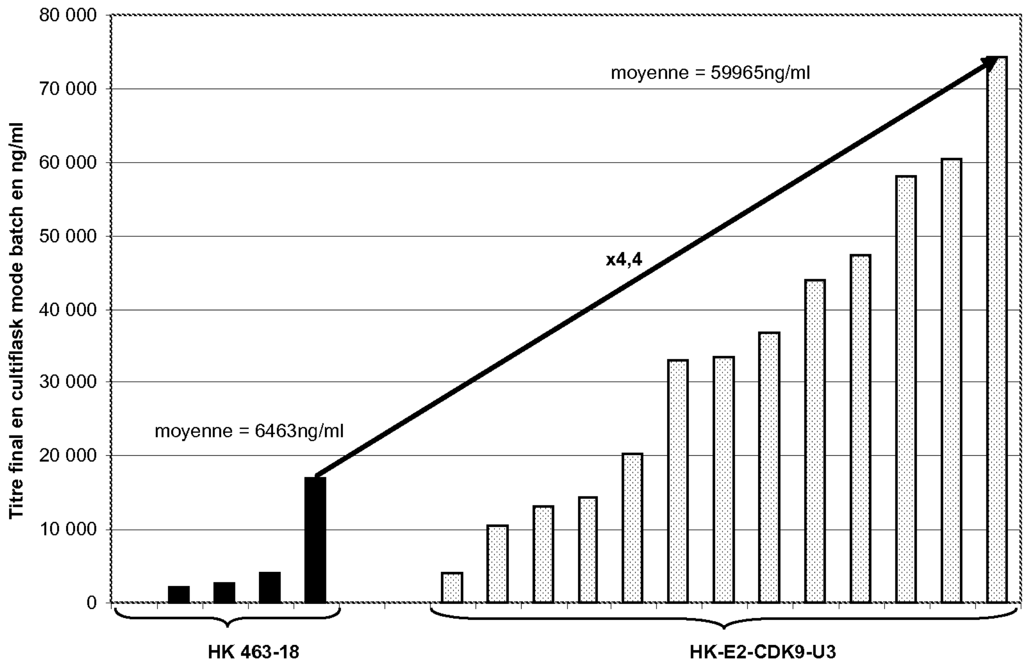
80 000
70 000
60 000
50 000
40 000
30 000
20 000
10 000
0
Titre final en cultiflask mode batch en ng/ml
moyenne = 59965ng/ml
x4,4
moyenne = 6463ng/ml
HK 463-18
HK-E2-CDK9-U3

Figure 33

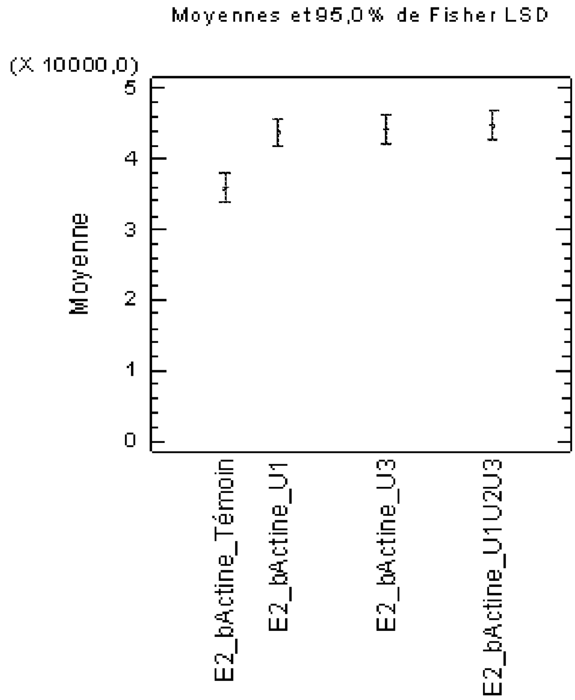


Figure 34

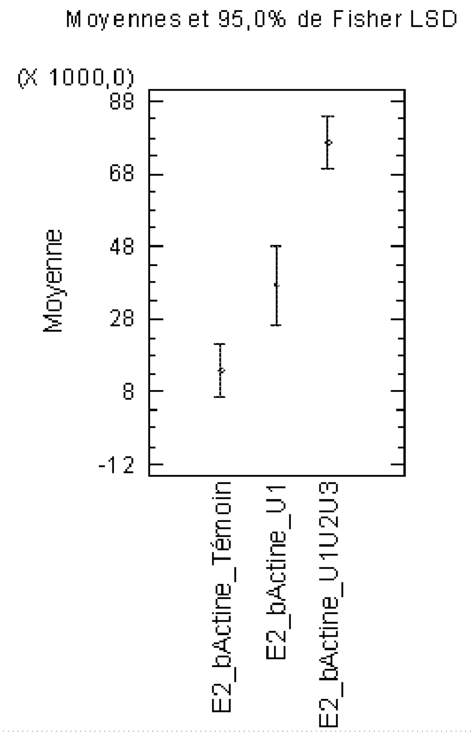

Figure 35

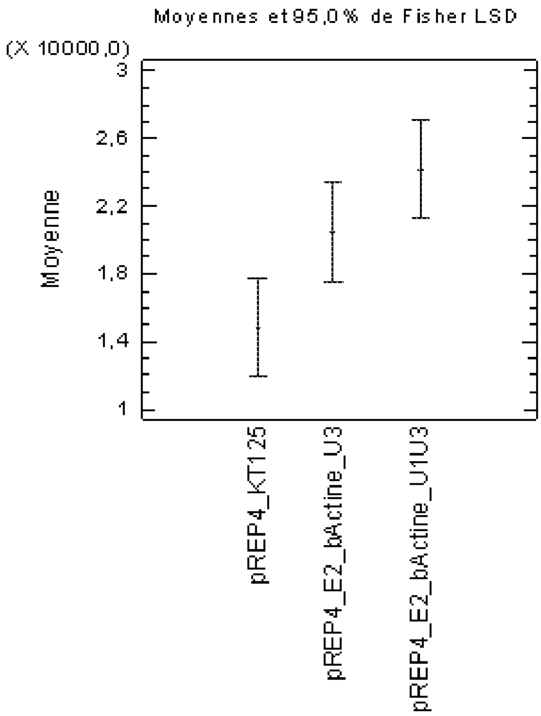


Figure 36

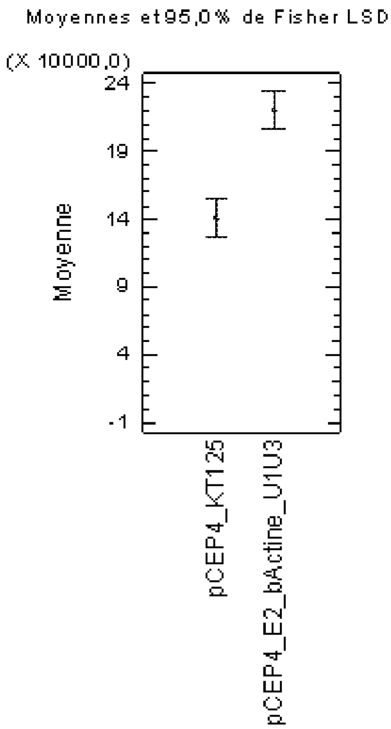

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2011062298 A1


### Littérature non-brevet citée dans la description


- **LIU et al.** *Gene,* 2000, vol. 252, 51-59
- **FRITZ et al.** *Sci. STKE,* 05 Décembre 2000, vol. 2000 (61), 11

SEQUENCE LISTING

<110> Laboratoire français du franctionnement et des biotechnologies

<120> UNITES DE TRANSCRIPTION ET LEUR UTILISATION DANS DES VECTEURS D'EXPRESSION

<130> WOB 11 CP LFB CHOS

<160> 75

<170> PatentIn version 3.5

<210> 1
<211> 306
<212> DNA
<213> Cytomegalovirus

<400> 1

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatgg                                                                306
```

<210> 2
<211> 352
<212> DNA
<213> Homo sapiens

<400> 2

```
catgcagcgg gacgcgccac cccgagcccc agctccggcg ccccggctcc ccgcgccccc      60
gatcggggcc gccgctagta gtggcggcgg cggaggcggg ggcagcggcg gcggcggcgg     120
aggcgcctct gcagctccgg ctccccctgg cctctcggga actacaagtc ccaggggggcc    180
tggcggtggg cggcgggcgg aagaggcggg gtcggcgccg cgaggccgga agtggccgtg     240
gaggcggaag tggcgcggcc gcggaggggc ctggagtgcg gcggcggcgg gacccggagc     300
aggagcggcg gcagcagcga ctgggggcgg cggcggcgcg ttggaggcgg cc             352
```

<210> 3
<211> 283
<212> DNA
<213> Gallus gallus

<400> 3

```
catggtcgag gtgagcccca cgttctgctt cactctcccc atctcccccc cctccccacc      60
cccaattttg tatttattta ttttttaatt attttgtgca gcgatggggg cggggggggg     120
gggggggcgc gcgccaggcg gggcggggcg gggcgagggg cggggcgggg cgaggcggag     180
```

aggtgcggcg gcagccaatc agagcggcgc gctccgaaag tttcctttta tggcgaggcg 240

gcggcggcgg cggccctata aaaagcgaag cgcgcggcgg gcg 283

<210> 4
<211> 267
<212> DNA
<213> Homo sapiens

<400> 4

ggctcgcatc tctccttcac gcgcccgccg ccctacctga ggccgccatc cacgccggtt 60

gagtcgcgtt ctgccgcctc ccgcctgtgg tgcctcctga actgcgtccg ccgtctaggt 120

aagtttaaag ctcaggtcga gaccgggcct ttgtccggcg ctcccttgga gcctacctag 180

actcagccgg ctctccacgc tttgcctgac cctgcttgct caactctacg tctttgtttc 240

gttttctgtt ctgcgccgtt acagatc 267

<210> 5
<211> 653
<212> DNA
<213> Homo sapiens

<400> 5

cagagtaatg acatggttcc ttccatcctc caaaggtgac caataatagt ttgtaagtat 60

cattatgaac taatgaattt tcaacatatt tgatatattt caatccattg ccatcattgt 120

tcttatcgat atttgagttg gctcactttg ccagtaagag tctattcaaa ttggcttctg 180

agtccatttg acacaacacc tttgatcttt gacagtttcc ttggttttag gtgctagatg 240

atttctcagg ctcaccttag acatttcctg ccacagactt agaatcagcc atttctctaa 300

ggaccctgat tccatttcat gagaaatgat agagaccaca atcaaaacaa gtcatgaatt 360

tatactgata ttttcaattc aaattaaaga tgaggttttt gctaaatttt tttgagttta 420

tatttgtatg tcttatgctg aaaaatcttg tttcctaatt agtaacataa ttattcattt 480

gatgggtaaa tattttaggg ccgattcttt ggttttatag ccaagatacc ctgttgataa 540

agtcttgtgg gagcaattat aagactggct tattttgaag ctttttaaaa aagacatcct 600

tacctgtttt aactgtagat tatattaact taaataggta cagcccacgc ttg 653

<210> 6
<211> 314
<212> DNA
<213> Homo sapiens

<400> 6

gctggtgggt agggatgagg gagggagggg cattgtgatg tacagggctg ctctgtgaga 60
tcaagggtct cttaagggtg ggagctgggg cagggactac gagagcagcc agatgggctg 120
aaagtggaac tcaaggggtt tctggcacct acctacctgc ttcccgctgg ggggtgggga 180
gttggcccag agtcttaaga ttggggcagg gtggagaggt gggctcttcc tgcttcccac 240
tcatcttata gctttctttc cccagatccg aattcgagat ccaaaccaag gaggaaagga 300
tatcacagag gaga 314

<210> 7
<211> 933
<212> DNA
<213> Artificial Sequence

<220>
<223> U1U2

<400> 7

ggctcgcatc tctccttcac gcgcccgccg ccctacctga ggccgccatc cacgccggtt 60
gagtcgcgtt ctgccgcctc ccgcctgtgg tgcctcctga actgcgtccg ccgtctaggt 120
aagtttaaag ctcaggtcga gaccgggcct ttgtccggcg ctcccttgga gcctacctag 180
actcagccgg ctctccacgc tttgcctgac cctgcttgct caactctacg tctttgtttc 240
gttttctgtt ctgcgccgtt acagatcact agtgtttaaa cagagtaatg acatggttcc 300
ttccatcctc caaaggtgac caataatagt ttgtaagtat cattatgaac taatgaattt 360
tcaacatatt tgatatattt caatccattg ccatcattgt tcttatcgat atttgagttg 420
gctcactttg ccagtaagag tctattcaaa ttggcttctg agtccatttg acacaacacc 480
tttgatcttt gacagtttcc ttggttttag gtgctagatg atttctcagg ctcaccttag 540
acatttcctg ccacagactt agaatcagcc atttctctaa ggaccctgat tccatttcat 600
gagaaatgat agagaccaca atcaaaacaa gtcatgaatt tatactgata ttttcaattc 660
aaattaaaga tgaggttttt gctaaatttt tttgagttta tatttgtatg tcttatgctg 720
aaaaatcttg tttcctaatt agtaacataa ttattcattt gatgggtaaa tattttaggg 780
ccgattcttt ggttttatag ccaagatacc ctgttgataa agtcttgtgg gagcaattat 840
aagactggct tattttgaag ctttttaaaa aagacatcct tacctgtttt aactgtagat 900
tatattaact taaataggta cagcccacgc ttg 933

<210> 8
<211> 591
<212> DNA
<213> Artificial Sequence

<220>
<223> U1U3

<400> 8

```
ggctcgcatc tctccttcac gcgcccgccg ccctacctga ggccgccatc cacgccggtt      60
gagtcgcgtt ctgccgcctc ccgcctgtgg tgcctcctga actgcgtccg ccgtctaggt     120
aagtttaaag ctcaggtcga gaccgggcct ttgtccggcg ctcccttgga gcctacctag     180
actcagccgg ctctccacgc tttgcctgac cctgcttgct caactctacg tctttgtttc     240
gttttctgtt ctgcgccgtt acagatcact agttaacgct ggtgggtagg gatgagggag     300
ggaggggcat tgtgatgtac agggctgctc tgtgagatca agggtctctt aagggtggga     360
gctggggcag ggactacgag agcagccaga tgggctgaaa gtggaactca aggggtttct     420
ggcacctacc tacctgcttc ccgctggggg gtggggagtt ggcccagagt cttaagattg     480
gggcagggtg gagaggtggg ctcttcctgc ttcccactca tcttatagct ttctttcccc     540
agatccgaat tcgagatcca aaccaaggag gaaaggatat cacagaggag a              591
```

<210> 9
<211> 977
<212> DNA
<213> Artificial Sequence

<220>
<223> U2U3

<400> 9

```
cagagtaatg acatggttcc ttccatcctc caaaggtgac caataatagt ttgtaagtat        60
cattatgaac taatgaattt tcaacatatt tgatatattt caatccattg ccatcattgt       120
tcttatcgat atttgagttg gctcactttg ccagtaagag tctattcaaa ttggcttctg       180
agtccatttg acacaacacc tttgatcttt gacagtttcc ttggttttag gtgctagatg       240
atttctcagg ctcaccttag acatttcctg ccacagactt agaatcagcc atttctctaa       300
ggaccctgat tccatttcat gagaaatgat agagaccaca atcaaaacaa gtcatgaatt       360
tatactgata ttttcaattc aaattaaaga tgaggttttt gctaaatttt tttgagttta       420
tatttgtatg tcttatgctg aaaaatcttg tttcctaatt agtaacataa ttattcattt       480
gatgggtaaa tattttaggg ccgattcttt ggttttatag ccaagatacc ctgttgataa       540
agtcttgtgg gagcaattat aagactggct tattttgaag ctttttaaaa aagacatcct       600
tacctgtttt aactgtagat tatattaact taaataggta cagcccacgc ttgactagtt       660
aacgctggtg ggtagggatg agggagggag gggcattgtg atgtacaggg ctgctctgtg       720
agatcaaggg tctcttaagg gtgggagctg gggcagggac tacgagagca gccagatggg       780
ctgaaagtgg aactcaaggg gtttctggca cctacctacc tgcttcccgc tgggggggtgg       840
ggagttggcc cagagtctta agattggggc agggtggaga ggtgggctct tcctgcttcc       900
cactcatctt atagctttct ttccccagat ccgaattcga gatccaaacc aaggaggaaa       960
ggatatcaca gaggaga                                                      977
```

<210> 10
<211> 1257
<212> DNA
<213> Artificial Sequence

<220>
<223> U1U2U3

<400> 10

ggctcgcatc tctccttcac gcgcccgccg ccctacctga ggccgccatc cacgccggtt 60
gagtcgcgtt ctgccgcctc ccgcctgtgg tgcctcctga actgcgtccg ccgtctaggt 120
aagtttaaag ctcaggtcga gaccgggcct ttgtccggcg ctcccttgga gcctacctag 180
actcagccgg ctctccacgc tttgcctgac cctgcttgct caactctacg tctttgtttc 240
gttttctgtt ctgcgccgtt acagatcact agtgtttaaa cagagtaatg acatggttcc 300
ttccatcctc caaaggtgac caataatagt ttgtaagtat cattatgaac taatgaattt 360
tcaacatatt tgatatattt caatccattg ccatcattgt tcttatcgat atttgagttg 420
gctcactttg ccagtaagag tctattcaaa ttggcttctg agtccatttg acacaacacc 480
tttgatcttt gacagtttcc ttggttttag gtgctagatg atttctcagg ctcaccttag 540
acatttcctg ccacagactt agaatcagcc atttctctaa ggaccctgat tccatttcat 600
gagaaatgat agagaccaca atcaaaacaa gtcatgaatt tatactgata ttttcaattc 660
aaattaaaga tgaggttttt gctaaatttt tttgagttta tatttgtatg tcttatgctg 720
aaaaatcttg tttcctaatt agtaacataa ttattcattt gatgggtaaa tattttaggg 780
ccgattcttt ggttttatag ccaagatacc ctgttgataa agtcttgtgg gagcaattat 840
aagactggct tattttgaag ctttttaaaa aagacatcct tacctgtttt aactgtagat 900
tatattaact taaataggta cagcccacgc ttgactagtt aacgctggtg ggtagggatg 960
agggagggag gggcattgtg atgtacaggg ctgctctgtg agatcaaggg tctcttaagg 1020
gtgggagctg gggcagggac tacgagagca gccagatggg ctgaaagtgg aactcaaggg 1080
gtttctggca cctacctacc tgcttcccgc tgggggtgg ggagttggcc cagagtctta 1140
agattggggc agggtggaga ggtgggctct tcctgcttcc cactcatctt atagctttct 1200
ttccccagat ccgaattcga gatccaaacc aaggaggaaa ggatatcaca gaggaga 1257

<210> 11
<211> 939
<212> DNA
<213> Homo sapiens

<400> 11

gtaagtgccg tgtgtggttc ccgcgggcct ggcctcttta cgggttatgg cccttgcgtg 60
ccttgaatta cttccacctg gctgcagtac gtgattcttg atcccgagct tcgggttgga 120
agtgggtggg agagttcgag gccttgcgct taaggagccc cttcgcctcg tgcttgagtt 180
gaggcctggc ctgggcgctg ggccgccgc gtgcgaatct ggtggcacct tcgcgcctgt 240

ctcgctgctt tcgataagtc tctagccatt taaaattttt gatgacctgc tgcgacgctt 300

tttttctggc aagatagtct tgtaaatgcg ggccaagatc tgcacactgg tatttcggtt 360

tttggggccg cgggcggcga cggggcccgt gcgtcccagc gcacatgttc ggcgaggcgg 420

ggcctgcgag cgcggccacc gagaatcgga cgggggtagt ctcaagctgg ccggcctgct 480

ctggtgcctg gcctcgcgcc gccgtgtatc gccccgccct gggcggcaag gctggcccgg 540

tcggcaccag ttgcgtgagc ggaaagatgg ccgcttcccg gccctgctgc agggagctca 600

aaatggagga cgcggcgctc gggagagcgg gcgggtgagt cacccacaca aaggaaaagg 660

gcctttccgt cctcagccgt cgcttcatgt gactccacgg agtaccgggc gccgtccagg 720

cacctcgatt agttctcgag cttttggagt acgtcgtctt taggttgggg ggaggggttt 780

tatgcgatgg agtttcccca cactgagtgg gtggagactg aagttaggcc agcttggcac 840

ttgatgtaat tctccttgga atttgccctt tttgagtttg gatcttggtt cattctcaag 900

cctcagacag tggttcaaag ttttttttctt ccatttcag 939

<210> 12
<211> 351
<212> DNA
<213> Mus musculus

<400> 12

gtaggggatc gggactctgg cgggagggcg gcttggtgcg tttgcgggga tgggcggccg 60

cggcaggccc tccgagcgtg gtggagccgt tctgtgagac agccgggtac gagtcgtgac 120

gctggaaggg gcaagcgggt ggtgggcagg aatgcggtcc gccctgcagc aaccggaggg 180

ggagggagaa gggagcggaa aagtctccac cggacgcggc catggctcgg gggggggggg 240

gcagcggagg agcgcttccg gccgacgtct cgtcgctgat tggcttcttt tcctcccgcc 300

gtgtgtgaaa acacaattgt actaaccttc ttctctttcc tctcctgaca g 351

<210> 13
<211> 1217
<212> DNA
<213> Homo sapiens

<400> 13

gtaggggagc ggaactctgg tgggagggga ggtgcggtgc actgggggga tgggtggcta 60
ggggggccgt ctggtggctt gcgggggttg cctttcccgt gggaagtcgg gaacataatg 120
tttgttacgt tgggagggaa aggggtggct ggatgcaggc gggagggagg cccgccctgc 180
ggcaaccgga gggggaggga gaagggagcg gaaaatgctc gaaaccggac ggagccattg 240
ctctcgcaga gggaggagcg cttccggcta gcctcttgtc gccgattggc cgtttctcct 300
cccgccgtgt gtgaaaacac aaatggcgta ttctggttgg agtaaagctc ctgtcagtta 360
caccgtcggg agtacgcagc cgcttagcga ctctcgcgtt gccccctggg tggggcgggt 420

aggtaggtgg ggtgtagaga tgctgggtgt gcgggcgcgg ccggcctcct gcggcgggag 480
gggagggtca gtgaaattgg ctctggcgcg ggcgtcctcc caccctcccc ttccttcggg 540
ggagtcggtt tacccgccgc ctgcttgtct tcgacacctg attggctgtc gaagctgtgg 600
gaccgggccc ttgctactgg ctcgagtctc acatgagcga aaccactgcg cggggcgcgg 660
gggtggcggg gaggcgggcg ttggtacggt cctccccgag gccgagcgcc gcagtgtctg 720
gccccgcgcc cctgcgcaac gtggcaggaa gcgcgcgctg gaggcggggg cgggctgccg 780
gccgagactt ctggatggcg gcggccgcgg ctccgccccg ggttcccacc gcctgaaggg 840
cgagacaagc ccgacctgct acaggcactc gtgggggtgg gggaggagcg ggggtcggtc 900
cggctggttt gtgggtggga ggcgcttgtt ctccaaaaac cggcgcgagc tgcaatcctg 960
agggagctgc ggtggaggag gtggagagaa ggccgcaccc ttctgggcag gggagggga 1020
gtgccgcaat acctttatgg gagttctttg ctgcctcccg tcttgtaagg accgccctgg 1080
gcctggaaga agccctccct cctttcctcc tcgcgtgatc tcgtcatcgc ctccatgtcg 1140
agtcgcttct cgattatggg cgggattctt ttgcctagac aattgtacta accttcttct 1200
ctttcctctc ctgacag 1217

<210> 14
<211> 951
<212> DNA
<213> Artificial Sequence

<220>
<223> CMV-CDK9-U1

<400> 14

```
gcgttacata acttacggta aatggcccgc ctggctgacc gcccaacgac ccccgcccat        60
tgacgtcaat aatgacgtat gttcccatag taacgccaat agggactttc cattgacgtc       120
aatgggtgga gtatttacgg taaactgccc acttggcagt acatcaagtg tatcatatgc       180
caagtacgcc ccctattgac gtcaatgacg gtaaatggcc cgcctggcat tatgcccagt       240
acatgacctt atgggacttt cctacttggc agtacatcta cgtattagtc atcgctatta       300
ccatggcccg ggtcgcgaca tgcagcggga cgcgccaccc cgagccccag ctccggcgcc       360
ccggctcccc gcgcccccga tcggggccgc cgctagtagt ggcggcggcg gaggcggggg       420
cagcggcggc ggcggcggag gcgcctctgc agctccggct ccccctggcc tctcgggaac       480
tacaagtccc agggggcctg gcggtgggcg gcgggcggaa gaggcggggt cggcgccgcg       540
aggccggaag tggccgtgga ggcggaagtg gcgcggccgc ggaggggcct ggagtgcggc       600
ggcggcggga cccggagcag gagcggcggc agcagcgact gggggcggcg gcggcgcgtt       660
ggaggcggcc ggatccgttt aaacggctcg catctctcct tcacgcgccc gccgccctac       720
ctgaggccgc catccacgcc ggttgagtcg cgttctgccg cctcccgcct gtggtgcctc       780
ctgaactgcg tccgccgtct aggtaagttt aaagctcagg tcgagaccgg gcctttgtcc       840
ggcgctccct tggagcctac ctagactcag ccggctctcc acgctttgcc tgaccctgct       900
tgctcaactc tacgtctttg tttcgttttc tgttctgcgc cgttacagat c                951
```

<210> 15
<211> 1336
<212> DNA
<213> Artificial Sequence

<220>
<223> CMV-CDK9-U2

<400> 15

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
tacaagtccc agggggcctg gcggtgggcg gcgggcggaa gaggcggggt cggcgccgcg     540
aggccggaag tggccgtgga ggcggaagtg gcgcggccgc ggaggggcct ggagtgcggc     600
ggcggcggga cccggagcag gagcggcggc agcagcgact gggggcggcg gcggcgcgtt     660
ggaggcggcc ggatccgttt aaacagagta atgacatggt tccttccatc ctccaaaggt     720
gaccaataat agtttgtaag tatcattatg aactaatgaa ttttcaacat atttgatata     780
tttcaatcca ttgccatcat tgttcttatc gatatttgag ttggctcact ttgccagtaa     840
gagtctattc aaattggctt ctgagtccat ttgacacaac acctttgatc tttgacagtt     900
tccttggttt taggtgctag atgatttctc aggctcacct tagacatttc ctgccacaga     960
cttagaatca gccatttctc taaggaccct gattccattt catgagaaat gatagagacc    1020
acaatcaaaa caagtcatga atttatactg atattttcaa ttcaaattaa agatgaggtt    1080
tttgctaaat ttttttgagt ttatatttgt atgtcttatg ctgaaaaatc ttgtttccta    1140
attagtaaca taattattca tttgatgggt aaatatttta gggccgattc tttggtttta    1200
tagccaagat accctgttga taaagtcttg tgggagcaat tataagactg gcttattttg    1260
aagcttttta aaaaagacat ccttacctgt tttaactgta gattatatta acttaaatag    1320
gtacagccca cgcttg                                                    1336
```

<210> 16
<211> 997
<212> DNA
<213> Artificial Sequence

<220>
<223> CMV-CDK9-U3

<400> 16

gcgttacata acttacggta aatggcccgc ctggctgacc gcccaacgac ccccgcccat 60
tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct ccccctggcc tctcgggaac 480
tacaagtccc agggggcctg gcggtgggcg gcgggcggaa gaggcggggt cggcgccgcg 540
aggccggaag tggccgtgga ggcggaagtg gcgcggccgc ggaggggcct ggagtgcggc 600
ggcggcggga cccggagcag gagcggcggc agcagcgact gggggcggcg gcggcgcgtt 660
ggaggcggcc ggatccgttt aacgctggtg ggtagggatg agggagggag gggcattgtg 720
atgtacaggg ctgctctgtg agatcaaggg tctcttaagg gtgggagctg gggcagggac 780
tacgagagca gccagatggg ctgaaagtgg aactcaaggg gtttctggca cctacctacc 840
tgcttcccgc tgggggtgg ggagttggcc cagagtctta agattggggc agggtggaga 900
ggtgggctct tcctgcttcc cactcatctt atagctttct ttccccagat ccgaattcga 960
gatccaaacc aaggaggaaa ggatatcaca gaggaga 997

<210> 17
<211> 1617
<212> DNA
<213> Artificial Sequence

<220>
<223> CMV-CDK9-U1U2

<400> 17

gcgttacata acttacggta aatggcccgc ctggctgacc gcccaacgac ccccgcccat 60
tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240

acatgacctt atgggacttt cctacttggc agtacatcta cgtattagtc atcgctatta 300
ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct ccccctggcc tctcgggaac 480
tacaagtccc agggggcctg gcggtgggcg gcgggcggaa gaggcggggt cggcgccgcg 540
aggccggaag tggccgtgga ggcggaagtg gcgcggccgc ggagggggcct ggagtgcggc 600
ggcggcggga cccggagcag gagcggcggc agcagcgact gggggcggcg gcggcgcgtt 660
ggaggcggcc ggatccgttt aaacggctcg catctctcct tcacgcgccc gccgccctac 720
ctgaggccgc catccacgcc ggttgagtcg cgttctgccg cctcccgcct gtggtgcctc 780
ctgaactgcg tccgccgtct aggtaagttt aaagctcagg tcgagaccgg gcctttgtcc 840
ggcgctccct tggagcctac ctagactcag ccggctctcc acgctttgcc tgaccctgct 900
tgctcaactc tacgtctttg tttcgttttc tgttctgcgc cgttacagat cactagtgtt 960
taaacagagt aatgacatgg ttccttccat cctccaaagg tgaccaataa tagtttgtaa 1020
gtatcattat gaactaatga attttcaaca tatttgatat atttcaatcc attgccatca 1080
ttgttcttat cgatatttga gttggctcac tttgccagta agagtctatt caaattggct 1140
tctgagtcca tttgacacaa cacctttgat ctttgacagt ttccttggtt ttaggtgcta 1200
gatgatttct caggctcacc ttagacattt cctgccacag acttagaatc agccatttct 1260
ctaaggaccc tgattccatt tcatgagaaa tgatagagac cacaatcaaa acaagtcatg 1320
aatttatact gatattttca attcaaatta aagatgaggt ttttgctaaa tttttttgag 1380
tttatatttg tatgtcttat gctgaaaaat cttgtttcct aattagtaac ataattattc 1440
atttgatggg taaatatttt agggccgatt ctttggtttt atagccaaga taccctgttg 1500
ataaagtctt gtgggagcaa ttataagact ggcttatttt gaagcttttt aaaaaagaca 1560
tccttacctg ttttaactgt agattatatt aacttaaata ggtacagccc acgcttg 1617

<210> 18
<211> 1275
<212> DNA
<213> Artificial Sequence

<220>
<223> CMV-CDK9-U1U3

<400> 18

```
gcgttacata acttacggta aatggcccgc ctggctgacc gcccaacgac ccccgcccat       60
tgacgtcaat aatgacgtat gttcccatag taacgccaat agggactttc cattgacgtc      120
aatgggtgga gtatttacgg taaactgccc acttggcagt acatcaagtg tatcatatgc      180
caagtacgcc ccctattgac gtcaatgacg gtaaatggcc cgcctggcat tatgcccagt      240
acatgacctt atgggacttt cctacttggc agtacatcta cgtattagtc atcgctatta      300
ccatggcccg ggtcgcgaca tgcagcggga cgcgccaccc cgagccccag ctccggcgcc      360
ccggctcccc gcgcccccga tcggggccgc cgctagtagt ggcggcggcg gaggcggggg      420
cagcggcggc ggcggcggag gcgcctctgc agctccggct ccccctggcc tctcgggaac      480
tacaagtccc agggggcctg gcggtgggcg gcgggcggaa gaggcggggt cggcgccgcg      540
aggccggaag tggccgtgga ggcggaagtg gcgcggccgc ggaggggcct ggagtgcggc      600
ggcggcggga cccggagcag gagcggcggc agcagcgact gggggcggcg gcggcgcgtt      660
ggaggcggcc ggatccgttt aaacggctcg catctctcct tcacgcgccc gccgccctac      720
ctgaggccgc catccacgcc ggttgagtcg cgttctgccg cctcccgcct gtggtgcctc      780
ctgaactgcg tccgccgtct aggtaagttt aaagctcagg tcgagaccgg gcctttgtcc      840
ggcgctccct tggagcctac ctagactcag ccggctctcc acgctttgcc tgaccctgct      900
tgctcaactc tacgtctttg tttcgttttc tgttctgcgc cgttacagat cactagttaa      960
cgctggtggg tagggatgag ggagggaggg gcattgtgat gtacagggct gctctgtgag     1020
atcaagggtc tcttaagggt gggagctggg gcagggacta cgagagcagc cagatgggct     1080
gaaagtggaa ctcaaggggt ttctggcacc tacctacctg cttcccgctg gggggtgggg     1140
agttggccca gagtcttaag attggggcag ggtggagagg tgggctcttc ctgcttccca     1200
ctcatcttat agctttcttt ccccagatcc gaattcgaga tccaaaccaa ggaggaaagg     1260
atatcacaga ggaga                                                      1275
```

<210> 19
<211> 1660
<212> DNA
<213> Artificial Sequence

<220>
<223> CMV-CDK9-U2U3

<400> 19

gcgttacata acttacggta aatggcccgc ctggctgacc gcccaacgac ccccgcccat 60
tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct ccccctggcc tctcgggaac 480

tacaagtccc agggggcctg gcggtgggcg gcgggcggaa gaggcggggt cggcgccgcg 540
aggccggaag tggccgtgga ggcggaagtg gcgcggccgc ggaggggcct ggagtgcggc 600
ggcggcggga cccggagcag gagcggcggc agcagcgact gggggcggcg gcggcgcgtt 660
ggaggcggcc ggatccgttt aaacagagta atgacatggt tccttccatc ctccaaaggt 720
gaccaataat agtttgtaag tatcattatg aactaatgaa ttttcaacat atttgatata 780
tttcaatcca ttgccatcat tgttcttatc gatatttgag ttggctcact ttgccagtaa 840
gagtctattc aaattggctt ctgagtccat ttgacacaac acctttgatc tttgacagtt 900
tccttggttt taggtgctag atgatttctc aggctcacct tagacatttc ctgccacaga 960
cttagaatca gccatttctc taaggaccct gattccattt catgagaaat gatagagacc 1020
acaatcaaaa caagtcatga atttatactg atattttcaa ttcaaattaa agatgaggtt 1080
tttgctaaat ttttttgagt ttatatttgt atgtcttatg ctgaaaaatc ttgtttccta 1140
attagtaaca taattattca tttgatgggt aaatatttta gggccgattc tttggtttta 1200
tagccaagat accctgttga taaagtcttg tgggagcaat tataagactg gcttattttg 1260
aagcttttta aaaaagacat ccttacctgt tttaactgta gattatatta acttaaatag 1320
gtacagccca cgcttgacta gttaacgctg gtgggtaggg atgagggagg gaggggcatt 1380
gtgatgtaca gggctgctct gtgagatcaa gggtctctta agggtgggag ctggggcagg 1440
gactacgaga gcagccagat gggctgaaag tggaactcaa ggggtttctg gcacctacct 1500
acctgcttcc cgctgggggg tggggagttg gcccagagtc ttaagattgg ggcagggtgg 1560
agaggtgggc tcttcctgct tcccactcat cttatagctt tctttcccca gatccgaatt 1620
cgagatccaa accaaggagg aaaggatatc acagaggaga 1660

<210> 20
<211> 1941
<212> DNA
<213> Artificial Sequence

<220>
<223> CMV-CDK9-U1U2U3

<400> 20

```
gcgttacata acttacggta aatggcccgc ctggctgacc gcccaacgac ccccgcccat       60
tgacgtcaat aatgacgtat gttcccatag taacgccaat agggactttc cattgacgtc      120
aatgggtgga gtatttacgg taaactgccc acttggcagt acatcaagtg tatcatatgc      180
caagtacgcc ccctattgac gtcaatgacg gtaaatggcc cgcctggcat tatgcccagt      240
acatgacctt atgggacttt cctacttggc agtacatcta cgtattagtc atcgctatta      300
ccatggcccg ggtcgcgaca tgcagcggga cgcgccaccc cgagccccag ctccggcgcc      360
ccggctcccc gcgcccccga tcggggccgc cgctagtagt ggcggcggcg gaggcggggg      420
```

```
cagcggcggc ggcggcggag gcgcctctgc agctccggct ccccctggcc tctcgggaac        480
tacaagtccc aggggggcctg gcggtgggcg gcgggcggaa gaggcggggt cggcgccgcg        540
aggccggaag tggccgtgga ggcggaagtg gcgcggccgc ggaggggcct ggagtgcggc        600
ggcggcggga cccggagcag gagcggcggc agcagcgact gggggcggcg gcggcgcgtt        660
ggaggcggcc ggatccgttt aaacggctcg catctctcct tcacgcgccc gccgccctac        720
ctgaggccgc catccacgcc ggttgagtcg cgttctgccg cctcccgcct gtggtgcctc        780
ctgaactgcg tccgccgtct aggtaagttt aaagctcagg tcgagaccgg gcctttgtcc        840
ggcgctccct tggagcctac ctagactcag ccggctctcc acgctttgcc tgaccctgct        900
tgctcaactc tacgtctttg tttcgttttc tgttctgcgc cgttacagat cactagtgtt        960
taaacagagt aatgacatgg ttccttccat cctccaaagg tgaccaataa tagtttgtaa       1020
gtatcattat gaactaatga attttcaaca tatttgatat atttcaatcc attgccatca       1080
ttgttcttat cgatatttga gttggctcac tttgccagta agagtctatt caaattggct       1140
tctgagtcca tttgacacaa cacctttgat ctttgacagt ttccttggtt ttaggtgcta       1200
gatgatttct caggctcacc ttagacattt cctgccacag acttagaatc agccatttct       1260
ctaaggaccc tgattccatt tcatgagaaa tgatagagac cacaatcaaa acaagtcatg       1320
aatttatact gatattttca attcaaatta aagatgaggt ttttgctaaa tttttttgag       1380
tttatatttg tatgtcttat gctgaaaaat cttgtttcct aattagtaac ataattattc       1440
atttgatggg taaatatttt agggccgatt ctttggtttt atagccaaga taccctgttg       1500
ataaagtctt gtgggagcaa ttataagact ggcttatttt gaagcttttt aaaaaagaca       1560
tccttacctg ttttaactgt agattatatt aacttaaata ggtacagccc acgcttgact       1620
agttaacgct ggtgggtagg gatgagggag ggaggggcat tgtgatgtac agggctgctc       1680
tgtgagatca agggtctctt aagggtggga gctggggcag ggactacgag agcagccaga       1740
tgggctgaaa gtggaactca aggggtttct ggcacctacc tacctgcttc ccgctggggg       1800
gtggggagtt ggcccagagt cttaagattg gggcagggtg gagaggtggg ctcttcctgc       1860
ttcccactca tcttatagct ttctttcccc agatccgaat tcgagatcca aaccaaggag       1920
gaaaggatat cacagaggag a                                                 1941
```

<210> 21
<211> 881
<212> DNA
<213> Artificial Sequence

<220>
<223> CMV-bActine-U1

<400> 21

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tggtcgaggt gagccccacg ttctgcttca ctctccccat     360
ctcccccccc tccccacccc caattttgta tttatttatt ttttaattat tttgtgcagc     420
gatgggggcg gggggggggg gggggcgcgc gccaggcggg gcggggcggg gcgaggggcg     480
gggcggggcg aggcggagag gtgcggcggc agccaatcag agcggcgcgc tccgaaagtt     540
tccttttatg gcgaggcggc ggcggcggcg gccctataaa aagcgaagcg cgcggcgggc     600
ggatccgttt aaacggctcg catctctcct tcacgcgccc gccgccctac ctgaggccgc     660
catccacgcc ggttgagtcg cgttctgccg cctcccgcct gtggtgcctc ctgaactgcg     720
tccgccgtct aggtaagttt aaagctcagg tcgagaccgg gcctttgtcc ggcgctccct     780
tggagcctac ctagactcag ccggctctcc acgctttgcc tgaccctgct tgctcaactc     840
tacgtctttg tttcgttttc tgttctgcgc cgttacagat c                         881
```

<210> 22
<211> 1266
<212> DNA
<213> Artificial Sequence

<220>
<223> CMV-bActine-U2

<400> 22

gcgttacata acttacggta aatggcccgc ctggctgacc gcccaacgac ccccgcccat 60
tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tggtcgaggt gagccccacg ttctgcttca ctctccccat 360
ctcccccccc tccccacccc caattttgta tttatttatt ttttaattat tttgtgcagc 420
gatgggggcg gggggggggg gggggcgcgc gccaggcggg gcggggcggg gcgaggggcg 480
gggcggggcg aggcggagag gtgcggcggc agccaatcag agcggcgcgc tccgaaagtt 540
tccttttatg gcgaggcggc ggcggcggcg gccctataaa aagcgaagcg cgcggcgggc 600
ggatccgttt aaacagagta atgacatggt tccttccatc ctccaaaggt gaccaataat 660
agtttgtaag tatcattatg aactaatgaa ttttcaacat atttgatata tttcaatcca 720
ttgccatcat tgttcttatc gatatttgag ttggctcact ttgccagtaa gagtctattc 780
aaattggctt ctgagtccat ttgacacaac acctttgatc tttgacagtt tccttggttt 840
taggtgctag atgatttctc aggctcacct tagacatttc ctgccacaga cttagaatca 900
gccatttctc taaggaccct gattccattt catgagaaat gatagagacc acaatcaaaa 960
caagtcatga atttatactg atattttcaa ttcaaattaa agatgaggtt tttgctaaat 1020
ttttttgagt ttatatttgt atgtcttatg ctgaaaaatc ttgtttccta attagtaaca 1080
taattattca tttgatgggt aaatatttta gggccgattc tttggtttta tagccaagat 1140
accctgttga taaagtcttg tgggagcaat tataagactg gcttattttg aagcttttta 1200
aaaaagacat ccttacctgt tttaactgta gattatatta acttaaatag gtacagccca 1260
cgcttg 1266

<210> 23
<211> 927
<212> DNA
<213> Artificial Sequence

<220>
<223> CMV-bActine-U3

<400> 23

gcgttacata acttacggta aatggcccgc ctggctgacc gcccaacgac ccccgcccat 60

tgacgtcaat aatgacgtat gttcccatag taacgccaat agggactttc cattgacgtc 120

aatgggtgga gtatttacgg taaactgccc acttggcagt acatcaagtg tatcatatgc 180

caagtacgcc ccctattgac gtcaatgacg gtaaatggcc cgcctggcat tatgcccagt 240

acatgacctt atgggacttt cctacttggc agtacatcta cgtattagtc atcgctatta 300

ccatggcccg ggtcgcgaca tggtcgaggt gagccccacg ttctgcttca ctctccccat 360

ctcccccccc tccccacccc caattttgta tttatttatt ttttaattat tttgtgcagc 420

gatgggggcg gggggggggg gggggcgcgc gccaggcggg gcggggcggg gcgaggggcg 480

gggcggggcg aggcggagag gtgcggcggc agccaatcag agcggcgcgc tccgaaagtt 540

tccttttatg gcgaggcggc ggcggcggcg gccctataaa aagcgaagcg cgcggcgggc 600

ggatccgttt aacgctggtg ggtagggatg agggagggag gggcattgtg atgtacaggg 660

ctgctctgtg agatcaaggg tctcttaagg gtgggagctg gggcagggac tacgagagca 720

gccagatggg ctgaaagtgg aactcaaggg gtttctggca cctacctacc tgcttcccgc 780

tgggggggtgg ggagttggcc cagagtctta agattggggc agggtggaga ggtgggctct 840

tcctgcttcc cactcatctt atagctttct ttccccagat ccgaattcga gatccaaacc 900

aaggaggaaa ggatatcaca gaggaga 927

<210> 24
<211> 1547
<212> DNA
<213> Artificial Sequence

<220>
<223> CMV-bActine-U1U2

<400> 24

gcgttacata acttacggta aatggcccgc ctggctgacc gcccaacgac ccccgcccat 60
tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tggtcgaggt gagccccacg ttctgcttca ctctccccat 360
ctcccccccc tccccacccc caattttgta tttatttatt ttttaattat tttgtgcagc 420
gatgggggcg gggggggggg gggggcgcgc gccaggcggg gcggggcggg gcgaggggcg 480
gggcggggcg aggcggagag gtgcggcggc agccaatcag agcggcgcgc tccgaaagtt 540
tccttttatg gcgaggcggc ggcggcggcg gccctataaa aagcgaagcg cgcggcgggc 600
ggatccgttt aaacggctcg catctctcct tcacgcgccc gccgccctac ctgaggccgc 660
catccacgcc ggttgagtcg cgttctgccg cctcccgcct gtggtgcctc ctgaactgcg 720
tccgccgtct aggtaagttt aaagctcagg tcgagaccgg gcctttgtcc ggcgctccct 780
tggagcctac ctagactcag ccggctctcc acgctttgcc tgaccctgct tgctcaactc 840
tacgtctttg tttcgttttc tgttctgcgc cgttacagat cactagtgtt taaacagagt 900
aatgacatgg ttccttccat cctccaaagg tgaccaataa tagtttgtaa gtatcattat 960
gaactaatga attttcaaca tatttgatat atttcaatcc attgccatca ttgttcttat 1020
cgatatttga gttggctcac tttgccagta agagtctatt caaattggct tctgagtcca 1080
tttgacacaa cacctttgat ctttgacagt ttccttggtt ttaggtgcta gatgatttct 1140
caggctcacc ttagacattt cctgccacag acttagaatc agccatttct ctaaggaccc 1200
tgattccatt tcatgagaaa tgatagagac cacaatcaaa acaagtcatg aatttatact 1260
gatattttca attcaaatta aagatgaggt ttttgctaaa ttttttttgag tttatatttg 1320
tatgtcttat gctgaaaaat cttgtttcct aattagtaac ataattattc atttgatggg 1380
taaatatttt agggccgatt ctttggtttt atagccaaga taccctgttg ataaagtctt 1440
gtgggagcaa ttataagact ggcttatttt gaagcttttt aaaaaagaca tccttacctg 1500
ttttaactgt agattatatt aacttaaata ggtacagccc acgcttg 1547

<210> 25
<211> 1205
<212> DNA
<213> Artificial Sequence

<220>
<223> CMV-bActine-U1U3

<400> 25

gcgttacata acttacggta aatggcccgc ctggctgacc gcccaacgac ccccgcccat 60

tgacgtcaat aatgacgtat gttcccatag taacgccaat agggactttc cattgacgtc 120

aatgggtgga gtatttacgg taaactgccc acttggcagt acatcaagtg tatcatatgc 180

caagtacgcc ccctattgac gtcaatgacg gtaaatggcc cgcctggcat tatgcccagt 240

acatgacctt atgggacttt cctacttggc agtacatcta cgtattagtc atcgctatta 300

ccatggcccg ggtcgcgaca tggtcgaggt gagccccacg ttctgcttca ctctccccat 360

ctcccccccc tccccacccc caattttgta tttatttatt ttttaattat tttgtgcagc 420

gatgggggcg gggggggggg gggggcgcgc gccaggcggg gcggggcggg gcgaggggcg 480

gggcggggcg aggcggagag gtgcggcggc agccaatcag agcggcgcgc tccgaaagtt 540

tccttttatg gcgaggcggc ggcggcggcg gccctataaa aagcgaagcg cgcggcgggc 600

ggatccgttt aaacggctcg catctctcct tcacgcgccc gccgccctac ctgaggccgc 660

catccacgcc ggttgagtcg cgttctgccg cctcccgcct gtggtgcctc ctgaactgcg 720

tccgccgtct aggtaagttt aaagctcagg tcgagaccgg gcctttgtcc ggcgctccct 780

tggagcctac ctagactcag ccggctctcc acgctttgcc tgaccctgct tgctcaactc 840

tacgtctttg tttcgttttc tgttctgcgc cgttacagat cactagttaa cgctggtggg 900

tagggatgag ggagggaggg gcattgtgat gtacagggct gctctgtgag atcaagggtc 960

tcttaagggt gggagctggg gcagggacta cgagagcagc cagatgggct gaaagtggaa 1020

ctcaaggggt ttctggcacc tacctacctg cttcccgctg gggggtgggg agttggccca 1080

gagtcttaag attggggcag ggtggagagg tgggctcttc ctgcttccca ctcatcttat 1140

agctttcttt ccccagatcc gaattcgaga tccaaaccaa ggaggaaagg atatcacaga 1200

ggaga 1205

<210> 26
<211> 1590
<212> DNA
<213> Artificial Sequence

<220>
<223> CMV-bActine-U2U3

<400> 26

gcgttacata acttacggta aatggcccgc ctggctgacc gcccaacgac ccccgcccat 60

tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tggtcgaggt gagccccacg ttctgcttca ctctccccat 360
ctcccccccc tccccacccc caattttgta tttatttatt ttttaattat tttgtgcagc 420
gatgggggcg gggggggggg ggggcgcgc gccaggcggg gcggggcggg gcgaggggcg 480
gggcggggcg aggcggagag gtgcggcggc agccaatcag agcggcgcgc tccgaaagtt 540
tccttttatg gcgaggcggc ggcggcggcg gccctataaa aagcgaagcg cgcggcgggc 600
ggatccgttt aaacagagta atgacatggt tccttccatc ctccaaaggt gaccaataat 660
agtttgtaag tatcattatg aactaatgaa ttttcaacat atttgatata tttcaatcca 720
ttgccatcat tgttcttatc gatatttgag ttggctcact ttgccagtaa gagtctattc 780
aaattggctt ctgagtccat ttgacacaac acctttgatc tttgacagtt tccttggttt 840
taggtgctag atgatttctc aggctcacct tagacatttc ctgccacaga cttagaatca 900
gccatttctc taaggaccct gattccattt catgagaaat gatagagacc acaatcaaaa 960
caagtcatga atttatactg atattttcaa ttcaaattaa agatgaggtt tttgctaaat 1020
ttttttgagt ttatatttgt atgtcttatg ctgaaaaatc ttgtttccta attagtaaca 1080
taattattca tttgatgggt aaatatttta gggccgattc tttggtttta tagccaagat 1140
accctgttga taaagtcttg tgggagcaat tataagactg gcttattttg aagcttttta 1200
aaaaagacat ccttacctgt tttaactgta gattatatta acttaaatag gtacagccca 1260
cgcttgacta gttaacgctg gtgggtaggg atgagggagg gaggggcatt gtgatgtaca 1320
gggctgctct gtgagatcaa gggtctctta agggtgggag ctggggcagg gactacgaga 1380
gcagccagat gggctgaaag tggaactcaa ggggtttctg gcacctacct acctgcttcc 1440
cgctgggggg tggggagttg gcccagagtc ttaagattgg ggcagggtgg agaggtgggc 1500
tcttcctgct tcccactcat cttatagctt tctttcccca gatccgaatt cgagatccaa 1560
accaaggagg aaaggatatc acagaggaga 1590

<210> 27
<211> 1871
<212> DNA
<213> Artificial Sequence

<220>
<223> CMV-bActine-U1U2U3

<400> 27

gcgttacata acttacggta aatggcccgc ctggctgacc gcccaacgac ccccgcccat 60

```
tgacgtcaat aatgacgtat gttcccatag taacgccaat agggactttc cattgacgtc      120
aatgggtgga gtatttacgg taaactgccc acttggcagt acatcaagtg tatcatatgc      180
caagtacgcc ccctattgac gtcaatgacg gtaaatggcc cgcctggcat tatgcccagt      240
acatgacctt atgggacttt cctacttggc agtacatcta cgtattagtc atcgctatta      300
ccatggcccg ggtcgcgaca tggtcgaggt gagccccacg ttctgcttca ctctccccat      360
ctcccccccc tccccacccc caattttgta tttatttatt ttttaattat tttgtgcagc      420
gatgggggcg gggggggggg gggggcgcgc gccaggcggg gcggggcggg gcgaggggcg      480
gggcggggcg aggcggagag gtgcggcggc agccaatcag agcggcgcgc tccgaaagtt      540
tccttttatg gcgaggcggc ggcggcggcg gccctataaa aagcgaagcg cgcggcgggc      600
ggatccgttt aaacggctcg catctctcct tcacgcgccc gccgccctac ctgaggccgc      660
catccacgcc ggttgagtcg cgttctgccg cctcccgcct gtggtgcctc ctgaactgcg      720
tccgccgtct aggtaagttt aaagctcagg tcgagaccgg gcctttgtcc ggcgctccct      780
tggagcctac ctagactcag ccggctctcc acgctttgcc tgaccctgct tgctcaactc      840
tacgtctttg tttcgttttc tgttctgcgc cgttacagat cactagtgtt taaacagagt      900
aatgacatgg ttccttccat cctccaaagg tgaccaataa tagtttgtaa gtatcattat      960
gaactaatga attttcaaca tatttgatat atttcaatcc attgccatca ttgttcttat     1020
cgatatttga gttggctcac tttgccagta agagtctatt caaattggct tctgagtcca     1080
tttgacacaa cacctttgat ctttgacagt ttccttggtt ttaggtgcta gatgatttct     1140
caggctcacc ttagacattt cctgccacag acttagaatc agccatttct ctaaggaccc     1200
tgattccatt tcatgagaaa tgatagagac cacaatcaaa acaagtcatg aatttatact     1260
gatattttca attcaaatta aagatgaggt tttgctaaa ttttttttgag tttatatttg     1320
tatgtcttat gctgaaaaat cttgtttcct aattagtaac ataattattc atttgatggg     1380
taaatatttt agggccgatt ctttggtttt atagccaaga taccctgttg ataaagtctt     1440
gtgggagcaa ttataagact ggcttatttt gaagcttttt aaaaaagaca tccttacctg     1500
ttttaactgt agattatatt aacttaaata ggtacagccc acgcttgact agttaacgct     1560
ggtgggtagg gatgagggag ggaggggcat tgtgatgtac agggctgctc tgtgagatca     1620
agggtctctt aagggtggga gctggggcag ggactacgag agcagccaga tgggctgaaa     1680
gtggaactca aggggtttct ggcacctacc tacctgcttc ccgctggggg gtggggagtt     1740
ggcccagagt cttaagattg gggcagggtg gagaggtggg ctcttcctgc ttcccactca     1800
tcttatagct ttctttcccc agatccgaat tcgagatcca aaccaaggag gaaaggatat     1860
cacagaggag a                                                          1871
```

<210> 28
<211> 1643
<212> DNA
<213> Artificial Sequence

<220>
<223> CMV-CDK9-EF1

<400> 28

gcgttacata acttacggta aatggcccgc ctggctgacc gcccaacgac ccccgcccat 60
tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct ccccctggcc tctcgggaac 480
tacaagtccc aggggggcctg gcggtgggcg gcgggcggaa gaggcggggt cggcgccgcg 540
aggccggaag tggccgtgga ggcggaagtg gcgcggccgc ggaggggcct ggagtgcggc 600
ggcggcggga cccggagcag gagcggcggc agcagcgact gggggcggcg gcggcgcgtt 660
ggaggcggcc ggatccacta gtcgggtttg ccgccagaac acaggtaagt gccgtgtgtg 720
gttcccgcgg gcctggcctc tttacgggtt atggcccttg cgtgccttga attacttcca 780
cctggctgca gtacgtgatt cttgatcccg agcttcgggt tggaagtggg tgggagagtt 840
cgaggccttg cgcttaagga gccccttcgc ctcgtgcttg agttgaggcc tggcctgggc 900
gctggggccg ccgcgtgcga atctggtggc accttcgcgc ctgtctcgct gctttcgata 960
agtctctagc catttaaaat ttttgatgac ctgctgcgac gctttttttc tggcaagata 1020
gtcttgtaaa tgcgggccaa gatctgcaca ctggtatttc ggtttttggg gccgcgggcg 1080
gcgacggggc ccgtgcgtcc cagcgcacat gttcggcgag gcggggcctg cgagcgcggc 1140
caccgagaat cggacggggg tagtctcaag ctggccggcc tgctctggtg cctggcctcg 1200
cgccgccgtg tatcgccccg ccctgggcgg caaggctggc ccggtcggca ccagttgcgt 1260
gagcggaaag atggccgctt cccggccctg ctgcagggag ctcaaaatgg aggacgcggc 1320
gctcgggaga gcgggcgggt gagtcaccca cacaaaggaa aagggccttt ccgtcctcag 1380
ccgtcgcttc atgtgactcc acggagtacc gggcgccgtc caggcacctc gattagttct 1440
cgagcttttg gagtacgtcg tctttaggtt gggggggaggg gttttatgcg atggagtttc 1500
cccacactga gtgggtggag actgaagtta ggccagcttg gcacttgatg taattctcct 1560
tggaatttgc cctttttgag tttggatctt ggttcattct caagcctcag acagtggttc 1620

aaagtttttt tcttccattt cag 1643

<210> 29
<211> 1051
<212> DNA
<213> Artificial Sequence

<220>
<223> CMV-CDK9-mROSA

<400> 29

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct cccccctggcc tctcgggaac    480
tacaagtccc aggggggcctg gcggtgggcg gcgggcggaa gaggcggggt cggcgccgcg   540
aggccggaag tggccgtgga ggcggaagtg gcgcggccgc ggagggggcct ggagtgcggc   600
ggcggcggga cccggagcag gagcggcggc agcagcgact gggggcggcg gcggcgcgtt    660
ggaggcggcc ggatccacta gttcagagag cctcggctag gtaggggatc gggactctgg     720
cgggagggcg gcttggtgcg tttgcgggga tgggcggccg cggcaggccc tccgagcgtg     780
gtggagccgt tctgtgagac agccgggtac gagtcgtgac gctggaaggg gcaagcgggt     840
ggtgggcagg aatgcggtcc gccctgcagc aaccggaggg ggagggagaa gggagcggaa     900
aagtctccac cggacgcggc catggctcgg gggggggggg gcagcggagg agcgcttccg     960
gccgacgtct cgtcgctgat tggcttcttt tcctcccgcc gtgtgtgaaa acacaattgt    1020
actaaccttc ttctctttcc tctcctgaca g                                  1051
```

<210> 30
<211> 1918
<212> DNA
<213> Artificial Sequence

<220>
<223> CMV-CDK9-hROSA

<400> 30

```
gcgttacata acttacggta aatggcccgc ctggctgacc gcccaacgac ccccgcccat      60
tgacgtcaat aatgacgtat gttcccatag taacgccaat agggactttc cattgacgtc     120
```

```
aatgggtgga gtatttacgg taaactgccc acttggcagt acatcaagtg tatcatatgc      180
caagtacgcc ccctattgac gtcaatgacg gtaaatggcc cgcctggcat tatgcccagt      240
acatgacctt atgggacttt cctacttggc agtacatcta cgtattagtc atcgctatta      300
ccatggcccg ggtcgcgaca tgcagcggga cgcgccaccc cgagccccag ctccggcgcc      360
ccggctcccc gcgcccccga tcggggccgc cgctagtagt ggcggcggcg gaggcggggg      420
cagcggcggc ggcggcggag gcgcctctgc agctccggct ccccctggcc tctcgggaac      480
tacaagtccc aggggcctg gcggtgggcg gcgggcggaa gaggcggggt cggcgccgcg      540
aggccggaag tggccgtgga ggcggaagtg gcgcggccgc ggaggggcct ggagtgcggc      600
ggcggcggga cccggagcag gagcggcggc agcagcgact gggggcggcg gcggcgcgtt      660
ggaggcggcc ggatccacta gtctcagaga gcctcggcta ggtaggggag cggaactctg      720
gtgggagggg aggtgcggtg cactgggggg atgggtggct aggggggccg tctggtggct      780
tgcgggggtt gcctttcccg tgggaagtcg ggaacataat gtttgttacg ttgggaggga      840
aaggggtggc tggatgcagg cgggagggag gcccgccctg cggcaaccgg agggggaggg      900
agaagggagc ggaaaatgct cgaaaccgga cggagccatt gctctcgcag agggaggagc      960
gcttccggct agcctcttgt cgccgattgg ccgtttctcc tcccgccgtg tgtgaaaaca     1020
caaatggcgt attctggttg gagtaaagct cctgtcagtt acaccgtcgg gagtacgcag     1080
ccgcttagcg actctcgcgt tgccccctgg gtggggcggg taggtaggtg ggtgtagag      1140
atgctgggtg tgcgggcgcg gccggcctcc tgcggcggga ggggagggtc agtgaaattg     1200
gctctggcgc gggcgtcctc ccaccctccc cttccttcgg gggagtcggt ttacccgccg     1260
cctgcttgtc ttcgacacct gattggctgt cgaagctgtg ggaccgggcc cttgctactg     1320
gctcgagtct cacatgagcg aaaccactgc gcggggcgcg ggggtggcgg ggaggcgggc     1380
gttggtacgg tcctccccga ggccgagcgc cgcagtgtct ggccccgcgc ccctgcgcaa     1440
cgtggcagga agcgcgcgct ggaggcgggg gcgggctgcc ggccgagact tctggatggc     1500
ggcggccgcg gctccgcccc gggttcccac cgcctgaagg gcgagacaag cccgacctgc     1560
tacaggcact cgtgggggtg ggggaggagc gggggtcggt ccggctggtt tgtgggtggg     1620
aggcgcttgt tctccaaaaa ccggcgcgag ctgcaatcct gagggagctg cggtggagga     1680
ggtggagaga aggccgcacc cttctgggca gggggagggg agtgccgcaa tacctttatg     1740
ggagttcttt gctgcctccc gtcttgtaag gaccgccctg ggcctggaag aagccctccc     1800
tcctttcctc ctcgcgtgat ctcgtcatcg cctccatgtc gagtcgcttc tcgattatgg     1860
gcgggattct tttgcctaga caattgtact aaccttcttc tctttcctct cctgacag       1918
```

<210> 31

<211> 1573
<212> DNA
<213> Artificial Sequence

<220>
<223> CMV-bActine-EF1

<400> 31

gcgttacata acttacggta aatggcccgc ctggctgacc gcccaacgac ccccgcccat 60
tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tggtcgaggt gagccccacg ttctgcttca ctctccccat 360
ctcccccccc tccccacccc caattttgta tttatttatt ttttaattat tttgtgcagc 420
gatgggggcg gggggggggg gggggcgcgc gccaggcggg gcggggcggg gcgaggggcg 480
gggcggggcg aggcggagag gtgcggcggc agccaatcag agcggcgcgc tccgaaagtt 540
tccttttatg gcgaggcggc ggcggcggcg gccctataaa aagcgaagcg cgcggcgggc 600
ggatccacta gtcgggtttg ccgccagaac acaggtaagt gccgtgtgtg gttcccgcgg 660
gcctggcctc tttacgggtt atggcccttg cgtgccttga attacttcca cctggctgca 720
gtacgtgatt cttgatcccg agcttcgggt tggaagtggg tgggagagtt cgaggccttg 780
cgcttaagga gccccttcgc ctcgtgcttg agttgaggcc tggcctgggc gctggggccg 840
ccgcgtgcga atctggtggc accttcgcgc ctgtctcgct gctttcgata agtctctagc 900
catttaaaat ttttgatgac ctgctgcgac gctttttttc tggcaagata gtcttgtaaa 960
tgcgggccaa gatctgcaca ctggtatttc ggtttttggg gccgcgggcg gcgacggggc 1020
ccgtgcgtcc cagcgcacat gttcggcgag gcggggcctg cgagcgcggc caccgagaat 1080
cggacggggg tagtctcaag ctggccggcc tgctctggtg cctggcctcg cgccgccgtg 1140
tatcgccccg ccctgggcgg caaggctggc ccggtcggca ccagttgcgt gagcggaaag 1200
atggccgctt cccggccctg ctgcagggag ctcaaaatgg aggacgcggc gctcgggaga 1260
gcgggcgggt gagtcaccca cacaaaggaa aagggccttt ccgtcctcag ccgtcgcttc 1320
atgtgactcc acggagtacc gggcgccgtc caggcacctc gattagttct cgagcttttg 1380
gagtacgtcg tctttaggtt ggggggaggg gttttatgcg atggagtttc cccacactga 1440
gtgggtggag actgaagtta ggccagcttg gcacttgatg taattctcct tggaatttgc 1500
ccttttttgag tttggatctt ggttcattct caagcctcag acagtggttc aaagtttttt 1560
tcttccattt cag 1573

<210> 32
<211> 981
<212> DNA
<213> Artificial Sequence

<220>
<223> CMV-bActine-mROSA

<400> 32

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tggtcgaggt gagccccacg ttctgcttca ctctccccat     360
ctcccccccc tccccacccc caattttgta tttatttatt ttttaattat tttgtgcagc     420
gatgggggcg gggggggggg ggggcgcgcc gccaggcggg gcggggcggg gcgaggggcg     480
gggcggggcg aggcggagag gtgcggcggc agccaatcag agcggcgcgc tccgaaagtt     540
tccttttatg gcgaggcggc ggcggcggcg gccctataaa aagcgaagcg cgcggcgggc     600
ggatccacta gttcagagag cctcggctag gtaggggatc gggactctgg cgggagggcg     660
gcttggtgcg tttgcgggga tgggcggccg cggcaggccc tccgagcgtg gtggagccgt     720
tctgtgagac agccgggtac gagtcgtgac gctggaaggg gcaagcgggt ggtgggcagg     780
aatgcggtcc gccctgcagc aaccggaggg ggagggagaa gggagcggaa aagtctccac     840
cggacgcggc catggctcgg gggggggggg gcagcggagg agcgcttccg gccgacgtct     900
cgtcgctgat tggcttcttt tcctcccgcc gtgtgtgaaa acacaattgt actaaccttc     960
ttctctttcc tctcctgaca g                                               981
```

<210> 33
<211> 1848
<212> DNA
<213> Artificial Sequence

<220>
<223> CMV-bActine-hROSA

<400> 33

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tggtcgaggt gagccccacg ttctgcttca ctctccccat     360
```

ctcccccccc tccccacccc caattttgta tttatttatt ttttaattat tttgtgcagc 420

gatgggggcg gggggggggg gggggcgcgc gccaggcggg gcggggcggg gcgaggggcg 480

gggcggggcg aggcggagag gtgcggcggc agccaatcag agcggcgcgc tccgaaagtt 540

tccttttatg gcgaggcggc ggcggcggcg gccctataaa aagcgaagcg cgcggcgggc 600

ggatccacta gtctcagaga gcctcggcta ggtaggggag cggaactctg gtgggagggg 660

aggtgcggtg cactgggggg atgggtggct agggggccg tctggtggct tgcgggggtt 720

gcctttcccg tgggaagtcg ggaacataat gtttgttacg ttgggaggga aaggggtggc 780

tggatgcagg cgggagggag gcccgccctg cggcaaccgg agggggaggg agaagggagc 840

ggaaaatgct cgaaaccgga cggagccatt gctctcgcag agggaggagc gcttccggct 900

agcctcttgt cgccgattgg ccgtttctcc tcccgccgtg tgtgaaaaca caaatggcgt 960

attctggttg gagtaaagct cctgtcagtt acaccgtcgg gagtacgcag ccgcttagcg 1020

actctcgcgt tgccccctgg gtggggcggg taggtaggtg gggtgtagag atgctgggtg 1080

tgcgggcgcg gccggcctcc tgcggcggga ggggagggtc agtgaaattg gctctggcgc 1140

gggcgtcctc ccaccctccc cttccttcgg gggagtcggt ttacccgccg cctgcttgtc 1200

ttcgacacct gattggctgt cgaagctgtg ggaccgggcc cttgctactg gctcgagtct 1260

cacatgagcg aaaccactgc gcggggcgcg gggtggcgg ggaggcgggc gttggtacgg 1320

tcctccccga ggccgagcgc cgcagtgtct ggccccgcgc ccctgcgcaa cgtggcagga 1380

agcgcgcgct ggaggcgggg gcgggctgcc ggccgagact tctggatggc ggcggccgcg 1440

gctccgcccc gggttcccac cgcctgaagg gcgagacaag cccgacctgc tacaggcact 1500

cgtgggggtg ggggaggagc gggggtcggt ccggctggtt tgtgggtggg aggcgcttgt 1560

tctccaaaaa ccggcgcgag ctgcaatcct gagggagctg cggtggagga ggtggagaga 1620

aggccgcacc cttctgggca gggggagggg agtgccgcaa tacctttatg ggagttcttt 1680

gctgcctccc gtcttgtaag gaccgccctg ggcctggaag aagccctccc tcctttcctc 1740

ctcgcgtgat ctcgtcatcg cctccatgtc gagtcgcttc tcgattatgg gcgggattct 1800

tttgcctaga caattgtact aaccttcttc tctttcctct cctgacag 1848

<210> 34
<211> 1918
<212> DNA
<213> Artificial Sequence

<220>
<223> CMV-CDK9-U1-EF1

<400> 34

gcgttacata acttacggta aatggcccgc ctggctgacc gcccaacgac ccccgcccat 60

tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct ccccctggcc tctcgggaac 480
tacaagtccc aggggggcctg gcggtgggcg gcgggcggaa gaggcggggt cggcgccgcg 540
aggccggaag tggccgtgga ggcggaagtg gcgcggccgc ggaggggcct ggagtgcggc 600
ggcggcggga cccggagcag gagcggcggc agcagcgact gggggcggcg gcggcgcgtt 660
ggaggcggcc ggatccgttt aaacggctcg catctctcct tcacgcgccc gccgccctac 720
ctgaggccgc catccacgcc ggttgagtcg cgttctgccg cctcccgcct gtggtgcctc 780
ctgaactgcg tccgccgtct aggtaagttt aaagctcagg tcgagaccgg gcctttgtcc 840
ggcgctccct tggagcctac ctagactcag ccggctctcc acgctttgcc tgaccctgct 900
tgctcaactc tacgtctttg tttcgttttc tgttctgcgc cgttacagat cactagtcgg 960
gtttgccgcc agaacacagg taagtgccgt gtgtggttcc cgcgggcctg gcctctttac 1020
gggttatggc ccttgcgtgc cttgaattac ttccacctgg ctgcagtacg tgattcttga 1080
tcccgagctt cgggttggaa gtgggtggga gagttcgagg ccttgcgctt aaggagcccc 1140
ttcgcctcgt gcttgagttg aggcctggcc tgggcgctgg ggccgccgcg tgcgaatctg 1200
gtggcacctt cgcgcctgtc tcgctgcttt cgataagtct ctagccattt aaaatttttg 1260
atgacctgct gcgacgcttt ttttctggca agatagtctt gtaaatgcgg gccaagatct 1320
gcacactggt atttcggttt ttggggccgc gggcggcgac ggggcccgtg cgtcccagcg 1380
cacatgttcg gcgaggcggg gcctgcgagc gcggccaccg agaatcggac gggggtagtc 1440
tcaagctggc cggcctgctc tggtgcctgg cctcgcgccg ccgtgtatcg ccccgccctg 1500
ggcggcaagg ctggcccggt cggcaccagt tgcgtgagcg gaaagatggc cgcttcccgg 1560
ccctgctgca gggagctcaa aatggaggac gcggcgctcg ggagagcggg cgggtgagtc 1620
acccacacaa aggaaaaggg cctttccgtc ctcagccgtc gcttcatgtg actccacgga 1680
gtaccgggcg ccgtccaggc acctcgatta gttctcgagc ttttggagta cgtcgtcttt 1740
aggttggggg gaggggtttt atgcgatgga gtttccccac actgagtggg tggagactga 1800
agttaggcca gcttggcact tgatgtaatt ctccttggaa tttgcccttt ttgagtttgg 1860
atcttggttc attctcaagc ctcagacagt ggttcaaagt ttttttcttc catttcag 1918

<210> 35
<211> 1326
<212> DNA
<213> Artificial Sequence

<220>
<223> CMV-CDK9-U1-mROSA

<400> 35

gcgttacata acttacggta aatggcccgc ctggctgacc gcccaacgac ccccgcccat 60
tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct ccccctggcc tctcgggaac 480
tacaagtccc agggggcctg gcggtgggcg gcgggcggaa gaggcggggt cggcgccgcg 540
aggccggaag tggccgtgga ggcggaagtg gcgcggccgc ggagggggcct ggagtgcggc 600
ggcggcggga cccggagcag gagcggcggc agcagcgact gggggcggcg gcggcgcgtt 660
ggaggcggcc ggatccgttt aaacggctcg catctctcct tcacgcgccc gccgccctac 720
ctgaggccgc catccacgcc ggttgagtcg cgttctgccg cctcccgcct gtggtgcctc 780
ctgaactgcg tccgccgtct aggtaagttt aaagctcagg tcgagaccgg gcctttgtcc 840
ggcgctccct tggagcctac ctagactcag ccggctctcc acgctttgcc tgaccctgct 900
tgctcaactc tacgtctttg tttcgttttc tgttctgcgc cgttacagat cactagttca 960
gagagcctcg gctaggtagg ggatcgggac tctggcggga gggcggcttg gtgcgtttgc 1020
ggggatgggc ggccgcggca ggccctccga gcgtggtgga gccgttctgt gagacagccg 1080
ggtacgagtc gtgacgctgg aaggggcaag cgggtggtgg gcaggaatgc ggtccgccct 1140
gcagcaaccg gagggggagg gagaagggag cggaaaagtc tccaccggac gcggccatgg 1200
ctcggggggg ggggggcagc ggaggagcgc ttccggccga cgtctcgtcg ctgattggct 1260
tcttttcctc ccgccgtgtg tgaaaacaca attgtactaa ccttcttctc tttcctctcc 1320
tgacag 1326

<210> 36
<211> 2193
<212> DNA
<213> Artificial Sequence

<220>
<223> CMV-CDK9-U1-hROSA

<400> 36

gcgttacata acttacggta aatggcccgc ctggctgacc gcccaacgac ccccgcccat 60

tgacgtcaat aatgacgtat gttcccatag taacgccaat agggactttc cattgacgtc 120

aatgggtgga gtatttacgg taaactgccc acttggcagt acatcaagtg tatcatatgc 180

caagtacgcc ccctattgac gtcaatgacg gtaaatggcc cgcctggcat tatgcccagt 240

acatgacctt atgggacttt cctacttggc agtacatcta cgtattagtc atcgctatta 300

ccatggcccg ggtcgcgaca tgcagcggga cgcgccaccc cgagccccag ctccggcgcc 360

ccggctcccc gcgcccccga tcggggccgc cgctagtagt ggcggcggcg gaggcggggg 420

cagcggcggc ggcggcggag gcgcctctgc agctccggct ccccctggcc tctcgggaac 480

tacaagtccc aggggggcctg gcggtgggcg gcgggcggaa gaggcggggt cggcgccgcg 540

aggccggaag tggccgtgga ggcggaagtg gcgcggccgc ggagggggcct ggagtgcggc 600

ggcggcggga cccggagcag gagcggcggc agcagcgact gggggcggcg gcggcgcgtt 660

ggaggcggcc ggatccgttt aaacggctcg catctctcct tcacgcgccc gccgccctac 720

ctgaggccgc catccacgcc ggttgagtcg cgttctgccg cctcccgcct gtggtgcctc 780

ctgaactgcg tccgccgtct aggtaagttt aaagctcagg tcgagaccgg gcctttgtcc 840

ggcgctccct tggagcctac ctagactcag ccggctctcc acgctttgcc tgaccctgct 900

tgctcaactc tacgtctttg tttcgttttc tgttctgcgc cgttacagat cactagtctc 960

agagagcctc ggctaggtag gggagcggaa ctctggtggg aggggaggtg cggtgcactg 1020

gggggatggg tggctagggg ggccgtctgg tggcttgcgg gggttgcctt tcccgtggga 1080

agtcgggaac ataatgtttg ttacgttggg agggaaaggg gtggctggat gcaggcggga 1140

gggaggcccg ccctgcggca accggagggg gagggagaag ggagcggaaa atgctcgaaa 1200

ccggacggag ccattgctct cgcagaggga ggagcgcttc cggctagcct cttgtcgccg 1260

attggccgtt tctcctcccg ccgtgtgtga aaacacaaat ggcgtattct ggttggagta 1320

aagctcctgt cagttacacc gtcgggagta cgcagccgct tagcgactct cgcgttgccc 1380

cctgggtggg gcgggtaggt aggtggggtg tagagatgct gggtgtgcgg gcgcggccgg 1440

cctcctgcgg cgggaggggga gggtcagtga aattggctct ggcgcgggcg tcctcccacc 1500

ctccccttcc ttcgggggag tcggtttacc cgccgcctgc ttgtcttcga cacctgattg 1560

gctgtcgaag ctgtgggacc gggcccttgc tactggctcg agtctcacat gagcgaaacc 1620

actgcgcggg gcgcgggggt ggcggggagg cgggcgttgg tacggtcctc cccgaggccg 1680

agcgccgcag tgtctggccc cgcgcccctg cgcaacgtgg caggaagcgc gcgctggagg 1740

cgggggcggg ctgccggccg agacttctgg atggcggcgg ccgcggctcc gccccgggtt 1800

cccaccgcct gaagggcgag acaagcccga cctgctacag gcactcgtgg gggtggggga 1860

ggagcggggg tcggtccggc tggtttgtgg gtgggaggcg cttgttctcc aaaaaccggc 1920

gcgagctgca atcctgaggg agctgcggtg gaggaggtgg agagaaggcc gcacccttct 1980

gggcaggggg aggggagtgc cgcaatacct ttatgggagt tctttgctgc ctcccgtctt 2040

gtaaggaccg ccctgggcct ggaagaagcc ctccctcctt tcctcctcgc gtgatctcgt 2100

catcgcctcc atgtcgagtc gcttctcgat tatgggcggg attcttttgc ctagacaatt 2160

gtactaacct tcttctcttt cctctcctga cag 2193

<210> 37
<211> 2309
<212> DNA
<213> Artificial Sequence

<220>
<223> CMV-CDK9-U2-EF1

<400> 37

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
tacaagtccc agggggcctg gcggtgggcg gcgggcggaa gaggcggggt cggcgccgcg     540
aggccggaag tggccgtgga ggcggaagtg gcgcggccgc ggaggggcct ggagtgcggc     600
ggcggcggga cccggagcag gagcggcggc agcagcgact gggggcggcg gcggcgcgtt     660
ggaggcggcc ggatccacta gtgtttaaac agagtaatga catggttcct tccatcctcc     720
aaaggtgacc aataatagtt tgtaagtatc attatgaact aatgaatttt caacatattt     780
gatatatttc aatccattgc catcattgtt cttatcgata tttgagttgg ctcactttgc     840
cagtaagagt ctattcaaat tggcttctga gtccatttga cacaacacct ttgatctttg     900
acagtttcct tggttttagg tgctagatga tttctcaggc tcaccttaga catttcctgc     960
cacagactta gaatcagcca tttctctaag gaccctgatt ccatttcatg agaaatgata    1020
gagaccacaa tcaaaacaag tcatgaattt atactgatat tttcaattca aattaaagat    1080
gaggtttttg ctaaattttt ttgagtttat atttgtatgt cttatgctga aaatcttgt     1140
ttcctaatta gtaacataat tattcatttg atgggtaaat attttagggc cgattctttg    1200
```

gttttatagc caagataccc tgttgataaa gtcttgtggg agcaattata agactggctt 1260

attttgaagc tttttaaaaa agacatcctt acctgtttta actgtagatt atattaactt 1320

aaataggtac agcccacgct tgactagtcg ggtttgccgc cagaacacag gtaagtgccg 1380

tgtgtggttc ccgcgggcct ggcctcttta cgggttatgg cccttgcgtg ccttgaatta 1440

cttccacctg gctgcagtac gtgattcttg atcccgagct tcgggttgga agtgggtggg 1500

agagttcgag gccttgcgct taaggagccc cttcgcctcg tgcttgagtt gaggcctggc 1560

ctgggcgctg gggccgccgc gtgcgaatct ggtggcacct tcgcgcctgt ctcgctgctt 1620

tcgataagtc tctagccatt taaaattttt gatgacctgc tgcgacgctt tttttctggc 1680

aagatagtct tgtaaatgcg ggccaagatc tgcacactgg tatttcggtt tttggggccg 1740

cgggcggcga cggggcccgt gcgtcccagc gcacatgttc ggcgaggcgg ggcctgcgag 1800

cgcggccacc gagaatcgga cgggggtagt ctcaagctgg ccggcctgct ctggtgcctg 1860

gcctcgcgcc gccgtgtatc gccccgccct gggcggcaag gctggcccgg tcggcaccag 1920

ttgcgtgagc ggaaagatgg ccgcttcccg gccctgctgc agggagctca aaatggagga 1980

cgcggcgctc gggagagcgg gcgggtgagt cacccacaca aaggaaaagg gcctttccgt 2040

cctcagccgt cgcttcatgt gactccacgg agtaccgggc gccgtccagg cacctcgatt 2100

agttctcgag cttttggagt acgtcgtctt taggttgggg ggaggggttt tatgcgatgg 2160

agtttcccca cactgagtgg gtggagactg aagttaggcc agcttggcac ttgatgtaat 2220

tctccttgga atttgccctt tttgagtttg gatcttggtt cattctcaag cctcagacag 2280

tggttcaaag tttttttctt ccatttcag 2309

<210> 38
<211> 1717
<212> DNA
<213> Artificial Sequence

<220>
<223> CMV-CDK9-U2-mROSA

<400> 38

gcgttacata acttacggta aatggcccgc ctggctgacc gcccaacgac ccccgcccat 60
tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct ccccctggcc tctcgggaac 480

tacaagtccc agggggcctg gcggtgggcg gcgggcggaa gaggcggggt cggcgccgcg 540
aggccggaag tggccgtgga ggcggaagtg gcgcggccgc ggagggggct ggagtgcggc 600
ggcggcggga cccggagcag gagcggcggc agcagcgact gggggcggcg gcggcgcgtt 660
ggaggcggcc ggatccacta gtgtttaaac agagtaatga catggttcct tccatcctcc 720
aaaggtgacc aataatagtt tgtaagtatc attatgaact aatgaatttt caacatattt 780
gatatatttc aatccattgc catcattgtt cttatcgata tttgagttgg ctcactttgc 840
cagtaagagt ctattcaaat tggcttctga gtccatttga cacaacacct ttgatctttg 900
acagtttcct tggttttagg tgctagatga tttctcaggc tcaccttaga catttcctgc 960
cacagactta gaatcagcca tttctctaag gaccctgatt ccatttcatg agaaatgata 1020
gagaccacaa tcaaaacaag tcatgaattt atactgatat tttcaattca aattaaagat 1080
gaggtttttg ctaaattttt ttgagtttat atttgtatgt cttatgctga aaaatcttgt 1140
ttcctaatta gtaacataat tattcatttg atgggtaaat attttagggc cgattctttg 1200
gttttatagc caagataccc tgttgataaa gtcttgtggg agcaattata agactggctt 1260
attttgaagc tttttaaaaa agacatcctt acctgtttta actgtagatt atattaactt 1320
aaataggtac agcccacgct tgactagttc agagagcctc ggctaggtag gggatcggga 1380
ctctggcggg agggcggctt ggtgcgtttg cggggatggg cggccgcggc aggccctccg 1440
agcgtggtgg agccgttctg tgagacagcc gggtacgagt cgtgacgctg gaaggggcaa 1500
gcgggtggtg ggcaggaatg cggtccgccc tgcagcaacc ggagggggag ggagaaggga 1560
gcggaaaagt ctccaccgga cgcggccatg gctcgggggg gggggggcag cggaggagcg 1620
cttccggccg acgtctcgtc gctgattggc ttcttttcct cccgccgtgt gtgaaaacac 1680
aattgtacta accttcttct ctttcctctc ctgacag 1717

<210> 39
<211> 2584

<212> DNA
<213> Artificial Sequence

<220>
<223> CMV-CDK9-U2-hROSA

<400> 39

```
gcgttacata acttacggta aatggcccgc ctggctgacc gcccaacgac ccccgcccat        60
tgacgtcaat aatgacgtat gttcccatag taacgccaat agggactttc cattgacgtc       120
aatgggtgga gtatttacgg taaactgccc acttggcagt acatcaagtg tatcatatgc       180
caagtacgcc ccctattgac gtcaatgacg gtaaatggcc cgcctggcat tatgcccagt       240
acatgacctt atgggacttt cctacttggc agtacatcta cgtattagtc atcgctatta       300
```

ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct cccccctggcc tctcgggaac 480
tacaagtccc agggggcctg gcggtgggcg gcgggcggaa gaggcggggt cggcgccgcg 540
aggccggaag tggccgtgga ggcggaagtg gcgcggccgc ggaggggcct ggagtgcggc 600
ggcggcggga cccggagcag gagcggcggc agcagcgact gggggcggcg gcggcgcgtt 660
ggaggcggcc ggatccacta gtgtttaaac agagtaatga catggttcct tccatcctcc 720
aaaggtgacc aataatagtt tgtaagtatc attatgaact aatgaatttt caacatattt 780
gatatatttc aatccattgc catcattgtt cttatcgata tttgagttgg ctcactttgc 840
cagtaagagt ctattcaaat tggcttctga gtccatttga cacaacacct ttgatctttg 900
acagtttcct tggttttagg tgctagatga tttctcaggc tcaccttaga catttcctgc 960
cacagactta gaatcagcca tttctctaag gaccctgatt ccatttcatg agaaatgata 1020
gagaccacaa tcaaaacaag tcatgaattt atactgatat tttcaattca aattaaagat 1080
gaggtttttg ctaaattttt ttgagtttat atttgtatgt cttatgctga aaaatcttgt 1140
ttcctaatta gtaacataat tattcatttg atgggtaaat attttagggc cgattctttg 1200
gttttatagc caagataccc tgttgataaa gtcttgtggg agcaattata agactggctt 1260
attttgaagc tttttaaaaa agacatcctt acctgtttta actgtagatt atattaactt 1320
aaataggtac agcccacgct tgactagtct cagagagcct cggctaggta ggggagcgga 1380
actctggtgg gaggggaggt gcggtgcact gggggggatgg gtggctaggg gggccgtctg 1440
gtggcttgcg gggggttgcct ttcccgtggg aagtcgggaa cataatgttt gttacgttgg 1500
gagggaaagg ggtggctgga tgcaggcggg agggaggccc gccctgcggc aaccggaggg 1560
ggagggagaa gggagcggaa aatgctcgaa accggacgga gccattgctc tcgcagaggg 1620
aggagcgctt ccggctagcc tcttgtcgcc gattggccgt ttctcctccc gccgtgtgtg 1680
aaaacacaaa tggcgtattc tggttggagt aaagctcctg tcagttacac cgtcgggagt 1740
acgcagccgc ttagcgactc tcgcgttgcc ccctgggtgg ggcgggtagg taggtggggt 1800
gtagagatgc tgggtgtgcg ggcgcggccg gcctcctgcg gcgggagggg agggtcagtg 1860
aaattggctc tggcgcgggc gtcctcccac cctcccccttc cttcgggggga gtcggtttac 1920
ccgccgcctg cttgtcttcg acacctgatt ggctgtcgaa gctgtgggac cgggcccttg 1980
ctactggctc gagtctcaca tgagcgaaac cactgcgcgg ggcgcggggg tggcggggag 2040
gcgggcgttg gtacggtcct ccccgaggcc gagcgccgca gtgtctggcc ccgcgcccct 2100
gcgcaacgtg gcaggaagcg cgcgctggag gcgggggcgg gctgccggcc gagacttctg 2160
gatggcggcg gccgcggctc cgccccgggt tcccaccgcc tgaagggcga gacaagcccg 2220

acctgctaca ggcactcgtg ggggtggggg aggagcgggg gtcggtccgg ctggtttgtg 2280

ggtgggaggc gcttgttctc caaaaaccgg cgcgagctgc aatcctgagg gagctgcggt 2340

ggaggaggtg gagagaaggc cgcacccttc tgggcagggg gaggggagtg ccgcaatacc 2400

tttatgggag ttctttgctg cctcccgtct tgtaaggacc gccctgggcc tggaagaagc 2460

cctccctcct ttcctcctcg cgtgatctcg tcatcgcctc catgtcgagt cgcttctcga 2520

ttatgggcgg gattcttttg cctagacaat tgtactaacc ttcttctctt tcctctcctg 2580

acag 2584

<210> 40
<211> 1964
<212> DNA
<213> Artificial Sequence

<220>
<223> CMV-CDK9-U3-EF1

<400> 40

gcgttacata acttacggta aatggcccgc ctggctgacc gcccaacgac ccccgcccat 60
tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct ccccctggcc tctcgggaac 480
tacaagtccc agggggcctg gcggtgggcg gcgggcggaa gaggcggggt cggcgccgcg 540
aggccggaag tggccgtgga ggcggaagtg gcgcggccgc ggaggggcct ggagtgcggc 600
ggcggcggga cccggagcag gagcggcggc agcagcgact gggggcggcg gcggcgcgtt 660
ggaggcggcc ggatccgttt aacgctggtg ggtagggatg agggagggag gggcattgtg 720
atgtacaggg ctgctctgtg agatcaaggg tctcttaagg gtgggagctg gggcagggac 780
tacgagagca gccagatggg ctgaaagtgg aactcaaggg gtttctggca cctacctacc 840
tgcttcccgc tggggggtgg ggagttggcc cagagtctta agattggggc agggtggaga 900
ggtgggctct tcctgcttcc cactcatctt atagctttct ttccccagat ccgaattcga 960
gatccaaacc aaggaggaaa ggatatcaca gaggagagct agtcgggttt gccgccagaa 1020
cacaggtaag tgccgtgtgt ggttcccgcg ggcctggcct ctttacgggt tatggccctt 1080
gcgtgccttg aattacttcc acctggctgc agtacgtgat tcttgatccc gagcttcggg 1140

ttggaagtgg gtgggagagt tcgaggcctt gcgcttaagg agccccttcg cctcgtgctt 1200

gagttgaggc ctggcctggg cgctggggcc gccgcgtgcg aatctggtgg caccttcgcg 1260

cctgtctcgc tgctttcgat aagtctctag ccatttaaaa tttttgatga cctgctgcga 1320

cgcttttttt ctggcaagat agtcttgtaa atgcgggcca agatctgcac actggtattt 1380

cggtttttgg ggccgcgggc ggcgacgggg cccgtgcgtc ccagcgcaca tgttcggcga 1440

ggcggggcct gcgagcgcgg ccaccgagaa tcggacgggg gtagtctcaa gctggccggc 1500

ctgctctggt gcctggcctc gcgccgccgt gtatcgcccc gccctgggcg gcaaggctgg 1560

cccggtcggc accagttgcg tgagcggaaa gatggccgct tcccggccct gctgcaggga 1620

gctcaaaatg gaggacgcgg cgctcgggag agcgggcggg tgagtcaccc acacaaagga 1680

aaagggcctt tccgtcctca gccgtcgctt catgtgactc cacggagtac cgggcgccgt 1740

ccaggcacct cgattagttc tcgagctttt ggagtacgtc gtctttaggt tggggggagg 1800

ggttttatgc gatggagttt ccccacactg agtgggtgga gactgaagtt aggccagctt 1860

ggcacttgat gtaattctcc ttggaatttg ccctttttga gtttggatct tggttcattc 1920

tcaagcctca gacagtggtt caaagttttt ttcttccatt tcag 1964

<210> 41
<211> 1372
<212> DNA
<213> Artificial Sequence

<220>
<223> DMV-CDK9-U3-mROSA

<400> 41

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
tacaagtccc agggggcctg gcggtgggcg gcgggcggaa gaggcggggt cggcgccgcg     540
aggccggaag tggccgtgga ggcggaagtg gcgcggccgc ggaggggcct ggagtgcggc     600
ggcggcggga cccggagcag gagcggcggc agcagcgact gggggcggcg gcggcgcgtt     660
ggaggcggcc ggatccgttt aacgctggtg ggtagggatg agggagggag gggcattgtg     720
atgtacaggg ctgctctgtg agatcaaggg tctcttaagg gtgggagctg gggcagggac     780
tacgagagca gccagatggg ctgaaagtgg aactcaaggg gtttctggca cctacctacc     840
tgcttcccgc tgggggggtgg ggagttggcc cagagtctta agattggggc agggtggaga     900
ggtgggctct tcctgcttcc cactcatctt atagctttct ttccccagat ccgaattcga     960
gatccaaacc aaggaggaaa ggatatcaca gaggagagct agttcagaga gcctcggcta    1020
ggtaggggat cgggactctg gcgggagggc ggcttggtgc gtttgcgggg atgggcggcc    1080
gcggcaggcc ctccgagcgt ggtggagccg ttctgtgaga cagccgggta cgagtcgtga    1140
cgctggaagg ggcaagcggg tggtgggcag gaatgcggtc cgccctgcag caaccggagg    1200
gggagggaga agggagcgga aaagtctcca ccggacgcgg ccatggctcg gggggggggg    1260
ggcagcggag gagcgcttcc ggccgacgtc tcgtcgctga ttggcttctt ttcctcccgc    1320
cgtgtgtgaa aacacaattg tactaacctt cttctctttc ctctcctgac ag            1372
```

<210> 42
<211> 2239
<212> DNA
<213> Artificial Sequence

<220>
<223> CMV-CDK9-U3-hROSA

<400> 42

gcgttacata acttacggta aatggcccgc ctggctgacc gcccaacgac ccccgcccat 60
tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct ccccctggcc tctcgggaac 480
tacaagtccc agggggcctg gcggtgggcg gcgggcggaa gaggcggggt cggcgccgcg 540
aggccggaag tggccgtgga ggcggaagtg gcgcggccgc ggagggggcct ggagtgcggc 600
ggcggcggga cccggagcag gagcggcggc agcagcgact gggggcggcg gcggcgcgtt 660
ggaggcggcc ggatccgttt aacgctggtg ggtagggatg agggagggag gggcattgtg 720
atgtacaggg ctgctctgtg agatcaaggg tctcttaagg gtgggagctg gggcagggac 780
tacgagagca gccagatggg ctgaaagtgg aactcaaggg gtttctggca cctacctacc 840
tgcttcccgc tgggggggtgg ggagttggcc cagagtctta agattggggc agggtggaga 900
ggtgggctct tcctgcttcc cactcatctt atagctttct ttccccagat ccgaattcga 960

gatccaaacc aaggaggaaa ggatatcaca gaggagagct agtctcagag agcctcggct 1020

aggtagggga gcggaactct ggtgggaggg gaggtgcggt gcactggggg gatgggtggc 1080

tagggggggcc gtctggtggc ttgcgggggt tgcctttccc gtgggaagtc gggaacataa 1140

tgtttgttac gttgggaggg aaaggggtgg ctggatgcag gcgggaggga ggcccgccct 1200

gcggcaaccg gagggggagg gagaagggag cggaaaatgc tcgaaaccgg acggagccat 1260

tgctctcgca gagggaggag cgcttccggc tagcctcttg tcgccgattg gccgtttctc 1320

ctcccgccgt gtgtgaaaac acaaatggcg tattctggtt ggagtaaagc tcctgtcagt 1380

tacaccgtcg ggagtacgca gccgcttagc gactctcgcg ttgcccctg ggtggggcgg 1440

gtaggtaggt ggggtgtaga gatgctgggt gtgcgggcgc ggccggcctc ctgcggcggg 1500

aggggagggt cagtgaaatt ggctctggcg cgggcgtcct cccaccctcc ccttccttcg 1560

ggggagtcgg tttacccgcc gcctgcttgt cttcgacacc tgattggctg tcgaagctgt 1620

gggaccgggc ccttgctact ggctcgagtc tcacatgagc gaaaccactg cgcggggcgc 1680

gggggtggcg gggaggcggg cgttggtacg gtcctccccg aggccgagcg ccgcagtgtc 1740

tggccccgcg cccctgcgca acgtggcagg aagcgcgcgc tggaggcggg ggcgggctgc 1800

cggccgagac ttctggatgg cggcggccgc ggctccgccc cgggttccca ccgcctgaag 1860

ggcgagacaa gcccgacctg ctacaggcac tcgtgggggt gggggaggag cgggggtcgg 1920

tccggctggt ttgtgggtgg gaggcgcttg ttctccaaaa accggcgcga gctgcaatcc 1980

tgagggagct gcggtggagg aggtggagag aaggccgcac ccttctgggc agggggaggg 2040

gagtgccgca ataccttat gggagttctt tgctgcctcc cgtcttgtaa ggaccgccct 2100

gggcctggaa gaagccctcc ctcctttcct cctcgcgtga tctcgtcatc gcctccatgt 2160

cgagtcgctt ctcgattatg ggcgggattc ttttgcctag acaattgtac taaccttctt 2220

ctctttcctc tcctgacag 2239

<210> 43
<211> 2584
<212> DNA
<213> Artificial Sequence

<220>
<223> CMV-CDK9-U1U2-EF1

<400> 43

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
```

ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct cccccctggcc tctcgggaac 480
tacaagtccc agggggcctg gcggtgggcg gcgggcggaa gaggcggggt cggcgccgcg 540
aggccggaag tggccgtgga ggcggaagtg gcgcggccgc ggaggggcct ggagtgcggc 600
ggcggcggga cccggagcag gagcggcggc agcagcgact gggggcggcg gcggcgcgtt 660
ggaggcggcc ggatccgttt aaacggctcg catctctcct tcacgcgccc gccgccctac 720
ctgaggccgc catccacgcc ggttgagtcg cgttctgccg cctcccgcct gtggtgcctc 780
ctgaactgcg tccgccgtct aggtaagttt aaagctcagg tcgagaccgg gcctttgtcc 840
ggcgctccct tggagcctac ctagactcag ccggctctcc acgctttgcc tgaccctgct 900
tgctcaactc tacgtctttg tttcgttttc tgttctgcgc cgttacagat cactagtgtt 960
taaacagagt aatgacatgg ttccttccat cctccaaagg tgaccaataa tagtttgtaa 1020
gtatcattat gaactaatga attttcaaca tatttgatat atttcaatcc attgccatca 1080
ttgttcttat cgatatttga gttggctcac tttgccagta agagtctatt caaattggct 1140
tctgagtcca tttgacacaa cacctttgat ctttgacagt ttccttggtt ttaggtgcta 1200
gatgatttct caggctcacc ttagacattt cctgccacag acttagaatc agccatttct 1260
ctaaggaccc tgattccatt tcatgagaaa tgatagagac cacaatcaaa acaagtcatg 1320
aatttatact gatattttca attcaaatta aagatgaggt tttgctaaa ttttttttgag 1380
tttatatttg tatgtcttat gctgaaaaat cttgtttcct aattagtaac ataattattc 1440
atttgatggg taaatatttt agggccgatt ctttggtttt atagccaaga taccctgttg 1500
ataaagtctt gtgggagcaa ttataagact ggcttatttt gaagcttttt aaaaaagaca 1560
tccttacctg ttttaactgt agattatatt aacttaaata ggtacagccc acgcttgact 1620
agtcgggttt gccgccagaa cacaggtaag tgccgtgtgt ggttcccgcg ggcctggcct 1680
ctttacgggt tatggccctt gcgtgccttg aattacttcc acctggctgc agtacgtgat 1740
tcttgatccc gagcttcggg ttggaagtgg gtgggagagt tcgaggcctt gcgcttaagg 1800
agccccttcg cctcgtgctt gagttgaggc ctggcctggg cgctggggcc gccgcgtgcg 1860
aatctggtgg caccttcgcg cctgtctcgc tgctttcgat aagtctctag ccatttaaaa 1920
tttttgatga cctgctgcga cgcttttttt ctggcaagat agtcttgtaa atgcgggcca 1980
agatctgcac actggtattt cggtttttgg ggccgcgggc ggcgacgggg cccgtgcgtc 2040
ccagcgcaca tgttcggcga ggcggggcct gcgagcgcgg ccaccgagaa tcggacgggg 2100
gtagtctcaa gctggccggc ctgctctggt gcctggcctc gcgccgccgt gtatcgcccc 2160

gccctgggcg gcaaggctgg cccggtcggc accagttgcg tgagcggaaa gatggccgct 2220

tcccggccct gctgcaggga gctcaaaatg gaggacgcgg cgctcgggag agcgggcggg 2280

tgagtcaccc acacaaagga aaagggcctt tccgtcctca gccgtcgctt catgtgactc 2340

cacggagtac cgggcgccgt ccaggcacct cgattagttc tcgagctttt ggagtacgtc 2400

gtctttaggt tggggggagg ggttttatgc gatggagttt ccccacactg agtgggtgga 2460

gactgaagtt aggccagctt ggcacttgat gtaattctcc ttggaatttg ccctttttga 2520

gtttggatct tggttcattc tcaagcctca gacagtggtt caaagttttt ttcttccatt 2580

tcag 2584

<210> 44
<211> 1992
<212> DNA
<213> Artificial Sequence

<220>
<223> CMV-CDK9-U1U2-mROSA

<400> 44

gcgttacata acttacggta aatggcccgc ctggctgacc gcccaacgac ccccgcccat 60
tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct ccccctggcc tctcgggaac 480
tacaagtccc aggggggcctg gcggtgggcg gcgggcggaa gaggcggggt cggcgccgcg 540
aggccggaag tggccgtgga ggcggaagtg gcgcggccgc ggaggggcct ggagtgcggc 600
ggcggcggga cccggagcag gagcggcggc agcagcgact gggggcggcg gcggcgcgtt 660
ggaggcggcc ggatccgttt aaacggctcg catctctcct tcacgcgccc gccgccctac 720
ctgaggccgc catccacgcc ggttgagtcg cgttctgccg cctcccgcct gtggtgcctc 780
ctgaactgcg tccgccgtct aggtaagttt aaagctcagg tcgagaccgg gcctttgtcc 840
ggcgctccct tggagcctac ctagactcag ccggctctcc acgctttgcc tgaccctgct 900
tgctcaactc tacgtctttg tttcgttttc tgttctgcgc cgttacagat cactagtgtt 960
taaacagagt aatgacatgg ttccttccat cctccaaagg tgaccaataa tagtttgtaa 1020
gtatcattat gaactaatga attttcaaca tatttgatat atttcaatcc attgccatca 1080
ttgttcttat cgatatttga gttggctcac tttgccagta agagtctatt caaattggct 1140

tctgagtcca tttgacacaa cacctttgat ctttgacagt ttccttggtt ttaggtgcta 1200

gatgatttct caggctcacc ttagacattt cctgccacag acttagaatc agccatttct 1260

ctaaggaccc tgattccatt tcatgagaaa tgatagagac cacaatcaaa acaagtcatg 1320

aatttatact gatattttca attcaaatta aagatgaggt ttttgctaaa ttttttttgag 1380

tttatatttg tatgtcttat gctgaaaaat cttgtttcct aattagtaac ataattattc 1440

atttgatggg taaatatttt agggccgatt ctttggtttt atagccaaga taccctgttg 1500

ataaagtctt gtgggagcaa ttataagact ggcttatttt gaagcttttt aaaaaagaca 1560

tccttacctg ttttaactgt agattatatt aacttaaata ggtacagccc acgcttgact 1620

agttcagaga gcctcggcta ggtaggggat cgggactctg gcgggagggc ggcttggtgc 1680

gtttgcgggg atgggcggcc gcggcaggcc ctccgagcgt ggtggagccg ttctgtgaga 1740

cagccgggta cgagtcgtga cgctggaagg ggcaagcggg tggtgggcag gaatgcggtc 1800

cgccctgcag caaccggagg gggagggaga agggagcgga aaagtctcca ccggacgcgg 1860

ccatggctcg gggggggggg ggcagcggag gagcgcttcc ggccgacgtc tcgtcgctga 1920

ttggcttctt ttcctcccgc cgtgtgtgaa aacacaattg tactaacctt cttctctttc 1980

ctctcctgac ag 1992

<210> 45
<211> 2859
<212> DNA
<213> Artificial Sequence

<220>
<223> CMV-CDK9-U1U2-hROSA

<400> 45

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
tacaagtccc aggggcctg gcggtgggcg gcgggcggaa gaggcggggt cggcgccgcg      540
aggccggaag tggccgtgga ggcggaagtg gcgcggccgc ggaggggcct ggagtgcggc     600
ggcggcggga cccggagcag gagcggcggc agcagcgact gggggcggcg gcggcgcgtt     660
```

ggaggcggcc ggatccgttt aaacggctcg catctctcct tcacgcgccc gccgccctac 720
ctgaggccgc catccacgcc ggttgagtcg cgttctgccg cctcccgcct gtggtgcctc 780
ctgaactgcg tccgccgtct aggtaagttt aaagctcagg tcgagaccgg gcctttgtcc 840
ggcgctccct tggagcctac ctagactcag ccggctctcc acgctttgcc tgaccctgct 900
tgctcaactc tacgtctttg tttcgttttc tgttctgcgc cgttacagat cactagtgtt 960
taaacagagt aatgacatgg ttccttccat cctccaaagg tgaccaataa tagtttgtaa 1020
gtatcattat gaactaatga attttcaaca tatttgatat atttcaatcc attgccatca 1080
ttgttcttat cgatatttga gttggctcac tttgccagta agagtctatt caaattggct 1140
tctgagtcca tttgacacaa cacctttgat ctttgacagt ttccttggtt ttaggtgcta 1200
gatgatttct caggctcacc ttagacattt cctgccacag acttagaatc agccatttct 1260
ctaaggaccc tgattccatt tcatgagaaa tgatagagac cacaatcaaa acaagtcatg 1320
aatttatact gatattttca attcaaatta aagatgaggt ttttgctaaa ttttttttgag 1380
tttatatttg tatgtcttat gctgaaaaat cttgtttcct aattagtaac ataattattc 1440
atttgatggg taaatatttt agggccgatt ctttggtttt atagccaaga taccctgttg 1500
ataaagtctt gtgggagcaa ttataagact ggcttatttt gaagcttttt aaaaaagaca 1560
tccttacctg ttttaactgt agattatatt aacttaaata ggtacagccc acgcttgact 1620
agtctcagag agcctcggct aggtagggga gcggaactct ggtgggaggg gaggtgcggt 1680
gcactggggg gatgggtggc taggggggcc gtctggtggc ttgcgggggt tgcctttccc 1740
gtgggaagtc gggaacataa tgtttgttac gttgggaggg aaaggggtgg ctggatgcag 1800
gcgggaggga ggcccgccct gcggcaaccg gagggggagg gagaagggag cggaaaatgc 1860
tcgaaaccgg acggagccat tgctctcgca gagggaggag cgcttccggc tagcctcttg 1920
tcgccgattg gccgtttctc ctcccgccgt gtgtgaaaac acaaatggcg tattctggtt 1980
ggagtaaagc tcctgtcagt tacaccgtcg ggagtacgca gccgcttagc gactctcgcg 2040
ttgcccctg ggtggggcgg gtaggtaggt ggggtgtaga gatgctgggt gtgcgggcgc 2100
ggccggcctc ctgcggcggg aggggagggt cagtgaaatt ggctctggcg cgggcgtcct 2160
cccaccctcc ccttccttcg ggggagtcgg tttacccgcc gcctgcttgt cttcgacacc 2220
tgattggctg tcgaagctgt gggaccgggc ccttgctact ggctcgagtc tcacatgagc 2280
gaaaccactg cgcggggcgc gggggtggcg gggaggcggg cgttggtacg gtcctccccg 2340
aggccgagcg ccgcagtgtc tggccccgcg cccctgcgca acgtggcagg aagcgcgcgc 2400
tggaggcggg ggcgggctgc cggccgagac ttctggatgg cggcggccgc ggctccgccc 2460
cgggttccca ccgcctgaag ggcgagacaa gcccgacctg ctacaggcac tcgtgggggt 2520
gggggaggag cgggggtcgg tccggctggt ttgtgggtgg gaggcgcttg ttctccaaaa 2580

accggcgcga gctgcaatcc tgagggagct gcggtggagg aggtggagag aaggccgcac 2640

ccttctgggc agggggaggg gagtgccgca ataccttat gggagttctt tgctgcctcc 2700

cgtcttgtaa ggaccgccct gggcctggaa gaagccctcc ctcctttcct cctcgcgtga 2760

tctcgtcatc gcctccatgt cgagtcgctt ctcgattatg ggcgggattc ttttgcctag 2820

acaattgtac taaccttctt ctctttcctc tcctgacag 2859

<210> 46
<211> 2242
<212> DNA
<213> Artificial Sequence

<220>
<223> CMV-CDK9-U1U3-EF1

<400> 46

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
tacaagtccc agggggcctg gcggtgggcg gcgggcggaa gaggcggggt cggcgccgcg     540
aggccggaag tggccgtgga ggcggaagtg gcgcggccgc ggaggggcct ggagtgcggc     600
ggcggcggga cccggagcag gagcggcggc agcagcgact gggggcggcg gcggcgcgtt     660
ggaggcggcc ggatccgttt aaacggctcg catctctcct tcacgcgccc gccgccctac     720
ctgaggccgc catccacgcc ggttgagtcg cgttctgccg cctcccgcct gtggtgcctc     780
ctgaactgcg tccgccgtct aggtaagttt aaagctcagg tcgagaccgg gcctttgtcc     840
ggcgctccct tggagcctac ctagactcag ccggctctcc acgctttgcc tgaccctgct     900
tgctcaactc tacgtctttg tttcgttttc tgttctgcgc cgttacagat cactagttaa     960
cgctggtggg tagggatgag ggagggaggg gcattgtgat gtacagggct gctctgtgag    1020
atcaagggtc tcttaagggt gggagctggg gcagggacta cgagagcagc cagatgggct    1080
gaaagtggaa ctcaaggggt ttctggcacc tacctacctg cttcccgctg gggggtgggg    1140
agttggccca gagtcttaag attggggcag ggtggagagg tgggctcttc ctgcttccca    1200
ctcatcttat agctttcttt ccccagatcc gaattcgaga tccaaaccaa ggaggaaagg    1260
```

```
atatcacaga ggagagctag tcgggtttgc cgccagaaca caggtaagtg ccgtgtgtgg      1320
ttcccgcggg cctggcctct ttacgggtta tggcccttgc gtgccttgaa ttacttccac      1380
ctggctgcag tacgtgattc ttgatcccga gcttcgggtt ggaagtgggt gggagagttc      1440
gaggccttgc gcttaaggag ccccttcgcc tcgtgcttga gttgaggcct ggcctgggcg      1500
ctggggccgc cgcgtgcgaa tctggtggca ccttcgcgcc tgtctcgctg ctttcgataa      1560
gtctctagcc atttaaaatt tttgatgacc tgctgcgacg ctttttttct ggcaagatag      1620
tcttgtaaat gcgggccaag atctgcacac tggtatttcg gtttttgggg ccgcgggcgg      1680
cgacggggcc cgtgcgtccc agcgcacatg ttcggcgagg cggggcctgc gagcgcggcc      1740
accgagaatc ggacgggggt agtctcaagc tggccggcct gctctggtgc ctggcctcgc      1800
gccgccgtgt atcgccccgc cctgggcggc aaggctggcc cggtcggcac cagttgcgtg      1860
agcggaaaga tggccgcttc ccggccctgc tgcagggagc tcaaaatgga ggacgcggcg      1920
ctcgggagag cgggcgggtg agtcacccac acaaaggaaa agggcctttc cgtcctcagc      1980
cgtcgcttca tgtgactcca cggagtaccg ggcgccgtcc aggcacctcg attagttctc      2040
gagcttttgg agtacgtcgt ctttaggttg gggggagggg ttttatgcga tggagtttcc      2100
ccacactgag tgggtggaga ctgaagttag gccagcttgg cacttgatgt aattctcctt      2160
ggaatttgcc ctttttgagt ttggatcttg gttcattctc aagcctcaga cagtggttca      2220
aagttttttt cttccatttc ag                                               2242
```

<210> 47
<211> 1650
<212> DNA
<213> Artificial Sequence

<220>
<223> CMV-CDK9-U1U3-mROSA

<400> 47

gcgttacata acttacggta aatggcccgc ctggctgacc gcccaacgac ccccgcccat 60
tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct ccccctggcc tctcgggaac 480
tacaagtccc agggggcctg gcggtgggcg gcgggcggaa gaggcggggt cggcgccgcg 540
aggccggaag tggccgtgga ggcggaagtg gcgcggccgc ggaggggcct ggagtgcggc 600
ggcggcggga cccggagcag gagcggcggc agcagcgact gggggcggcg gcggcgcgtt 660
ggaggcggcc ggatccgttt aaacggctcg catctctcct tcacgcgccc gccgccctac 720
ctgaggccgc catccacgcc ggttgagtcg cgttctgccg cctcccgcct gtggtgcctc 780
ctgaactgcg tccgccgtct aggtaagttt aaagctcagg tcgagaccgg gcctttgtcc 840
ggcgctccct tggagcctac ctagactcag ccggctctcc acgctttgcc tgaccctgct 900
tgctcaactc tacgtctttg tttcgttttc tgttctgcgc cgttacagat cactagttaa 960
cgctggtggg tagggatgag ggagggaggg gcattgtgat gtacagggct gctctgtgag 1020
atcaagggtc tcttaagggt gggagctggg gcagggacta cgagagcagc cagatgggct 1080
gaaagtggaa ctcaaggggt ttctggcacc tacctacctg cttcccgctg gggggtgggg 1140
agttggccca gagtcttaag attggggcag ggtggagagg tgggctcttc ctgcttccca 1200
ctcatcttat agctttcttt ccccagatcc gaattcgaga tccaaaccaa ggaggaaagg 1260
atatcacaga ggagagctag ttcagagagc ctcggctagg taggggatcg ggactctggc 1320
gggagggcgg cttggtgcgt ttgcggggat gggcggccgc ggcaggccct ccgagcgtgg 1380
tggagccgtt ctgtgagaca gccgggtacg agtcgtgacg ctggaagggg caagcgggtg 1440
gtgggcagga atgcggtccg ccctgcagca accggagggg gagggagaag ggagcggaaa 1500
agtctccacc ggacgcggcc atggctcggg gggggggggg cagcggagga gcgcttccgg 1560
ccgacgtctc gtcgctgatt ggcttctttt cctcccgccg tgtgtgaaaa cacaattgta 1620
ctaaccttct tctctttcct ctcctgacag 1650

<210> 48
<211> 2517
<212> DNA
<213> Artificial Sequence

<220>
<223> CMV-CDK9-U1U3-hROSA

<400> 48

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
```

tacaagtccc aggggggcctg gcggtgggcg gcgggcggaa gaggcggggt cggcgccgcg 540

aggccggaag tggccgtgga ggcggaagtg gcgcggccgc ggaggggcct ggagtgcggc 600

ggcggcggga cccggagcag gagcggcggc agcagcgact gggggcggcg gcggcgcgtt 660

ggaggcggcc ggatccgttt aaacggctcg catctctcct tcacgcgccc gccgccctac 720

ctgaggccgc catccacgcc ggttgagtcg cgttctgccg cctcccgcct gtggtgcctc 780

ctgaactgcg tccgccgtct aggtaagttt aaagctcagg tcgagaccgg gcctttgtcc 840

ggcgctccct tggagcctac ctagactcag ccggctctcc acgctttgcc tgaccctgct 900

tgctcaactc tacgtctttg tttcgttttc tgttctgcgc cgttacagat cactagttaa 960

cgctggtggg tagggatgag ggagggaggg gcattgtgat gtacagggct gctctgtgag 1020

atcaagggtc tcttaagggt gggagctggg gcagggacta cgagagcagc cagatgggct 1080

gaaagtggaa ctcaaggggt ttctggcacc tacctacctg cttcccgctg gggggtgggg 1140

agttggccca gagtcttaag attggggcag ggtggagagg tgggctcttc ctgcttccca 1200

ctcatcttat agctttcttt ccccagatcc gaattcgaga tccaaaccaa ggaggaaagg 1260

atatcacaga ggagagctag tctcagagag cctcggctag gtaggggagc ggaactctgg 1320

tgggagggga ggtgcggtgc actgggggga tgggtggcta ggggggccgt ctggtggctt 1380

gcgggggttg cctttcccgt gggaagtcgg gaacataatg tttgttacgt tgggagggaa 1440

aggggtggct ggatgcaggc gggagggagg cccgccctgc ggcaaccgga gggggaggga 1500

gaagggagcg gaaaatgctc gaaaccggac ggagccattg ctctcgcaga gggaggagcg 1560

cttccggcta gcctcttgtc gccgattggc cgtttctcct cccgccgtgt gtgaaaacac 1620

aaatggcgta ttctggttgg agtaaagctc ctgtcagtta caccgtcggg agtacgcagc 1680

cgcttagcga ctctcgcgtt gccccctggg tggggcgggt aggtaggtgg ggtgtagaga 1740

tgctgggtgt gcgggcgcgg ccggcctcct gcggcgggag gggagggtca gtgaaattgg 1800

ctctggcgcg ggcgtcctcc caccctcccc ttccttcggg ggagtcggtt tacccgccgc 1860

ctgcttgtct tcgacacctg attggctgtc gaagctgtgg gaccgggccc ttgctactgg 1920

ctcgagtctc acatgagcga aaccactgcg cggggcgcgg gggtggcggg gaggcgggcg 1980

ttggtacggt cctccccgag gccgagcgcc gcagtgtctg gccccgcgcc cctgcgcaac 2040

gtggcaggaa gcgcgcgctg gaggcggggg cgggctgccg gccgagactt ctggatggcg 2100

gcggccgcgg ctccgccccg ggttcccacc gcctgaaggg cgagacaagc ccgacctgct 2160

acaggcactc gtgggggtgg gggaggagcg ggggtcggtc cggctggttt gtgggtggga 2220

ggcgcttgtt ctccaaaaac cggcgcgagc tgcaatcctg agggagctgc ggtggaggag 2280

gtggagagaa ggccgcaccc ttctgggcag ggggagggga gtgccgcaat acctttatgg 2340

gagttctttg ctgcctcccg tcttgtaagg accgccctgg gcctggaaga agccctccct 2400

cctttcctcc tcgcgtgatc tcgtcatcgc ctccatgtcg agtcgcttct cgattatggg 2460

cgggattctt ttgcctagac aattgtacta accttcttct ctttcctctc ctgacag 2517

<210> 49
<211> 2627
<212> DNA
<213> Artificial Sequence

<220>
<223> CMV-CDK9-U2U3-EF1

<400> 49

gcgttacata acttacggta aatggcccgc ctggctgacc gcccaacgac ccccgcccat 60
tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct ccccctggcc tctcgggaac 480
tacaagtccc agggggcctg gcggtgggcg gcgggcggaa gaggcggggt cggcgccgcg 540
aggccggaag tggccgtgga ggcggaagtg gcgcggccgc ggaggggcct ggagtgcggc 600
ggcggcggga cccggagcag gagcggcggc agcagcgact gggggcggcg gcggcgcgtt 660
ggaggcggcc ggatccgttt aaacagagta atgacatggt tccttccatc ctccaaaggt 720
gaccaataat agtttgtaag tatcattatg aactaatgaa ttttcaacat atttgatata 780
tttcaatcca ttgccatcat tgttcttatc gatatttgag ttggctcact ttgccagtaa 840
gagtctattc aaattggctt ctgagtccat ttgacacaac acctttgatc tttgacagtt 900
tccttggttt taggtgctag atgatttctc aggctcacct tagacatttc ctgccacaga 960
cttagaatca gccatttctc taaggaccct gattccattt catgagaaat gatagagacc 1020
acaatcaaaa caagtcatga atttatactg atattttcaa ttcaaattaa agatgaggtt 1080
tttgctaaat ttttttgagt ttatatttgt atgtcttatg ctgaaaaatc ttgtttccta 1140
attagtaaca taattattca tttgatgggt aaatatttta gggccgattc tttggtttta 1200
tagccaagat accctgttga taaagtcttg tgggagcaat tataagactg gcttattttg 1260
aagcttttta aaaaagacat ccttacctgt tttaactgta gattatatta acttaaatag 1320
gtacagccca cgcttgacta gttaacgctg gtgggtaggg atgagggagg gaggggcatt 1380
gtgatgtaca gggctgctct gtgagatcaa gggtctctta agggtgggag ctggggcagg 1440

gactacgaga gcagccagat gggctgaaag tggaactcaa ggggtttctg gcacctacct 1500

acctgcttcc cgctgggggg tggggagttg gcccagagtc ttaagattgg ggcagggtgg 1560

agaggtgggc tcttcctgct tcccactcat cttatagctt tctttcccca gatccgaatt 1620

cgagatccaa accaaggagg aaaggatatc acagaggaga gctagtcggg tttgccgcca 1680

gaacacaggt aagtgccgtg tgtggttccc gcgggcctgg cctctttacg ggttatggcc 1740

cttgcgtgcc ttgaattact tccacctggc tgcagtacgt gattcttgat cccgagcttc 1800

gggttggaag tgggtgggag agttcgaggc cttgcgctta aggagcccct tcgcctcgtg 1860

cttgagttga ggcctggcct gggcgctggg gccgccgcgt gcgaatctgg tggcaccttc 1920

gcgcctgtct cgctgctttc gataagtctc tagccattta aaatttttga tgacctgctg 1980

cgacgctttt tttctggcaa gatagtcttg taaatgcggg ccaagatctg cacactggta 2040

tttcggtttt tggggccgcg ggcggcgacg gggcccgtgc gtcccagcgc acatgttcgg 2100

cgaggcgggg cctgcgagcg cggccaccga gaatcggacg ggggtagtct caagctggcc 2160

ggcctgctct ggtgcctggc ctcgcgccgc cgtgtatcgc cccgccctgg gcggcaaggc 2220

tggcccggtc ggcaccagtt gcgtgagcgg aaagatggcc gcttcccggc cctgctgcag 2280

ggagctcaaa atggaggacg cggcgctcgg gagagcgggc gggtgagtca cccacacaaa 2340

ggaaaagggc ctttccgtcc tcagccgtcg cttcatgtga ctccacggag taccgggcgc 2400

cgtccaggca cctcgattag ttctcgagct tttggagtac gtcgtcttta ggttgggggg 2460

aggggtttta tgcgatggag tttccccaca ctgagtgggt ggagactgaa gttaggccag 2520

cttggcactt gatgtaattc tccttggaat ttgccctttt tgagtttgga tcttggttca 2580

ttctcaagcc tcagacagtg gttcaaagtt tttttcttcc atttcag 2627

<210> 50
<211> 2035
<212> DNA
<213> Artificial Sequence

<220>
<223> CMV-CDK9-U2U3-mROSA

<400> 50

```
gcgttacata acttacggta aatggcccgc ctggctgacc gcccaacgac ccccgcccat        60
tgacgtcaat aatgacgtat gttcccatag taacgccaat agggactttc cattgacgtc       120
aatgggtgga gtatttacgg taaactgccc acttggcagt acatcaagtg tatcatatgc       180
caagtacgcc ccctattgac gtcaatgacg gtaaatggcc cgcctggcat tatgcccagt       240
acatgacctt atgggacttt cctacttggc agtacatcta cgtattagtc atcgctatta       300
ccatggcccg ggtcgcgaca tgcagcggga cgcgccaccc cgagccccag ctccggcgcc       360
ccggctcccc gcgcccccga tcggggccgc cgctagtagt ggcggcggcg gaggcggggg       420
```

cagcggcggc ggcggcggag gcgcctctgc agctccggct ccccctggcc tctcgggaac 480

tacaagtccc agggggcctg gcggtgggcg gcgggcggaa gaggcggggt cggcgccgcg 540

aggccggaag tggccgtgga ggcggaagtg gcgcggccgc ggaggggcct ggagtgcggc 600

ggcggcggga cccggagcag gagcggcggc agcagcgact gggggcggcg gcggcgcgtt 660

ggaggcggcc ggatccgttt aaacagagta atgacatggt tccttccatc ctccaaaggt 720

gaccaataat agtttgtaag tatcattatg aactaatgaa ttttcaacat atttgatata 780

tttcaatcca ttgccatcat tgttcttatc gatatttgag ttggctcact ttgccagtaa 840

gagtctattc aaattggctt ctgagtccat ttgacacaac acctttgatc tttgacagtt 900

tccttggttt taggtgctag atgatttctc aggctcacct tagacatttc ctgccacaga 960

cttagaatca gccatttctc taaggaccct gattccattt catgagaaat gatagagacc 1020

acaatcaaaa caagtcatga atttatactg atattttcaa ttcaaattaa agatgaggtt 1080

tttgctaaat ttttttgagt ttatatttgt atgtcttatg ctgaaaaatc ttgtttccta 1140

attagtaaca taattattca tttgatgggt aaatatttta gggccgattc tttggtttta 1200

tagccaagat accctgttga taaagtcttg tgggagcaat tataagactg gcttattttg 1260

aagcttttta aaaaagacat ccttacctgt tttaactgta gattatatta acttaaatag 1320

gtacagccca cgcttgacta gttaacgctg gtgggtaggg atgagggagg gaggggcatt 1380

gtgatgtaca gggctgctct gtgagatcaa gggtctctta agggtgggag ctggggcagg 1440

gactacgaga gcagccagat gggctgaaag tggaactcaa ggggtttctg gcacctacct 1500

acctgcttcc cgctgggggg tggggagttg gcccagagtc ttaagattgg ggcagggtgg 1560

agaggtgggc tcttcctgct tcccactcat cttatagctt tctttcccca gatccgaatt 1620

cgagatccaa accaaggagg aaaggatatc acagaggaga gctagttcag agagcctcgg 1680

ctaggtaggg gatcgggact ctggcgggag ggcggcttgg tgcgtttgcg ggatgggcg 1740

gccgcggcag gccctccgag cgtggtggag ccgttctgtg agacagccgg gtacgagtcg 1800

tgacgctgga aggggcaagc gggtggtggg caggaatgcg gtccgccctg cagcaaccgg 1860

agggggaggg agaagggagc ggaaaagtct ccaccggacg cggccatggc tcgggggggg 1920

gggggcagcg gaggagcgct tccggccgac gtctcgtcgc tgattggctt cttttcctcc 1980

cgccgtgtgt gaaaacacaa ttgtactaac cttcttctct ttcctctcct gacag 2035

<210> 51
<211> 2902
<212> DNA
<213> Artificial Sequence

<220>
<223> CMV-CDK9-U2U3-hROSA

<400> 51

gcgttacata acttacggta aatggcccgc ctggctgacc gcccaacgac ccccgcccat 60

tgacgtcaat aatgacgtat gttcccatag taacgccaat agggactttc cattgacgtc 120

aatgggtgga gtatttacgg taaactgccc acttggcagt acatcaagtg tatcatatgc 180

caagtacgcc ccctattgac gtcaatgacg gtaaatggcc cgcctggcat tatgcccagt 240

acatgacctt atgggacttt cctacttggc agtacatcta cgtattagtc atcgctatta 300

ccatggcccg ggtcgcgaca tgcagcggga cgcgccaccc cgagccccag ctccggcgcc 360

ccggctcccc gcgcccccga tcggggccgc cgctagtagt ggcggcggcg gaggcggggg 420

cagcggcggc ggcggcggag gcgcctctgc agctccggct ccccctggcc tctcgggaac 480

tacaagtccc aggggggcctg gcggtgggcg gcgggcggaa gaggcggggt cggcgccgcg 540

aggccggaag tggccgtgga ggcggaagtg gcgcggccgc ggagggggcct ggagtgcggc 600

ggcggcggga cccggagcag gagcggcggc agcagcgact gggggcggcg gcggcgcgtt 660

ggaggcggcc ggatccgttt aaacagagta atgacatggt tccttccatc ctccaaaggt 720

gaccaataat agtttgtaag tatcattatg aactaatgaa ttttcaacat atttgatata 780

tttcaatcca ttgccatcat tgttcttatc gatatttgag ttggctcact ttgccagtaa 840

gagtctattc aaattggctt ctgagtccat ttgacacaac acctttgatc tttgacagtt 900

tccttggttt taggtgctag atgatttctc aggctcacct tagacatttc ctgccacaga 960

cttagaatca gccatttctc taaggaccct gattccattt catgagaaat gatagagacc 1020

acaatcaaaa caagtcatga atttatactg atattttcaa ttcaaattaa agatgaggtt 1080

tttgctaaat ttttttgagt ttatatttgt atgtcttatg ctgaaaaatc ttgtttccta 1140

attagtaaca taattattca tttgatgggt aaatatttta gggccgattc tttggtttta 1200

tagccaagat accctgttga taaagtcttg tgggagcaat tataagactg gcttattttg 1260

aagcttttta aaaaagacat ccttacctgt tttaactgta gattatatta acttaaatag 1320

gtacagccca cgcttgacta gttaacgctg gtgggtaggg atgagggagg gaggggcatt 1380

gtgatgtaca gggctgctct gtgagatcaa gggtctctta agggtgggag ctggggcagg 1440

gactacgaga gcagccagat gggctgaaag tggaactcaa ggggtttctg gcacctacct 1500

acctgcttcc cgctgggggg tggggagttg gcccagagtc ttaagattgg ggcagggtgg 1560

agaggtgggc tcttcctgct tcccactcat cttatagctt tctttcccca gatccgaatt 1620

cgagatccaa accaaggagg aaaggatatc acagaggaga gctagtctca gagagcctcg 1680

gctaggtagg ggagcggaac tctggtggga ggggaggtgc ggtgcactgg ggggatgggt 1740

ggctaggggg gccgtctggt ggcttgcggg ggttgccttt cccgtgggaa gtcgggaaca 1800

taatgtttgt tacgttggga gggaaagggg tggctggatg caggcgggag ggaggcccgc 1860

cctgcggcaa ccggaggggg agggagaagg gagcggaaaa tgctcgaaac cggacggagc 1920

cattgctctc gcagagggag gagcgcttcc ggctagcctc ttgtcgccga ttggccgttt 1980

ctcctcccgc cgtgtgtgaa aacacaaatg gcgtattctg gttggagtaa agctcctgtc 2040

agttacaccg tcgggagtac gcagccgctt agcgactctc gcgttgcccc ctgggtgggg 2100

cgggtaggta ggtggggtgt agagatgctg ggtgtgcggg cgcggccggc ctcctgcggc 2160

gggaggggag ggtcagtgaa attggctctg gcgcgggcgt cctcccaccc tccccttcct 2220

tcgggggagt cggtttaccc gccgcctgct tgtcttcgac acctgattgg ctgtcgaagc 2280

tgtgggaccg ggcccttgct actggctcga gtctcacatg agcgaaacca ctgcgcgggg 2340

cgcgggggtg gcggggaggc gggcgttggt acggtcctcc ccgaggccga gcgccgcagt 2400

gtctggcccc gcgcccctgc gcaacgtggc aggaagcgcg cgctggaggc gggggcgggc 2460

tgccggccga gacttctgga tggcggcggc cgcggctccg ccccgggttc ccaccgcctg 2520

aagggcgaga caagcccgac ctgctacagg cactcgtggg ggtgggggag gagcggggt 2580

cggtccggct ggtttgtggg tgggaggcgc ttgttctcca aaaaccggcg cgagctgcaa 2640

tcctgaggga gctgcggtgg aggaggtgga gagaaggccg caccccttctg ggcaggggga 2700

ggggagtgcc gcaatacctt tatgggagtt ctttgctgcc tcccgtcttg taaggaccgc 2760

cctgggcctg gaagaagccc tccctccttt cctcctcgcg tgatctcgtc atcgcctcca 2820

tgtcgagtcg cttctcgatt atgggcggga ttcttttgcc tagacaattg tactaacctt 2880

cttctctttc ctctcctgac ag 2902

<210> 52
<211> 2908
<212> DNA
<213> Artificial Sequence

<220>
<223> CMV-CDK9-U1U2U3-EF1

<400> 52

```
gcgttacata acttacggta aatggcccgc ctggctgacc gcccaacgac ccccgcccat        60
tgacgtcaat aatgacgtat gttcccatag taacgccaat agggactttc cattgacgtc       120
aatgggtgga gtatttacgg taaactgccc acttggcagt acatcaagtg tatcatatgc       180
caagtacgcc ccctattgac gtcaatgacg gtaaatggcc cgcctggcat tatgcccagt       240
acatgacctt atgggacttt cctacttggc agtacatcta cgtattagtc atcgctatta       300
ccatggcccg ggtcgcgaca tgcagcggga cgcgccaccc cgagccccag ctccggcgcc       360
ccggctcccc gcgcccccga tcggggccgc cgctagtagt ggcggcggcg gaggcggggg       420
cagcggcggc ggcggcggag gcgcctctgc agctccggct ccccctggcc tctcgggaac       480
```

tacaagtccc aggggggcctg gcggtgggcg gcgggcggaa gaggcggggt cggcgccgcg 540
aggccggaag tggccgtgga ggcggaagtg gcgcggccgc ggaggggcct ggagtgcggc 600
ggcggcggga cccggagcag gagcggcggc agcagcgact gggggcggcg gcggcgcgtt 660
ggaggcggcc ggatccgttt aaacggctcg catctctcct tcacgcgccc gccgccctac 720
ctgaggccgc catccacgcc ggttgagtcg cgttctgccg cctcccgcct gtggtgcctc 780
ctgaactgcg tccgccgtct aggtaagttt aaagctcagg tcgagaccgg gcctttgtcc 840
ggcgctccct tggagcctac ctagactcag ccggctctcc acgctttgcc tgaccctgct 900
tgctcaactc tacgtctttg tttcgttttc tgttctgcgc cgttacagat cactagtgtt 960
taaacagagt aatgacatgg ttccttccat cctccaaagg tgaccaataa tagtttgtaa 1020
gtatcattat gaactaatga attttcaaca tatttgatat atttcaatcc attgccatca 1080
ttgttcttat cgatatttga gttggctcac tttgccagta agagtctatt caaattggct 1140
tctgagtcca tttgacacaa cacctttgat ctttgacagt ttccttggtt ttaggtgcta 1200
gatgatttct caggctcacc ttagacattt cctgccacag acttagaatc agccatttct 1260
ctaaggaccc tgattccatt tcatgagaaa tgatagagac cacaatcaaa acaagtcatg 1320
aatttatact gatattttca attcaaatta aagatgaggt ttttgctaaa tttttttgag 1380
tttatatttg tatgtcttat gctgaaaaat cttgtttcct aattagtaac ataattattc 1440
atttgatggg taaatatttt agggccgatt ctttggtttt atagccaaga taccctgttg 1500
ataaagtctt gtgggagcaa ttataagact ggcttatttt gaagcttttt aaaaaagaca 1560
tccttacctg ttttaactgt agattatatt aacttaaata ggtacagccc acgcttgact 1620
agttaacgct ggtgggtagg gatgagggag ggaggggcat tgtgatgtac agggctgctc 1680
tgtgagatca agggtctctt aagggtggga gctggggcag ggactacgag agcagccaga 1740
tgggctgaaa gtggaactca aggggtttct ggcacctacc tacctgcttc ccgctggggg 1800
gtggggagtt ggcccagagt cttaagattg ggcagggtg gagaggtggg ctcttcctgc 1860
ttcccactca tcttatagct ttctttcccc agatccgaat tcgagatcca aaccaaggag 1920
gaaaggatat cacagaggag agctagtcgg gtttgccgcc agaacacagg taagtgccgt 1980
gtgtggttcc cgcgggcctg gcctctttac gggttatggc ccttgcgtgc cttgaattac 2040
ttccacctgg ctgcagtacg tgattcttga tcccgagctt cgggttggaa gtgggtggga 2100
gagttcgagg ccttgcgctt aaggagcccc ttcgcctcgt gcttgagttg aggcctggcc 2160
tgggcgctgg ggccgccgcg tgcgaatctg gtggcacctt cgcgcctgtc tcgctgcttt 2220
cgataagtct ctagccattt aaaatttttg atgacctgct gcgacgcttt ttttctggca 2280
agatagtctt gtaaatgcgg gccaagatct gcacactggt atttcggttt ttggggccgc 2340
gggcggcgac ggggcccgtg cgtcccagcg cacatgttcg gcgaggcggg gcctgcgagc 2400

gcggccaccg agaatcggac gggggtagtc tcaagctggc cggcctgctc tggtgcctgg 2460
cctcgcgccg ccgtgtatcg ccccgccctg ggcggcaagg ctggcccggt cggcaccagt 2520
tgcgtgagcg gaaagatggc cgcttcccgg ccctgctgca gggagctcaa aatggaggac 2580
gcggcgctcg ggagagcggg cgggtgagtc acccacacaa aggaaaaggg cctttccgtc 2640
ctcagccgtc gcttcatgtg actccacgga gtaccgggcg ccgtccaggc acctcgatta 2700
gttctcgagc ttttggagta cgtcgtcttt aggttggggg gaggggtttt atgcgatgga 2760
gtttccccac actgagtggg tggagactga agttaggcca gcttggcact tgatgtaatt 2820
ctccttggaa tttgcccttt ttgagtttgg atcttggttc attctcaagc ctcagacagt 2880
ggttcaaagt ttttttcttc catttcag 2908

<210> 53
<211> 2316
<212> DNA
<213> Artificial Sequence

<220>
<223> CMV-CDK9-U1U2U3-mROSA

<400> 53

gcgttacata acttacggta aatggcccgc ctggctgacc gcccaacgac ccccgcccat 60
tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct ccccctggcc tctcgggaac 480
tacaagtccc agggggcctg gcggtgggcg gcgggcggaa gaggcggggt cggcgccgcg 540
aggccggaag tggccgtgga ggcggaagtg gcgcggccgc ggaggggcct ggagtgcggc 600
ggcggcggga cccggagcag gagcggcggc agcagcgact gggggcggcg gcggcgcgtt 660
ggaggcggcc ggatccgttt aaacggctcg catctctcct tcacgcgccc gccgccctac 720
ctgaggccgc catccacgcc ggttgagtcg cgttctgccg cctcccgcct gtggtgcctc 780
ctgaactgcg tccgccgtct aggtaagttt aaagctcagg tcgagaccgg gcctttgtcc 840
ggcgctccct tggagcctac ctagactcag ccggctctcc acgctttgcc tgaccctgct 900
tgctcaactc tacgtctttg tttcgttttc tgttctgcgc cgttacagat cactagtgtt 960
taaacagagt aatgacatgg ttccttccat cctccaaagg tgaccaataa tagtttgtaa 1020

gtatcattat gaactaatga attttcaaca tatttgatat atttcaatcc attgccatca 1080
ttgttcttat cgatatttga gttggctcac tttgccagta agagtctatt caaattggct 1140
tctgagtcca tttgacacaa cacctttgat ctttgacagt ttccttggtt ttaggtgcta 1200
gatgatttct caggctcacc ttagacattt cctgccacag acttagaatc agccatttct 1260
ctaaggaccc tgattccatt tcatgagaaa tgatagagac cacaatcaaa acaagtcatg 1320
aatttatact gatattttca attcaaatta aagatgaggt ttttgctaaa ttttttttgag 1380
tttatatttg tatgtcttat gctgaaaaat cttgtttcct aattagtaac ataattattc 1440
atttgatggg taaatatttt agggccgatt ctttggtttt atagccaaga taccctgttg 1500
ataaagtctt gtgggagcaa ttataagact ggcttatttt gaagcttttt aaaaaagaca 1560
tccttacctg ttttaactgt agattatatt aacttaaata ggtacagccc acgcttgact 1620
agttaacgct ggtgggtagg gatgagggag ggaggggcat tgtgatgtac agggctgctc 1680
tgtgagatca agggtctctt aagggtggga gctggggcag ggactacgag agcagccaga 1740
tgggctgaaa gtggaactca aggggtttct ggcacctacc tacctgcttc ccgctggggg 1800
gtggggagtt ggcccagagt cttaagattg gggcagggtg gagaggtggg ctcttcctgc 1860
ttcccactca tcttatagct ttctttcccc agatccgaat tcgagatcca aaccaaggag 1920
gaaaggatat cacagaggag agctagttca gagagcctcg gctaggtagg ggatcgggac 1980
tctggcggga gggcggcttg gtgcgtttgc ggggatgggc ggccgcggca ggccctccga 2040
gcgtggtgga gccgttctgt gagacagccg ggtacgagtc gtgacgctgg aaggggcaag 2100
cgggtggtgg gcaggaatgc ggtccgccct gcagcaaccg gagggggagg gagaagggag 2160
cggaaaagtc tccaccggac gcggccatgg ctcggggggg gggggcagc ggaggagcgc 2220
ttccggccga cgtctcgtcg ctgattggct tcttttcctc ccgccgtgtg tgaaaacaca 2280
attgtactaa ccttcttctc tttcctctcc tgacag 2316

<210> 54
<211> 3183
<212> DNA
<213> Artificial Sequence

<220>
<223> CMV-CDK9-U12U3-hROSA

<400> 54

gcgttacata acttacggta aatggcccgc ctggctgacc gcccaacgac ccccgcccat 60

tgacgtcaat aatgacgtat gttcccatag taacgccaat agggactttc cattgacgtc 120

aatgggtgga gtatttacgg taaactgccc acttggcagt acatcaagtg tatcatatgc 180

caagtacgcc ccctattgac gtcaatgacg gtaaatggcc cgcctggcat tatgcccagt 240

acatgacctt atgggacttt cctacttggc agtacatcta cgtattagtc atcgctatta 300

ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct cccccctggcc tctcgggaac 480
tacaagtccc agggggcctg gcggtgggcg gcgggcggaa gaggcggggt cggcgccgcg 540
aggccggaag tggccgtgga ggcggaagtg gcgcggccgc ggaggggcct ggagtgcggc 600
ggcggcggga cccggagcag gagcggcggc agcagcgact gggggcggcg gcggcgcgtt 660
ggaggcggcc ggatccgttt aaacggctcg catctctcct tcacgcgccc gccgccctac 720
ctgaggccgc catccacgcc ggttgagtcg cgttctgccg cctcccgcct gtggtgcctc 780
ctgaactgcg tccgccgtct aggtaagttt aaagctcagg tcgagaccgg gcctttgtcc 840
ggcgctccct tggagcctac ctagactcag ccggctctcc acgctttgcc tgaccctgct 900
tgctcaactc tacgtctttg tttcgttttc tgttctgcgc cgttacagat cactagtgtt 960
taaacagagt aatgacatgg ttccttccat cctccaaagg tgaccaataa tagtttgtaa 1020
gtatcattat gaactaatga attttcaaca tatttgatat atttcaatcc attgccatca 1080
ttgttcttat cgatatttga gttggctcac tttgccagta agagtctatt caaattggct 1140
tctgagtcca tttgacacaa cacctttgat ctttgacagt ttccttggtt ttaggtgcta 1200
gatgatttct caggctcacc ttagacattt cctgccacag acttagaatc agccatttct 1260
ctaaggaccc tgattccatt tcatgagaaa tgatagagac cacaatcaaa acaagtcatg 1320
aatttatact gatattttca attcaaatta aagatgaggt tttgctaaa ttttttttgag 1380
tttatatttg tatgtcttat gctgaaaaat cttgtttcct aattagtaac ataattattc 1440
atttgatggg taaatatttt agggccgatt ctttggtttt atagccaaga taccctgttg 1500
ataaagtctt gtgggagcaa ttataagact ggcttatttt gaagcttttt aaaaaagaca 1560
tccttacctg ttttaactgt agattatatt aacttaaata ggtacagccc acgcttgact 1620
agttaacgct ggtgggtagg gatgagggag ggagggggcat tgtgatgtac agggctgctc 1680
tgtgagatca agggtctctt aagggtggga gctggggcag ggactacgag agcagccaga 1740
tgggctgaaa gtggaactca aggggtttct ggcacctacc tacctgcttc ccgctggggg 1800
gtggggagtt ggcccagagt cttaagattg gggcagggtg gagaggtggg ctcttcctgc 1860
ttcccactca tcttatagct ttctttcccc agatccgaat tcgagatcca aaccaaggag 1920
gaaaggatat cacagaggag agctagtctc agagagcctc ggctaggtag gggagcggaa 1980
ctctggtggg aggggaggtg cggtgcactg gggggatggg tggctagggg ggccgtctgg 2040
tggcttgcgg gggttgcctt tcccgtggga agtcgggaac ataatgtttg ttacgttggg 2100
agggaaaggg gtggctggat gcaggcggga gggaggcccg ccctgcggca accggagggg 2160

gagggagaag ggagcggaaa atgctcgaaa ccggacggag ccattgctct cgcagaggga 2220

ggagcgcttc cggctagcct cttgtcgccg attggccgtt tctcctcccg ccgtgtgtga 2280

aaacacaaat ggcgtattct ggttggagta aagctcctgt cagttacacc gtcgggagta 2340

cgcagccgct tagcgactct cgcgttgccc cctgggtggg gcgggtaggt aggtggggtg 2400

tagagatgct gggtgtgcgg gcgcggccgg cctcctgcgg cgggagggga gggtcagtga 2460

aattggctct ggcgcgggcg tcctcccacc ctccccttcc ttcgggggag tcggtttacc 2520

cgccgcctgc ttgtcttcga cacctgattg gctgtcgaag ctgtgggacc gggcccttgc 2580

tactggctcg agtctcacat gagcgaaacc actgcgcggg gcgcgggggt ggcggggagg 2640

cgggcgttgg tacggtcctc cccgaggccg agcgccgcag tgtctggccc cgcgcccctg 2700

cgcaacgtgg caggaagcgc gcgctggagg cgggggcggg ctgccggccg agacttctgg 2760

atggcggcgg ccgcggctcc gccccgggtt cccaccgcct gaagggcgag acaagcccga 2820

cctgctacag gcactcgtgg gggtggggga ggagcggggg tcggtccggc tggtttgtgg 2880

gtgggaggcg cttgttctcc aaaaaccggc gcgagctgca atcctgaggg agctgcggtg 2940

gaggaggtgg agagaaggcc gcacccttct gggcaggggg aggggagtgc cgcaatacct 3000

ttatgggagt tctttgctgc ctcccgtctt gtaaggaccg ccctgggcct ggaagaagcc 3060

ctccctcctt tcctcctcgc gtgatctcgt catcgcctcc atgtcgagtc gcttctcgat 3120

tatgggcggg attcttttgc ctagacaatt gtactaacct tcttctcttt cctctcctga 3180

cag 3183

<210> 55
<211> 1848
<212> **DNA**
<213> Artificial Sequence

<220>
<223> CMV-bActine-U1-EF1

<400> 55

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tggtcgaggt gagccccacg ttctgcttca ctctccccat     360
ctcccccccc tccccacccc caattttgta tttatttatt ttttaattat tttgtgcagc     420
gatgggggcg gggggggggg gggggcgcgc gccaggcggg gcggggcggg gcgaggggcg     480
gggcggggcg aggcggagag gtgcggcggc agccaatcag agcggcgcgc tccgaaagtt     540
```

tccttttatg gcgaggcggc ggcggcggcg gccctataaa aagcgaagcg cgcggcgggc 600

ggatccgttt aaacggctcg catctctcct tcacgcgccc gccgccctac ctgaggccgc 660

catccacgcc ggttgagtcg cgttctgccg cctcccgcct gtggtgcctc ctgaactgcg 720

tccgccgtct aggtaagttt aaagctcagg tcgagaccgg gcctttgtcc ggcgctccct 780

tggagcctac ctagactcag ccggctctcc acgctttgcc tgaccctgct tgctcaactc 840

tacgtctttg tttcgttttc tgttctgcgc cgttacagat cactagtcgg gtttgccgcc 900

agaacacagg taagtgccgt gtgtggttcc cgcgggcctg gcctctttac gggttatggc 960

ccttgcgtgc cttgaattac ttccacctgg ctgcagtacg tgattcttga tcccgagctt 1020

cgggttggaa gtgggtggga gagttcgagg ccttgcgctt aaggagcccc ttcgcctcgt 1080

gcttgagttg aggcctggcc tgggcgctgg ggccgccgcg tgcgaatctg gtggcacctt 1140

cgcgcctgtc tcgctgcttt cgataagtct ctagccattt aaaatttttg atgacctgct 1200

gcgacgcttt ttttctggca agatagtctt gtaaatgcgg gccaagatct gcacactggt 1260

atttcggttt ttggggccgc gggcggcgac ggggcccgtg cgtcccagcg cacatgttcg 1320

gcgaggcggg gcctgcgagc gcggccaccg agaatcggac gggggtagtc tcaagctggc 1380

cggcctgctc tggtgcctgg cctcgcgccg ccgtgtatcg ccccgccctg ggcggcaagg 1440

ctggcccggt cggcaccagt tgcgtgagcg gaaagatggc cgcttcccgg ccctgctgca 1500

gggagctcaa aatggaggac gcggcgctcg ggagagcggg cgggtgagtc acccacacaa 1560

aggaaaaggg cctttccgtc ctcagccgtc gcttcatgtg actccacgga gtaccgggcg 1620

ccgtccaggc acctcgatta gttctcgagc ttttggagta cgtcgtcttt aggttggggg 1680

gaggggtttt atgcgatgga gtttccccac actgagtggg tggagactga agttaggcca 1740

gcttggcact tgatgtaatt ctccttggaa tttgcccttt ttgagtttgg atcttggttc 1800

attctcaagc ctcagacagt ggttcaaagt ttttttcttc catttcag 1848

<210> 56
<211> 1256
<212> DNA
<213> Artificial Sequence

<220>
<223> CMV-bActine-U1-mROSA

<400> 56

```
gcgttacata acttacggta aatggcccgc ctggctgacc gcccaacgac ccccgcccat        60
tgacgtcaat aatgacgtat gttcccatag taacgccaat agggactttc cattgacgtc       120
aatgggtgga gtatttacgg taaactgccc acttggcagt acatcaagtg tatcatatgc       180
caagtacgcc ccctattgac gtcaatgacg gtaaatggcc cgcctggcat tatgcccagt       240
acatgacctt atgggacttt cctacttggc agtacatcta cgtattagtc atcgctatta       300
ccatggcccg ggtcgcgaca tggtcgaggt gagccccacg ttctgcttca ctctccccat       360
ctcccccccc tccccacccc caattttgta tttatttatt ttttaattat tttgtgcagc       420
gatgggggcg gggggggggg gggggcgcgc gccaggcggg gcggggcggg gcgaggggcg       480
gggcggggcg aggcggagag gtgcggcggc agccaatcag agcggcgcgc tccgaaagtt       540
tccttttatg gcgaggcggc ggcggcggcg gccctataaa aagcgaagcg cgcggcgggc       600
ggatccgttt aaacggctcg catctctcct tcacgcgccc gccgccctac ctgaggccgc       660
catccacgcc ggttgagtcg cgttctgccg cctcccgcct gtggtgcctc ctgaactgcg       720
tccgccgtct aggtaagttt aaagctcagg tcgagaccgg gcctttgtcc ggcgctccct       780
tggagcctac ctagactcag ccggctctcc acgctttgcc tgaccctgct tgctcaactc       840
tacgtctttg tttcgttttc tgttctgcgc cgttacagat cactagttca gagagcctcg       900
gctaggtagg ggatcgggac tctggcggga gggcggcttg gtgcgtttgc ggggatgggc       960
ggccgcggca ggccctccga gcgtggtgga gccgttctgt gagacagccg ggtacgagtc      1020
gtgacgctgg aaggggcaag cgggtggtgg gcaggaatgc ggtccgccct gcagcaaccg      1080
gagggggagg gagaagggag cggaaaagtc tccaccggac gcggccatgg ctcggggggg      1140
gggggcagc ggaggagcgc ttccggccga cgtctcgtcg ctgattggct tcttttcctc      1200
ccgccgtgtg tgaaaacaca attgtactaa ccttcttctc tttcctctcc tgacag          1256
```

<210> 57
<211> 2123
<212> DNA
<213> Artificial Sequence

<220>
<223> CMV-bActine-U1-hROSA

<400> 57

```
gcgttacata acttacggta aatggcccgc ctggctgacc gcccaacgac ccccgcccat        60
tgacgtcaat aatgacgtat gttcccatag taacgccaat agggactttc cattgacgtc       120
aatgggtgga gtatttacgg taaactgccc acttggcagt acatcaagtg tatcatatgc       180
caagtacgcc ccctattgac gtcaatgacg gtaaatggcc cgcctggcat tatgcccagt       240
acatgacctt atgggacttt cctacttggc agtacatcta cgtattagtc atcgctatta       300
ccatggcccg ggtcgcgaca tggtcgaggt gagccccacg ttctgcttca ctctccccat       360
ctcccccccc tccccacccc caattttgta tttatttatt ttttaattat tttgtgcagc       420
gatgggggcg gggggggggg gggggcgcgc gccaggcggg gcggggcggg gcgaggggcg       480
gggcggggcg aggcggagag gtgcggcggc agccaatcag agcggcgcgc tccgaaagtt       540
tccttttatg gcgaggcggc ggcggcggcg gccctataaa aagcgaagcg cgcggcgggc       600
```

ggatccgttt aaacggctcg catctctcct tcacgcgccc gccgccctac ctgaggccgc 660

catccacgcc ggttgagtcg cgttctgccg cctcccgcct gtggtgcctc ctgaactgcg 720

tccgccgtct aggtaagttt aaagctcagg tcgagaccgg gcctttgtcc ggcgctccct 780

tggagcctac ctagactcag ccggctctcc acgctttgcc tgaccctgct tgctcaactc 840

tacgtctttg tttcgttttc tgttctgcgc cgttacagat cactagtctc agagagcctc 900

ggctaggtag gggagcggaa ctctggtggg aggggaggtg cggtgcactg gggggatggg 960

tggctagggg ggccgtctgg tggcttgcgg gggttgcctt tcccgtggga agtcgggaac 1020

ataatgtttg ttacgttggg agggaaaggg gtggctggat gcaggcggga gggaggcccg 1080

ccctgcggca accggagggg gagggagaag ggagcggaaa atgctcgaaa ccggacggag 1140

ccattgctct cgcagaggga ggagcgcttc cggctagcct cttgtcgccg attggccgtt 1200

tctcctcccg ccgtgtgtga aaacacaaat ggcgtattct ggttggagta aagctcctgt 1260

cagttacacc gtcgggagta cgcagccgct tagcgactct cgcgttgccc cctgggtggg 1320

gcgggtaggt aggtggggtg tagagatgct gggtgtgcgg gcgcggccgg cctcctgcgg 1380

cgggagggga gggtcagtga aattggctct ggcgcgggcg tcctcccacc ctccccttcc 1440

ttcgggggag tcggtttacc cgccgcctgc ttgtcttcga cacctgattg gctgtcgaag 1500

ctgtgggacc gggcccttgc tactggctcg agtctcacat gagcgaaacc actgcgcggg 1560

gcgcgggggt ggcggggagg cgggcgttgg tacggtcctc cccgaggccg agcgccgcag 1620

tgtctggccc cgcgcccctg cgcaacgtgg caggaagcgc gcgctggagg cgggggcggg 1680

ctgccggccg agacttctgg atggcggcgg ccgcggctcc gccccgggtt cccaccgcct 1740

gaagggcgag acaagcccga cctgctacag gcactcgtgg gggtggggga ggagcggggg 1800

tcggtccggc tggtttgtgg gtgggaggcg cttgttctcc aaaaaccggc gcgagctgca 1860

atcctgaggg agctgcggtg gaggaggtgg agagaaggcc gcacccttct gggcaggggg 1920

aggggagtgc cgcaatacct ttatgggagt tctttgctgc ctcccgtctt gtaaggaccg 1980

ccctgggcct ggaagaagcc ctccctcctt tcctcctcgc gtgatctcgt catcgcctcc 2040

atgtcgagtc gcttctcgat tatgggcggg attcttttgc ctagacaatt gtactaacct 2100

tcttctcttt cctctcctga cag 2123

<210> 58
<211> 2239
<212> DNA
<213> Artificial Sequence

<220>
<223> CMV-bActine-U2-EF1

<400> 58

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tggtcgaggt gagccccacg ttctgcttca ctctccccat     360
ctcccccccc tccccacccc caattttgta tttatttatt ttttaattat tttgtgcagc     420
gatgggggcg gggggggggg ggggcgcgc gccaggcggg gcggggcggg gcgaggggcg      480
gggcggggcg aggcggagag gtgcggcggc agccaatcag agcggcgcgc tccgaaagtt     540
tccttttatg gcgaggcggc ggcggcggcg gccctataaa aagcgaagcg cgcggcgggc     600
ggatccacta gtgtttaaac agagtaatga catggttcct tccatcctcc aaaggtgacc     660
aataatagtt tgtaagtatc attatgaact aatgaatttt caacatattt gatatatttc     720
aatccattgc catcattgtt cttatcgata tttgagttgg ctcactttgc cagtaagagt     780
ctattcaaat tggcttctga gtccatttga cacaacacct ttgatctttg acagtttcct     840
tggttttagg tgctagatga tttctcaggc tcaccttaga catttcctgc cacagactta     900
gaatcagcca tttctctaag gaccctgatt ccatttcatg agaaatgata gagaccacaa     960
tcaaaacaag tcatgaattt atactgatat tttcaattca aattaaagat gaggtttttg    1020
ctaaattttt ttgagtttat atttgtatgt cttatgctga aaaatcttgt ttcctaatta    1080
gtaacataat tattcatttg atgggtaaat attttagggc cgattctttg gttttatagc    1140
caagataccc tgttgataaa gtcttgtggg agcaattata agactggctt attttgaagc    1200
tttttaaaaa agacatcctt acctgtttta actgtagatt atattaactt aaataggtac    1260
agcccacgct tgactagtcg ggtttgccgc cagaacacag gtaagtgccg tgtgtggttc    1320
ccgcgggcct ggcctcttta cgggttatgg cccttgcgtg ccttgaatta cttccacctg    1380
gctgcagtac gtgattcttg atcccgagct tcgggttgga agtgggtggg agagttcgag    1440
gccttgcgct taaggagccc cttcgcctcg tgcttgagtt gaggcctggc ctgggcgctg    1500
gggccgccgc gtgcgaatct ggtggcacct tcgcgcctgt ctcgctgctt tcgataagtc    1560
tctagccatt taaaattttt gatgacctgc tgcgacgctt tttttctggc aagatagtct    1620
tgtaaatgcg ggccaagatc tgcacactgg tatttcggtt tttggggccg cgggcggcga    1680
cggggcccgt gcgtcccagc gcacatgttc ggcgaggcgg ggcctgcgag cgcggccacc    1740
gagaatcgga cgggggtagt ctcaagctgg ccggcctgct ctggtgcctg gcctcgcgcc    1800
gccgtgtatc gccccgccct gggcggcaag gctggcccgg tcggcaccag ttgcgtgagc    1860
ggaaagatgg ccgcttcccg gccctgctgc agggagctca aaatggagga cgcggcgctc    1920
```

```
gggagagcgg gcgggtgagt cacccacaca aaggaaaagg gcctttccgt cctcagccgt     1980
cgcttcatgt gactccacgg agtaccgggc gccgtccagg cacctcgatt agttctcgag     2040
cttttggagt acgtcgtctt taggttgggg ggaggggttt tatgcgatgg agtttcccca     2100
cactgagtgg gtggagactg aagttaggcc agcttggcac ttgatgtaat tctccttgga     2160
atttgccctt tttgagtttg gatcttggtt cattctcaag cctcagacag tggttcaaag     2220
tttttttctt ccatttcag                                                  2239
```

<210> 59
<211> 1647
<212> DNA
<213> Artificial Sequence

<220>
<223> CMV-bActine-U2-mROSA

<400> 59

gcgttacata acttacggta aatggcccgc ctggctgacc gcccaacgac ccccgcccat 60
tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tggtcgaggt gagccccacg ttctgcttca ctctccccat 360
ctccccccc tccccacccc caattttgta tttatttatt ttttaattat tttgtgcagc 420
gatgggggcg gggggggggg gggggcgcgc gccaggcggg gcggggcggg gcgaggggcg 480
gggcggggcg aggcggagag gtgcggcggc agccaatcag agcggcgcgc tccgaaagtt 540
tccttttatg gcgaggcggc ggcggcggcg gccctataaa aagcgaagcg cgcggcgggc 600
ggatccacta gtgtttaaac agagtaatga catggttcct tccatcctcc aaaggtgacc 660
aataatagtt tgtaagtatc attatgaact aatgaatttt caacatattt gatatatttc 720
aatccattgc catcattgtt cttatcgata tttgagttgg ctcactttgc cagtaagagt 780
ctattcaaat tggcttctga gtccatttga cacaacacct ttgatctttg acagtttcct 840
tggttttagg tgctagatga tttctcaggc tcaccttaga catttcctgc cacagactta 900
gaatcagcca tttctctaag gaccctgatt ccatttcatg agaaatgata gagaccacaa 960
tcaaaacaag tcatgaattt atactgatat tttcaattca aattaaagat gaggtttttg 1020
ctaaattttt ttgagtttat atttgtatgt cttatgctga aaaatcttgt ttcctaatta 1080
gtaacataat tattcatttg atgggtaaat attttagggc cgattctttg gttttatagc 1140
caagataccc tgttgataaa gtcttgtggg agcaattata agactggctt attttgaagc 1200

tttttaaaaa agacatcctt acctgtttta actgtagatt atattaactt aaataggtac 1260
agcccacgct tgactagttc agagagcctc ggctaggtag gggatcggga ctctggcggg 1320
agggcggctt ggtgcgtttg cggggatggg cggccgcggc aggccctccg agcgtggtgg 1380
agccgttctg tgagacagcc gggtacgagt cgtgacgctg gaaggggcaa gcgggtggtg 1440
ggcaggaatg cggtccgccc tgcagcaacc ggagggggag ggagaaggga gcggaaaagt 1500
ctccaccgga cgcggccatg gctcgggggg gggggggcag cggaggagcg cttccggccg 1560
acgtctcgtc gctgattggc ttcttttcct cccgccgtgt gtgaaaacac aattgtacta 1620
accttcttct ctttcctctc ctgacag 1647

<210> 60
<211> 2514
<212> DNA
<213> Artificial Sequence

<220>
<223> CMV-bActine-U2-hROSA

<400> 60

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tggtcgaggt gagccccacg ttctgcttca ctctccccat     360
ctcccccccc tccccacccc caattttgta tttatttatt ttttaattat tttgtgcagc     420
gatgggggcg gggggggggg gggggcgcgc gccaggcggg gcggggcggg gcgaggggcg     480
gggcggggcg aggcggagag gtgcggcggc agccaatcag agcggcgcgc tccgaaagtt     540
tccttttatg gcgaggcggc ggcggcggcg gccctataaa aagcgaagcg cgcggcgggc     600
ggatccacta gtgtttaaac agagtaatga catggttcct tccatcctcc aaaggtgacc     660
aataatagtt tgtaagtatc attatgaact aatgaatttt caacatattt gatatatttc     720
aatccattgc catcattgtt cttatcgata tttgagttgg ctcactttgc cagtaagagt     780
ctattcaaat tggcttctga gtccatttga cacaacacct ttgatctttg acagtttcct     840
tggttttagg tgctagatga tttctcaggc tcaccttaga catttcctgc cacagactta     900
gaatcagcca tttctctaag gaccctgatt ccatttcatg agaaatgata gagaccacaa     960
tcaaaacaag tcatgaattt atactgatat tttcaattca aattaaagat gaggtttttg    1020
ctaaattttt ttgagtttat atttgtatgt cttatgctga aaatcttgt ttcctaatta     1080
gtaacataat tattcatttg atgggtaaat attttagggc cgattctttg gttttatagc    1140
```

caagataccc tgttgataaa gtcttgtggg agcaattata agactggctt attttgaagc 1200
tttttaaaaa agacatcctt acctgtttta actgtagatt atattaactt aaataggtac 1260
agcccacgct tgactagtct cagagagcct cggctaggta ggggagcgga actctggtgg 1320
gaggggaggt gcggtgcact gggggggatgg gtggctaggg gggccgtctg gtggcttgcg 1380
ggggttgcct ttcccgtggg aagtcgggaa cataatgttt gttacgttgg gagggaaagg 1440
ggtggctgga tgcaggcggg agggaggccc gccctgcggc aaccggaggg ggagggagaa 1500
gggagcggaa aatgctcgaa accggacgga gccattgctc tcgcagaggg aggagcgctt 1560
ccggctagcc tcttgtcgcc gattggccgt ttctcctccc gccgtgtgtg aaaacacaaa 1620
tggcgtattc tggttggagt aaagctcctg tcagttacac cgtcgggagt acgcagccgc 1680
ttagcgactc tcgcgttgcc ccctgggtgg ggcgggtagg taggtggggt gtagagatgc 1740
tgggtgtgcg ggcgcggccg gcctcctgcg gcgggagggg agggtcagtg aaattggctc 1800
tggcgcgggc gtcctcccac cctccccttc cttcgggggga gtcggtttac ccgccgcctg 1860
cttgtcttcg acacctgatt ggctgtcgaa gctgtgggac cgggcccttg ctactggctc 1920
gagtctcaca tgagcgaaac cactgcgcgg ggcgcggggg tggcggggag gcgggcgttg 1980
gtacggtcct ccccgaggcc gagcgccgca gtgtctggcc ccgcgcccct gcgcaacgtg 2040
gcaggaagcg cgcgctggag gcgggggcgg gctgccggcc gagacttctg gatggcggcg 2100
gccgcggctc cgccccgggt tcccaccgcc tgaagggcga gacaagcccg acctgctaca 2160
ggcactcgtg gggggtgggg aggagcgggg gtcggtccgg ctggtttgtg ggtgggaggc 2220
gcttgttctc caaaaaccgg cgcgagctgc aatcctgagg gagctgcggt ggaggaggtg 2280
gagagaaggc cgcacccttc tgggcagggg gaggggagtg ccgcaatacc tttatgggag 2340
ttctttgctg cctcccgtct tgtaaggacc gccctgggcc tggaagaagc cctccctcct 2400
ttcctcctcg cgtgatctcg tcatcgcctc catgtcgagt cgcttctcga ttatgggcgg 2460
gattcttttg cctagacaat tgtactaacc ttcttctctt tcctctcctg acag 2514

<210> 61
<211> 1894
<212> DNA
<213> Artificial Sequence

<220>
<223> CMV-bActine-U3-EF1

<400> 61

gcgttacata acttacggta aatggcccgc ctggctgacc gcccaacgac ccccgcccat 60

tgacgtcaat aatgacgtat gttcccatag taacgccaat agggactttc cattgacgtc 120

aatgggtgga gtatttacgg taaactgccc acttggcagt acatcaagtg tatcatatgc 180

caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tggtcgaggt gagccccacg ttctgcttca ctctccccat 360
ctcccccccc tccccacccc caattttgta tttatttatt ttttaattat tttgtgcagc 420
gatgggggcg gggggggggg gggggcgcgc gccaggcggg gcggggcggg gcgaggggcg 480
gggcggggcg aggcggagag gtgcggcggc agccaatcag agcggcgcgc tccgaaagtt 540
tccttttatg gcgaggcggc ggcggcggcg gccctataaa aagcgaagcg cgcggcgggc 600
ggatccgttt aacgctggtg ggtagggatg agggagggag gggcattgtg atgtacaggg 660
ctgctctgtg agatcaaggg tctcttaagg gtgggagctg gggcagggac tacgagagca 720
gccagatggg ctgaaagtgg aactcaaggg gtttctggca cctacctacc tgcttcccgc 780
tgggggtgg ggagttggcc cagagtctta agattggggc agggtggaga ggtgggctct 840
tcctgcttcc cactcatctt atagctttct ttccccagat ccgaattcga gatccaaacc 900
aaggaggaaa ggatatcaca gaggagagct agtcgggttt gccgccagaa cacaggtaag 960
tgccgtgtgt ggttcccgcg ggcctggcct ctttacgggt tatggccctt gcgtgccttg 1020
aattacttcc acctggctgc agtacgtgat tcttgatccc gagcttcggg ttggaagtgg 1080
gtgggagagt tcgaggcctt gcgcttaagg agccccttcg cctcgtgctt gagttgaggc 1140
ctggcctggg cgctggggcc gccgcgtgcg aatctggtgg caccttcgcg cctgtctcgc 1200
tgctttcgat aagtctctag ccatttaaaa tttttgatga cctgctgcga cgcttttttt 1260
ctggcaagat agtcttgtaa atgcgggcca agatctgcac actggtattt cggtttttgg 1320
ggccgcgggc ggcgacgggg cccgtgcgtc ccagcgcaca tgttcggcga ggcggggcct 1380
gcgagcgcgg ccaccgagaa tcggacgggg gtagtctcaa gctggccggc ctgctctggt 1440
gcctggcctc gcgccgccgt gtatcgcccc gccctgggcg gcaaggctgg cccggtcggc 1500
accagttgcg tgagcggaaa gatggccgct tcccggccct gctgcaggga gctcaaaatg 1560
gaggacgcgg cgctcgggag agcgggcggg tgagtcaccc acacaaagga aaagggcctt 1620
tccgtcctca gccgtcgctt catgtgactc cacggagtac cgggcgccgt ccaggcacct 1680
cgattagttc tcgagctttt ggagtacgtc gtctttaggt tggggggagg ggttttatgc 1740
gatggagttt ccccacactg agtgggtgga gactgaagtt aggccagctt ggcacttgat 1800
gtaattctcc ttggaatttg ccctttttga gtttggatct tggttcattc tcaagcctca 1860
gacagtggtt caaagttttt ttcttccatt tcag 1894

<210> 62
<211> 1302
<212> DNA

<213> Artificial Sequence

<220>
<223> CMV-bActine-U3-mROSA

<400> 62

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tggtcgaggt gagccccacg ttctgcttca ctctccccat     360
ctcccccccc tccccacccc caattttgta tttatttatt ttttaattat tttgtgcagc     420
gatgggggcg gggggggggg gggggcgcgc gccaggcggg gcggggcggg gcgaggggcg     480
gggcggggcg aggcggagag gtgcggcggc agccaatcag agcggcgcgc tccgaaagtt     540
tccttttatg gcgaggcggc ggcggcggcg gccctataaa aagcgaagcg cgcggcgggc     600
ggatccgttt aacgctggtg ggtagggatg agggagggag gggcattgtg atgtacaggg     660
ctgctctgtg agatcaaggg tctcttaagg gtgggagctg gggcagggac tacgagagca     720
gccagatggg ctgaaagtgg aactcaaggg gtttctggca cctacctacc tgcttcccgc     780
tggggggtgg ggagttggcc cagagtctta agattggggc agggtggaga ggtgggctct     840
tcctgcttcc cactcatctt atagctttct ttccccagat ccgaattcga gatccaaacc     900
aaggaggaaa ggatatcaca gaggagagct agttcagaga gcctcggcta ggtaggggat     960
cgggactctg gcgggagggc ggcttggtgc gtttgcgggg atgggcggcc gcggcaggcc    1020
ctccgagcgt ggtggagccg ttctgtgaga cagccgggta cgagtcgtga cgctggaagg    1080
ggcaagcggg tggtgggcag gaatgcggtc cgccctgcag caaccggagg gggagggaga    1140
agggagcgga aaagtctcca ccggacgcgg ccatggctcg gggggggggg ggcagcggag    1200
gagcgcttcc ggccgacgtc tcgtcgctga ttggcttctt ttcctcccgc cgtgtgtgaa    1260
aacacaattg tactaacctt cttctctttc ctctcctgac ag                       1302
```

<210> 63
<211> 2169
<212> DNA
<213> Artificial Sequence

<220>
<223> CMV-bActine-U3-hROSA

<400> 63

```
gcgttacata acttacggta aatggcccgc ctggctgacc gcccaacgac ccccgcccat       60
tgacgtcaat aatgacgtat gttcccatag taacgccaat agggactttc cattgacgtc      120
```

aatgggtgga gtatttacgg taaactgccc acttggcagt acatcaagtg tatcatatgc 180

caagtacgcc ccctattgac gtcaatgacg gtaaatggcc cgcctggcat tatgcccagt 240

acatgacctt atgggacttt cctacttggc agtacatcta cgtattagtc atcgctatta 300

ccatggcccg ggtcgcgaca tggtcgaggt gagccccacg ttctgcttca ctctccccat 360

ctcccccccc tccccacccc caattttgta tttatttatt ttttaattat tttgtgcagc 420

gatgggggcg gggggggggg gggggcgcgc gccaggcggg gcggggcggg gcgaggggcg 480

gggcggggcg aggcggagag gtgcggcggc agccaatcag agcggcgcgc tccgaaagtt 540

tccttttatg gcgaggcggc ggcggcggcg gccctataaa aagcgaagcg cgcggcgggc 600

ggatccgttt aacgctggtg ggtagggatg agggagggag ggcattgtg atgtacaggg 660

ctgctctgtg agatcaaggg tctcttaagg gtgggagctg ggcagggac tacgagagca 720

gccagatggg ctgaaagtgg aactcaaggg gtttctggca cctacctacc tgcttcccgc 780

tggggggtgg ggagttggcc cagagtctta agattggggc agggtggaga ggtgggctct 840

tcctgcttcc cactcatctt atagctttct ttccccagat ccgaattcga gatccaaacc 900

aaggaggaaa ggatatcaca gaggagagct agtctcagag agcctcggct aggtagggga 960

gcggaactct ggtgggaggg gaggtgcggt gcactggggg gatgggtggc tagggggccc 1020

gtctggtggc ttgcgggggt tgcctttccc gtgggaagtc gggaacataa tgtttgttac 1080

gttgggaggg aaaggggtgg ctggatgcag gcgggaggga ggcccgccct gcggcaaccg 1140

gagggggagg gagaagggag cggaaaatgc tcgaaaccgg acggagccat tgctctcgca 1200

gagggaggag cgcttccggc tagcctcttg tcgccgattg gccgtttctc ctcccgccgt 1260

gtgtgaaaac acaaatggcg tattctggtt ggagtaaagc tcctgtcagt tacaccgtcg 1320

ggagtacgca gccgcttagc gactctcgcg ttgcccccctg ggtggggcgg gtaggtaggt 1380

ggggtgtaga gatgctgggt gtgcgggcgc ggccggcctc ctgcggcggg aggggagggt 1440

cagtgaaatt ggctctggcg cgggcgtcct cccaccctcc ccttccttcg gggagtcgg 1500

tttacccgcc gcctgcttgt cttcgacacc tgattggctg tcgaagctgt gggaccgggc 1560

ccttgctact ggctcgagtc tcacatgagc gaaaccactg cgcggggcgc gggggtggcg 1620

gggaggcggg cgttggtacg gtcctccccg aggccgagcg ccgcagtgtc tggccccgcg 1680

cccctgcgca acgtggcagg aagcgcgcgc tggaggcggg ggcgggctgc cggccgagac 1740

ttctggatgg cggcggccgc ggctccgccc cgggttccca ccgcctgaag ggcgagacaa 1800

gcccgacctg ctacaggcac tcgtgggggt gggggaggag cgggggtcgg tccggctggt 1860

ttgtgggtgg gaggcgcttg ttctccaaaa accggcgcga gctgcaatcc tgagggagct 1920

gcggtggagg aggtggagag aaggccgcac ccttctgggc agggggaggg gagtgccgca 1980

atacctttat gggagttctt tgctgcctcc cgtcttgtaa ggaccgccct gggcctggaa 2040

gaagccctcc ctcctttcct cctcgcgtga tctcgtcatc gcctccatgt cgagtcgctt 2100

ctcgattatg ggcgggattc ttttgcctag acaattgtac taaccttctt ctctttcctc 2160

tcctgacag 2169

<210> 64
<211> 2514
<212> DNA
<213> Artificial Sequence

<220>
<223> CMV-bActine-U1U2-EF1

<400> 64

gcgttacata acttacggta aatggcccgc ctggctgacc gcccaacgac ccccgcccat 60
tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tggtcgaggt gagccccacg ttctgcttca ctctccccat 360
ctcccccccc tccccacccc caattttgta tttatttatt ttttaattat tttgtgcagc 420
gatgggggcg gggggggggg gggggcgcgc gccaggcggg gcggggcggg gcgaggggcg 480
gggcggggcg aggcggagag gtgcggcggc agccaatcag agcggcgcgc tccgaaagtt 540
tccttttatg gcgaggcggc ggcggcggcg gccctataaa aagcgaagcg cgcggcgggc 600
ggatccgttt aaacggctcg catctctcct tcacgcgccc gccgccctac ctgaggccgc 660
catccacgcc ggttgagtcg cgttctgccg cctcccgcct gtggtgcctc ctgaactgcg 720
tccgccgtct aggtaagttt aaagctcagg tcgagaccgg gcctttgtcc ggcgctccct 780
tggagcctac ctagactcag ccggctctcc acgctttgcc tgaccctgct tgctcaactc 840
tacgtctttg tttcgttttc tgttctgcgc cgttacagat cactagtgtt taaacagagt 900
aatgacatgg ttccttccat cctccaaagg tgaccaataa tagtttgtaa gtatcattat 960
gaactaatga attttcaaca tatttgatat atttcaatcc attgccatca ttgttcttat 1020
cgatatttga gttggctcac tttgccagta agagtctatt caaattggct tctgagtcca 1080
tttgacacaa cacctttgat ctttgacagt ttccttggtt ttaggtgcta gatgatttct 1140
caggctcacc ttagacattt cctgccacag acttagaatc agccatttct ctaaggaccc 1200
tgattccatt tcatgagaaa tgatagagac cacaatcaaa acaagtcatg aatttatact 1260
gatattttca attcaaatta aagatgaggt ttttgctaaa ttttttgag tttatatttg 1320
tatgtcttat gctgaaaaat cttgtttcct aattagtaac ataattattc atttgatggg 1380

```
taaatatttt agggccgatt ctttggtttt atagccaaga taccctgttg ataaagtctt      1440
gtgggagcaa ttataagact ggcttatttt gaagcttttt aaaaaagaca tccttacctg      1500
ttttaactgt agattatatt aacttaaata ggtacagccc acgcttgact agtcgggttt      1560
gccgccagaa cacaggtaag tgccgtgtgt ggttcccgcg ggcctggcct ctttacgggt      1620
tatggccctt gcgtgccttg aattacttcc acctggctgc agtacgtgat tcttgatccc      1680
gagcttcggg ttggaagtgg gtgggagagt tcgaggcctt gcgcttaagg agccccttcg      1740
cctcgtgctt gagttgaggc ctggcctggg cgctggggcc gccgcgtgcg aatctggtgg      1800
caccttcgcg cctgtctcgc tgctttcgat aagtctctag ccatttaaaa tttttgatga      1860
cctgctgcga cgcttttttt ctggcaagat agtcttgtaa atgcgggcca agatctgcac      1920
actggtattt cggtttttgg ggccgcgggc ggcgacgggg cccgtgcgtc ccagcgcaca      1980
tgttcggcga ggcggggcct gcgagcgcgg ccaccgagaa tcggacgggg gtagtctcaa      2040
gctggccggc ctgctctggt gcctggcctc gcgccgccgt gtatcgcccc gccctgggcg      2100
gcaaggctgg cccggtcggc accagttgcg tgagcggaaa gatggccgct tcccggccct      2160
gctgcaggga gctcaaaatg gaggacgcgg cgctcgggag agcgggcggg tgagtcaccc      2220
acacaaagga aaagggcctt tccgtcctca gccgtcgctt catgtgactc cacggagtac      2280
cgggcgccgt ccaggcacct cgattagttc tcgagctttt ggagtacgtc gtctttaggt      2340
tgggggggagg ggttttatgc gatggagttt ccccacactg agtgggtgga gactgaagtt      2400
aggccagctt ggcacttgat gtaattctcc ttggaatttg ccctttttga gtttggatct      2460
tggttcattc tcaagcctca gacagtggtt caaagttttt ttcttccatt tcag            2514
```

<210> 65
<211> 1922
<212> DNA
<213> Artificial Sequence

<220>
<223> CMV-bActine-U1U2-mROSA

<400> 65

```
gcgttacata acttacggta aatggcccgc ctggctgacc gcccaacgac ccccgcccat        60
tgacgtcaat aatgacgtat gttcccatag taacgccaat agggactttc cattgacgtc       120
aatgggtgga gtatttacgg taaactgccc acttggcagt acatcaagtg tatcatatgc       180
caagtacgcc ccctattgac gtcaatgacg gtaaatggcc cgcctggcat tatgcccagt       240
acatgacctt atgggacttt cctacttggc agtacatcta cgtattagtc atcgctatta       300
ccatggcccg ggtcgcgaca tggtcgaggt gagccccacg ttctgcttca ctctccccat       360
ctccccccc tccccacccc caattttgta tttatttatt ttttaattat tttgtgcagc        420
gatgggggcg gggggggggg gggggcgcgc gccaggcggg gcggggcggg gcgaggggcg       480
```

gggcggggcg aggcggagag gtgcggcggc agccaatcag agcggcgcgc tccgaaagtt 540

tcctttttatg gcgaggcggc ggcggcggcg gccctataaa aagcgaagcg cgcggcgggc 600

ggatccgttt aaacggctcg catctctcct tcacgcgccc gccgccctac ctgaggccgc 660

catccacgcc ggttgagtcg cgttctgccg cctcccgcct gtggtgcctc ctgaactgcg 720

tccgccgtct aggtaagttt aaagctcagg tcgagaccgg gcctttgtcc ggcgctccct 780

tggagcctac ctagactcag ccggctctcc acgctttgcc tgaccctgct tgctcaactc 840

tacgtctttg tttcgttttc tgttctgcgc cgttacagat cactagtgtt taaacagagt 900

aatgacatgg ttccttccat cctccaaagg tgaccaataa tagtttgtaa gtatcattat 960

gaactaatga attttcaaca tatttgatat atttcaatcc attgccatca ttgttcttat 1020

cgatatttga gttggctcac tttgccagta agagtctatt caaattggct tctgagtcca 1080

tttgacacaa cacctttgat ctttgacagt ttccttggtt ttaggtgcta gatgatttct 1140

caggctcacc ttagacattt cctgccacag acttagaatc agccatttct ctaaggaccc 1200

tgattccatt tcatgagaaa tgatagagac cacaatcaaa acaagtcatg aatttatact 1260

gatattttca attcaaatta aagatgaggt ttttgctaaa ttttttttgag tttatatttg 1320

tatgtcttat gctgaaaaat cttgtttcct aattagtaac ataattattc atttgatggg 1380

taaatatttt agggccgatt ctttggtttt atagccaaga taccctgttg ataaagtctt 1440

gtgggagcaa ttataagact ggcttatttt gaagcttttt aaaaaagaca tccttacctg 1500

ttttaactgt agattatatt aacttaaata ggtacagccc acgcttgact agttcagaga 1560

gcctcggcta ggtaggggat cgggactctg gcgggagggc ggcttggtgc gtttgcgggg 1620

atgggcggcc gcggcaggcc ctccgagcgt ggtggagccg ttctgtgaga cagccgggta 1680

cgagtcgtga cgctggaagg ggcaagcggg tggtgggcag gaatgcggtc cgccctgcag 1740

caaccggagg gggagggaga agggagcgga aaagtctcca ccggacgcgg ccatggctcg 1800

gggggggggg ggcagcggag gagcgcttcc ggccgacgtc tcgtcgctga ttggcttctt 1860

ttcctcccgc cgtgtgtgaa aacacaattg tactaacctt cttctctttc ctctcctgac 1920

ag 1922

<210> 66
<211> 2789
<212> DNA
<213> Artificial Sequence

<220>
<223> CMV-bActine-U1U2-hROSA

<400> 66

gcgttacata acttacggta aatggcccgc ctggctgacc gcccaacgac ccccgcccat 60

tgacgtcaat aatgacgtat gttcccatag taacgccaat agggactttc cattgacgtc 120

aatgggtgga gtatttacgg taaactgccc acttggcagt acatcaagtg tatcatatgc 180

caagtacgcc ccctattgac gtcaatgacg gtaaatggcc cgcctggcat tatgcccagt 240

acatgacctt atgggacttt cctacttggc agtacatcta cgtattagtc atcgctatta 300

ccatggcccg ggtcgcgaca tggtcgaggt gagccccacg ttctgcttca ctctccccat 360

ctcccccccc tccccacccc caattttgta tttatttatt ttttaattat tttgtgcagc 420

gatgggggcg gggggggggg gggggcgcgc gccaggcggg gcggggcggg gcgaggggcg 480

gggcggggcg aggcggagag gtgcggcggc agccaatcag agcggcgcgc tccgaaagtt 540

tccttttatg gcgaggcggc ggcggcggcg gccctataaa aagcgaagcg cgcggcgggc 600

ggatccgttt aaacggctcg catctctcct tcacgcgccc gccgccctac ctgaggccgc 660

catccacgcc ggttgagtcg cgttctgccg cctcccgcct gtggtgcctc ctgaactgcg 720

tccgccgtct aggtaagttt aaagctcagg tcgagaccgg gcctttgtcc ggcgctccct 780

tggagcctac ctagactcag ccggctctcc acgctttgcc tgaccctgct tgctcaactc 840

tacgtctttg tttcgttttc tgttctgcgc cgttacagat cactagtgtt taaacagagt 900

aatgacatgg ttccttccat cctccaaagg tgaccaataa tagtttgtaa gtatcattat 960

gaactaatga attttcaaca tatttgatat atttcaatcc attgccatca ttgttcttat 1020

cgatatttga gttggctcac tttgccagta agagtctatt caaattggct tctgagtcca 1080

tttgacacaa cacctttgat ctttgacagt ttccttggtt ttaggtgcta gatgatttct 1140

caggctcacc ttagacattt cctgccacag acttagaatc agccatttct ctaaggaccc 1200

tgattccatt tcatgagaaa tgatagagac cacaatcaaa acaagtcatg aatttatact 1260

gatattttca attcaaatta aagatgaggt tttgctaaa tttttttgag tttatatttg 1320

tatgtcttat gctgaaaaat cttgtttcct aattagtaac ataattattc atttgatggg 1380

taaatatttt agggccgatt ctttggtttt atagccaaga taccctgttg ataaagtctt 1440

gtgggagcaa ttataagact ggcttatttt gaagcttttt aaaaaagaca tccttacctg 1500

ttttaactgt agattatatt aacttaaata ggtacagccc acgcttgact agtctcagag 1560

agcctcggct aggtagggga gcggaactct ggtgggaggg gaggtgcggt gcactggggg 1620

gatgggtggc taggggggcc gtctggtggc ttgcgggggt tgcctttccc gtgggaagtc 1680

gggaacataa tgtttgttac gttgggaggg aaaggggtgg ctggatgcag gcgggaggga 1740

ggcccgccct gcggcaaccg gagggggagg gagaagggag cggaaaatgc tcgaaaccgg 1800

acggagccat tgctctcgca gagggaggag cgcttccggc tagcctcttg tcgccgattg 1860

gccgtttctc ctcccgccgt gtgtgaaaac acaaatggcg tattctggtt ggagtaaagc 1920

tcctgtcagt tacaccgtcg ggagtacgca gccgcttagc gactctcgcg ttgccccctg 1980

ggtggggcgg gtaggtaggt ggggtgtaga gatgctgggt gtgcgggcgc ggccggcctc 2040

ctgcggcggg aggggagggt cagtgaaatt ggctctggcg cgggcgtcct cccaccctcc 2100

ccttccttcg ggggagtcgg tttacccgcc gcctgcttgt cttcgacacc tgattggctg 2160

tcgaagctgt gggaccgggc ccttgctact ggctcgagtc tcacatgagc gaaaccactg 2220

cgcggggcgc gggggtggcg gggaggcggg cgttggtacg gtcctccccg aggccgagcg 2280

ccgcagtgtc tggccccgcg cccctgcgca acgtggcagg aagcgcgcgc tggaggcggg 2340

ggcgggctgc cggccgagac ttctggatgg cggcggccgc ggctccgccc cgggttccca 2400

ccgcctgaag ggcgagacaa gcccgacctg ctacaggcac tcgtgggggt gggggaggag 2460

cgggggtcgg tccggctggt ttgtgggtgg gaggcgcttg ttctccaaaa accggcgcga 2520

gctgcaatcc tgagggagct gcggtggagg aggtggagag aaggccgcac ccttctgggc 2580

agggggaggg gagtgccgca ataccttat gggagttctt tgctgcctcc cgtcttgtaa 2640

ggaccgccct gggcctggaa gaagccctcc ctcctttcct cctcgcgtga tctcgtcatc 2700

gcctccatgt cgagtcgctt ctcgattatg ggcgggattc ttttgcctag acaattgtac 2760

taaccttctt ctctttcctc tcctgacag 2789

<210> 67
<211> 2172
<212> DNA
<213> Artificial Sequence

<220>
<223> CMV-bActine-U1U3-EF1

<400> 67

gcgttacata acttacggta aatggcccgc ctggctgacc gcccaacgac ccccgcccat 60
tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tggtcgaggt gagccccacg ttctgcttca ctctccccat 360
ctcccccccc tccccacccc caattttgta tttatttatt ttttaattat tttgtgcagc 420
gatgggggcg gggggggggg gggggcgcgc gccaggcggg gcggggcggg gcgaggggcg 480
gggcggggcg aggcggagag gtgcggcggc agccaatcag agcggcgcgc tccgaaagtt 540
tccttttatg gcgaggcggc ggcggcggcg gccctataaa aagcgaagcg cgcggcgggc 600
ggatccgttt aaacggctcg catctctcct tcacgcgccc gccgccctac ctgaggccgc 660
catccacgcc ggttgagtcg cgttctgccg cctcccgcct gtggtgcctc ctgaactgcg 720

tccgccgtct aggtaagttt aaagctcagg tcgagaccgg gcctttgtcc ggcgctccct 780
tggagcctac ctagactcag ccggctctcc acgctttgcc tgaccctgct tgctcaactc 840
tacgtctttg tttcgttttc tgttctgcgc cgttacagat cactagttaa cgctggtggg 900
tagggatgag ggagggaggg gcattgtgat gtacagggct gctctgtgag atcaagggtc 960
tcttaagggt gggagctggg gcagggacta cgagagcagc cagatgggct gaaagtggaa 1020
ctcaaggggt ttctggcacc tacctacctg cttcccgctg gggggtgggg agttggccca 1080
gagtcttaag attggggcag ggtggagagg tgggctcttc ctgcttccca ctcatcttat 1140
agctttcttt ccccagatcc gaattcgaga tccaaaccaa ggaggaaagg atatcacaga 1200
ggagagctag tcgggtttgc cgccagaaca caggtaagtg ccgtgtgtgg ttcccgcggg 1260
cctggcctct ttacgggtta tggcccttgc gtgccttgaa ttacttccac ctggctgcag 1320
tacgtgattc ttgatcccga gcttcgggtt ggaagtgggt gggagagttc gaggccttgc 1380
gcttaaggag ccccttcgcc tcgtgcttga gttgaggcct ggcctgggcg ctggggccgc 1440
cgcgtgcgaa tctggtggca ccttcgcgcc tgtctcgctg ctttcgataa gtctctagcc 1500
atttaaaatt tttgatgacc tgctgcgacg ctttttttct ggcaagatag tcttgtaaat 1560
gcgggccaag atctgcacac tggtatttcg gtttttgggg ccgcgggcgg cgacggggcc 1620
cgtgcgtccc agcgcacatg ttcggcgagg cggggcctgc gagcgcggcc accgagaatc 1680
ggacgggggt agtctcaagc tggccggcct gctctggtgc ctggcctcgc gccgccgtgt 1740
atcgccccgc cctgggcggc aaggctggcc cggtcggcac cagttgcgtg agcggaaaga 1800
tggccgcttc ccggccctgc tgcagggagc tcaaaatgga ggacgcggcg ctcgggagag 1860
cgggcgggtg agtcacccac acaaaggaaa agggcctttc cgtcctcagc cgtcgcttca 1920
tgtgactcca cggagtaccg ggcgccgtcc aggcacctcg attagttctc gagcttttgg 1980
agtacgtcgt ctttaggttg gggggagggg ttttatgcga tggagtttcc ccacactgag 2040
tgggtggaga ctgaagttag gccagcttgg cacttgatgt aattctcctt ggaatttgcc 2100
ctttttgagt ttggatcttg gttcattctc aagcctcaga cagtggttca aagttttttt 2160
cttccatttc ag 2172

<210> 68
<211> 1580
<212> DNA
<213> Artificial Sequence

<220>
<223> CMV-bActine-U1U3-mROSA

<400> 68

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tggtcgaggt gagccccacg ttctgcttca ctctccccat     360
ctcccccccc tccccacccc caattttgta tttatttatt ttttaattat tttgtgcagc     420
gatgggggcg gggggggggg gggggcgcgc gccaggcggg gcggggcggg gcgaggggcg     480
gggcggggcg aggcggagag gtgcggcggc agccaatcag agcggcgcgc tccgaaagtt     540
tccttttatg gcgaggcggc ggcggcggcg gccctataaa aagcgaagcg cgcggcgggc     600
ggatccgttt aaacggctcg catctctcct tcacgcgccc gccgccctac ctgaggccgc     660
catccacgcc ggttgagtcg cgttctgccg cctcccgcct gtggtgcctc ctgaactgcg     720
tccgccgtct aggtaagttt aaagctcagg tcgagaccgg gcctttgtcc ggcgctccct     780
tggagcctac ctagactcag ccggctctcc acgctttgcc tgaccctgct tgctcaactc     840
tacgtctttg tttcgttttc tgttctgcgc cgttacagat cactagttaa cgctggtggg     900
tagggatgag ggagggaggg gcattgtgat gtacagggct gctctgtgag atcaagggtc     960
tcttaagggt gggagctggg gcagggacta cgagagcagc cagatgggct gaaagtggaa    1020
ctcaaggggt ttctggcacc tacctacctg cttcccgctg gggggtgggg agttggccca    1080
gagtcttaag attggggcag ggtggagagg tgggctcttc ctgcttccca ctcatcttat    1140
agctttcttt ccccagatcc gaattcgaga tccaaaccaa ggaggaaagg atatcacaga    1200
ggagagctag ttcagagagc ctcggctagg taggggatcg ggactctggc gggagggcgg    1260
cttggtgcgt ttgcggggat gggcggccgc ggcaggccct ccgagcgtgg tggagccgtt    1320
ctgtgagaca gccgggtacg agtcgtgacg ctggaagggg caagcgggtg gtgggcagga    1380
atgcggtccg ccctgcagca accggagggg gagggagaag ggagcggaaa agtctccacc    1440
ggacgcggcc atggctcggg gggggggggg cagcggagga gcgcttccgg ccgacgtctc    1500
gtcgctgatt ggcttctttt cctcccgccg tgtgtgaaaa cacaattgta ctaaccttct    1560
tctctttcct ctcctgacag                                                1580
```

<210> 69
<211> 2447
<212> DNA
<213> Artificial Sequence

<220>

<223> CMV-bActine-U1U3-hROSA

<400> 69

gcgttacata acttacggta aatggcccgc ctggctgacc gcccaacgac ccccgcccat 60

tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tggtcgaggt gagccccacg ttctgcttca ctctccccat 360
ctcccccccc tccccacccc caattttgta tttatttatt ttttaattat tttgtgcagc 420
gatgggggcg gggggggggg gggggcgcgc gccaggcggg gcggggcggg gcgaggggcg 480
gggcggggcg aggcggagag gtgcggcggc agccaatcag agcggcgcgc tccgaaagtt 540
tccttttatg gcgaggcggc ggcggcggcg gccctataaa aagcgaagcg cgcggcgggc 600
ggatccgttt aaacggctcg catctctcct tcacgcgccc gccgccctac ctgaggccgc 660
catccacgcc ggttgagtcg cgttctgccg cctcccgcct gtggtgcctc ctgaactgcg 720
tccgccgtct aggtaagttt aaagctcagg tcgagaccgg gcctttgtcc ggcgctccct 780
tggagcctac ctagactcag ccggctctcc acgctttgcc tgaccctgct tgctcaactc 840
tacgtctttg tttcgttttc tgttctgcgc cgttacagat cactagttaa cgctggtggg 900
tagggatgag ggagggaggg gcattgtgat gtacagggct gctctgtgag atcaagggtc 960
tcttaagggt gggagctggg gcagggacta cgagagcagc cagatgggct gaaagtggaa 1020
ctcaaggggt ttctggcacc tacctacctg cttcccgctg gggggtgggg agttggccca 1080
gagtcttaag attggggcag ggtggagagg tgggctcttc ctgcttccca ctcatcttat 1140
agctttcttt ccccagatcc gaattcgaga tccaaaccaa ggaggaaagg atatcacaga 1200
ggagagctag tctcagagag cctcggctag gtaggggagc ggaactctgg tgggagggga 1260
ggtgcggtgc actgggggga tgggtggcta ggggggccgt ctggtggctt gcggggggttg 1320
cctttcccgt gggaagtcgg gaacataatg tttgttacgt tgggagggaa aggggtggct 1380
ggatgcaggc gggagggagg cccgccctgc ggcaaccgga gggggaggga gaagggagcg 1440
gaaaatgctc gaaaccggac ggagccattg ctctcgcaga gggaggagcg cttccggcta 1500
gcctcttgtc gccgattggc cgtttctcct cccgccgtgt gtgaaaacac aaatggcgta 1560
ttctggttgg agtaaagctc ctgtcagtta caccgtcggg agtacgcagc cgcttagcga 1620
ctctcgcgtt gccccctggg tggggcgggt aggtaggtgg ggtgtagaga tgctgggtgt 1680
gcgggcgcgg ccggcctcct gcggcgggag gggagggtca gtgaaattgg ctctggcgcg 1740
ggcgtcctcc caccctcccc ttccttcggg ggagtcggtt tacccgccgc ctgcttgtct 1800
tcgacacctg attggctgtc gaagctgtgg gaccgggccc ttgctactgg ctcgagtctc 1860
acatgagcga aaccactgcg cggggcgcgg gggtggcggg gaggcgggcg ttggtacggt 1920
cctccccgag gccgagcgcc gcagtgtctg gccccgcgcc cctgcgcaac gtggcaggaa 1980

```
gcgcgcgctg gaggcggggg cgggctgccg gccgagactt ctggatggcg gcggccgcgg      2040
ctccgccccg ggttcccacc gcctgaaggg cgagacaagc ccgacctgct acaggcactc      2100
gtgggggtgg gggaggagcg gggtcggtc cggctggttt gtgggtggga ggcgcttgtt      2160
ctccaaaaac cggcgcgagc tgcaatcctg agggagctgc ggtggaggag gtggagagaa      2220
ggccgcaccc ttctgggcag gggagggga gtgccgcaat acctttatgg gagttctttg      2280
ctgcctcccg tcttgtaagg accgccctgg gcctggaaga agccctccct cctttcctcc      2340
tcgcgtgatc tcgtcatcgc ctccatgtcg agtcgcttct cgattatggg cgggattctt      2400
ttgcctagac aattgtacta accttcttct ctttcctctc ctgacag                    2447
```

<210> 70
<211> 2557
<212> DNA
<213> Artificial Sequence

<220>
<223> CMV-bActine-U2U3-EF1

<400> 70

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tggtcgaggt gagccccacg ttctgcttca ctctccccat     360
ctcccccccc tccccacccc caattttgta tttatttatt ttttaattat tttgtgcagc     420
gatgggggcg gggggggggg gggggcgcgc gccaggcggg gcggggcggg gcgaggggcg     480
gggcggggcg aggcggagag gtgcggcggc agccaatcag agcggcgcgc tccgaaagtt     540
tccttttatg gcgaggcggc ggcggcggcg gccctataaa aagcgaagcg cgcggcgggc     600
ggatccgttt aaacagagta atgacatggt tccttccatc ctccaaaggt gaccaataat     660
agtttgtaag tatcattatg aactaatgaa ttttcaacat atttgatata tttcaatcca     720
ttgccatcat tgttcttatc gatatttgag ttggctcact ttgccagtaa gagtctattc     780
aaattggctt ctgagtccat ttgacacaac acctttgatc tttgacagtt tccttggttt     840
taggtgctag atgatttctc aggctcacct tagacatttc ctgccacaga cttagaatca     900
gccatttctc taaggaccct gattccattt catgagaaat gatagagacc acaatcaaaa     960
caagtcatga atttatactg atattttcaa ttcaaattaa agatgaggtt tttgctaaat    1020
ttttttgagt ttatatttgt atgtcttatg ctgaaaaatc ttgtttccta attagtaaca    1080
```

taattattca tttgatgggt aaatatttta gggccgattc tttggtttta tagccaagat 1140

accctgttga taaagtcttg tgggagcaat tataagactg gcttattttg aagcttttta 1200

aaaaagacat ccttacctgt tttaactgta gattatatta acttaaatag gtacagccca 1260

cgcttgacta gttaacgctg gtgggtaggg atgagggagg gaggggcatt gtgatgtaca 1320

gggctgctct gtgagatcaa gggtctctta agggtgggag ctggggcagg gactacgaga 1380

gcagccagat gggctgaaag tggaactcaa ggggtttctg gcacctacct acctgcttcc 1440

cgctgggggg tggggagttg gcccagagtc ttaagattgg ggcagggtgg agaggtgggc 1500

tcttcctgct tcccactcat cttatagctt tctttcccca gatccgaatt cgagatccaa 1560

accaaggagg aaaggatatc acagaggaga gctagtcggg tttgccgcca gaacacaggt 1620

aagtgccgtg tgtggttccc gcgggcctgg cctctttacg ggttatggcc cttgcgtgcc 1680

ttgaattact tccacctggc tgcagtacgt gattcttgat cccgagcttc gggttggaag 1740

tgggtgggag agttcgaggc cttgcgctta aggagcccct tcgcctcgtg cttgagttga 1800

ggcctggcct gggcgctggg gccgccgcgt gcgaatctgg tggcaccttc gcgcctgtct 1860

cgctgctttc gataagtctc tagccattta aaatttttga tgacctgctg cgacgctttt 1920

tttctggcaa gatagtcttg taaatgcggg ccaagatctg cacactggta tttcggtttt 1980

tggggccgcg ggcggcgacg gggcccgtgc gtcccagcgc acatgttcgg cgaggcgggg 2040

cctgcgagcg cggccaccga gaatcggacg ggggtagtct caagctggcc ggcctgctct 2100

ggtgcctggc ctcgcgccgc cgtgtatcgc cccgccctgg gcggcaaggc tggcccggtc 2160

ggcaccagtt gcgtgagcgg aaagatggcc gcttcccggc cctgctgcag ggagctcaaa 2220

atggaggacg cggcgctcgg gagagcgggc gggtgagtca cccacacaaa ggaaaagggc 2280

ctttccgtcc tcagccgtcg cttcatgtga ctccacggag taccgggcgc cgtccaggca 2340

cctcgattag ttctcgagct tttggagtac gtcgtcttta ggttgggggg aggggtttta 2400

tgcgatggag tttccccaca ctgagtgggt ggagactgaa gttaggccag cttggcactt 2460

gatgtaattc tccttggaat ttgccctttt tgagtttgga tcttggttca ttctcaagcc 2520

tcagacagtg gttcaaagtt tttttcttcc atttcag 2557

<210> 71
<211> 1965
<212> DNA
<213> Artificial Sequence

<220>
<223> CMV-bActine-U2U3-mROSA

<400> 71

gcgttacata acttacggta aatggcccgc ctggctgacc gcccaacgac ccccgcccat 60

tgacgtcaat aatgacgtat gttcccatag taacgccaat agggactttc cattgacgtc 120

aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tggtcgaggt gagccccacg ttctgcttca ctctccccat 360
ctccccccc tccccacccc caattttgta tttatttatt ttttaattat tttgtgcagc 420
gatgggggcg gggggggggg gggggcgcgc gccaggcggg gcggggcggg gcgaggggcg 480
gggcggggcg aggcggagag gtgcggcggc agccaatcag agcggcgcgc tccgaaagtt 540
tccttttatg gcgaggcggc ggcggcggcg gccctataaa aagcgaagcg cgcggcgggc 600
ggatccgttt aaacagagta atgacatggt tccttccatc ctccaaaggt gaccaataat 660
agtttgtaag tatcattatg aactaatgaa ttttcaacat atttgatata tttcaatcca 720
ttgccatcat tgttcttatc gatatttgag ttggctcact ttgccagtaa gagtctattc 780
aaattggctt ctgagtccat ttgacacaac acctttgatc tttgacagtt tccttggttt 840
taggtgctag atgatttctc aggctcacct tagacatttc ctgccacaga cttagaatca 900
gccatttctc taaggaccct gattccattt catgagaaat gatagagacc acaatcaaaa 960
caagtcatga atttatactg atattttcaa ttcaaattaa agatgaggtt tttgctaaat 1020
ttttttgagt ttatatttgt atgtcttatg ctgaaaaatc ttgtttccta attagtaaca 1080
taattattca tttgatgggt aaatatttta gggccgattc tttggtttta tagccaagat 1140
accctgttga taaagtcttg tgggagcaat tataagactg gcttattttg aagcttttta 1200
aaaaagacat ccttacctgt tttaactgta gattatatta acttaaatag gtacagccca 1260
cgcttgacta gttaacgctg gtgggtaggg atgagggagg gaggggcatt gtgatgtaca 1320
gggctgctct gtgagatcaa gggtctctta agggtgggag ctggggcagg gactacgaga 1380
gcagccagat gggctgaaag tggaactcaa ggggtttctg gcacctacct acctgcttcc 1440
cgctgggggg tggggagttg gcccagagtc ttaagattgg ggcagggtgg agaggtgggc 1500
tcttcctgct tcccactcat cttatagctt tctttcccca gatccgaatt cgagatccaa 1560
accaaggagg aaaggatatc acagaggaga gctagttcag agagcctcgg ctaggtaggg 1620
gatcgggact ctggcgggag ggcggcttgg tgcgtttgcg gggatgggcg gccgcggcag 1680
gccctccgag cgtggtggag ccgttctgtg agacagccgg gtacgagtcg tgacgctgga 1740
aggggcaagc gggtggtggg caggaatgcg gtccgccctg cagcaaccgg agggggaggg 1800
agaagggagc ggaaaagtct ccaccggacg cggccatggc tcgggggggg gggggcagcg 1860
gaggagcgct tccggccgac gtctcgtcgc tgattggctt cttttcctcc cgccgtgtgt 1920
gaaaacacaa ttgtactaac cttcttctct ttcctctcct gacag 1965

<210> 72
<211> 2832
<212> DNA
<213> Artificial Sequence

<220>
<223> CMV-bActine-U2U3-hROSA

<400> 72

gcgttacata acttacggta aatggcccgc ctggctgacc gcccaacgac ccccgcccat 60
tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tggtcgaggt gagccccacg ttctgcttca ctctccccat 360
ctcccccccc tccccacccc caattttgta tttatttatt ttttaattat tttgtgcagc 420
gatgggggcg gggggggggg gggggcgcgc gccaggcggg gcggggcggg gcgaggggcg 480
gggcggggcg aggcggagag gtgcggcggc agccaatcag agcggcgcgc tccgaaagtt 540
tccttttatg gcgaggcggc ggcggcggcg gccctataaa aagcgaagcg cgcggcgggc 600
ggatccgttt aaacagagta atgacatggt tccttccatc ctccaaaggt gaccaataat 660
agtttgtaag tatcattatg aactaatgaa ttttcaacat atttgatata tttcaatcca 720
ttgccatcat tgttcttatc gatatttgag ttggctcact ttgccagtaa gagtctattc 780
aaattggctt ctgagtccat ttgacacaac acctttgatc tttgacagtt tccttggttt 840
taggtgctag atgatttctc aggctcacct tagacatttc ctgccacaga cttagaatca 900
gccatttctc taaggaccct gattccattt catgagaaat gatagagacc acaatcaaaa 960
caagtcatga atttatactg atattttcaa ttcaaattaa agatgaggtt tttgctaaat 1020
ttttttgagt ttatatttgt atgtcttatg ctgaaaaatc ttgtttccta attagtaaca 1080
taattattca tttgatgggt aaatatttta gggccgattc tttggtttta tagccaagat 1140
accctgttga taaagtcttg tgggagcaat tataagactg gcttattttg aagcttttta 1200
aaaaagacat ccttacctgt tttaactgta gattatatta acttaaatag gtacagccca 1260
cgcttgacta gttaacgctg gtgggtaggg atgagggagg gaggggcatt gtgatgtaca 1320
gggctgctct gtgagatcaa gggtctctta agggtgggag ctggggcagg gactacgaga 1380
gcagccagat gggctgaaag tggaactcaa ggggtttctg gcacctacct acctgcttcc 1440
cgctgggggg tggggagttg gcccagagtc ttaagattgg ggcagggtgg agaggtgggc 1500
tcttcctgct tcccactcat cttatagctt tctttcccca gatccgaatt cgagatccaa 1560
accaaggagg aaaggatatc acagaggaga gctagtctca gagagcctcg gctaggtagg 1620

ggagcggaac tctggtggga ggggaggtgc ggtgcactgg ggggatgggt ggctaggggg 1680

gccgtctggt ggcttgcggg ggttgccttt cccgtgggaa gtcgggaaca taatgtttgt 1740

tacgttggga gggaaagggg tggctggatg caggcgggag ggaggcccgc cctgcggcaa 1800

ccggaggggg agggagaagg gagcggaaaa tgctcgaaac cggacggagc cattgctctc 1860

gcagagggag gagcgcttcc ggctagcctc ttgtcgccga ttggccgttt ctcctcccgc 1920

cgtgtgtgaa aacacaaatg gcgtattctg gttggagtaa agctcctgtc agttacaccg 1980

tcgggagtac gcagccgctt agcgactctc gcgttgcccc ctgggtgggg cgggtaggta 2040

ggtggggtgt agagatgctg ggtgtgcggg cgcggccggc ctcctgcggc gggaggggag 2100

ggtcagtgaa attggctctg gcgcgggcgt cctcccaccc tccccttcct tcgggggagt 2160

cggtttaccc gccgcctgct tgtcttcgac acctgattgg ctgtcgaagc tgtgggaccg 2220

ggcccttgct actggctcga gtctcacatg agcgaaacca ctgcgcgggg cgcgggggtg 2280

gcggggaggc gggcgttggt acggtcctcc ccgaggccga gcgccgcagt gtctggcccc 2340

gcgcccctgc gcaacgtggc aggaagcgcg cgctggaggc gggggcgggc tgccggccga 2400

gacttctgga tggcggcggc cgcggctccg ccccgggttc ccaccgcctg aagggcgaga 2460

caagcccgac ctgctacagg cactcgtggg ggtgggggag gagcgggggt cggtccggct 2520

ggtttgtggg tgggaggcgc ttgttctcca aaaaccggcg cgagctgcaa tcctgaggga 2580

gctgcggtgg aggaggtgga gagaaggccg caccccttctg ggcaggggga ggggagtgcc 2640

gcaatacctt tatgggagtt ctttgctgcc tcccgtcttg taaggaccgc cctgggcctg 2700

gaagaagccc tccctccttt cctcctcgcg tgatctcgtc atcgcctcca tgtcgagtcg 2760

cttctcgatt atgggcggga ttcttttgcc tagacaattg tactaacctt cttctctttc 2820

ctctcctgac ag 2832

<210> 73
<211> 2838
<212> DNA
<213> Artificial Sequence

<220>
<223> CMV-bActine-U1U2U3-EF1

<400> 73

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
```

ccatggcccg ggtcgcgaca tggtcgaggt gagccccacg ttctgcttca ctctccccat 360

ctcccccccc tccccacccc caattttgta tttatttatt ttttaattat tttgtgcagc 420

gatgggggcg gggggggggg gggggcgcgc gccaggcggg gcggggcggg gcgaggggcg 480

gggcggggcg aggcggagag gtgcggcggc agccaatcag agcggcgcgc tccgaaagtt 540

tccttttatg gcgaggcggc ggcggcggcg gccctataaa aagcgaagcg cgcggcgggc 600

ggatccgttt aaacggctcg catctctcct tcacgcgccc gccgccctac ctgaggccgc 660

catccacgcc ggttgagtcg cgttctgccg cctcccgcct gtggtgcctc ctgaactgcg 720

tccgccgtct aggtaagttt aaagctcagg tcgagaccgg gcctttgtcc ggcgctccct 780

tggagcctac ctagactcag ccggctctcc acgctttgcc tgaccctgct tgctcaactc 840

tacgtctttg tttcgttttc tgttctgcgc cgttacagat cactagtgtt taaacagagt 900

aatgacatgg ttccttccat cctccaaagg tgaccaataa tagtttgtaa gtatcattat 960

gaactaatga attttcaaca tatttgatat atttcaatcc attgccatca ttgttcttat 1020

cgatatttga gttggctcac tttgccagta agagtctatt caaattggct tctgagtcca 1080

tttgacacaa cacctttgat ctttgacagt ttccttggtt ttaggtgcta gatgatttct 1140

caggctcacc ttagacattt cctgccacag acttagaatc agccatttct ctaaggaccc 1200

tgattccatt tcatgagaaa tgatagagac cacaatcaaa acaagtcatg aatttatact 1260

gatattttca attcaaatta aagatgaggt tttgctaaa tttttttgag tttatatttg 1320

tatgtcttat gctgaaaaat cttgtttcct aattagtaac ataattattc atttgatggg 1380

taaatatttt agggccgatt ctttggtttt atagccaaga taccctgttg ataaagtctt 1440

gtgggagcaa ttataagact ggcttatttt gaagcttttt aaaaaagaca tccttacctg 1500

ttttaactgt agattatatt aacttaaata ggtacagccc acgcttgact agttaacgct 1560

ggtgggtagg gatgagggag ggagggggcat tgtgatgtac agggctgctc tgtgagatca 1620

agggtctctt aagggtggga gctggggcag ggactacgag agcagccaga tgggctgaaa 1680

gtggaactca aggggtttct ggcacctacc tacctgcttc ccgctggggg gtggggagtt 1740

ggcccagagt cttaagattg ggcagggtg gagaggtggg ctcttcctgc ttcccactca 1800

tcttatagct ttctttcccc agatccgaat tcgagatcca aaccaaggag gaaaggatat 1860

cacagaggag agctagtcgg gtttgccgcc agaacacagg taagtgccgt gtgtggttcc 1920

cgcgggcctg gcctctttac gggttatggc ccttgcgtgc cttgaattac ttccacctgg 1980

ctgcagtacg tgattcttga tcccgagctt cgggttggaa gtgggtggga gagttcgagg 2040

ccttgcgctt aaggagcccc ttcgcctcgt gcttgagttg aggcctggcc tgggcgctgg 2100

ggccgccgcg tgcgaatctg gtggcacctt cgcgcctgtc tcgctgcttt cgataagtct 2160

ctagccattt aaaattttg atgacctgct gcgacgcttt ttttctggca agatagtctt 2220

gtaaatgcgg gccaagatct gcacactggt atttcggttt ttggggccgc gggcggcgac 2280

ggggcccgtg cgtcccagcg cacatgttcg gcgaggcggg gcctgcgagc gcggccaccg 2340

agaatcggac gggggtagtc tcaagctggc cggcctgctc tggtgcctgg cctcgcgccg 2400

ccgtgtatcg ccccgccctg ggcggcaagg ctggcccggt cggcaccagt tgcgtgagcg 2460

gaaagatggc cgcttcccgg ccctgctgca gggagctcaa aatggaggac gcggcgctcg 2520

ggagagcggg cgggtgagtc acccacacaa aggaaaaggg cctttccgtc ctcagccgtc 2580

gcttcatgtg actccacgga gtaccgggcg ccgtccaggc acctcgatta gttctcgagc 2640

ttttggagta cgtcgtcttt aggttggggg gaggggtttt atgcgatgga gtttccccac 2700

actgagtggg tggagactga agttaggcca gcttggcact tgatgtaatt ctccttggaa 2760

tttgcccttt ttgagtttgg atcttggttc attctcaagc ctcagacagt ggttcaaagt 2820

tttttcttc catttcag 2838

<210> 74
<211> 2246
<212> DNA
<213> Artificial Sequence

<220>
<223> CMV-bActine-U1U2U3-mROSA

<400> 74

```
gcgttacata acttacggta aatggcccgc ctggctgacc gcccaacgac ccccgcccat       60
tgacgtcaat aatgacgtat gttcccatag taacgccaat agggactttc cattgacgtc      120
aatgggtgga gtatttacgg taaactgccc acttggcagt acatcaagtg tatcatatgc      180
caagtacgcc ccctattgac gtcaatgacg gtaaatggcc cgcctggcat tatgcccagt      240
acatgacctt atgggacttt cctacttggc agtacatcta cgtattagtc atcgctatta      300
ccatggcccg ggtcgcgaca tggtcgaggt gagccccacg ttctgcttca ctctccccat      360
ctcccccccc tccccacccc caattttgta tttatttatt ttttaattat tttgtgcagc      420
gatgggggcg gggggggggg gggggcgcgc gccaggcggg gcggggcggg gcgaggggcg      480
gggcggggcg aggcggagag gtgcggcggc agccaatcag agcggcgcgc tccgaaagtt      540
tccttttatg gcgaggcggc ggcggcggcg gccctataaa aagcgaagcg cgcggcgggc      600
ggatccgttt aaacggctcg catctctcct tcacgcgccc gccgccctac ctgaggccgc      660
catccacgcc ggttgagtcg cgttctgccg cctcccgcct gtggtgcctc ctgaactgcg      720
tccgccgtct aggtaagttt aaagctcagg tcgagaccgg gcctttgtcc ggcgctccct      780
tggagcctac ctagactcag ccggctctcc acgctttgcc tgaccctgct tgctcaactc      840
tacgtctttg tttcgttttc tgttctgcgc cgttacagat cactagtgtt taaacagagt      900
```

aatgacatgg ttccttccat cctccaaagg tgaccaataa tagtttgtaa gtatcattat 960

gaactaatga attttcaaca tatttgatat atttcaatcc attgccatca ttgttcttat 1020

cgatatttga gttggctcac tttgccagta agagtctatt caaattggct tctgagtcca 1080

tttgacacaa cacctttgat ctttgacagt ttccttggtt ttaggtgcta gatgatttct 1140

caggctcacc ttagacattt cctgccacag acttagaatc agccatttct ctaaggaccc 1200

tgattccatt tcatgagaaa tgatagagac cacaatcaaa acaagtcatg aatttatact 1260

gatattttca attcaaatta aagatgaggt tttgctaaa ttttttgag tttatatttg 1320

tatgtcttat gctgaaaaat cttgtttcct aattagtaac ataattattc atttgatggg 1380

taaatatttt agggccgatt ctttggtttt atagccaaga taccctgttg ataaagtctt 1440

gtgggagcaa ttataagact ggcttatttt gaagcttttt aaaaaagaca tccttacctg 1500

ttttaactgt agattatatt aacttaaata ggtacagccc acgcttgact agttaacgct 1560

ggtgggtagg gatgagggag ggaggggcat tgtgatgtac agggctgctc tgtgagatca 1620

agggtctctt aagggtggga gctggggcag ggactacgag agcagccaga tgggctgaaa 1680

gtggaactca aggggtttct ggcacctacc tacctgcttc ccgctggggg gtggggagtt 1740

ggcccagagt cttaagattg gggcagggtg gagaggtggg ctcttcctgc ttcccactca 1800

tcttatagct ttctttcccc agatccgaat tcgagatcca aaccaaggag gaaaggatat 1860

cacagaggag agctagttca gagagcctcg gctaggtagg ggatcgggac tctggcggga 1920

gggcggcttg gtgcgtttgc ggggatgggc ggccgcggca ggccctccga gcgtggtgga 1980

gccgttctgt gagacagccg ggtacgagtc gtgacgctgg aaggggcaag cgggtggtgg 2040

gcaggaatgc ggtccgccct gcagcaaccg gagggggagg gagaagggag cggaaaagtc 2100

tccaccggac gcggccatgg ctcggggggg ggggggcagc ggaggagcgc ttccggccga 2160

cgtctcgtcg ctgattggct tcttttcctc ccgccgtgtg tgaaaacaca attgtactaa 2220

ccttcttctc tttcctctcc tgacag 2246

<210> 75
<211> 3113
<212> DNA
<213> Artificial Sequence

<220>
<223> CMV-bActine-U1U2U3-hROSA

<400> 75

```
gcgttacata acttacggta aatggcccgc ctggctgacc gcccaacgac ccccgcccat        60
tgacgtcaat aatgacgtat gttcccatag taacgccaat agggactttc cattgacgtc       120
aatgggtgga gtatttacgg taaactgccc acttggcagt acatcaagtg tatcatatgc       180
caagtacgcc ccctattgac gtcaatgacg gtaaatggcc cgcctggcat tatgcccagt       240
```

acatgacctt atgggacttt cctacttggc agtacatcta cgtattagtc atcgctatta 300

ccatggcccg ggtcgcgaca tggtcgaggt gagccccacg ttctgcttca ctctccccat 360

ctcccccccc tccccacccc caattttgta tttatttatt ttttaattat tttgtgcagc 420

gatgggggcg gggggggggg gggggcgcgc gccaggcggg gcggggcggg gcgaggggcg 480

gggcggggcg aggcggagag gtgcggcggc agccaatcag agcggcgcgc tccgaaagtt 540

tccttttatg gcgaggcggc ggcggcggcg gccctataaa aagcgaagcg cgcggcgggc 600

ggatccgttt aaacggctcg catctctcct tcacgcgccc gccgccctac ctgaggccgc 660

catccacgcc ggttgagtcg cgttctgccg cctcccgcct gtggtgcctc ctgaactgcg 720

tccgccgtct aggtaagttt aaagctcagg tcgagaccgg gcctttgtcc ggcgctccct 780

tggagcctac ctagactcag ccggctctcc acgctttgcc tgaccctgct tgctcaactc 840

tacgtctttg tttcgttttc tgttctgcgc cgttacagat cactagtgtt taaacagagt 900

aatgacatgg ttccttccat cctccaaagg tgaccaataa tagtttgtaa gtatcattat 960

gaactaatga attttcaaca tatttgatat atttcaatcc attgccatca ttgttcttat 1020

cgatatttga gttggctcac tttgccagta agagtctatt caaattggct tctgagtcca 1080

tttgacacaa cacctttgat ctttgacagt ttccttggtt ttaggtgcta gatgatttct 1140

caggctcacc ttagacattt cctgccacag acttagaatc agccatttct ctaaggaccc 1200

tgattccatt tcatgagaaa tgatagagac cacaatcaaa acaagtcatg aatttatact 1260

gatattttca attcaaatta aagatgaggt tttgctaaa tttttttgag tttatatttg 1320

tatgtcttat gctgaaaaat cttgtttcct aattagtaac ataattattc atttgatggg 1380

taaatatttt agggccgatt ctttggtttt atagccaaga taccctgttg ataaagtctt 1440

gtgggagcaa ttataagact ggcttatttt gaagcttttt aaaaaagaca tccttacctg 1500

ttttaactgt agattatatt aacttaaata ggtacagccc acgcttgact agttaacgct 1560

ggtgggtagg gatgagggag ggaggggcat tgtgatgtac agggctgctc tgtgagatca 1620

agggtctctt aagggtggga gctggggcag ggactacgag agcagccaga tgggctgaaa 1680

gtggaactca aggggtttct ggcacctacc tacctgcttc ccgctggggg gtggggagtt 1740

ggcccagagt cttaagattg gggcagggtg gagaggtggg ctcttcctgc ttcccactca 1800

tcttatagct ttctttcccc agatccgaat tcgagatcca aaccaaggag gaaaggatat 1860

cacagaggag agctagtctc agagagcctc ggctaggtag gggagcggaa ctctggtggg 1920

aggggaggtg cggtgcactg gggggatggg tggctagggg ggccgtctgg tggcttgcgg 1980

gggttgcctt tcccgtggga agtcgggaac ataatgtttg ttacgttggg agggaaaggg 2040

gtggctggat gcaggcggga gggaggcccg ccctgcggca accggagggg gagggagaag 2100

```
ggagcggaaa atgctcgaaa ccggacggag ccattgctct cgcagaggga ggagcgcttc     2160
cggctagcct cttgtcgccg attggccgtt tctcctcccg ccgtgtgtga aaacacaaat     2220
ggcgtattct ggttggagta aagctcctgt cagttacacc gtcgggagta cgcagccgct     2280
tagcgactct cgcgttgccc cctgggtggg gcgggtaggt aggtggggtg tagagatgct     2340
gggtgtgcgg gcgcggccgg cctcctgcgg cgggagggga gggtcagtga aattggctct     2400
ggcgcgggcg tcctcccacc ctccccttcc ttcgggggag tcggtttacc cgccgcctgc     2460
ttgtcttcga cacctgattg gctgtcgaag ctgtgggacc gggcccttgc tactggctcg     2520
agtctcacat gagcgaaacc actgcgcggg gcgcgggggt ggcggggagg cgggcgttgg     2580
tacggtcctc cccgaggccg agcgccgcag tgtctggccc cgcgcccctg cgcaacgtgg     2640
caggaagcgc gcgctggagg cgggggcggg ctgccggccg agacttctgg atggcggcgg     2700
ccgcggctcc gccccgggtt cccaccgcct gaagggcgag acaagcccga cctgctacag     2760
gcactcgtgg gggtggggga ggagcggggg tcggtccggc tggtttgtgg gtgggaggcg     2820
cttgttctcc aaaaaccggc gcgagctgca atcctgaggg agctgcggtg gaggaggtgg     2880
agagaaggcc gcacccttct gggcaggggg aggggagtgc cgcaatacct ttatgggagt     2940
tctttgctgc ctcccgtctt gtaaggaccg ccctgggcct ggaagaagcc ctccctcctt     3000
tcctcctcgc gtgatctcgt catcgcctcc atgtcgagtc gcttctcgat tatgggcggg     3060
attcttttgc ctagacaatt gtactaacct tcttctcttt cctctcctga cag            3113
```